(19) Europäisches Patentamt / European Patent Office / Office européen des brevets

(11) EP 3 221 294 B1

(12) **EUROPÄISCHE PATENTSCHRIFT**

(45) Veröffentlichungstag und Bekanntmachung des Hinweises auf die Patenterteilung:
**05.09.2018 Patentblatt 2018/36**

(21) Anmeldenummer: **15790467.3**

(22) Anmeldetag: **03.11.2015**

(51) Int Cl.:
***C07D 209/86*** (2006.01) ***H01L 51/50*** (2006.01)

(86) Internationale Anmeldenummer:
**PCT/EP2015/002199**

(87) Internationale Veröffentlichungsnummer:
**WO 2016/078747 (26.05.2016 Gazette 2016/21)**

(54) **HETEROCYCLISCHE VERBINDUNGEN ZUR VERWENDUNG IN ELEKTRONISCHEN VORRICHTUNGEN**

HETEROCYCLIC COMPOUNDS FOR USE IN ELECTRONIC DEVICES

DERIVES HETEROCYCLIQUES POUR L'UTILISATION DANS DES DISPOSITIFS ELECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.11.2014 EP 14003925**

(43) Veröffentlichungstag der Anmeldung:
**27.09.2017 Patentblatt 2017/39**

(73) Patentinhaber: **Merck Patent GmbH**
**64293 Darmstadt (DE)**

(72) Erfinder:
- **JATSCH, Anja**
  **60489 Frankfurt am Main (DE)**
- **PARHAM, Amir Hossain**
  **60486 Frankfurt am Main (DE)**
- **EBERLE, Thomas**
  **76829 Landau (DE)**
- **GROSSMANN, Tobias**
  **64297 Darmstadt (DE)**
- **KROEBER, Jonas Valentin**
  **60311 Frankfurt am Main (DE)**

(56) Entgegenhaltungen:
**EP-A1- 2 772 483 JP-A- 2014 101 275**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft heterocyclischeVerbindungen, welche sich für den Einsatz in elektronischen Vorrichtungen eignen. Weiterhin betrifft die vorliegende Erfindung Verfahren zu deren Herstellung und elektronische Vorrichtungen.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische FeldeffektTransistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig von dem jeweiligen Verwendungszweck folgenden allgemeinen Schichtaufbau auf, der für die jeweilige Anwendung angepasst werden kann:

(1) Substrat,
(2) Elektrode, häufig metallisch oder anorganisch, aber auch aus organischen bzw. polymeren, leitfähigen Materialien,
(3) Ladungsinjektionsschicht/en bzw. Zwischenschicht/en, beispielsweise zum Ausgleich von Unebenheiten der Elektrode ("planarisation layer"), häufig aus einem leitfähigen, dotierten Polymer,
(4) Organische Halbleiter,
(5) evtl. weitere Ladungstransport-, Ladungsinjektions- bzw. Ladungsblockierschichten,
(6) Gegenelektrode, Materialien wie unter (2) genannt,
(7) Verkapselung.

Die obige Anordnung stellt den allgemeinen Aufbau einer organischen, elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs. Ein derartiges System ist beispielsweise in der WO 90/13148 A1 auf der Basis von Poly-(p-phenylenen) beschrieben.

Elektronische Vorrichtungen, die heterocyclische Verbindungen enthalten, sind unter anderem aus den Veröffentlichungen WO 2014/088047 A1, WO 2013/120577 A1, WO 2013/183851 A1, EP 2 468 725 A1, KR 2013 0134426 A und KR 101395080 B1 bekannt. In EP 2772483 A1 und JP 2014101275 A werden 4-Aminocarbazole und deren Anwendung in elektrolumineszenten Vorrichtungen beschrieben.

Bekannte elektronische Vorrichtungen weisen ein brauchbares Eigenschaftsprofil auf. Allerdings besteht die dauerhafte Notwendigkeit, die Eigenschaften dieser Vorrichtungen zu verbessern.

Zu diesen Eigenschaften gehört insbesondere die Energieeffizienz, mit der eine elektronische Vorrichtung die vorgegebene Aufgabe löst. Bei organischen Leuchtdioden, die sowohl auf niedermolekularen Verbindungen als auch auf polymeren Materialien basieren können, sollte insbesondere die Lichtausbeute hoch sein, so dass zum Erreichen eines bestimmten Lichtflusses möglichst wenig elektrische Leistung aufgebracht werden muss. Weiterhin sollte auch zum Erzielen einer vorgegebenen Leuchtdichte eine möglichst geringe Spannung notwendig sein. Ein weiteres Problem stellt insbesondere die Lebensdauer der elektronischen Vorrichtungen dar.

Aufgabe der vorliegenden Erfindung ist daher die Bereitstellung neuer Verbindungen, welche zu Elektronischen Vorrichtungen mit verbesserten Eigenschaften führen. Insbesondere ist die Aufgabe Elektronenblockiermaterialien, Lochtransportmaterialien, Lochinjektionsmaterialien und/oder Matrixmaterialien bereitzustellen, welche verbesserte Eigenschaften in Bezug auf Effizienz, Betriebsspannung und/oder Lebensdauer zeigen.

Weiterhin sollten sich die Verbindungen möglichst einfach verarbeiten lassen, insbesondere eine gute Löslichkeit und Filmbildung zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschenderweise wurde gefunden, dass diese sowie weitere nicht explizit genannte Aufgaben, die jedoch aus den hierin einleitend diskutierten Zusammenhängen ohne Weiteres ableitbar oder erschließbar sind, durch Verbindungen mit allen Merkmalen des Patentanspruchs 1 gelöst werden. Zweckmäßige Abwandlungen der erfindungsgemäßen Verbindungen werden in den auf Anspruch 1 rückbezogenen abhängigen Ansprüchen unter Schutz gestellt.

Gegenstand der Erfindung ist somit eine Verbindung, umfassend mindestens eine umfassend mindestens eine Struktur der Formel (I)

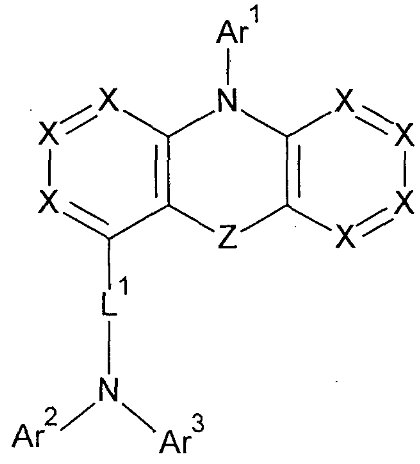

Formel (I)

wobei für die verwendeten Symbole gilt:

X ist bei jedem Auftreten gleich oder verschieden N oder $CR^1$, vorzugsweise CR1, mit der Maßgabe, dass nicht mehr als zwei der Gruppen X in einem Cyclus für N stehen;

Z ist eine Bindung, $C(R^1)_2$, O oder S;

$L^1$ ist eine Bindung, ein aromatisches Ringsystem mit 6 bis 60, vorzugsweise 6 bis 40, besonders bevorzugt 6 bis 20 C-Atomen oder ein heteroaromatisches Ringsystem mit 3 bis 60 C-Atomen, vorzugsweise 3 bis 40, besonders bevorzugt 3 bis 20 welches jeweils durch einen oder mehrere Reste $R^1$ substituiert sein kann, vorzugsweise eine Bindung, eine Arylgruppe mit 6 bis 60, vorzugsweise 6 bis 40, besonders bevorzugt 6 bis 20 C-Atomen oder eine Heteroarylgruppe mit 3 bis 60, vorzugsweise 6 bis 40, besonders bevorzugt 6 bis 20 C-Atomen ist, welche jeweils durch einen oder mehrere Reste $R^1$ substituiert sein kann;

$Ar^1$, $Ar^2$, $Ar^3$ ist eine Arylgruppe mit 6 bis 40, bevorzugt 6 bis 20 C-Atomen oder eine Heteroarylgruppe mit 3 bis 40, bevorzugt 3 bis 20 C-Atomen, welche jeweils durch einen oder mehrere Reste $R^1$ substituiert sein kann;

$R^1$ ist bei jedem Auftreten gleich oder verschieden H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden;

$R^2$ ist bei jedem Auftreten gleich oder verschieden H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C≡C-, $Si(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei

oder mehrere benachbarte Substituenten $R^2$ auch miteinander ein mono- oder polycyclisches, aliphatisches oder aromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

mit der Maßgabe, dass
mindestens einer der Reste $Ar^2$ und/oder $Ar^3$ eine Gruppe der Formel (IIa) oder (IIb) darstellt

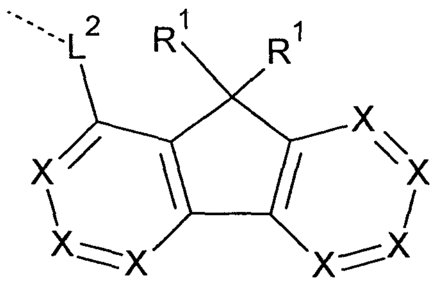

Formel (IIa)

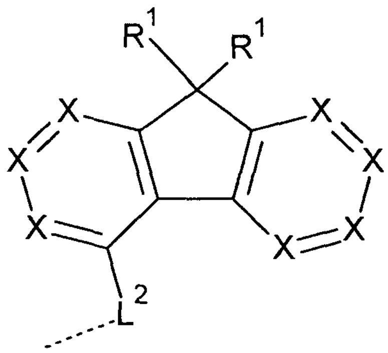

Formel (IIb)

worin

X bei jedem Auftreten gleich oder verschieden N oder $CR^1$ ist, vorzugsweise $CR^1$, mit der Maßgabe, dass nicht mehr als zwei der Gruppen X in einem Cyclus für N stehen;

$L^2$ eine Bindung, ein aromatisches Ringsystem mit 6 bis 60 vorzugsweise 6 bis 40, besonders bevorzugt 6 bis 20 C-Atomen oder ein heteroaromatisches Ringsystem mit 3 bis 60 vorzugsweise 3 bis 40, besonders bevorzugt 3 bis 20 C-Atomen, welches jeweils durch einen oder mehrere Reste $R^1$ substituiert sein kann, vorzugsweise eine Bindung, eine Arylgruppe mit 6 bis 60 vorzugsweise 6 bis 40, besonders bevorzugt 6 bis 20 C-Atomen oder eine Heteroarylgruppe mit 3 bis 60 vorzugsweise 3 bis 40, besonders bevorzugt 3 bis 20 C-Atomen ist, welche jeweils durch einen oder mehrere Reste $R^1$ substituiert sein kann;

$R^1$ die zuvor genannte Bedeutung hat und die gestrichelte Linie die Bindungsstelle darstellt, so dass $L^2$ an das gleiche Stickstoffatom bindet, wie $L^1$.

Dabei bedeutet "benachbarte Kohlenstoffatome", dass die Kohlenstoffatome direkt aneinander gebunden sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen

und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 3 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl oder Terphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 3 - 60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausgestaltung kann vorgesehen sein, dass eine Struktur gemäß Formel (Ia) gebildet wird

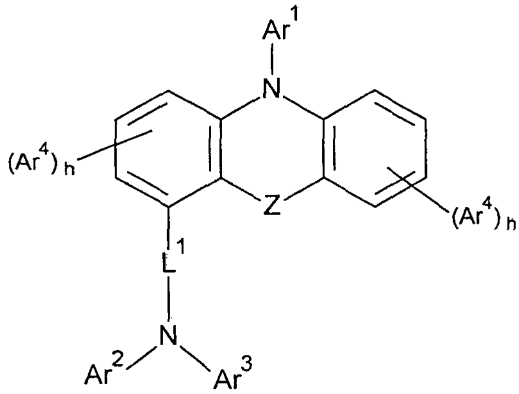

Formel (Ia)

worin

h bei jedem Auftreten unabhängig 0, 1, 2, 3 oder 4, vorzugsweise 0, 1 oder 2 und besonders bevorzugt 0 oder 1 ist;
$Ar^4$ eine Arylgruppe mit 6 bis 40 C-Atomen oder eine Heteroarylgruppe mit 3 bis 40 C-Atomen, welche jeweils durch einen oder mehrere Reste $R^1$ substituiert sein kann, und
die Symbole $Ar^1$, $Ar^2$, $Ar^3$, $L^1$ und Z die zuvor genannte Bedeutung annehmen können.

Ferner kann vorgesehen sein, dass die Gruppe Z in Formel (I) für eine Bindung steht, so dass eine Struktur der Formel (Ib) oder (Ic) gebildet wird

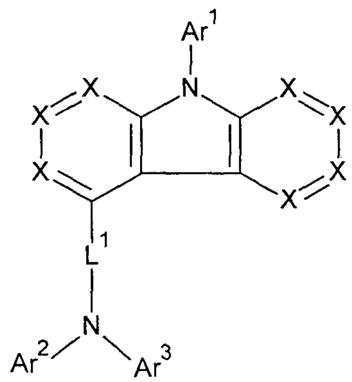

Formel (Ib)

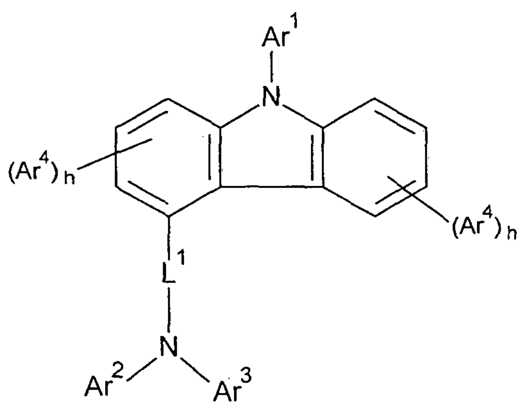

Formel (Ic)

worin die Symbole die zuvor dargelegten Bedeutungen annehmen können.

Zur Verdeutlichung der nachfolgenden Ausführungen wird untenstehend die Strukturformel von Fluoren abgebildet, zusammen mit der Nummerierung der Positionen. Bei Spirobifluorenderivaten ist die Nummerierung analog, mit dem einzigen Unterschied, dass die Position 9 nicht substituiert sein kann.

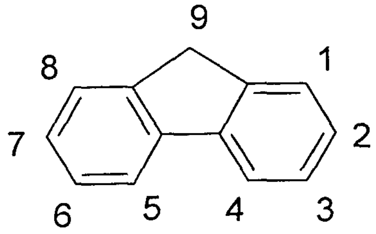

In Strukturen gemäß den Formeln (IIa) oder (IIb) kann vorgesehen sein, dass die beiden Reste $R^1$, die an das C-Atom in Position 9 in der Fluorenstruktur gebunden sind, identisch sind. Hierdurch kann eine symetrische Substitution an dieser Position erhalten werden.

Weiterhin kann vorgesehen sein, dass beiden Reste $R^1$, die an das C-Atom in Position 9 in der Fluorenstruktur gemäß Formel (IIa) oder (IIb) gebunden sind, verschieden sind. Hierbei kann der Unterschied darin bestehen, dass einer der beiden Reste $R^1$ ein aromatisches oder heteroaromatisches Ringsystem mit 5 bis 30 aromatischen Ringatomen, das mit einem oder mehreren Resten $R^2$ substituiert sein kann darstellt, wohingegen der andere Rest $R^1$ eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, , -C≡C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, darstellt. Ferner kann der Unterschied darin bestehen, dass die beiden Reste $R^1$, die an das C-Atom in Position 9 in der Fluorenstruktur gemäß Formel (IIa) oder (IIb) gebunden sind, verschiedene aromatische oder heteroaromatische Ringsysteme mit 5 bis 30 aromatischen Ringatomen, welche mit einem oder mehreren Resten $R^2$ substituiert sein können, darstellen. Darüber hinaus können die beiden Reste $R^1$, die an das C-Atom in Position 9 in der Fluorenstruktur gemäß Formel (IIa) oder (IIb) gebunden sind, verschiedene geradkettige Alkyl-, Alkoxy- oder Thioalkoxygruppen mit 1 bis 40 C-Atomen oder verzweigte oder cyclische Alkyl-, Alkoxy- oder Thioalkoxygruppen mit 3 bis 40 C-Atomen, die jeweils mit einem oder mehreren Resten $R^2$ substituiert sein können, wobei eine oder mehrere nicht benachbarte $CH_2$-Gruppen durch $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, darstellen.

Besonders bevorzugt sind die beiden Reste $R^1$, die an das C-Atom in Position 9 in der Fluorenstruktur gemäß Formel (IIa) oder (IIb) gebunden sind, gleich.

Vorzugsweise können die beiden Reste $R^1$, die an das C-Atom in Position 9 in der Fluorenstruktur gemäß Formel (IIa) oder (IIb) gebunden sind, jeweils ein aromatisches oder heteroaromatisches Ringsystem mit 5 bis 30 aromatischen Ringatomen, das mit einem oder mehreren Resten $R^1$ substituiert sein kann darstellen, wobei die zwei Ringsysteme miteinander verbunden sind.

Weiterhin zeigen Verbindungen gemäß Formel (I) überraschende Vorteile, bei denen die beiden Reste $R^1$, die an das C-Atom in Position 9 in der Fluorenstruktur gemäß Formel (IIa) oder (IIb) gebunden sind, jeweils ein aromatisches oder heteroaromatisches Ringsystem mit 5 bis 30 aromatischen Ringatomen, das mit einem oder mehreren Resten $R^1$ substituiert sein kann darstellen, wobei die zwei Ringsysteme miteinander verbunden sind.

Besonders bevorzugt sind hierbei Verbindungen gemäß Formel (I), worin mindestens einer der Reste $Ar^2$ und/oder $Ar^3$ eine Gruppe der Formel (IIc) oder (IId) umfasst

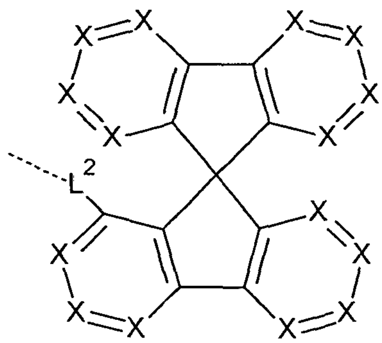

Formel (IIc)

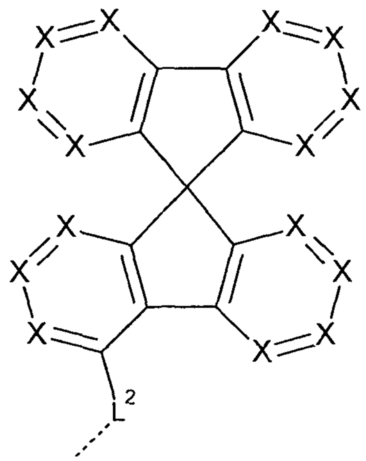

Formel (IId)

worin die Symbole die zuvor dargelegten Bedeutungen annehmen können.

Darüber hinaus sind Verbindungen bevorzugt, die dadurch gekennzeichnet sind, dass das die beiden Reste $R^1$, die an das C-Atom in Position 9 in der Fluorenstruktur gemäß Formel (IIa) oder (IIb) gebunden sind, jeweils ein aromatisches oder heteroaromatisches Ringsystem mit 5 bis 30 aromatischen Ringatomen, das mit einem oder mehreren Resten $R^1$ substituiert sein kann darstellen, wobei die zwei Ringsysteme nicht miteinander verbunden sind, oder eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können.

Besonders bevorzugt sind hierbei Verbindungen gemäß Formel (I), worin mindestens einer der Reste $Ar^2$ und/oder $Ar^3$ eine Gruppe der Formel (IIe) oder (IIf) umfasst

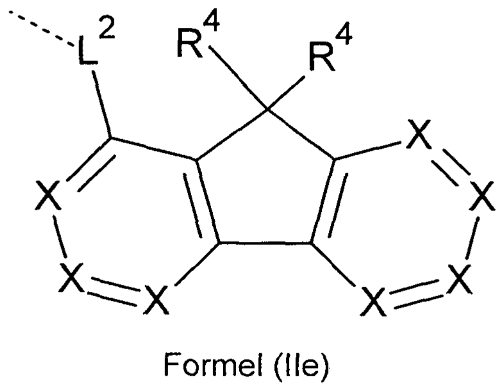

Formel (IIe)

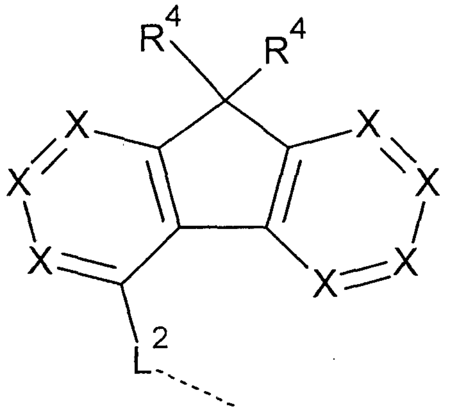

Formel (IIf)

worin die Symbole X und $L^2$ die zuvor dargelegte Bedeutung annehmen können und $R^4$ für H, ein aromatisches Ringsystem mit 6 bis 30 aromatischen Ringatomen, vorzugsweise eine Arylgruppe mit 6 bis 20 Kohlenstoffatomen, besonders bevorzugt Phenyl, Biphenyl, oder Naphthyl, oder eine Alkylgruppe mit 1 bis 20 Kohlenstoffatomen, vorzugsweise Methyl, Ethyl, Propyl oder Butyl, besonders bevorzugt Methyl steht.

In Strukturen gemäß den Formeln (IIe) oder (IIf) kann vorgesehen sein, dass die beiden Reste $R^4$, die an das C-Atom in Position 9 in der Fluorenstruktur gebunden sind, identisch sind. Weiterhin kann vorgesehen sein, dass beiden Reste $R^4$, die an das C-Atom in Position 9 in der Fluorenstruktur gemäß Formel (IIe) oder (IIf) gebunden sind, verschieden sind. Vorzugsweise sind die beiden Reste $R^4$, die an das C-Atom in Position 9 in der Fluorenstruktur gebunden sind, in Strukturen gemäß den Formeln (IIe) oder (IIf) identisch.

Weiterhin zeigen Verbindungen gemäß Formeln (I), (Ib), (IIa), (IIb), (IIc), (IId), (IIe) und Formel (IIf) überraschende Vorteile, bei nicht mehr als zwei, vorzugsweise nicht mehr als eine Gruppe X für N steht, vorzugsweise alle X für $CR^1$ stehen, wobei vorzugsweise höchstens 4, besonders bevorzugt höchstens 3 und speziell bevorzugt höchstens 2 der Gruppen $CR^1$ für die X steht, ungleich der Gruppe CH ist.

In den Strukturen gemäß Formeln (I), (Ia), (Ib), (Ic), (IIa), (IIb), (IIc), (IId), (IIe) und Formel (IIf) kann vorzugsweise vorgesehen sein, dass der Rest $L^1$ und/oder $L^2$, falls diese keine Bindung darstellt, über eine Arylgruppe oder Heteroarylgruppe an das Stickstoffatom, die Fluorengruppe (Formeln (IIa), (IIb), (IIc), (IId), (IIe) und (IIf)) und/oder den in Formeln (I), (Ia), (Ib), (Ic), dargestellten heterocyclischen Rest, vorzugsweise eine Carbazolgruppe, bindet. Falls die Reste $L^1$ und $L^2$ keine Bindung darstellen, binden diese Reste $L^1$ und $L^2$ vorzugsweise an das Stickstoffatom und die Fluorengruppe (Formeln (IIa), (IIb), (IIc), (IId), (IIe) und (IIf) und den in Formeln (I), (Ia), (Ib), (Ic), dargestellten heterocyclischen Rest, vorzugsweise eine Carbazolgruppe, über eine Arylgruppe oder Heteroarylgruppe.

Bevorzugt sind Verbindungen umfassend Strukturen der Formel (I), in denen mindestens mindestens eine Gruppe $L^1$ und/oder $L^2$ gemäß Formel (I), (IIa) und/oder (IIb) für eine Gruppe steht, die ausgewählt ist aus den Formeln (L-1) bis (L-78)

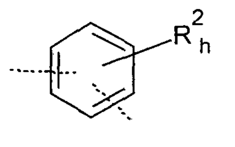

Formel (L-1)

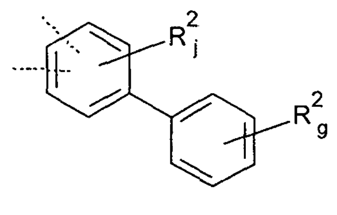

Formel (L-2)

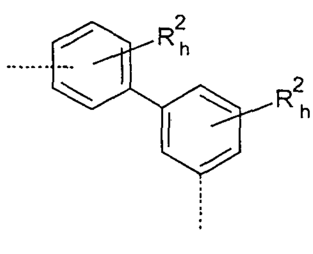

Formel (L-3)

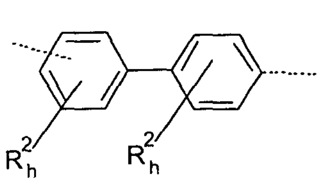

Formel (L-4)

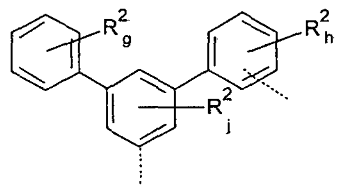

Formel (L-5)

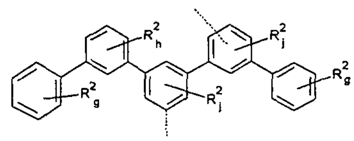

Formel (L-6)

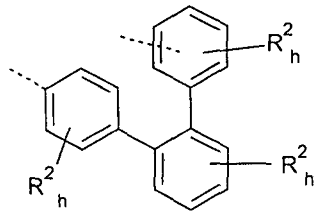

Formel (L-7)

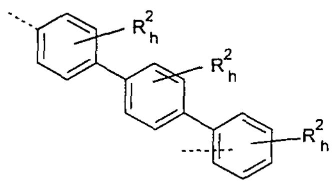

Formel (L-8)

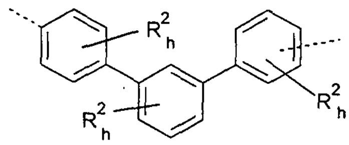

Formel (L-9)

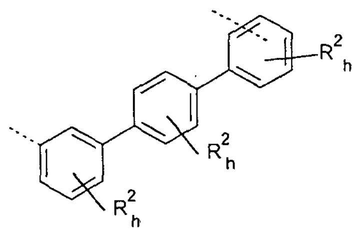

Formel (L-10)

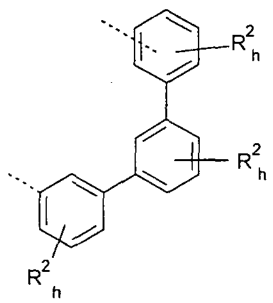

Formel (L-11)

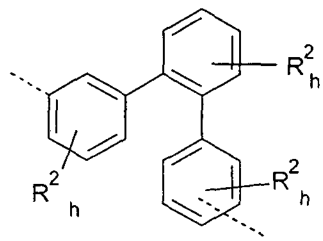

Formel (L-12)

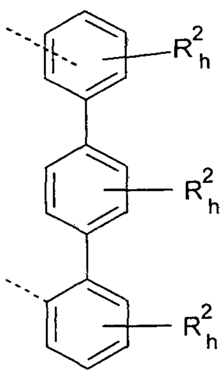

Formel (L-13)

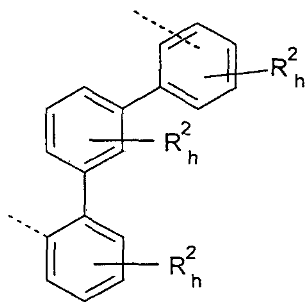

Formel (L-14)

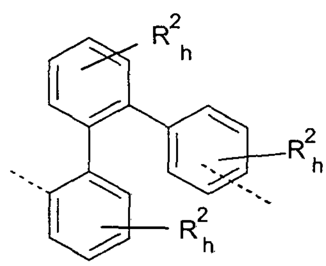

Formel (L-15)

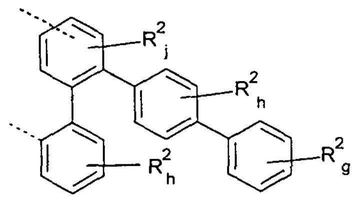

Formel (L-16)

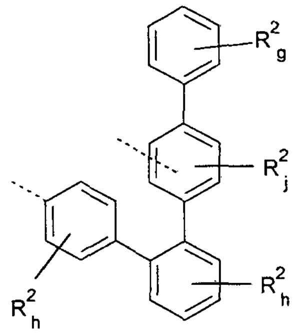

Formel (L-17)

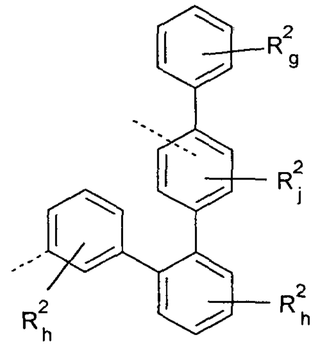

Formel (L-18)

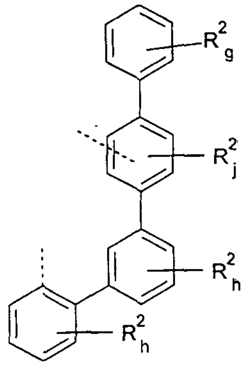

Formel (L-19)

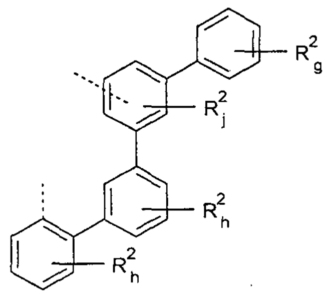

Formel (L-20)

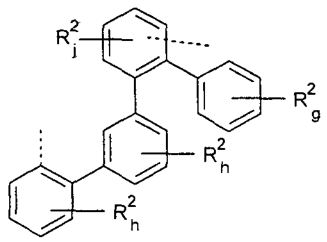

Formel (L-21)

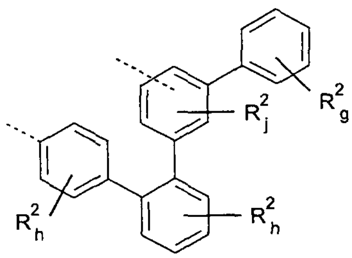

Formel (L-22)

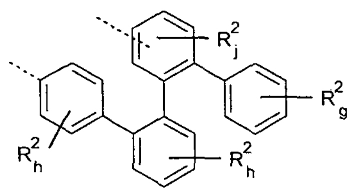

Formel (L-23)

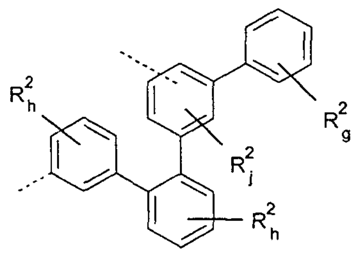

Formel (L-24)

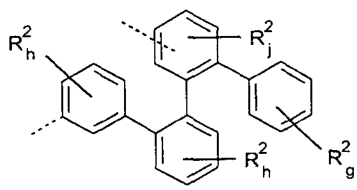

Formel (L-25)

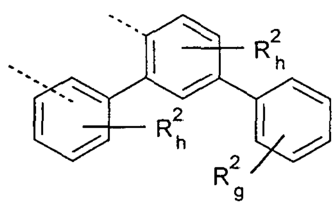

Formel (L-26)

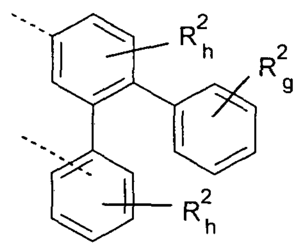

Formel (L-27)

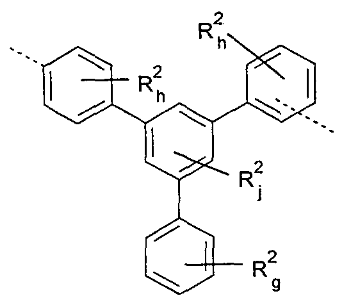

Formel (L-28)

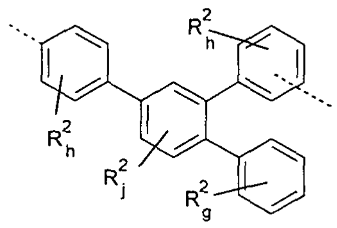

Formel (L-29)

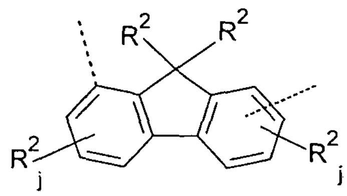

Formel (L-30)

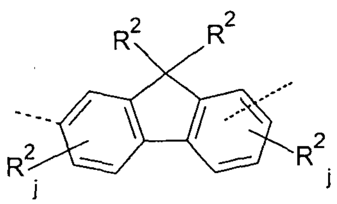

Formel (L-31)

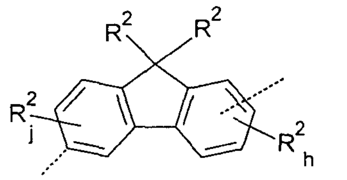

Formel (L-32)

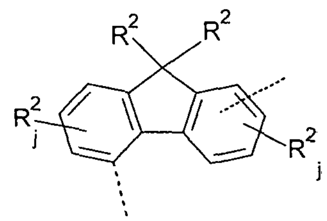

Formel (L-33)

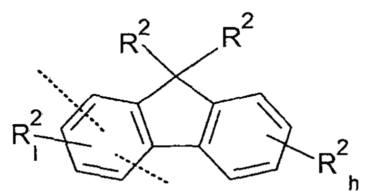

Formel (L-34)

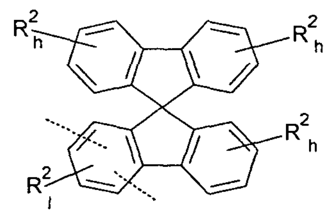

Formel (L-35)

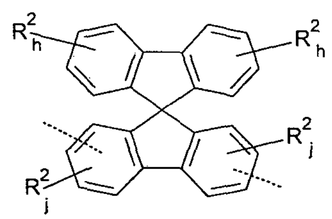

Formel (L-36)

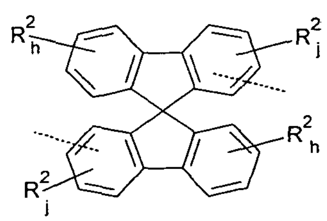

Formel (L-37)

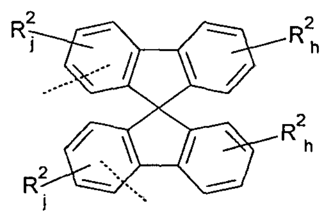

Formel (L-38)

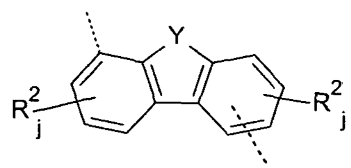

Formel (L-39)

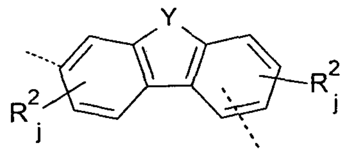

Formel (L-40)

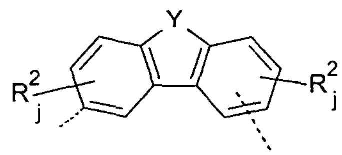

Formel (L-41)

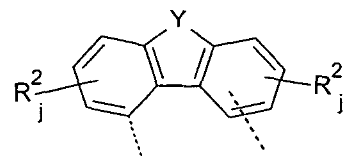

Formel (L-42)

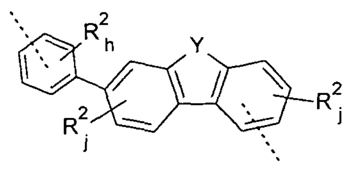

Formel (L-43)

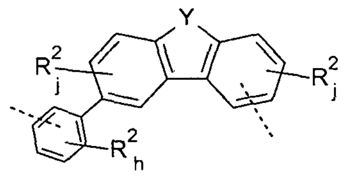

Formel (L-44)

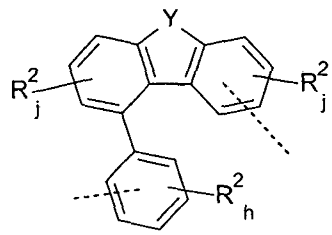

Formel (L-45)

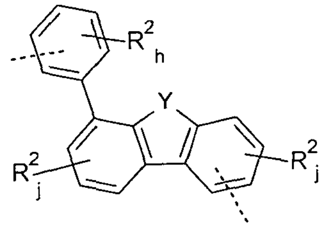

Formel (L-46)

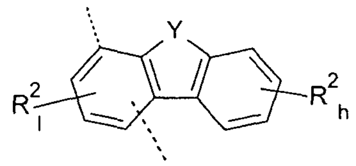

Formel (L-47)

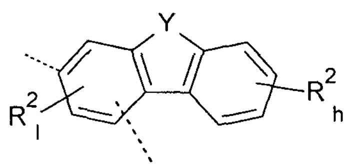

Formel (L-48)

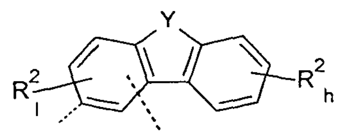

Formel (L-49)

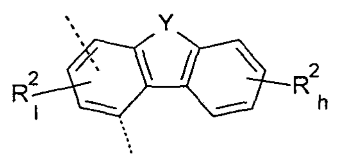

Formel (L-50)

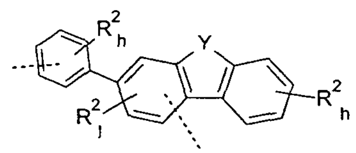

Formel (L-51)

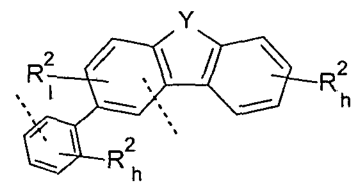

Formel (L-52)

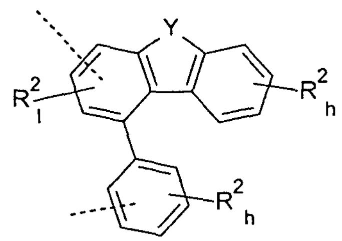

Formel (L-53)

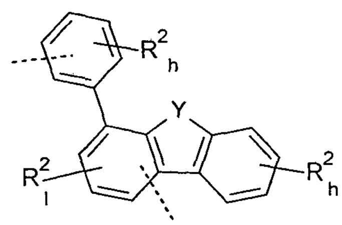

Formel (L-54)

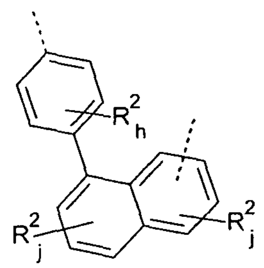

Formel (L-55)

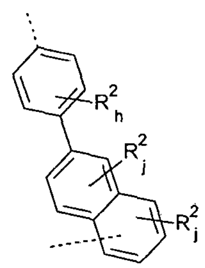

Formel (L-56)

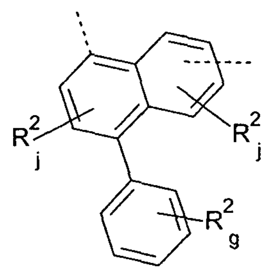

Formel (L-57)

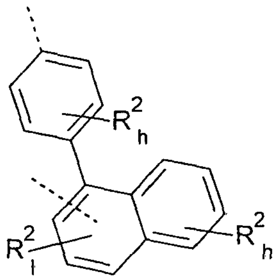

Formel (L-58)

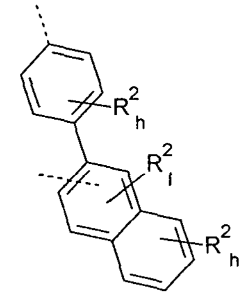

Formel (L-59)

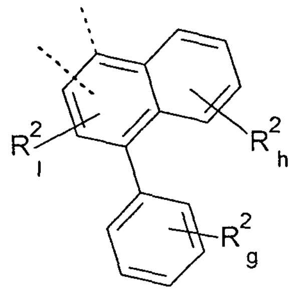

Formel (L-60)

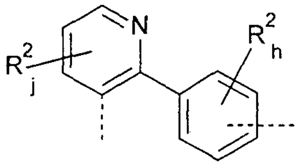

Formel (L-61)

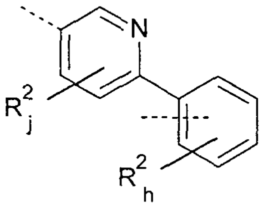

Formel (L-62)

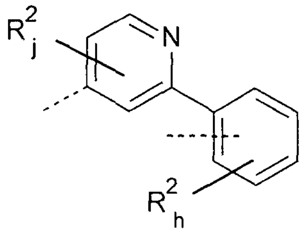

Formel (L-63)

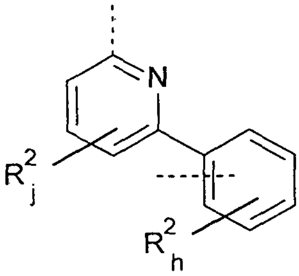

Formel (L-64)

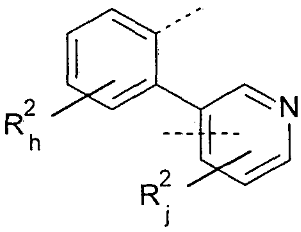

Formel (L-65)

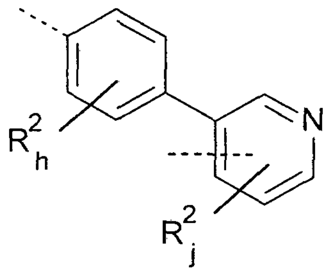

Formel (L-66)

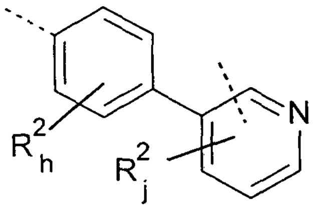

Formel (L-67)

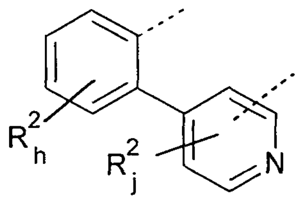

Formel (L-68)

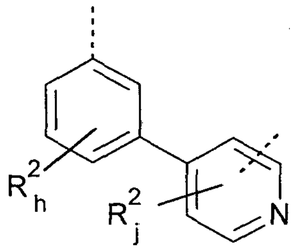

Formel (L-69)

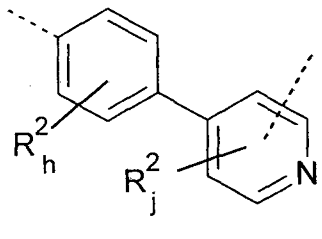

Formel (L-70)

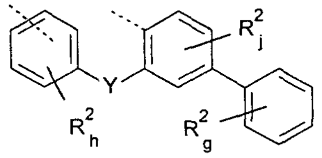

Formel (L-71)

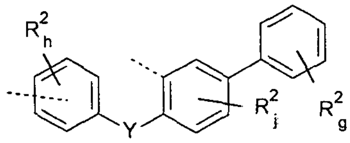

Formel (L-72)

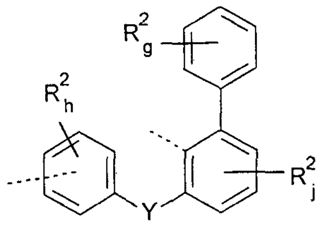

Formel (L-73)

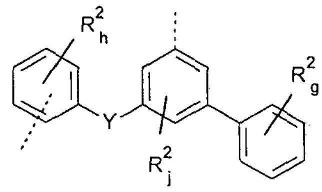

Formel (L-74)

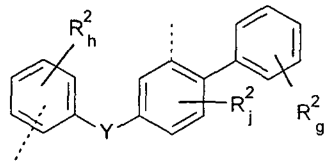

Formel (L-75)

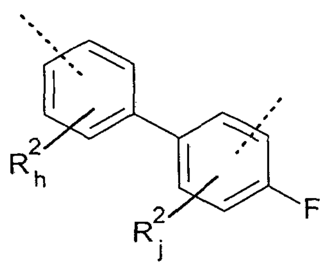

Formel (L-76)

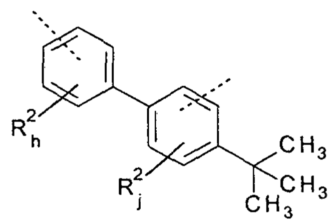

Formel (L-77)

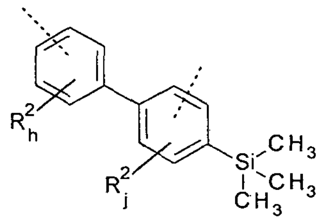

Formel (L-78)

worin, die gestrichelten Bindungen jeweils die Anbindungspositionen markieren, der Index l 0, 1 oder 2 ist, der Index g 0, 1, 2, 3, 4 oder 5 ist, j bei jedem Auftreten unabhängig 0, 1, 2 oder 3 ist; h bei jedem Auftreten unabhängig 0, 1, 2, 3 oder 4 ist; Y O, S oder $NR^2$, vorzugsweise O oder S ist; und $R^2$ die zuvor genannte Bedeutung annehmen kann.

Vorzugsweise kann vorgesehen sein, dass die Summe der Indices l, g, h und j in den Strukturen der Formel (L-1) bis (L-78) jeweils höchstens 3, vorzugsweise höchstens 2 und besonders bevorzugt höchstens 1 beträgt.

Besonders bevorzugt stellt die Gruppe $L^1$ und/oder $L^2$ gemäß Formel (I), (IIa) und/oder (IIb) einen aromatischen Rest mit 6 bis 18, vorzugsweise 6 bis 12 Kohlenstoffatomen dar, wobei Strukturen der Formel (L-1) bis (L-15) bevorzugt und Strukturen der Formel (L-1) bis (L-4) besonders bevorzugt sind.

Vorzugsweise kann die Verbindung mit Strukturen gemäß Formel (I) Reste $R^1$ umfassen, bei denen diese Reste $R^1$ bei jedem Auftreten gleich oder verschieden bevorzugt ausgewählt sind aus der Gruppe bestehend aus H, D, F, Br, I, CN, $Si(R^2)_3$, $B(OR^2)_2$, $C(=O)Ar^1$, einer geradkettigen Alkylgruppe mit 1 bis 10 C-Atomen oder einer geradkettigen Alkoxygruppe mit 1 bis 10 C-Atomen oder einer Alkenylgruppe mit 2 bis 10 C-Atomen oder einer verzweigten oder cyclischen Alkoxygruppe mit 3 bis 10 C-Atomen oder einer verzweigten oder cyclischen Alkylgruppe mit 3 bis 10 C-Atomen, die jeweils mit einem oder mehreren Resten $R^2$ substituiert sein kann, wobei ein oder mehrere H-Atome durch D oder F ersetzt sein können, oder einem aromatischen oder heteroaromatischen Ringsystem mit 5 bis 30 aromatischen Ringatomen, das jeweils durch einen oder mehrere Reste $R^2$ substituiert sein kann; dabei können zwei benachbarte Reste R bzw. $R^1$ oder R bzw. $R^1$ mit $R^2$ auch miteinander ein mono- oder polycyclisches, aliphatisches oder aromatisches Ringsystem bilden. Besonders bevorzugt sind diese Reste $R^1$ bei jedem Auftreten gleich oder verschieden ausgewählt aus der Gruppe bestehend aus H, D, F, einer geradkettigen Alkoxygruppe mit 1 bis 6 C-Atomen oder einer verzweigten oder cyclischen Alkoxygruppe mit 3 bis 10 C-Atomen, einer geradkettigen Alkylgruppe mit 1 bis 6 C-Atomen oder einer verzweigten oder cyclischen Alkylgruppe mit 3 bis 10 C-Atomen, wobei ein oder mehrere H-Atome durch D oder F ersetzt sein können, oder einem aromatischen oder heteroaromatischen Ringsystem mit 5 bis 24 aromatischen Ringatomen, das jeweils durch einen oder mehrere Reste $R^2$ substituiert sein kann; dabei können zwei benachbarte Reste

R bzw. $R^1$ oder R bzw. $R^1$ mit $R^2$ auch miteinander ein mono- oder polycyclisches, aliphatisches oder aromatisches Ringsystem bilden. Besonders bevorzugt kann mindestens einer der Reste R oder $R^1$ in Formel (I) eine Arylgruppe oder eine Heteroarylgruppe mit 6 bis 18 Kohlenstoffatomen darstellen, die mit bis zu drei Resten $R^2$ substituiert sein kann.

Vorzugsweise kann die Verbindung mit Strukturen gemäß Formel (I) Reste $R^2$ umfassen, bei denen diese Reste $R^2$ bei jedem Auftreten gleich oder verschieden bevorzugt ausgewählt sind aus der Gruppe bestehend aus H, D, F, Cl, Br, I, CHO, $C(=O)Ar^1$, $P(=O)(Ar^1)_2$, $S(=O)Ar^1$, $S(=O)_2Ar^1$, CN, $NO_2$, $Si(R^3)_3$, $B(OR^3)_2$, $OSO_2R^3$, eine geradkettige Alkyl-, Alkoxy- oder Thioalkoxygruppe mit 1 bis 10 C-Atomen oder eine verzweigte oder cyclische Alkyl-, Alkoxy- oder Thioalkoxygruppe mit 3 bis 10 C-Atomen, die jeweils mit einem oder mehreren Resten $R^3$ substituiert sein kann, wobei eine oder mehrere nicht benachbarte $CH_2$-Gruppen durch C≡C , $Si(R^3)_2$, $Ge(R^3)_2$, $Sn(R^3)_2$, C=O, C=S, C=Se, $P(=O)(R^3)$, SO, $SO_2$, O, S oder $CONR^3$ ersetzt sein können und wobei ein oder mehrere H-Atome durch D, F, Cl, Br, I, CN oder $NO_2$ ersetzt sein können,
oder ein aromatisches oder heteroaromatisches Ringsystem mit 5 bis 24 aromatischen Ringatomen, das jeweils durch einen oder mehrere Reste $R^3$ substituiert sein kann, oder eine Aryloxy- oder Heteroaryloxygruppe mit 5 bis 24 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden. Besonders bevorzugt kann mindestens einer der Reste $R^2$ in Formel (I) eine Arylgruppe oder eine Heteroarylgruppe mit 6 bis 18 Kohlenstoffatomen darstellen, die mit bis zu drei Resten $R^3$ substituiert sein kann.

Bevorzugt sind Verbindungen umfassend Strukturen der Formel (I), in denen mindestens ein Rest $R^1$, $Ar^2$, $Ar^3$ oder $Ar^4$ in den Strukturen gemäß Formeln (I), (Ib), (IIa), (IIb), (IIc), (IId), (IIe) oder Formel (IIf) für eine Gruppe steht, die ausgewählt ist aus den Formeln ($R^1$-1) bis ($R^1$-72)

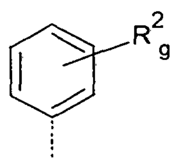

Formel ($R^1$-1)

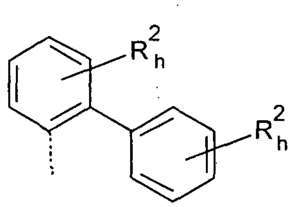

Formel ($R^1$-2)

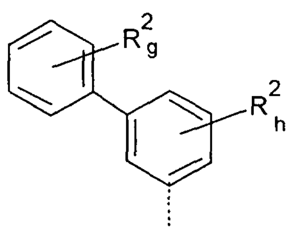

Formel ($R^1$-3)

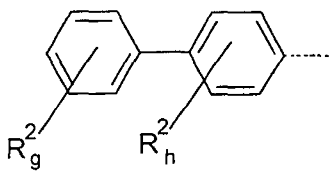

Formel ($R^1$-4)

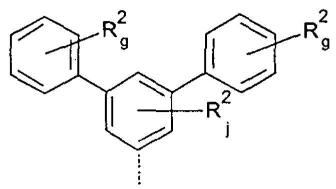

Formel ($R^1$-5)

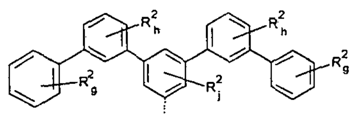

Formel ($R^1$-6)

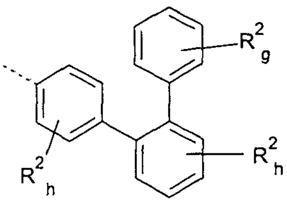

Formel ($R^1$-7)

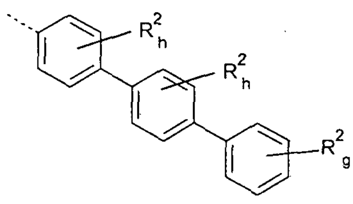

Formel ($R^1$-8)

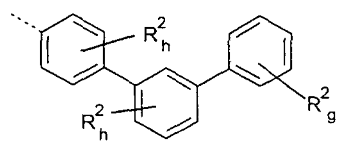

Formel ($R^1$-9)

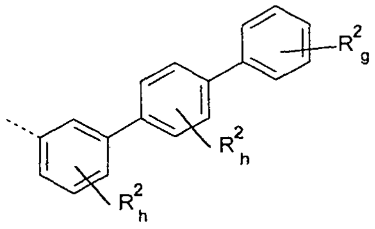
Formel ($R^1$-10)

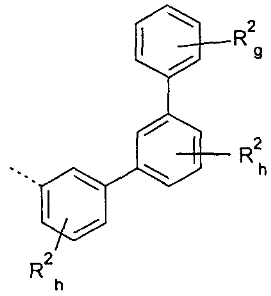
Formel ($R^1$-11)

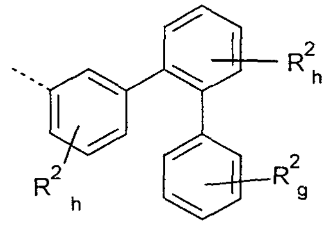
Formel ($R^1$-12)

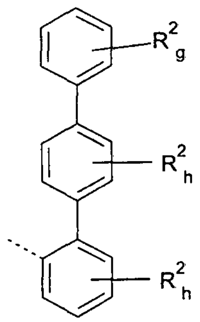
Formel ($R^1$-13)

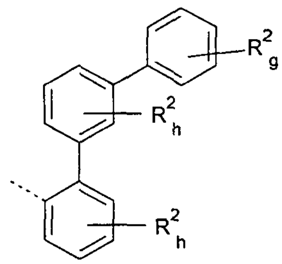
Formel ($R^1$-14)

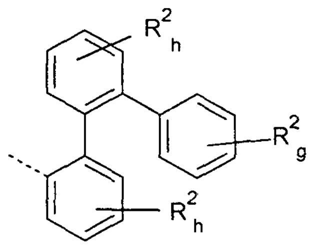
Formel ($R^1$-15)

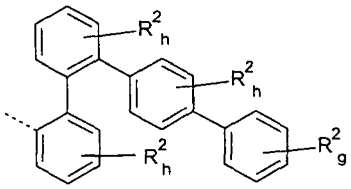
Formel ($R^1$-16)

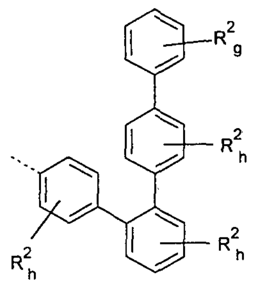
Formel ($R^1$-17)

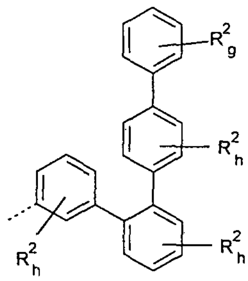
Formel ($R^1$-18)

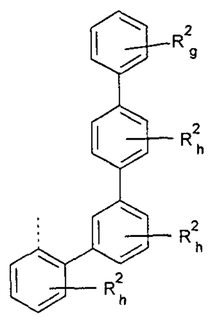
Formel ($R^1$-19)

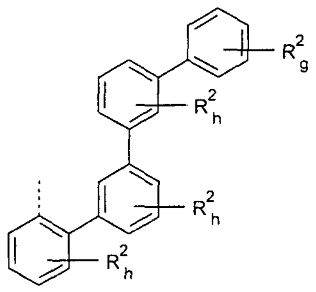
Formel ($R^1$-20)

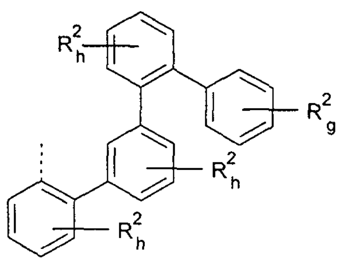
Formel ($R^1$-21)

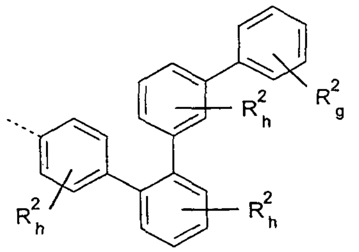

Formel ($R^1$-22)

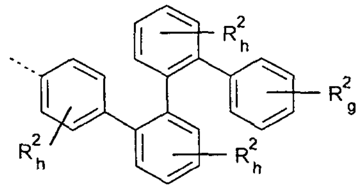

Formel ($R^1$-23)

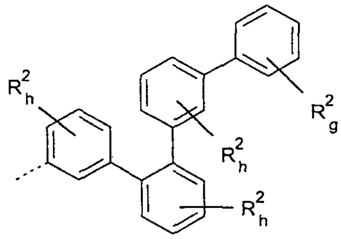

Formel ($R^1$-24)

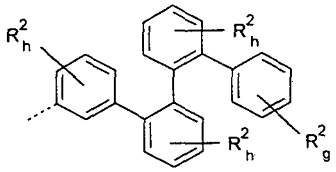

Formel ($R^1$-25)

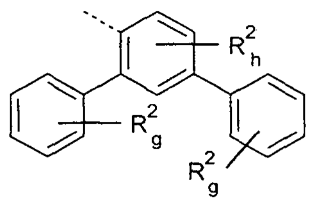

Formel ($R^1$-26)

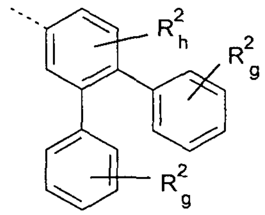

Formel ($R^1$-27)

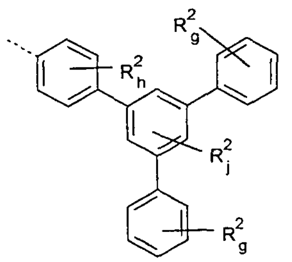

Formel ($R^1$-28)

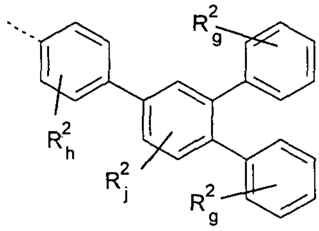

Formel ($R^1$-29)

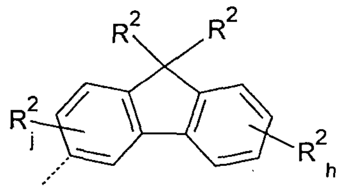

Formel ($R^1$-30)

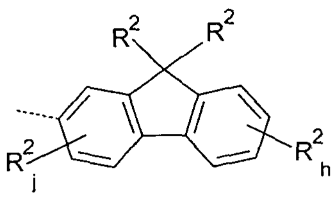

Formel ($R^1$-31)

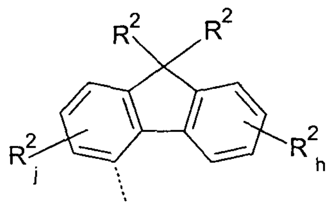

Formel ($R^1$-32)

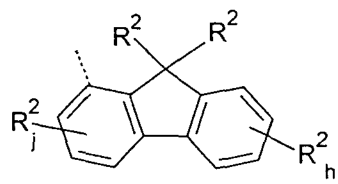

Formel ($R^1$-33)

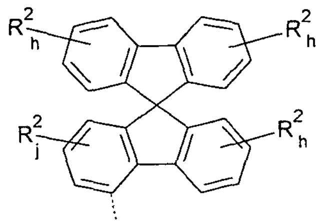

Formel ($R^1$-34)

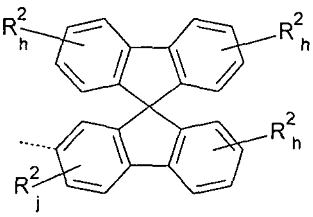

Formel ($R^1$-35)

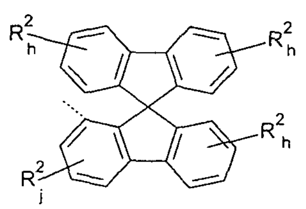

Formel ($R^1$-36)

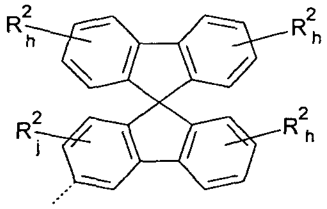

Formel ($R^1$-37)

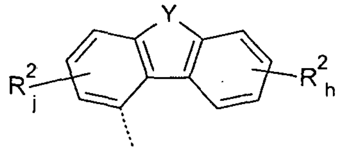

Formel ($R^1$-38)

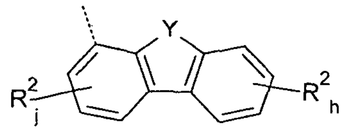

Formel ($R^1$-39)

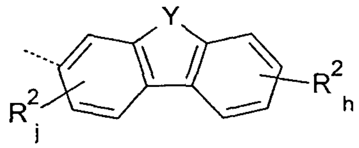

Formel ($R^1$-40)

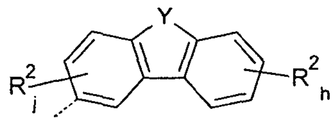

Formel ($R^1$-41)

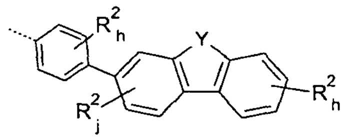

Formel ($R^1$-42)

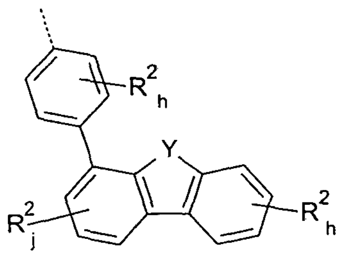

Formel ($R^1$-43)

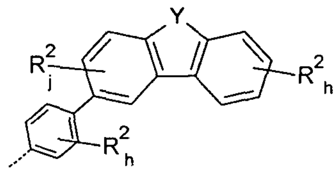

Formel ($R^1$-44)

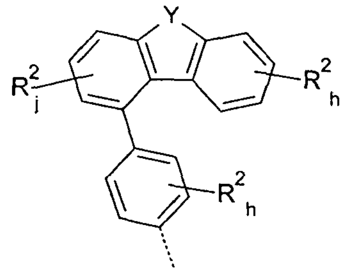

Formel ($R^1$-45)

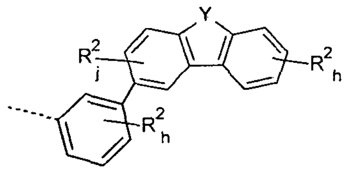

Formel ($R^1$-46)

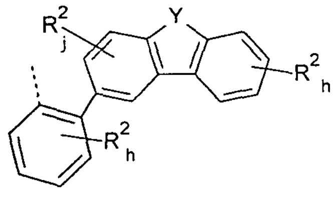

Formel ($R^1$-47)

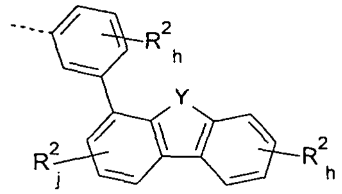

Formel ($R^1$-48)

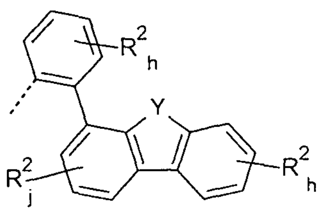

Formel ($R^1$-49)

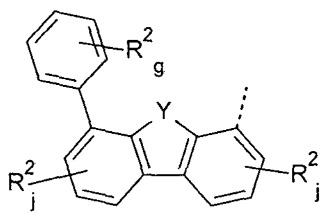

Formel ($R^1$-50)

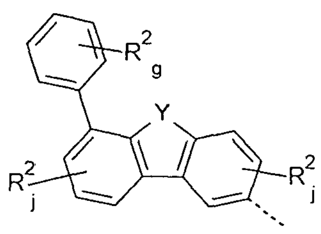

Formel ($R^1$-51)

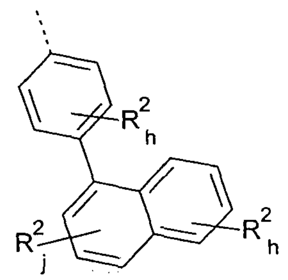

Formel ($R^1$-52)

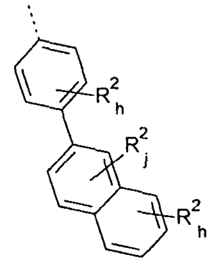

Formel ($R^1$-53)

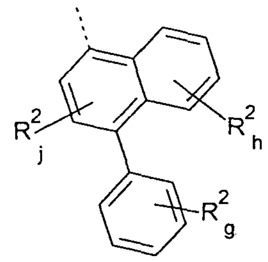

Formel ($R^1$-54)

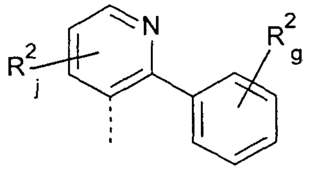

Formel ($R^1$-55)

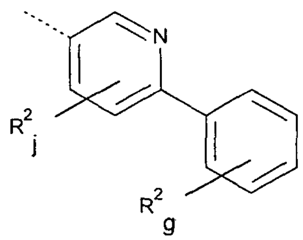

Formel ($R^1$-56)

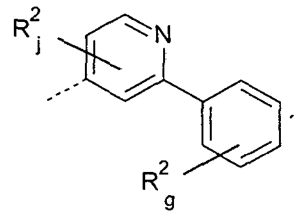

Formel ($R^1$-57)

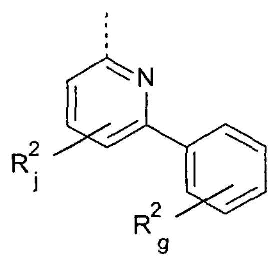

Formel ($R^1$-58)

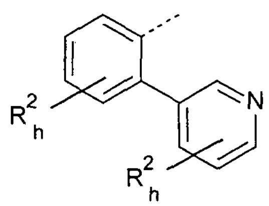

Formel ($R^1$-59)

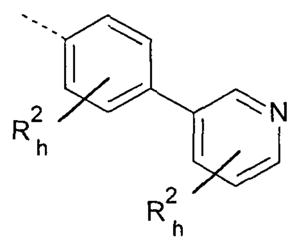

Formel ($R^1$-60)

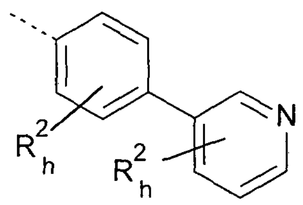

Formel ($R^1$-61)

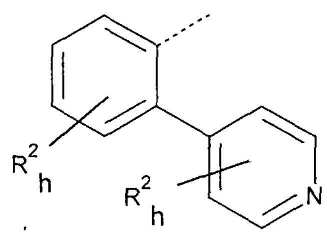

Formel ($R^1$-62)

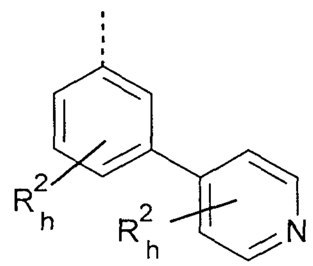

Formel ($R^1$-63)

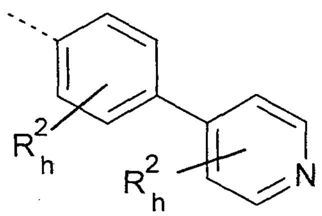

Formel ($R^1$-64)

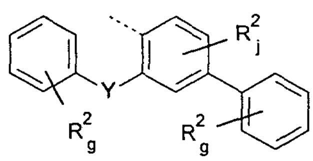

Formel ($R^1$-65)

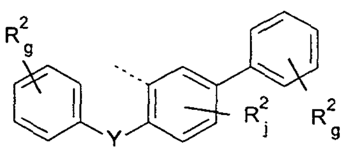

Formel ($R^1$-66)

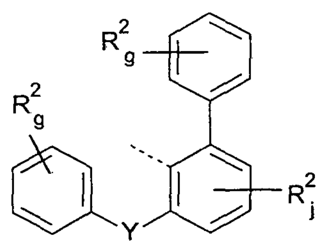
Formel ($R^1$-67)

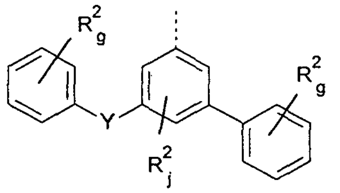
Formel ($R^1$-68)

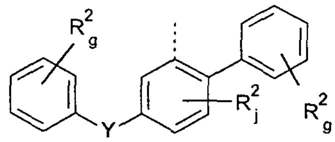
Formel ($R^1$-69)

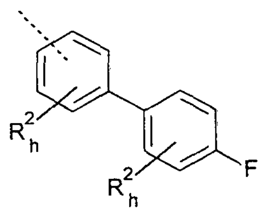
Formel ($R^1$-70)

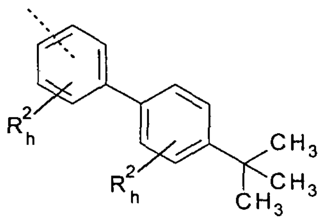
Formel ($R^1$-71)

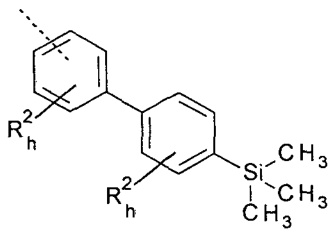
Formel ($R^1$-72)

wobei für die verwendeten Symbole gilt:

Y ist O, S oder $NR^2$, vorzugsweise O oder S;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5;

die gestrichelte Bindung markiert die Anbindungsposition; und

$R^2$ kann die zuvor genannte Bedeutung aufweisen.

Vorzugsweise kann vorgesehen sein, dass die Summe der Indices g, h und j in den Strukturen der Formel ($R^1$-1) bis ($R^1$-72) jeweils höchstens 3, vorzugsweise höchstens 2 und besonders bevorzugt höchstens 1 beträgt.
Besonders bevorzugt kann die Gruppe $R^1$, $Ar^2$, $Ar^3$ oder $Ar^4$ gemäß Formel (I), (IIa) und/oder (IIb) einen aromatischen Rest mit 6 bis 18, vorzugsweise 6 bis 12 Kohlenstoffatomen darstellen, wobei Strukturen der Formel ($R^1$-1) bis ($R^1$-15) bevorzugt und Strukturen der Formel ($R^1$-1) bis ($R^1$-4) besonders bevorzugt sind. Allerdings muss einer der Reste $Ar^2$ oder $Ar^3$ eine Gruppe der Formel (IIa) oder (IIb) darstellen.
Ferner kann vorgesehen sein, dass in Struktur gemäß Formel (I) die Gruppe $L^1$ für eine Bindung und in Formel (IIa) und/oder (IIb) die Gruppe $L^2$ für eine Gruppe steht, die ausgewählt ist aus den Formeln (L-1) bis (L-78), vorzugsweise aus den Formeln (L-1) bis (L-15), besonders bevorzugt aus den Formeln (L-1) bis (L- 5), wie zuvor beschrieben.
Ferner kann vorgesehen sein, dass in Struktur gemäß Formel (IIa) und/oder (IIb) die Gruppe $L^2$ für eine Bindung steht und in Formel (I) die Gruppe $L^1$ für eine Gruppe steht, die ausgewählt ist aus den Formeln (L-1) bis (L- 78), vorzugsweise aus den Formeln (L-1) bis (L-15), besonders bevorzugt aus den Formeln (L-1) bis (L- 5), wie zuvor beschrieben.
Ferner kann vorgesehen sein, dass in Struktur gemäß Formel (I), (IIa) und/oder (IIb) die Gruppen $L^1$ und $L^2$ für eine Bindung stehen.
Ferner kann vorgesehen sein, dass in Struktur gemäß Formel (I), (IIa) und/oder (IIb) die Gruppen $L^1$ und $L^2$ für eine Gruppe stehen, die ausgewählt ist aus den Formeln (L-1) bis (L- 78), vorzugsweise aus den Formeln (L-1) bis (L-15), besonders bevorzugt aus den Formeln (L-1) bis (L- 5), wie zuvor beschrieben.
Vorzugsweise kann der Rest $Ar^1$ und/oder $Ar^3$ in Struktren der Formel (I) ein aromatisches Ringsystem mit6 bis 18, vorzugsweise 6 bis 12 aromatischen Ringatomen darstellt, welches mit einem oder mehreren Resten $R^1$ substituiert sein kann, wobei vorzugsweise beide Reste $Ar^1$ und $Ar^3$ in Struktren der Formel (I) ein aromatisches Ringsystem mit 6 bis 12 aromatischen Ringatomen darstellen.
Besonders bevorzugte Verbindungen umfassen Strukuren gemäß den folgenden Formeln 1 bis 244:

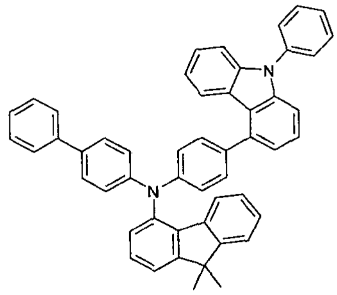

Formel 1

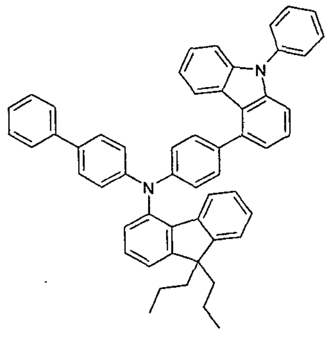

Formel 2

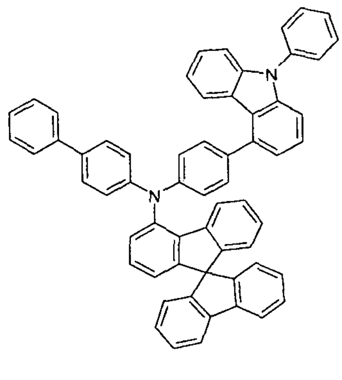

Formel 3

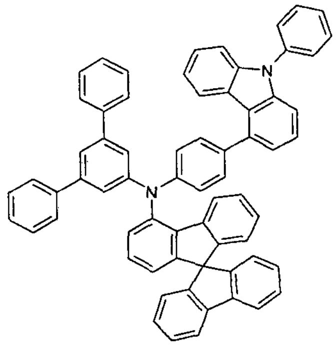

Formel 4

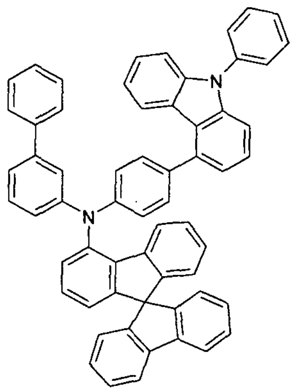

Formel 5

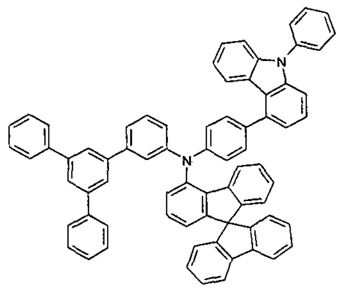

Formel 6

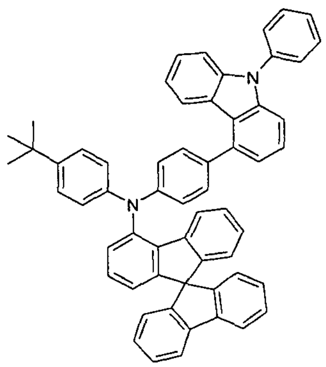

Formel 7

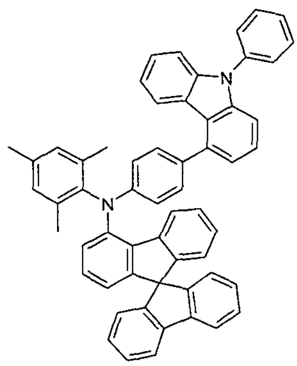

Formel 8

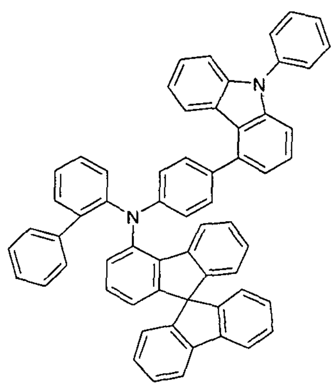

Formel 9

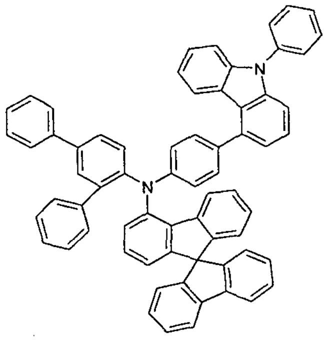

Formel 10

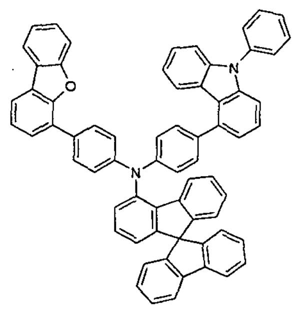

Formel 11

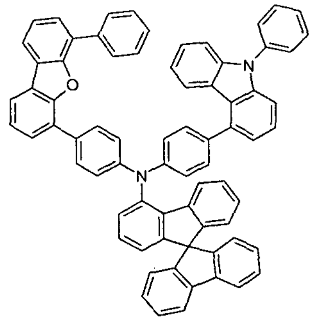

Formel 12

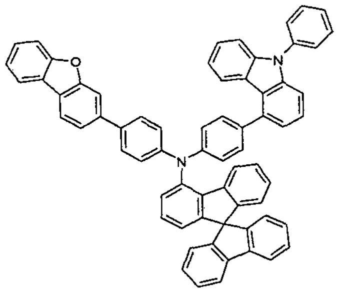

Formel 13

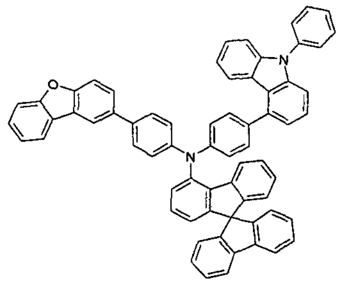

Formel 14

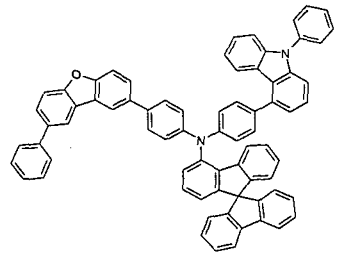

Formel 15

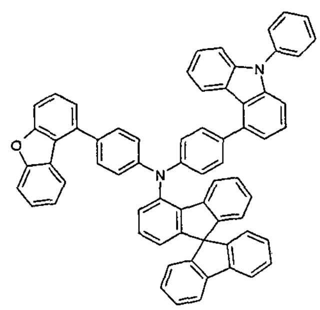

Formel 16

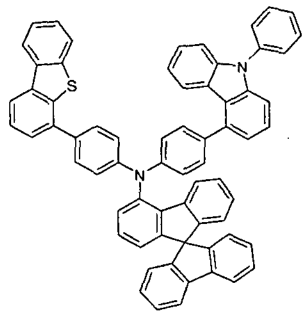

Formel 17

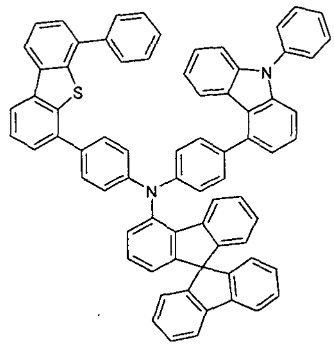

Formel 18

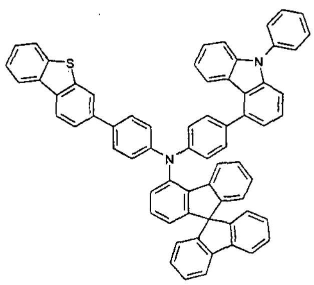

Formel 19

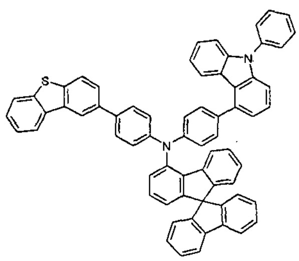

Formel 20

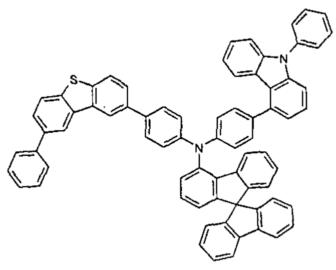

Formel 21

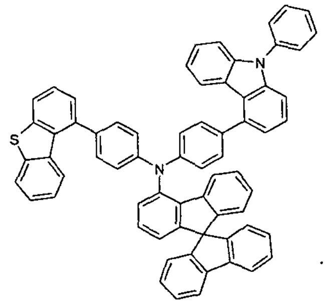

Formel 22

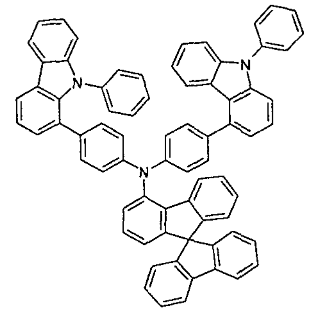

Formel 23

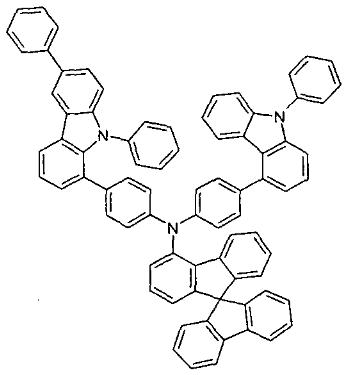

Formel 24

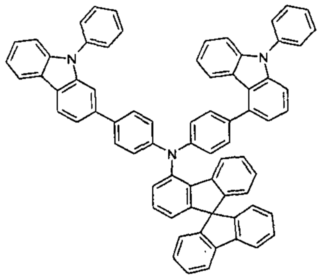

Formel 25

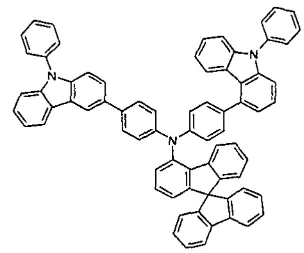

Formel 26

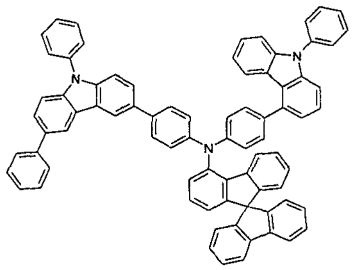

Formel 27

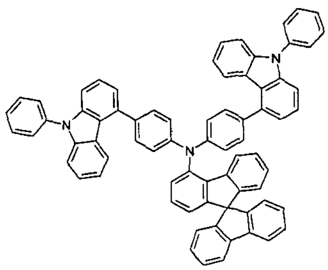

Formel 28

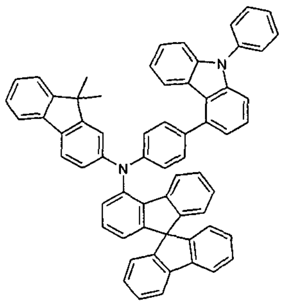

Formel 29

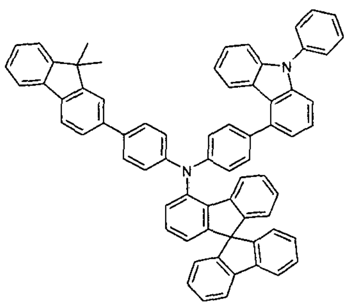

Formel 30

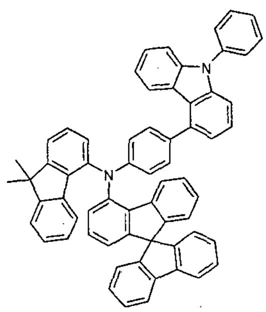

Formel 31

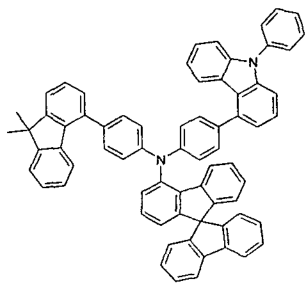

Formel 32

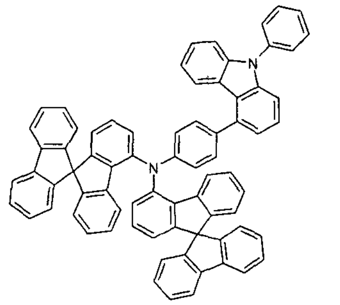

Formel 33

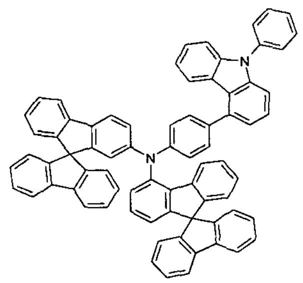

Formel 34

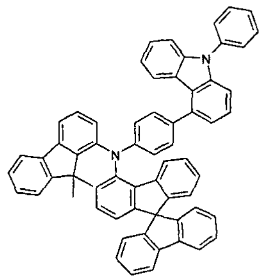

Formel 35

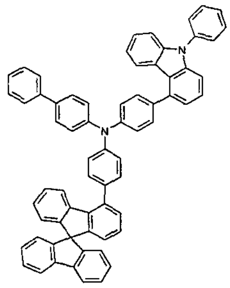

Formel 36

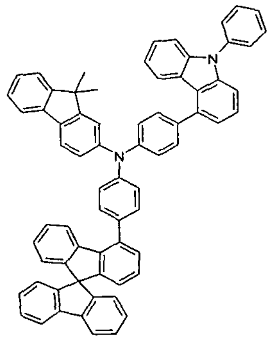

Formel 37

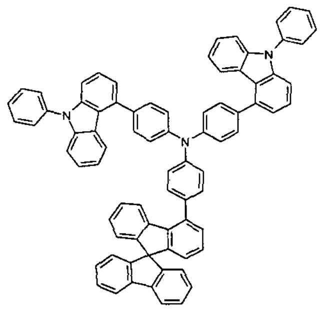

Formel 38

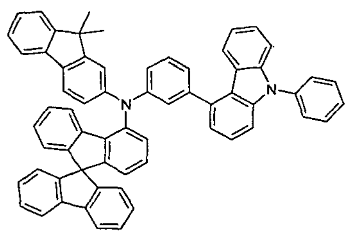

Formel 39

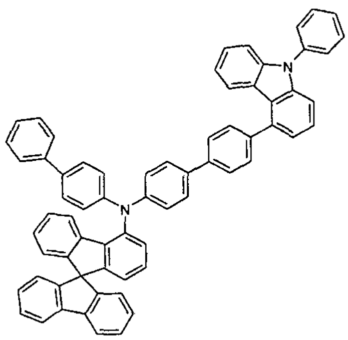

Formel 40

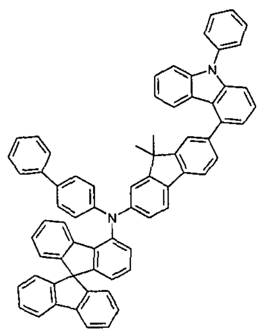

Formel 41

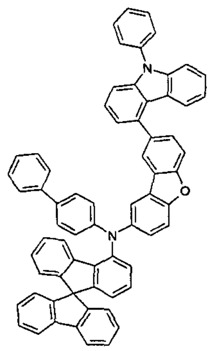

Formel 42

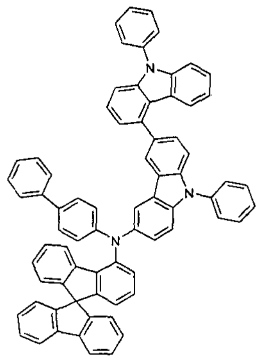

Formel 43

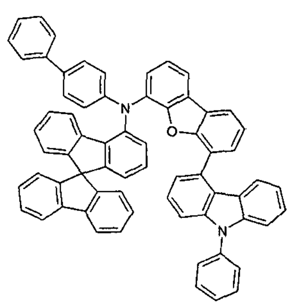

Formel 44

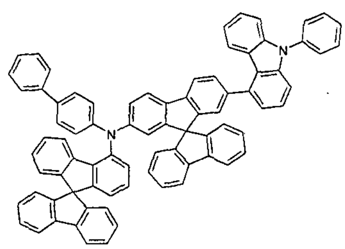

Formel 45

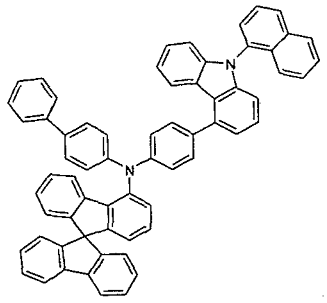

Formel 46

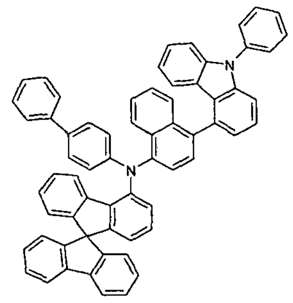

Formel 47

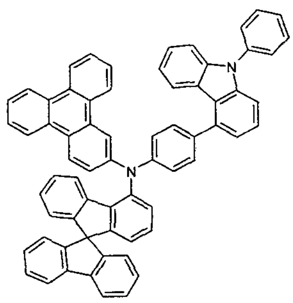

Formel 48

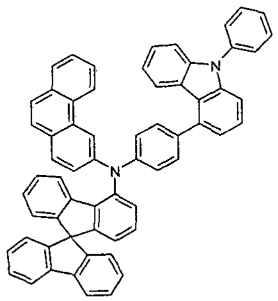

Formel 49

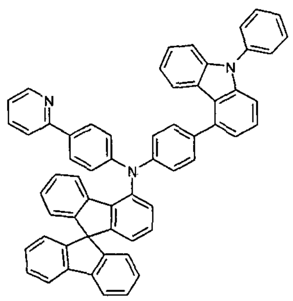

Formel 50

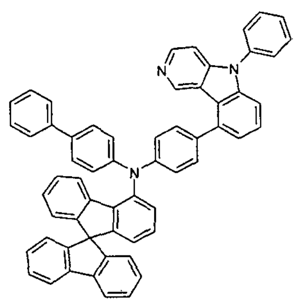

Formel 51

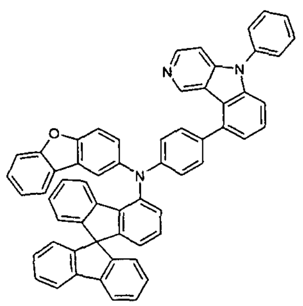

Formel 52

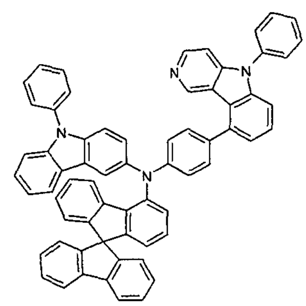

Formel 53

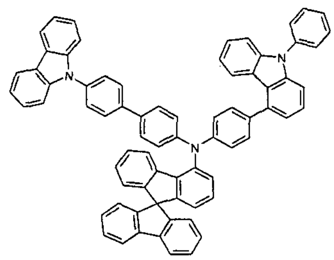

Formel 54

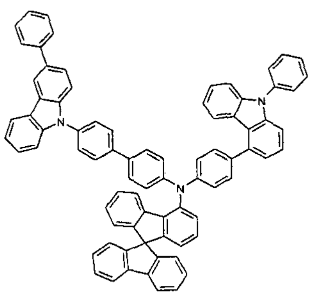

Formel 55

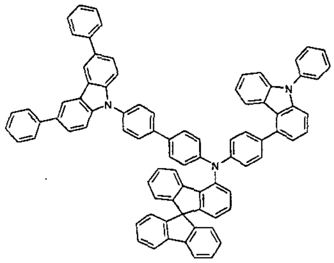

Formel 56

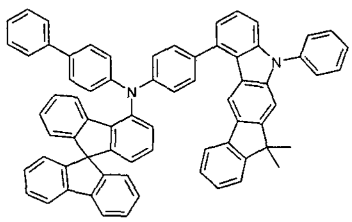

Formel 57

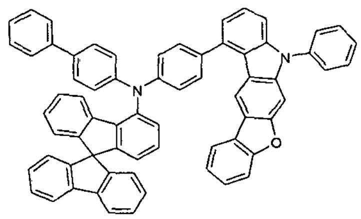
Formel 58

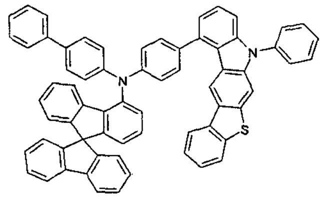
Formel 59

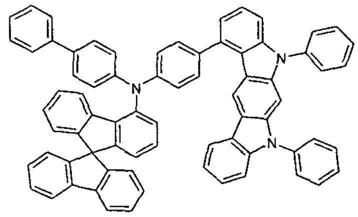
Formel 60

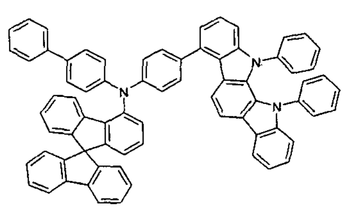
Formel 61

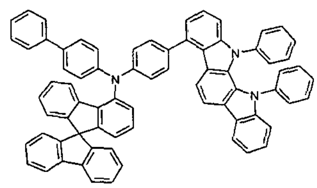
Formel 62

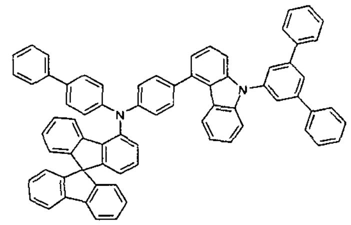
Formel 63

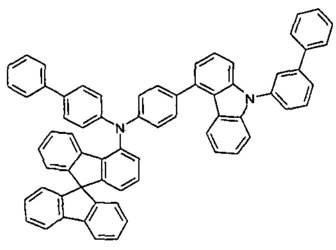
Formel 64

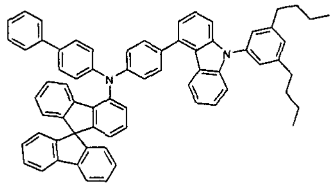
Formel 65

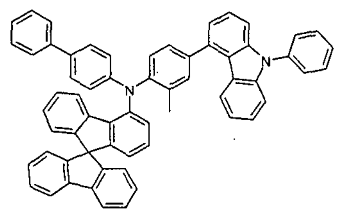
Formel 66

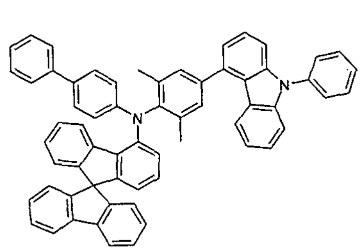
Formel 67

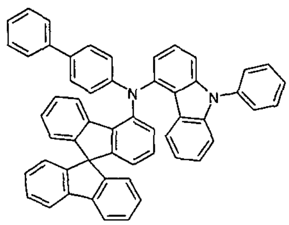
Formel 68

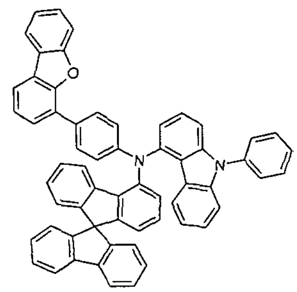
Formel 69

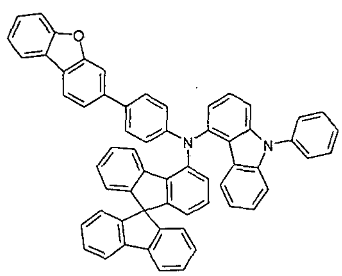

Formel 70

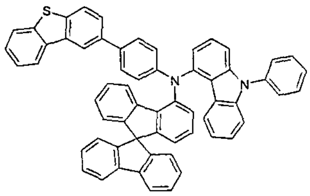

Formel 71

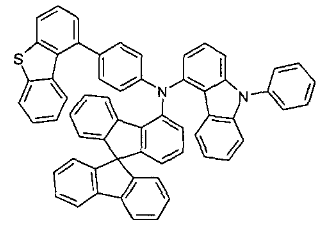

Formel 72

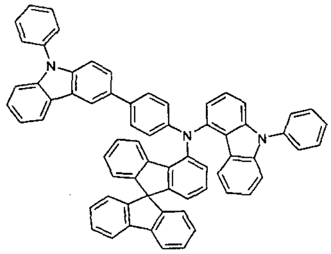

Formel 73

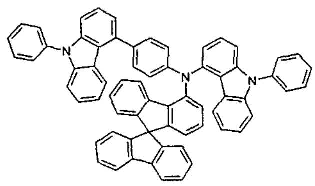

Formel 74

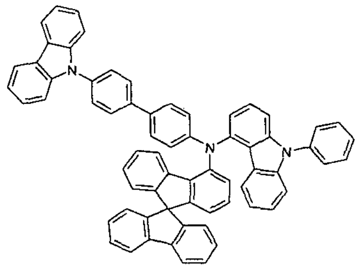

Formel 75

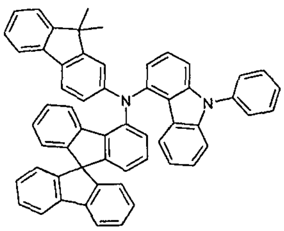

Formel 76

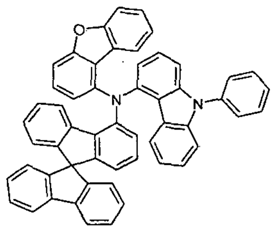

Formel 77

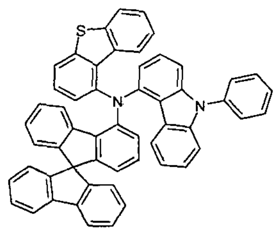

Formel 78

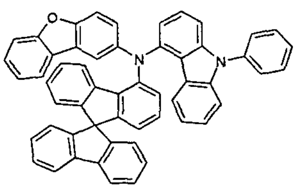

Formel 79

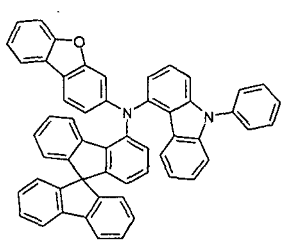

Formel 80

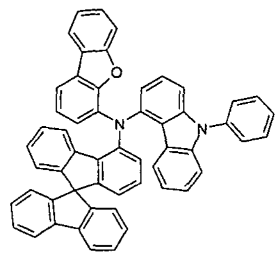

Formel 81

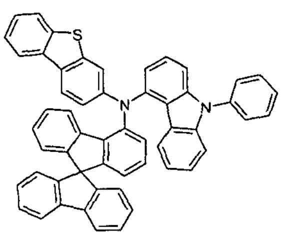
Formel 82

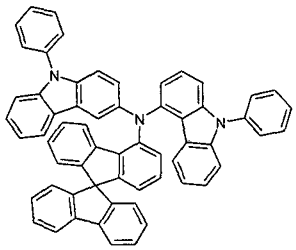
Formel 83

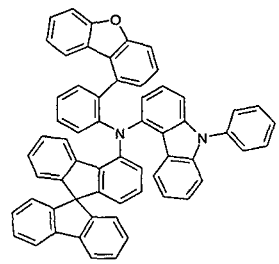
Formel 84

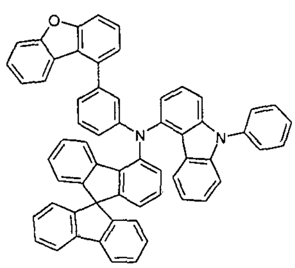
Formel 85

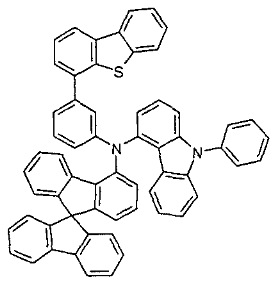
Formel 86

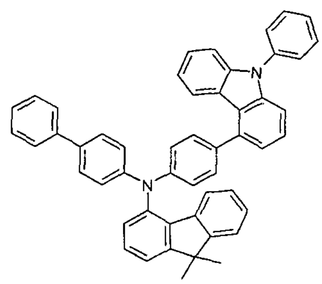
Formel 87

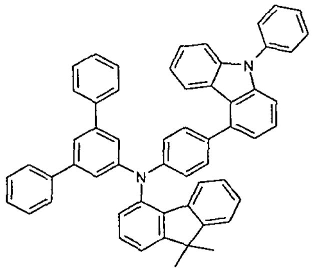
Formel 88

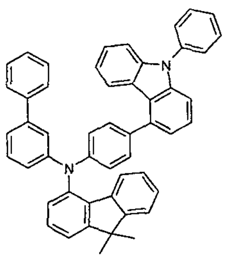
Formel 89

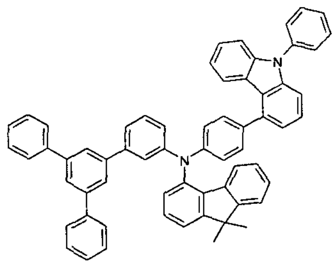
Formel 90

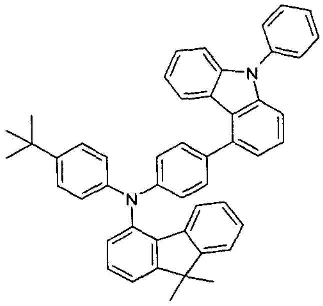
Formel 91

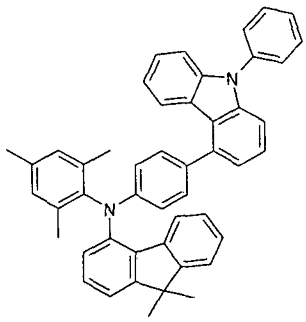
Formel 92

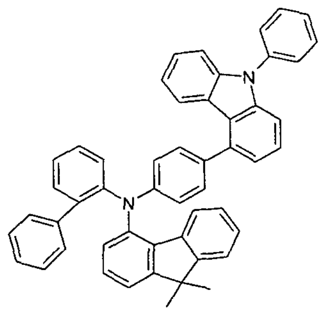
Formel 93

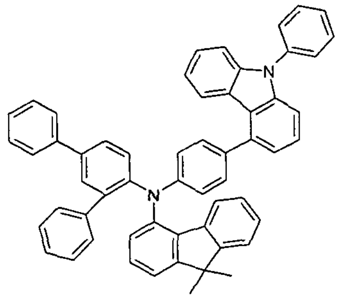

Formel 94

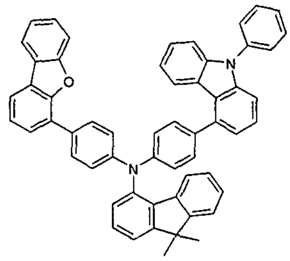

Formel 95

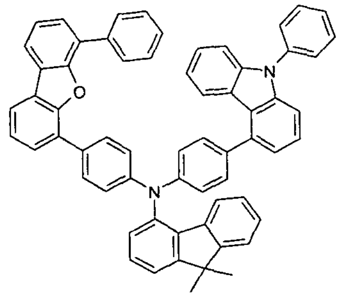

Formel 96

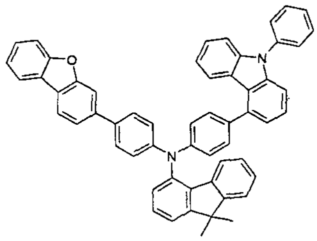

Formel 97

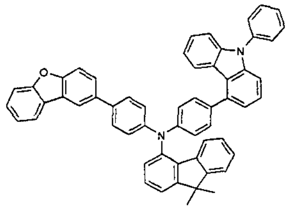

Formel 98

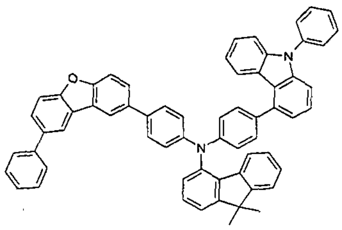

Formel 99

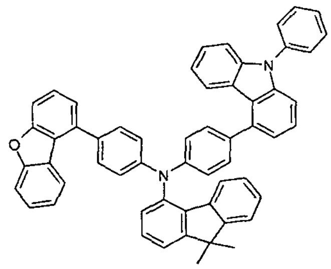

Formel 100

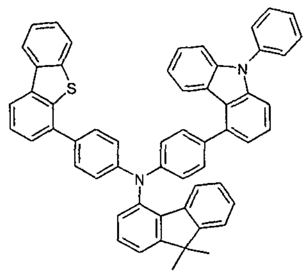

Formel 101

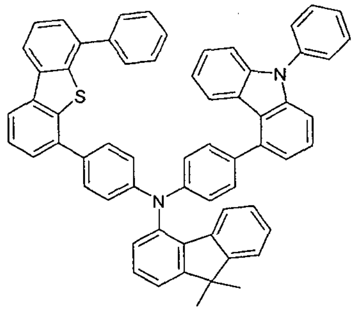

Formel 102

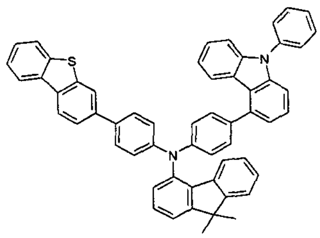

Formel 103

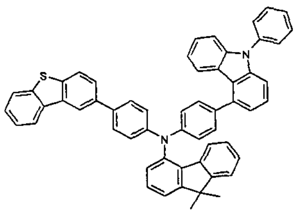

Formel 104

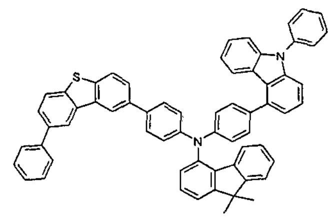

Formel 105

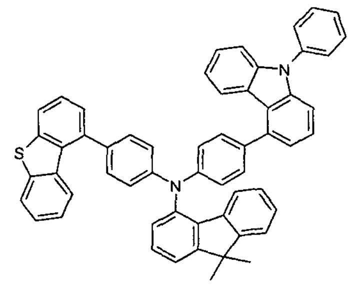
Formel 106

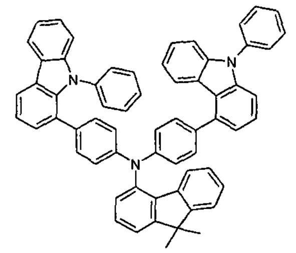
Formel 107

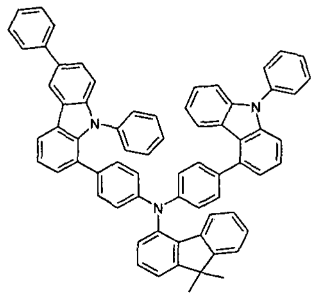
Formel 108

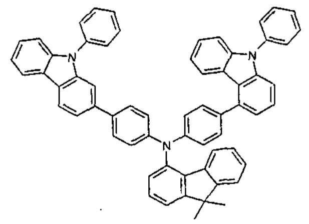
Formel 109

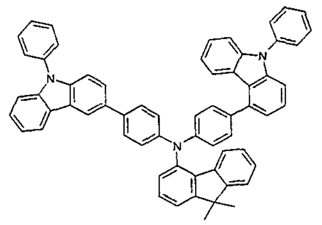
Formel 110

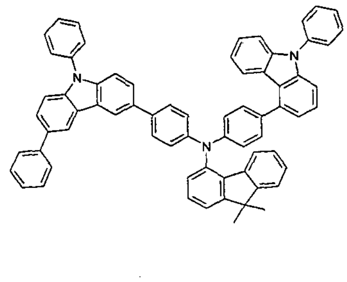
Formel 111

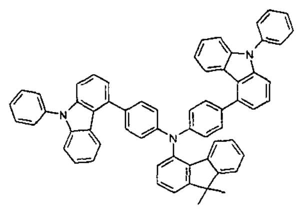
Formel 112

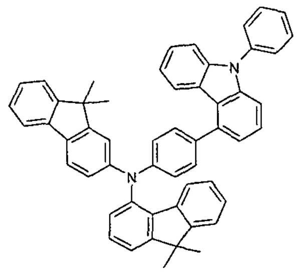
Formel 113

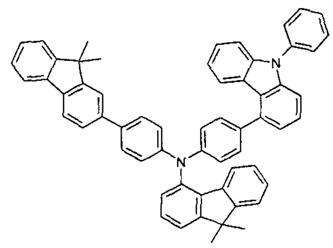
Formel 114

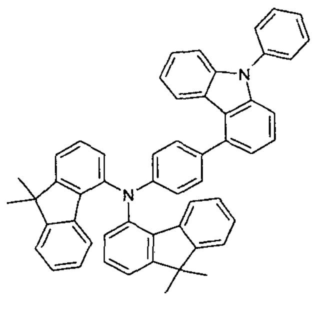
Formel 115

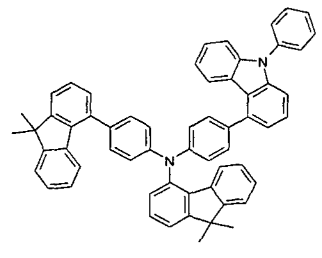
Formel 116

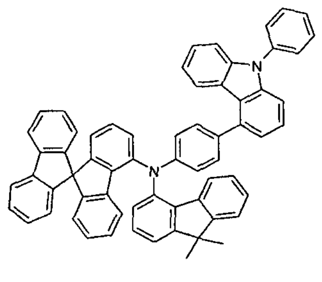
Formel 117

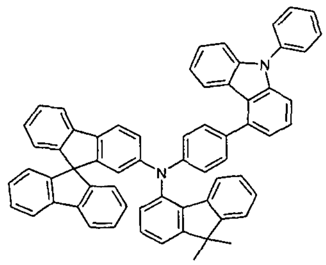

Formel 118

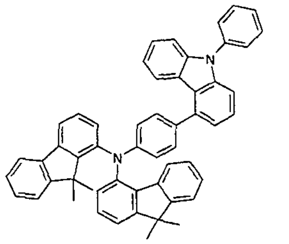

Formel 119

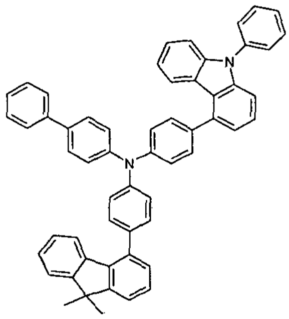

Formel 120

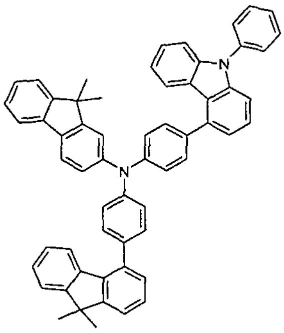

Formel 121

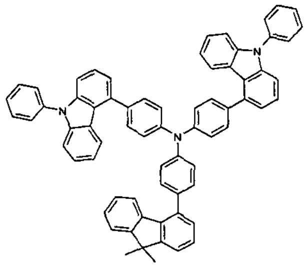

Formel 122

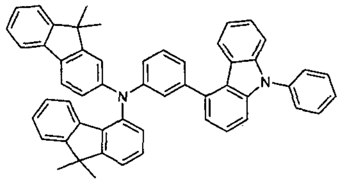

Formel 123

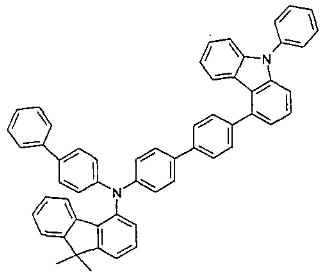

Formel 124

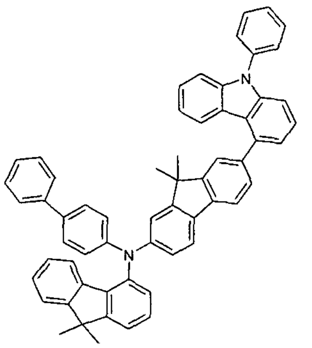

Formel 125

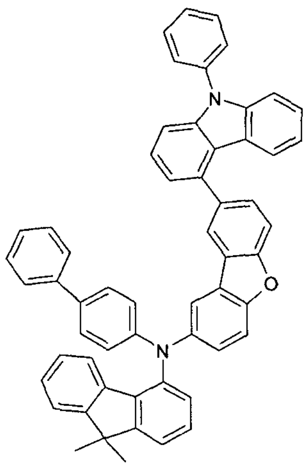

Formel 126

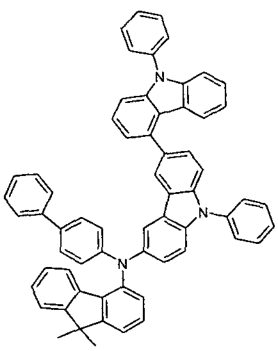

Formel 127

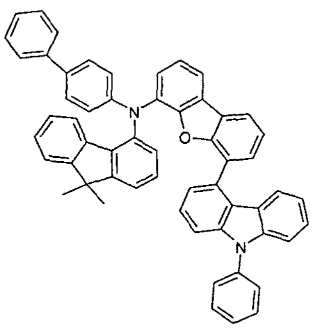

Formel 128

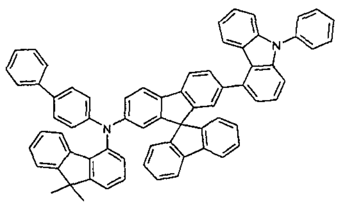

Formel 129

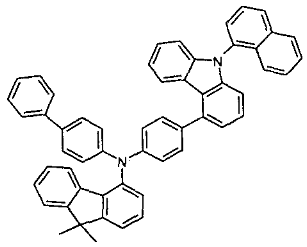

Formel 130

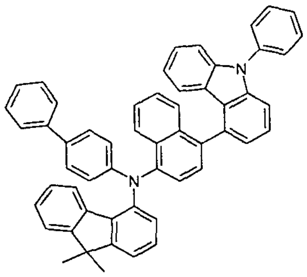

Formel 131

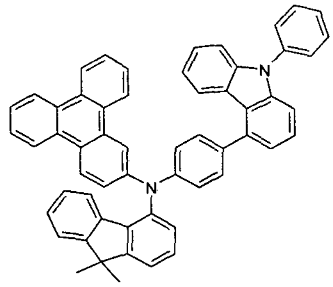

Formel 132

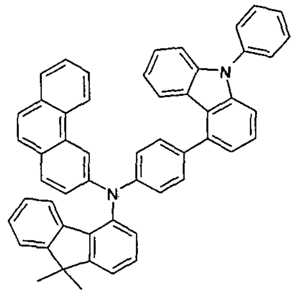

Formel 133

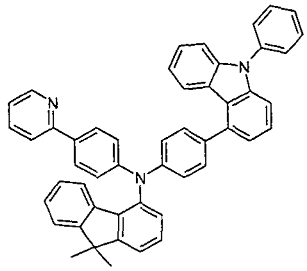

Formel 134

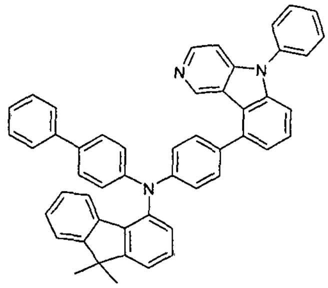

Formel 135

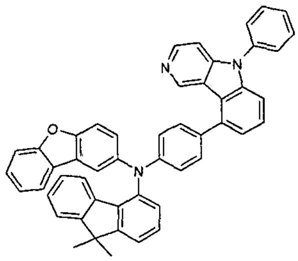

Formel 136

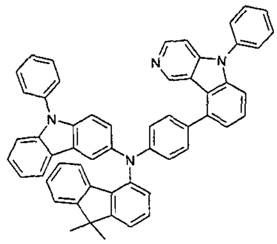

Formel 137

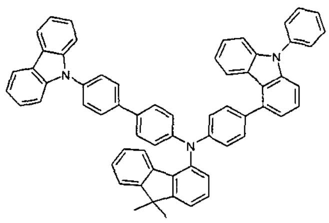

Formel 138

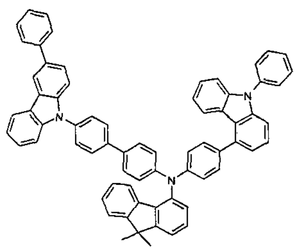

Formel 139

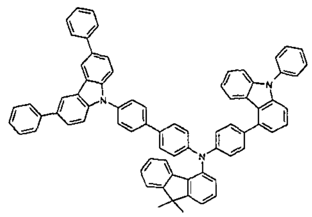

Formel 140

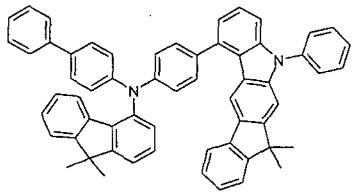

Formel 141

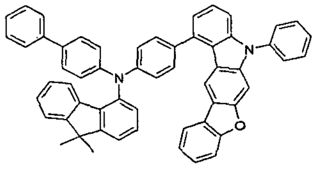

Formel 142

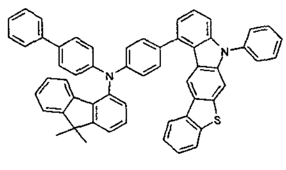

Formel 143

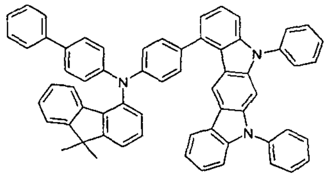

Formel 144

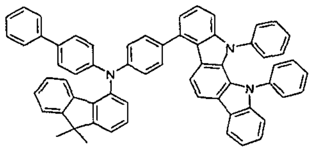

Formel 145

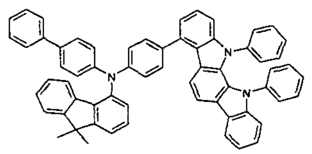

Formel 146

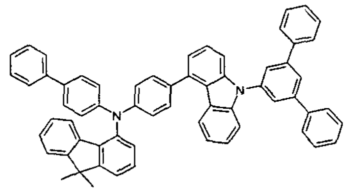

Formel 147

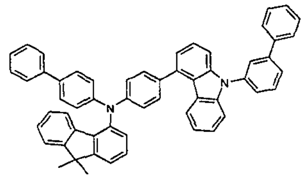

Formel 148

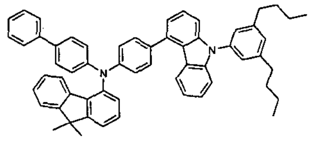

Formel 149

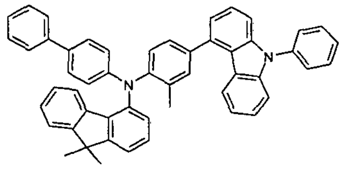

Formel 150

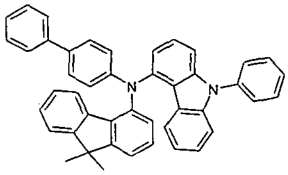

Formel 151

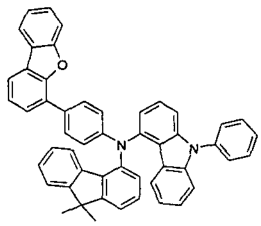

Formel 152

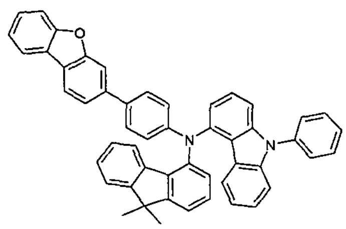

Formel 153

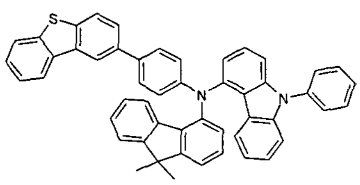

Formel 154

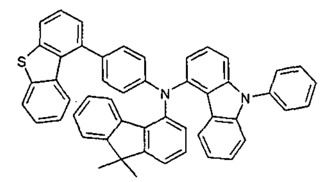

Formel 155

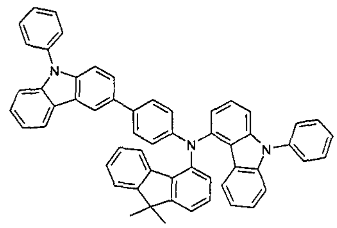

Formel 156

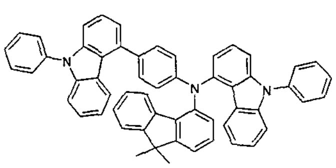

Formel 157

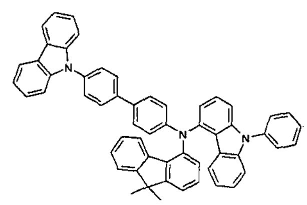

Formel 158

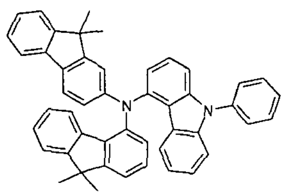

Formel 159

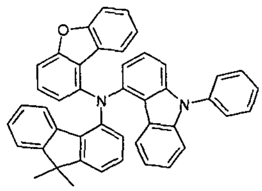
Formel 160

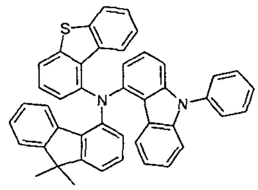
Formel 161

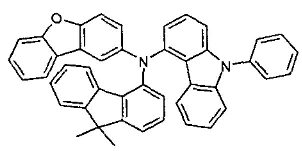
Formel 162

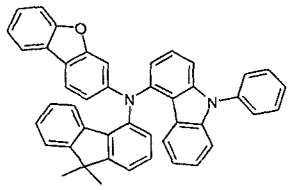
Formel 163

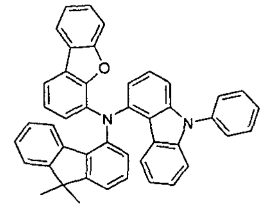
Formel 164

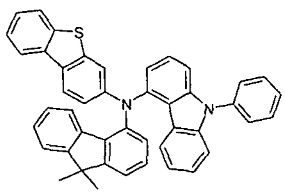
Formel 165

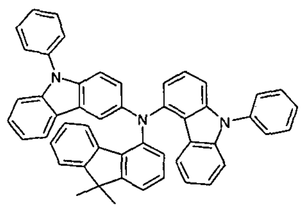
Formel 166

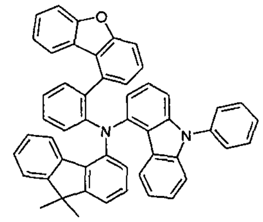
Formel 167

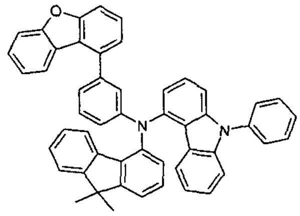
Formel 168

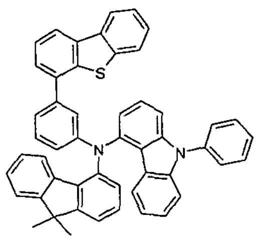
Formel 169

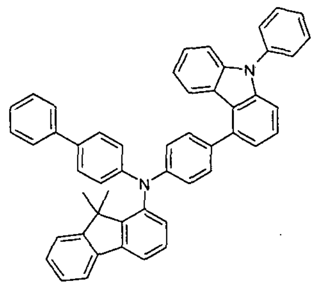
Formel 170

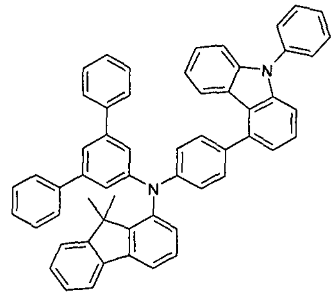
Formel 171

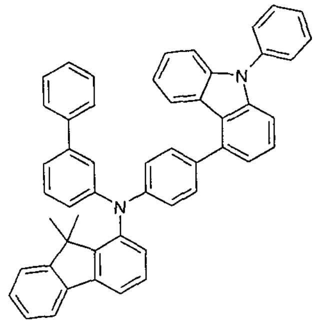
Formel 172

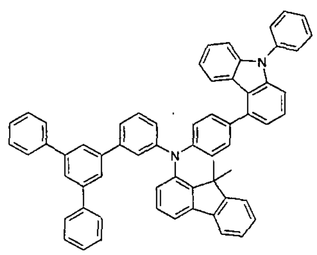
Formel 173

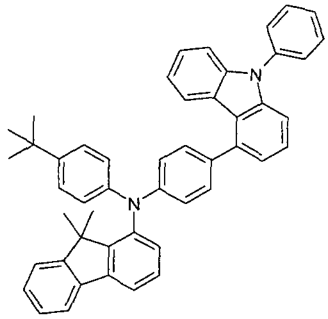
Formel 174

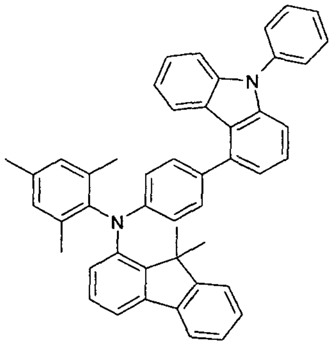

Formel 175

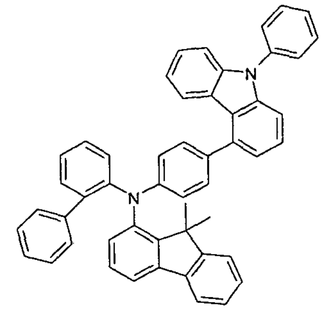

Formel 176

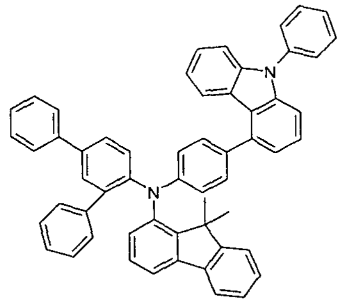

Formel 177

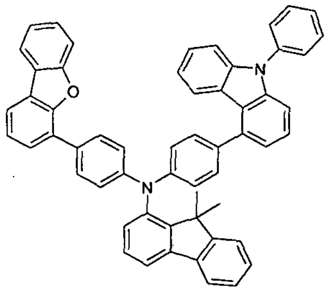

Formel 178

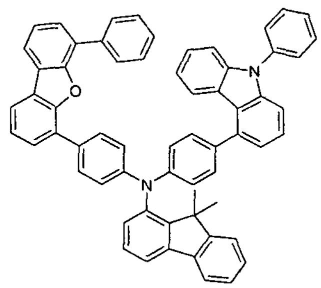

Formel 179

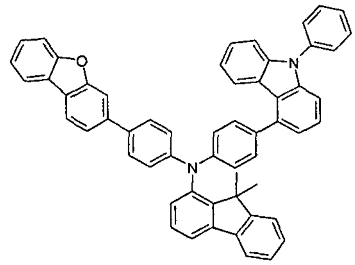

Formel 180

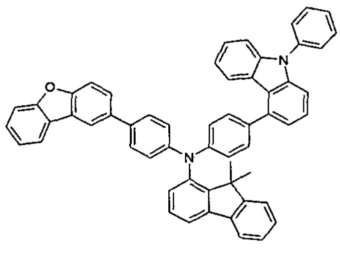

Formel 181

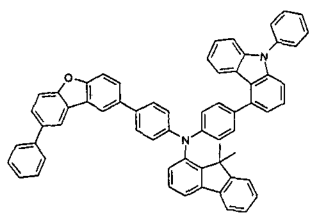

Formel 182

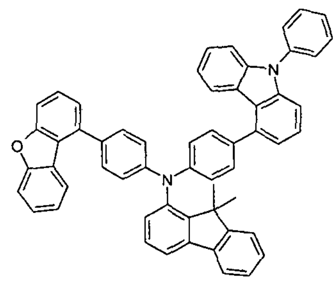

Formel 183

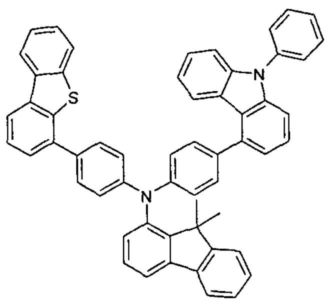

Formel 184

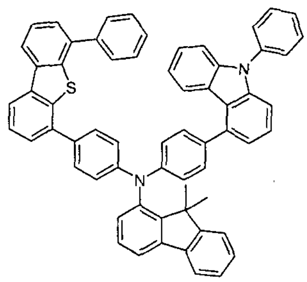

Formel 185

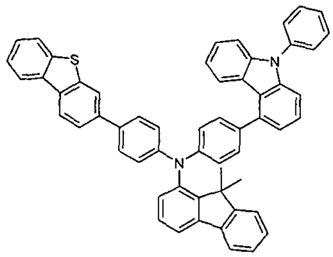

Formel 186

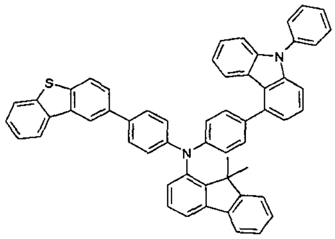

Formel 187

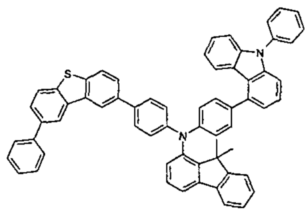

Formel 188

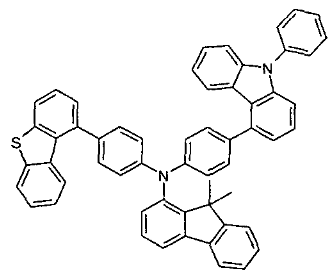

Formel 189

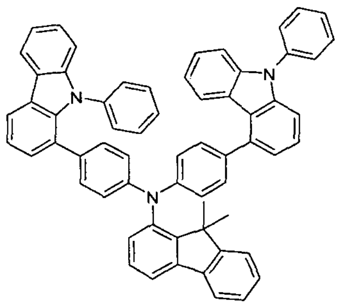

Formel 190

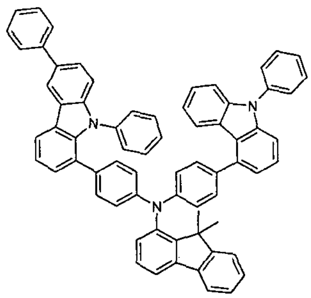

Formel 191

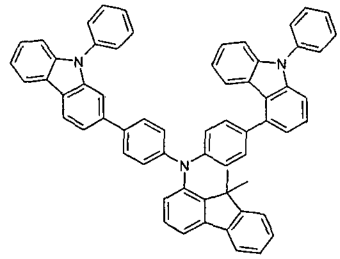

Formel 192

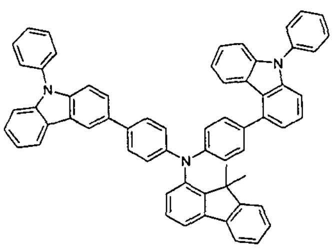

Formel 193

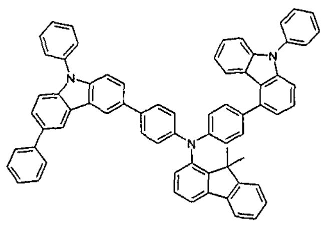

Formel 194

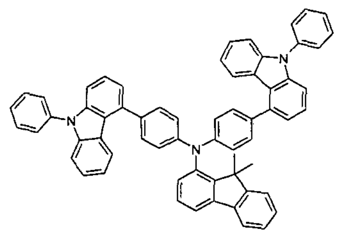

Formel 195

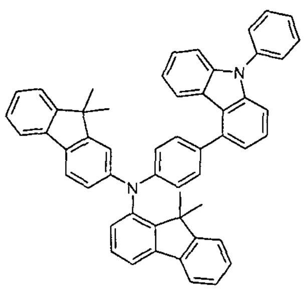

Formel 196

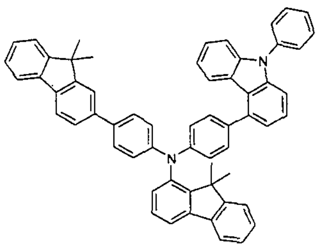

Formel 197

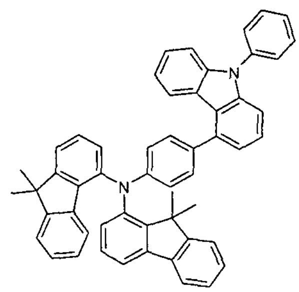

Formel 198

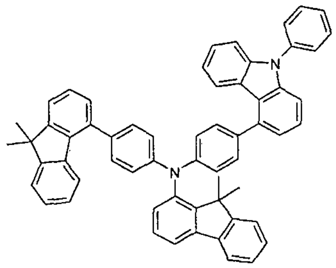

Formel 199

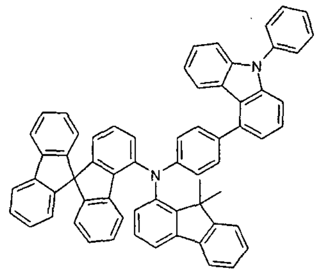

Formel 200

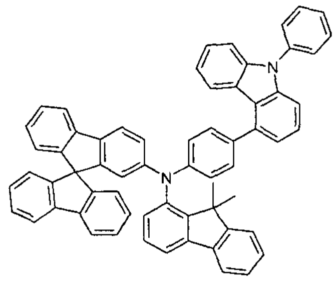

Formel 201

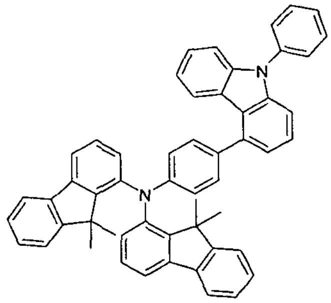

Formel 202

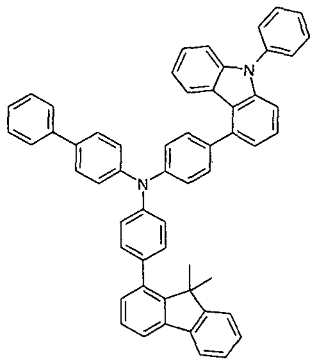

Formel 203

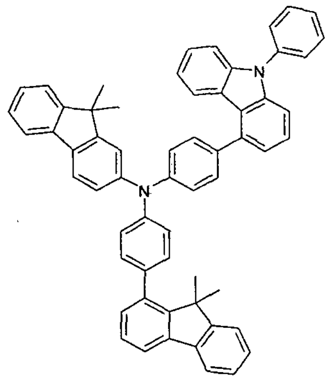

Formel 204

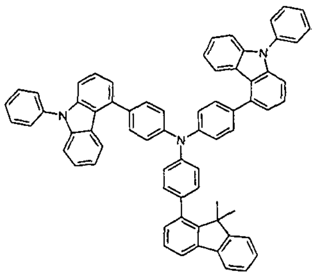

Formel 205

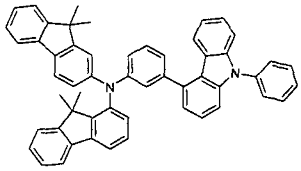

Formel 206

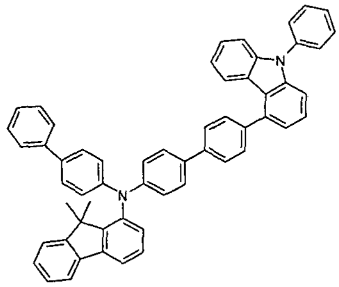

Formel 207

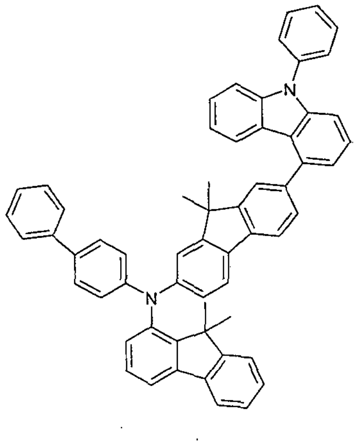

Formel 208

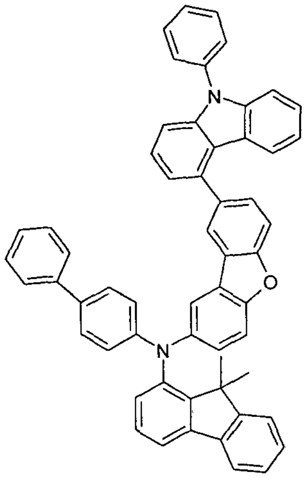

Formel 209

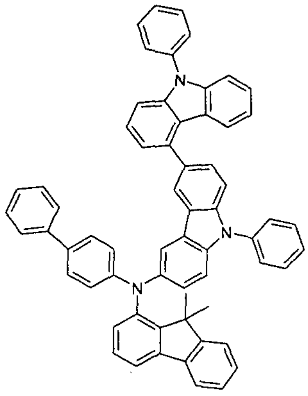

Formel 210

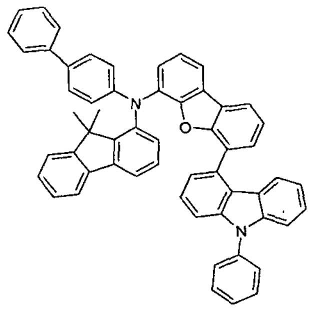

Formel 211

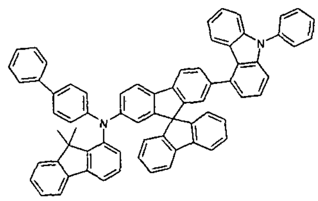

Formel 212

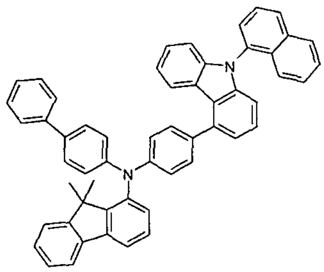

Formel 213

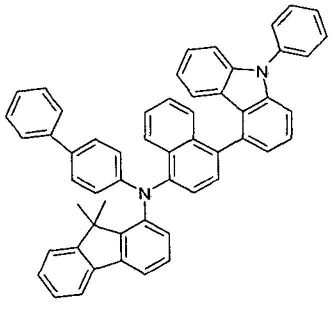

Formel 214

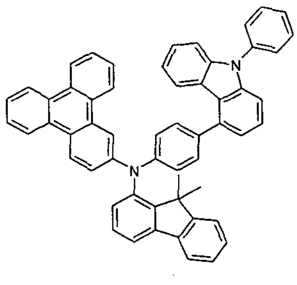

Formel 215

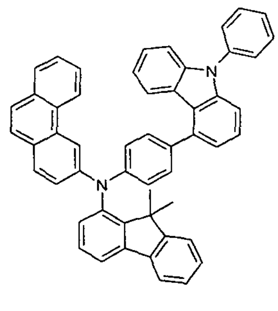

Formel 216

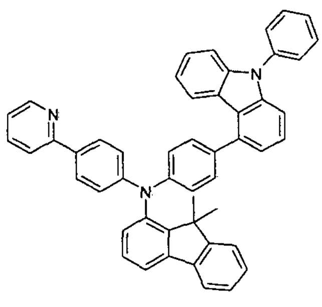

Formel 217

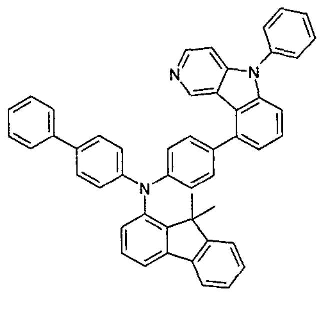

Formel 218

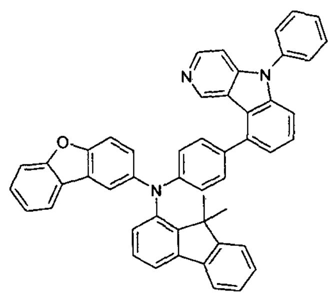

Formel 219

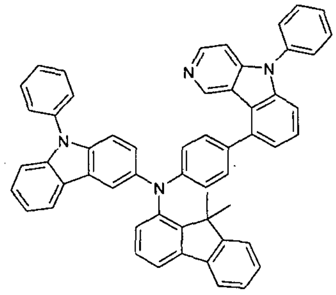

Formel 220

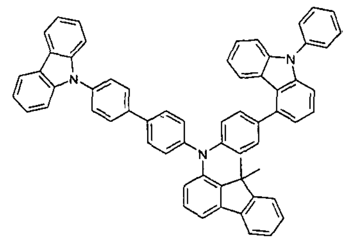

Formel 221

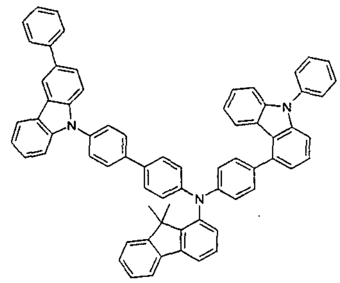

Formel 222

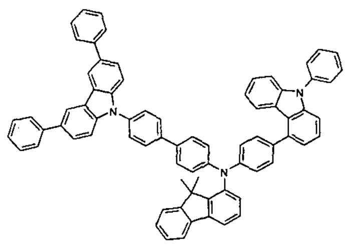

Formel 223

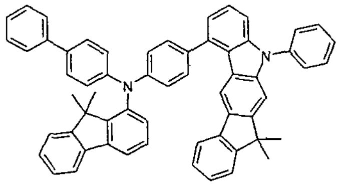

Formel 224

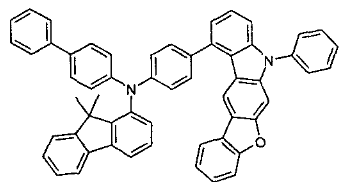

Formel 225

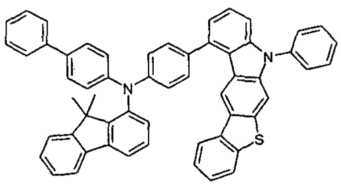

Formel 226

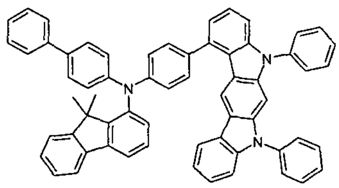

Formel 227

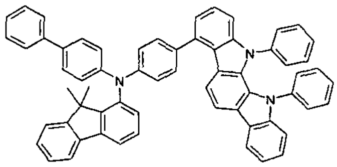

Formel 228

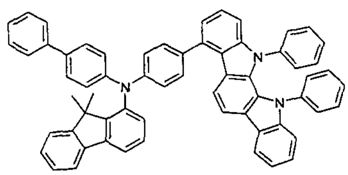

Formel 229

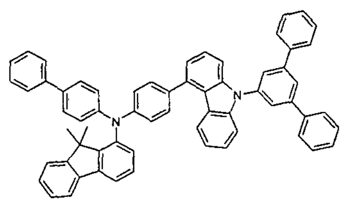

Formel 230

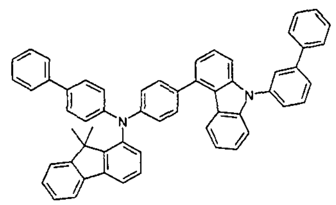

Formel 231

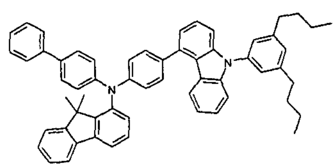

Formel 232

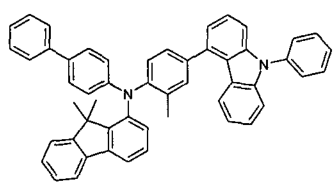

Formel 233

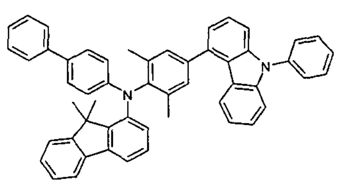

Formel 234

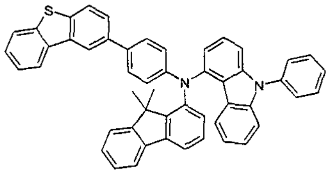

Formel 235

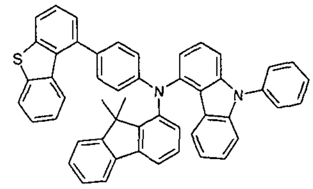

Formel 236

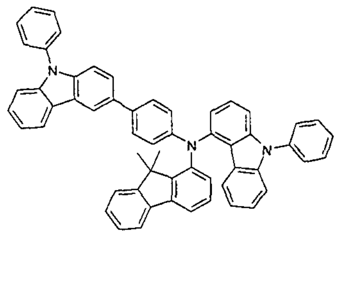

Formel 237

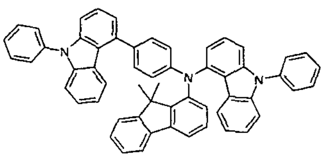

Formel 238

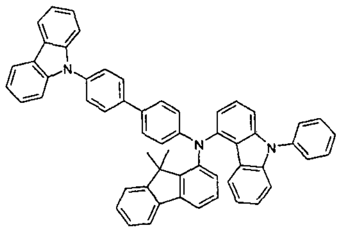

Formel 239

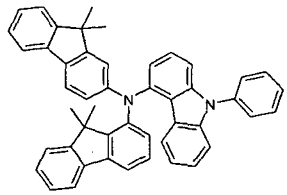

Formel 240

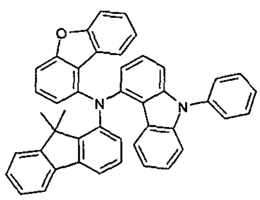

Formel 241

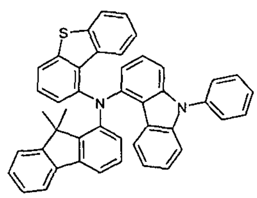

Formel 242

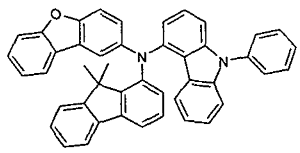

Formel 243

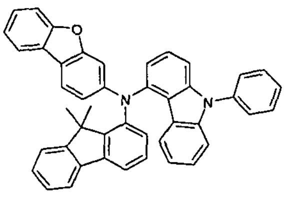

Formel 244

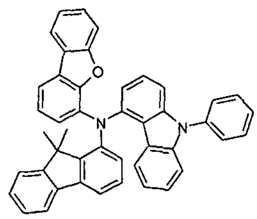

Formel 245

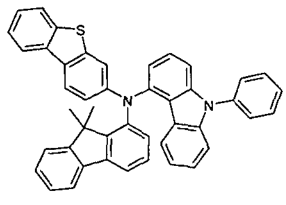

Formel 246

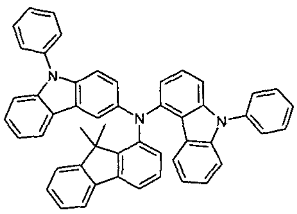

Formel 247

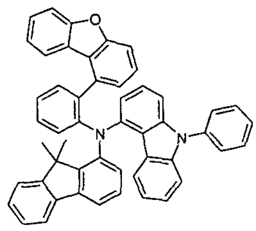

Formel 248

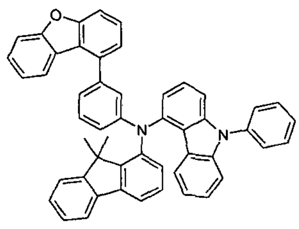

Formel 249

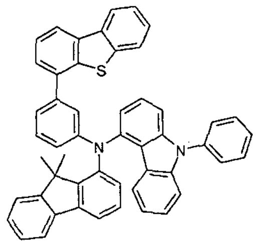

Formel 250

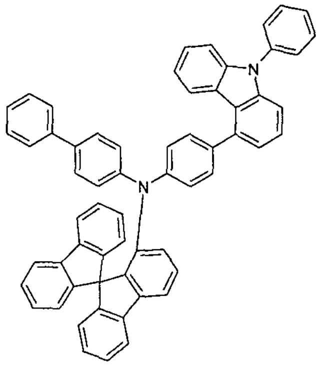

Formel 251

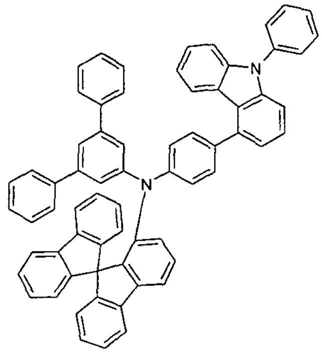

Formel 252

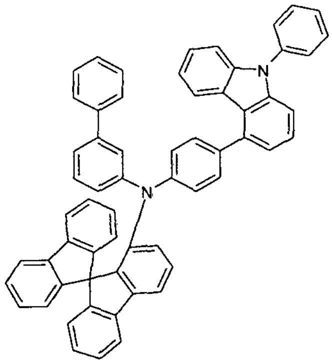

Formel 253

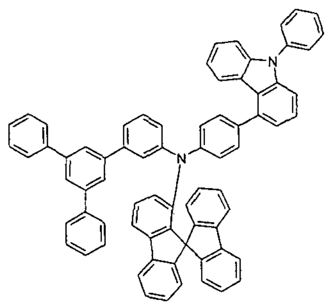

Formel 254

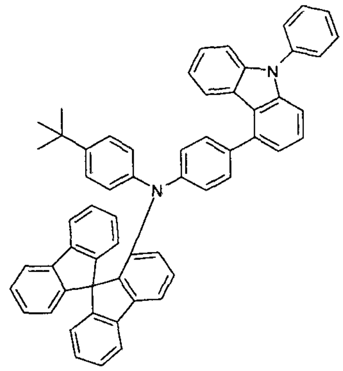

Formel 255

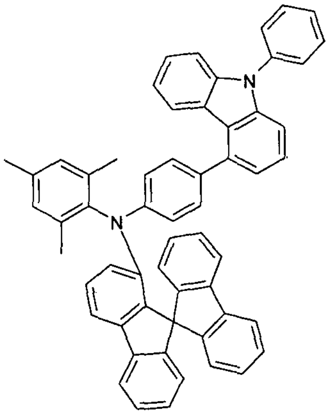

Formel 256

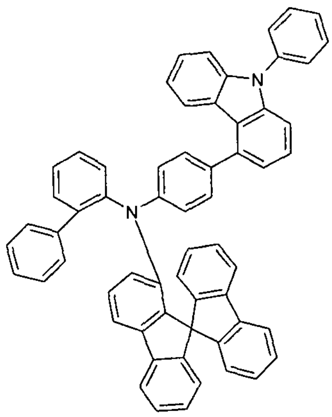

Formel 257

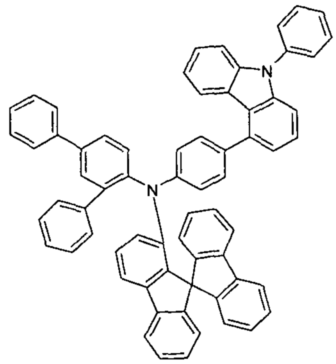

Formel 258

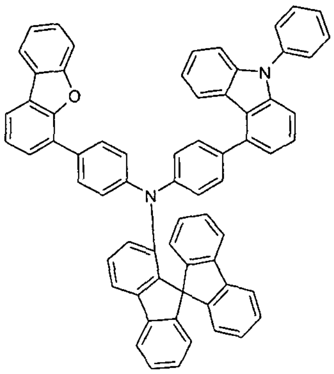
Formel 259

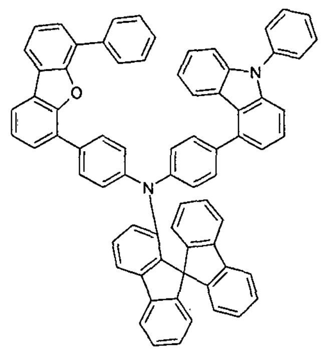
Formel 260

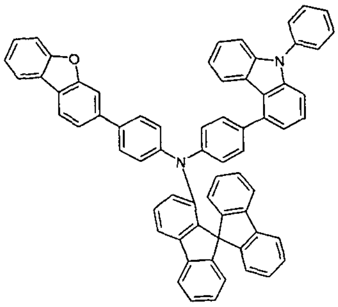
Formel 261

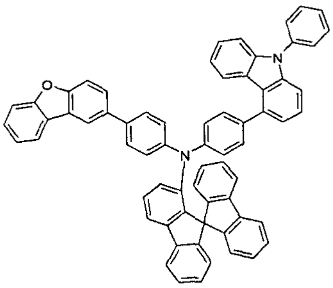
Formel 262

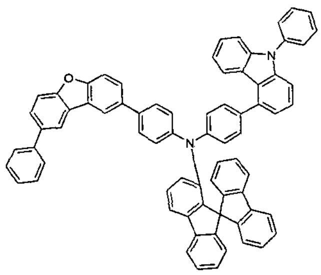
Formel 263

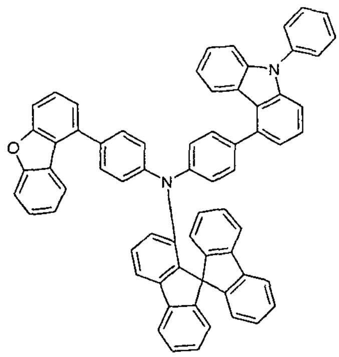
Formel 264

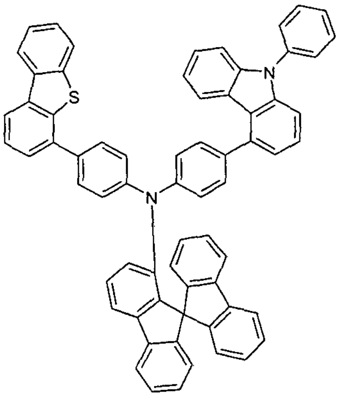
Formel 265

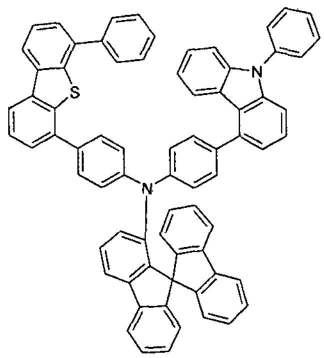
Formel 266

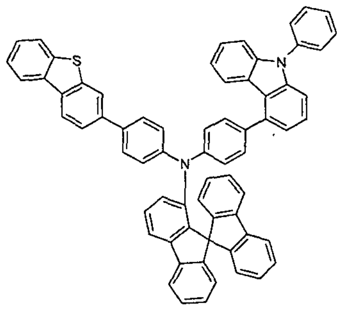
Formel 267

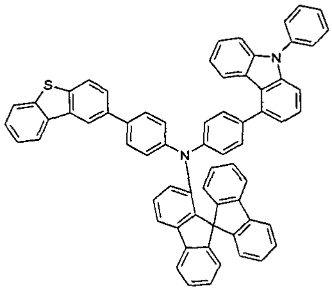

Formel 268

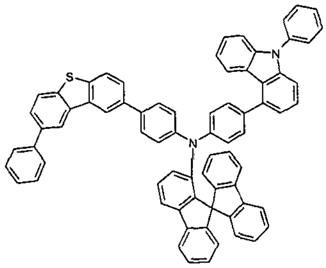

Formel 269

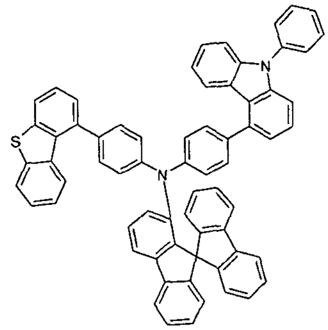

Formel 270

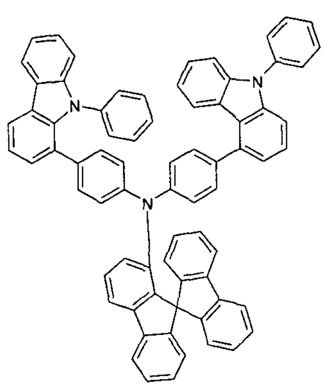

Formel 271

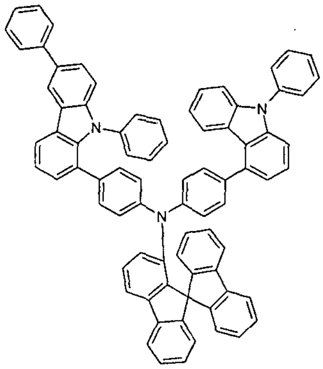

Formel 272

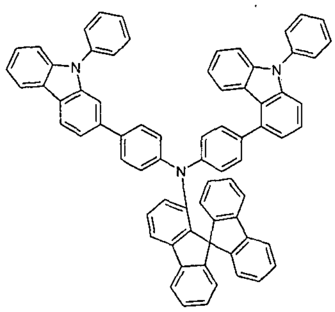

Formel 273

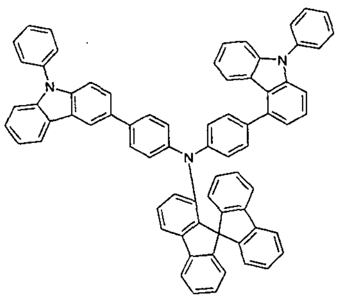

Formel 274

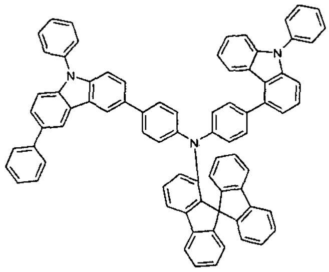

Formel 275

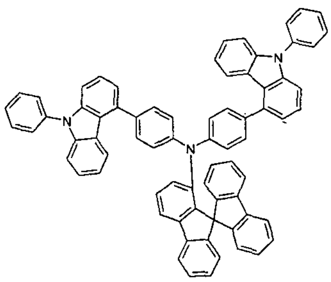

Formel 276

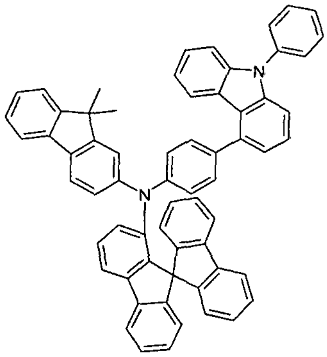
Formel 277

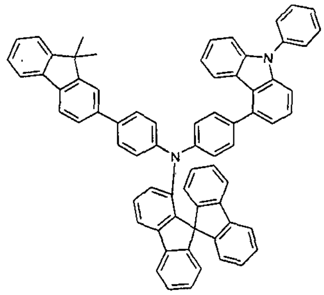
Formel 278

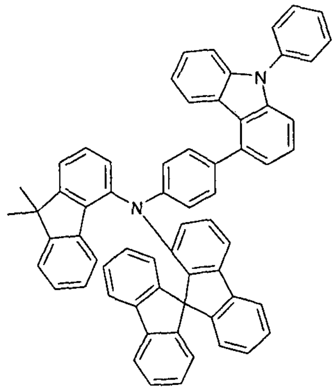
Formel 279

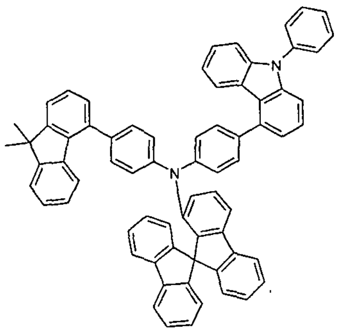
Formel 280

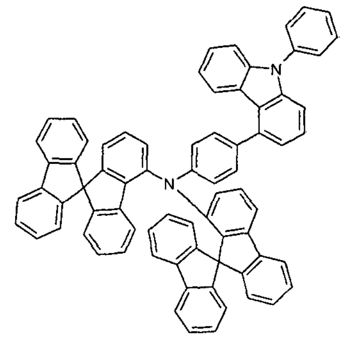
Formel 281

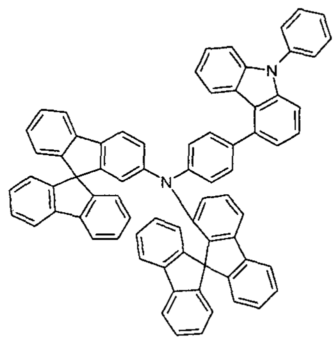
Formel 282

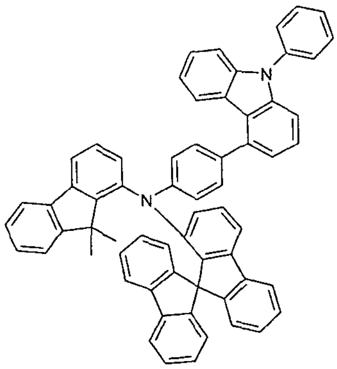
Formel 283

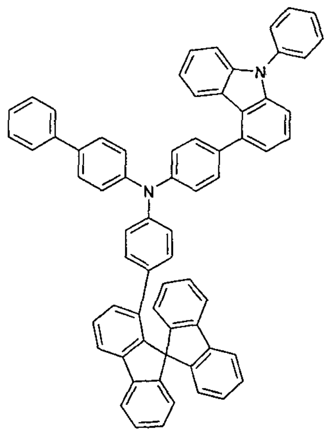
Formel 284

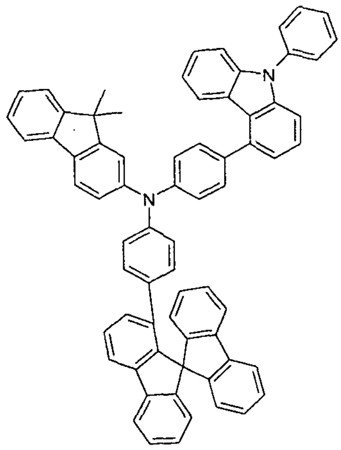
Formel 285

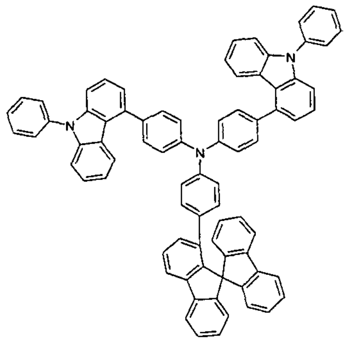

Formel 286

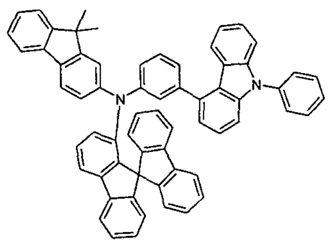

Formel 287

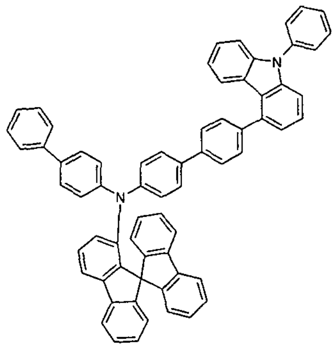

Formel 288

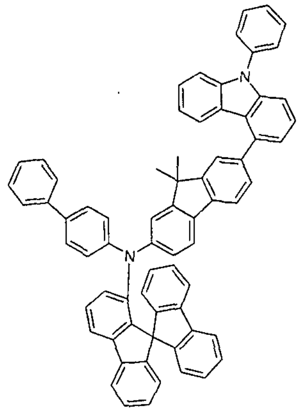

Formel 289

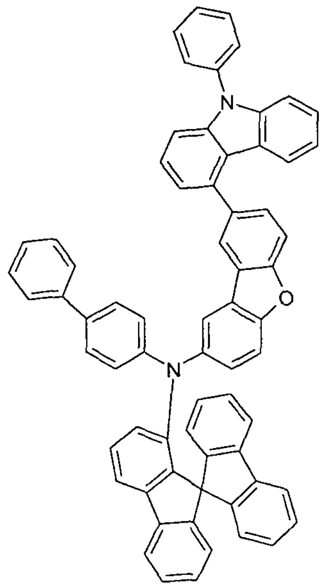

Formel 290

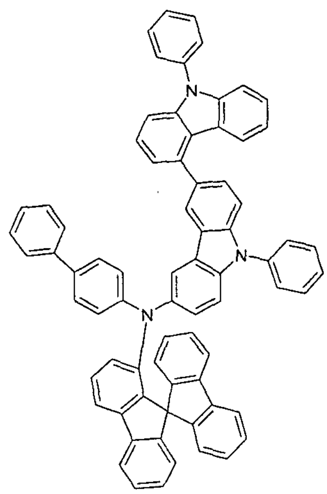

Formel 291

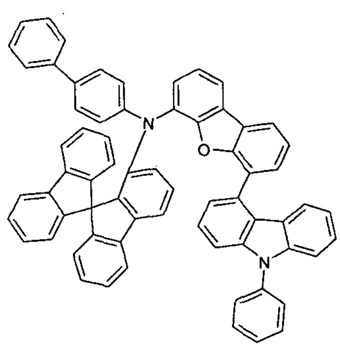

Formel 292

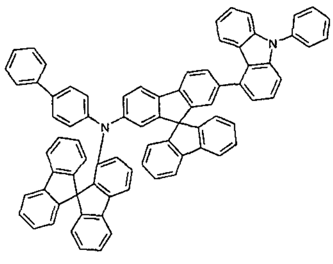

Formel 293

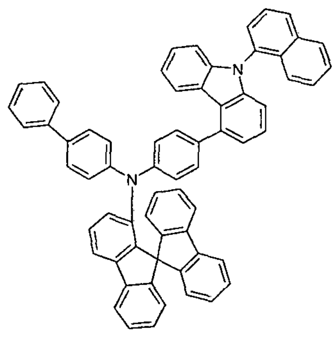

Formel 294

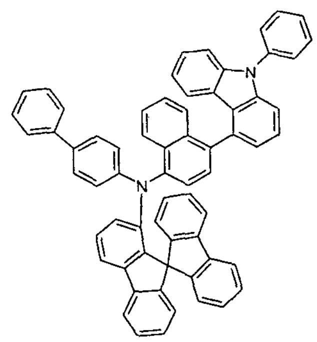

Formel 295

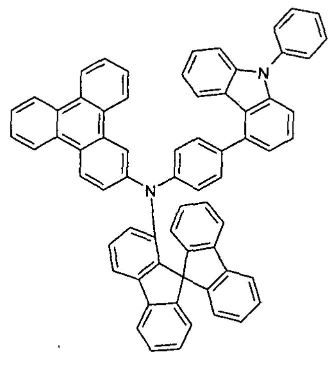

Formel 296

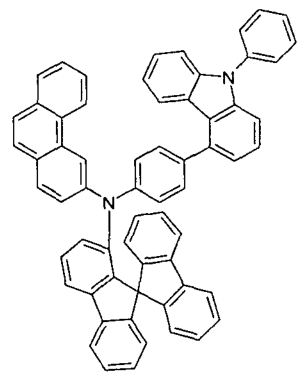

Formel 297

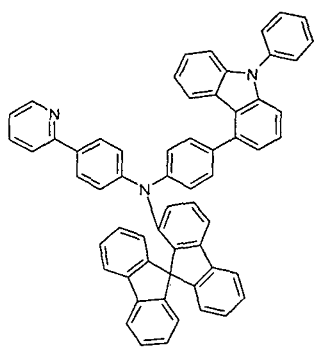

Formel 298

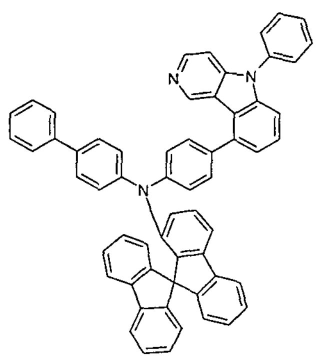

Formel 299

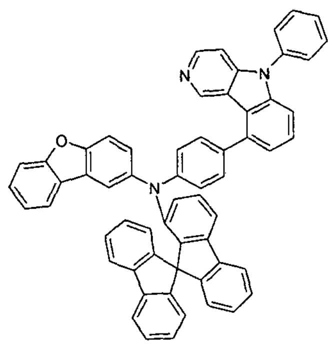

Formel 300

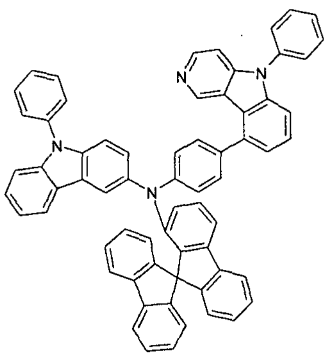

Formel 301

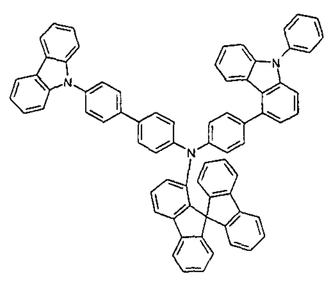

Formel 302

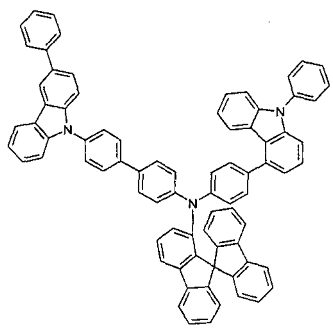

Formel 303

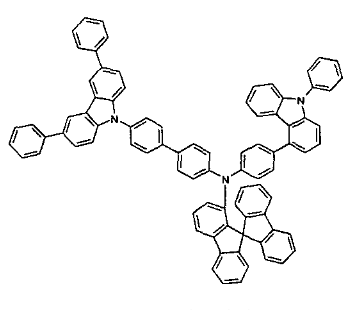

Formel 304

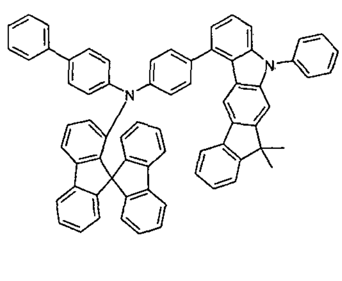

Formel 305

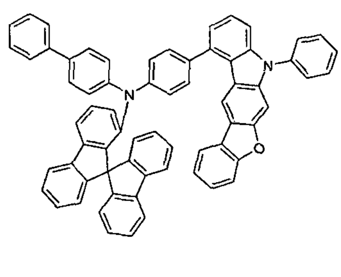

Formel 306

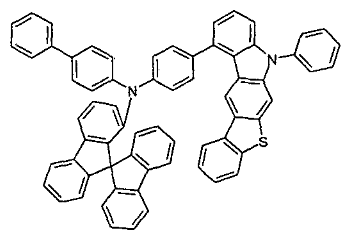

Formel 307

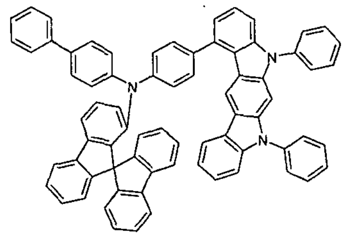

Formel 308

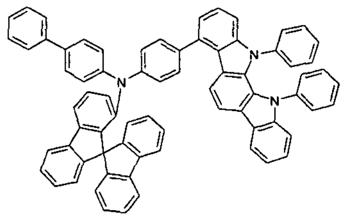

Formel 309

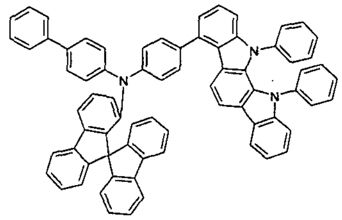

Formel 310

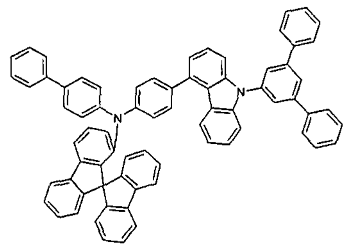

Formel 311

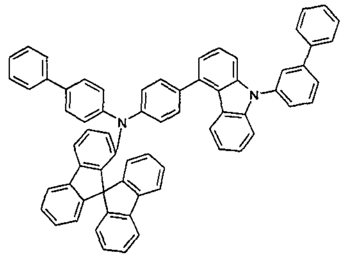

Formel 312

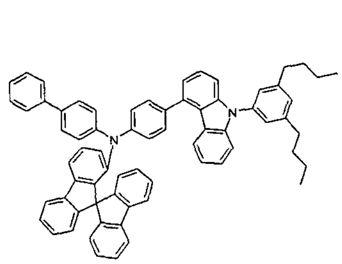

Formel 313

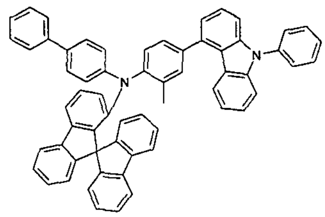

Formel 314

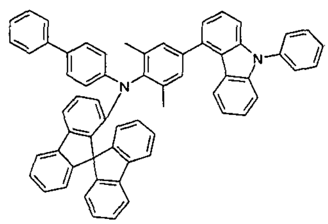

Formel 315

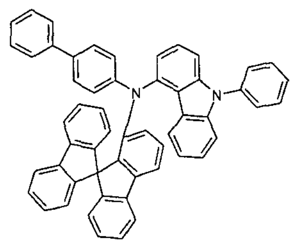

Formel 316

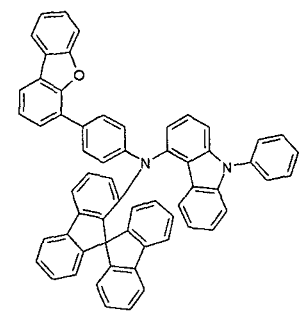

Formel 317

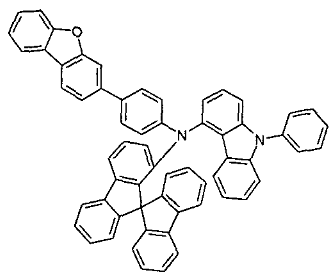

Formel 318

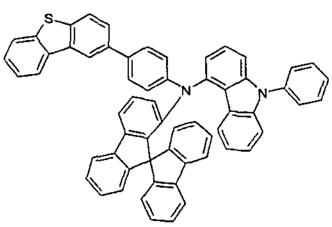

Formel 319

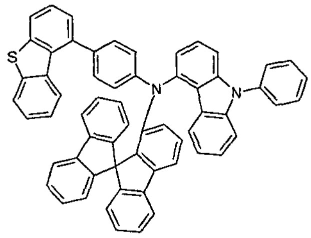

Formel 320

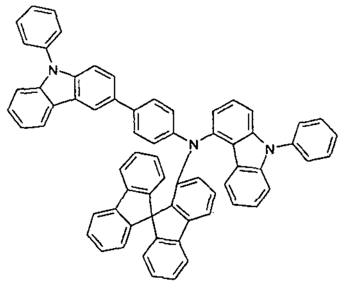

Formel 321

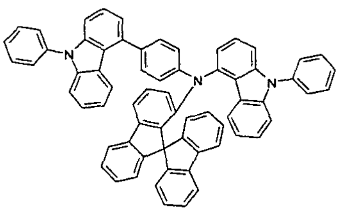

Formel 322

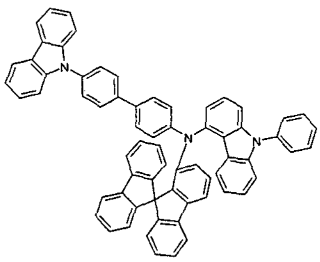

Formel 323

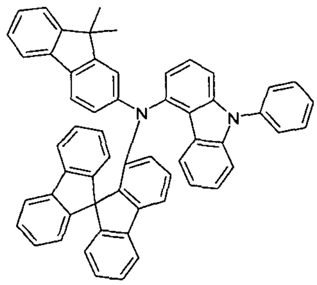

Formel 324

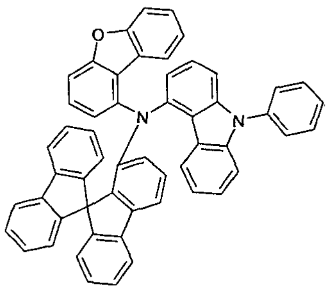

Formel 325

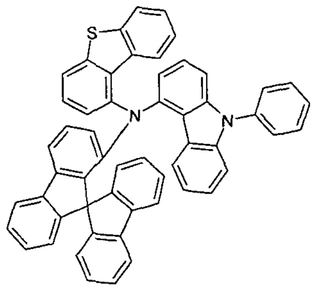

Formel 326

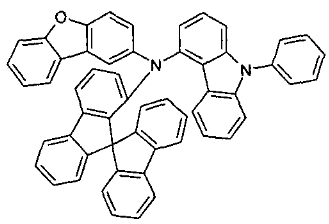

Formel 327

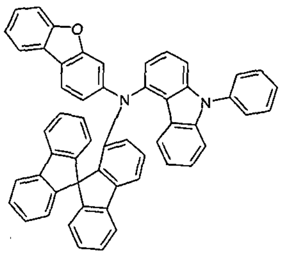

Formel 328

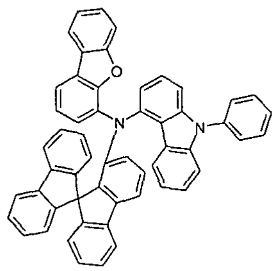

Formel 329

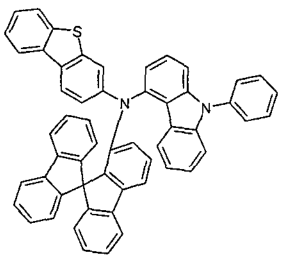

Formel 330

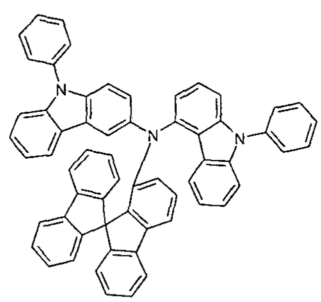

Formel 331

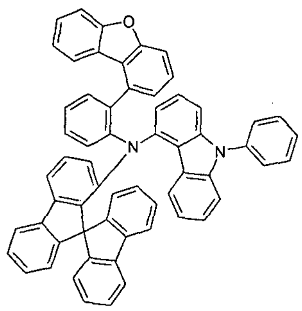

Formel 332

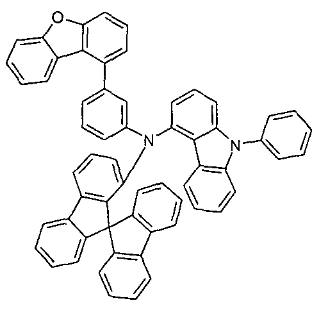

Formel 333

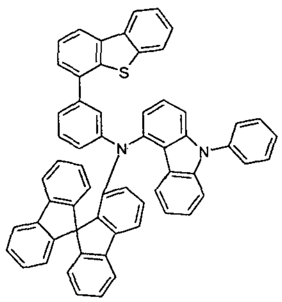

Formel 334

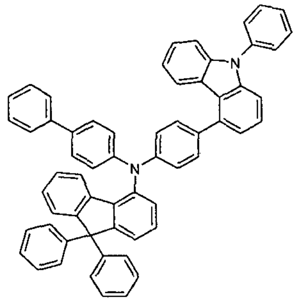

Formel 335

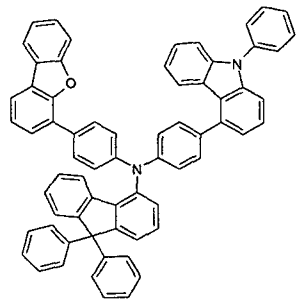

Formel 336

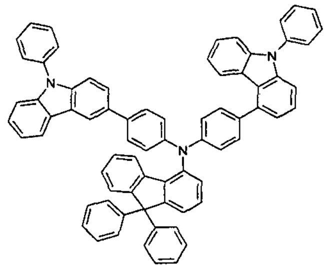

Formel 337

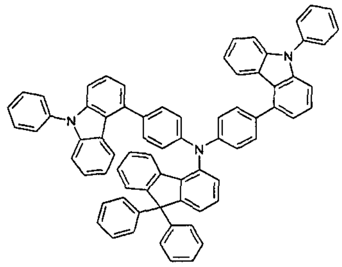

Formel 338

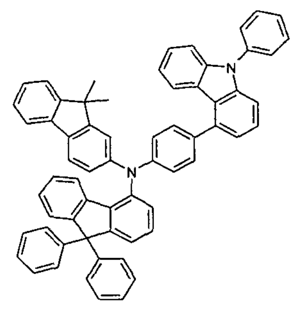

Formel 339

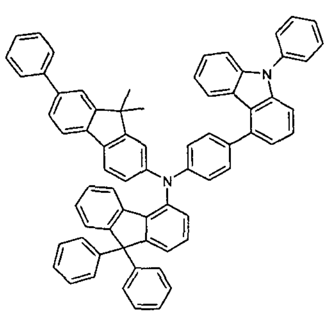

Formel 340

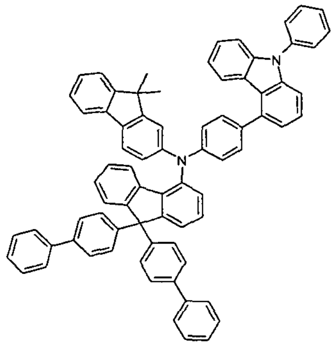

Formel 341

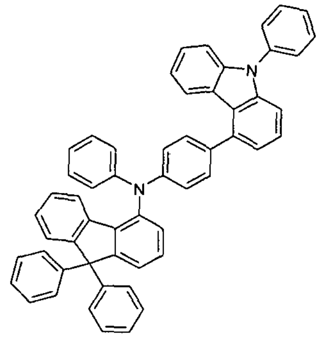

Formel 342

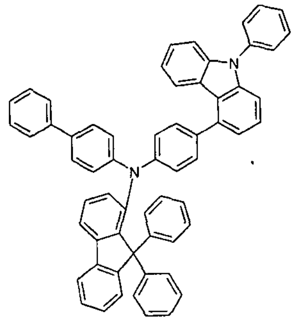
Formel 343

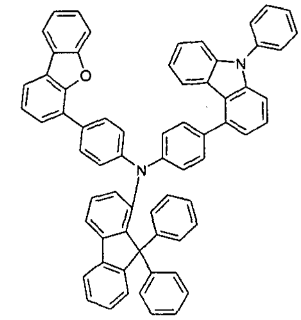
Formel 344

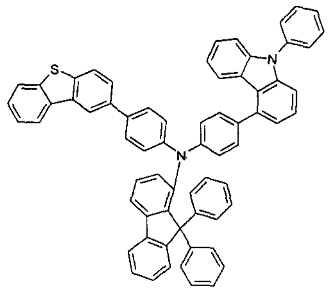
Formel 345

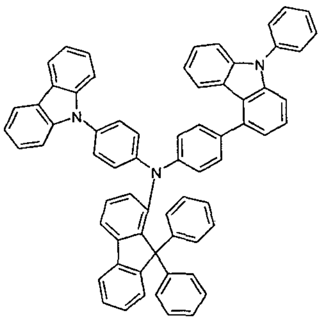
Formel 346

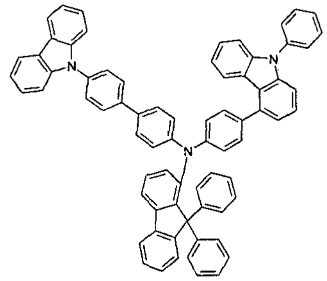
Formel 347

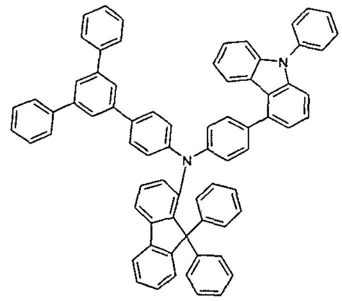
Formel 348

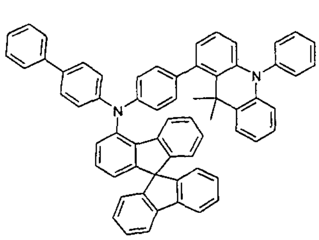
Formel 349

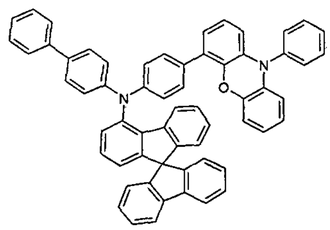
Formel 350

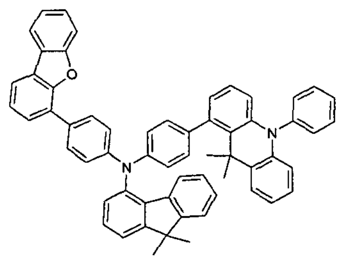
Formel 351

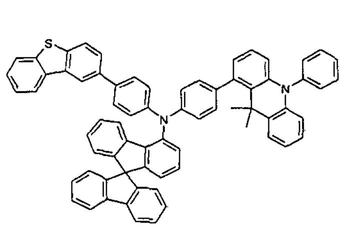
Formel 352

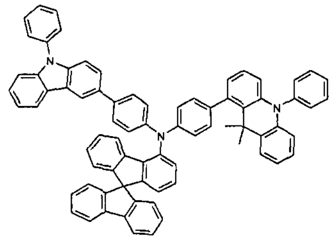
Formel 353

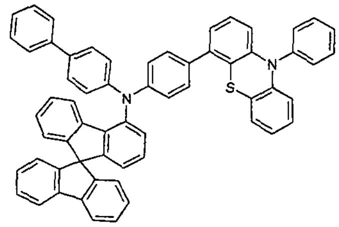
Formel 354

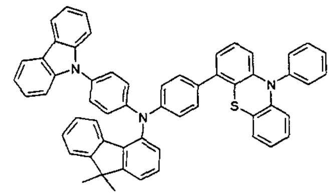

Formel 355

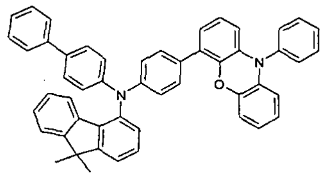

Formel 356

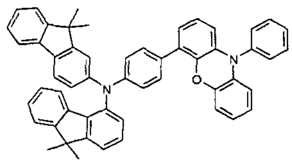

Formel 357

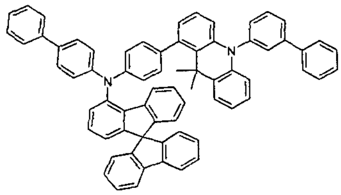

Formel 358

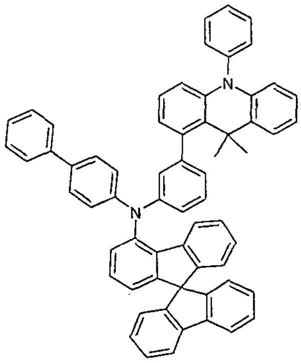

Formel 359

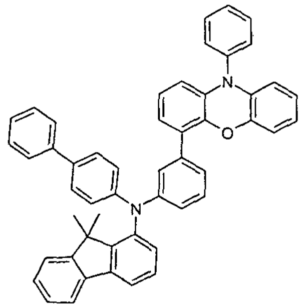

Formel 360

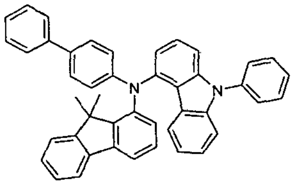

Formel 361

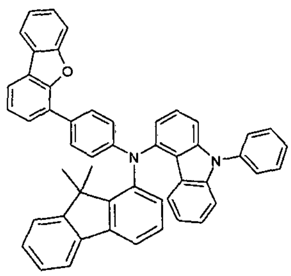

Formel 362

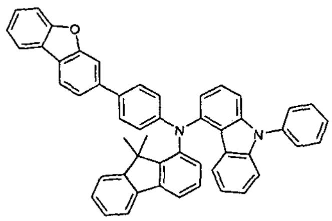

Formel 363

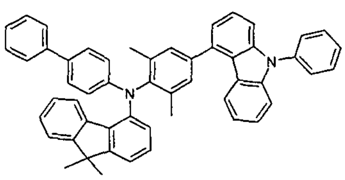

Formel 364

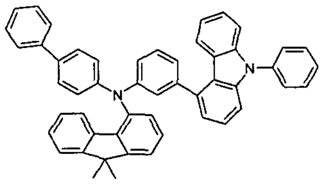

Formel 365

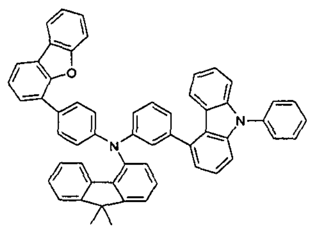

Formel 366

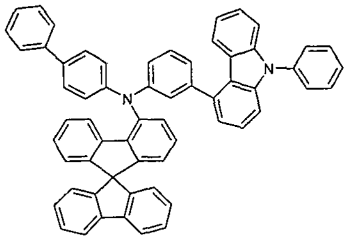

Formel 367

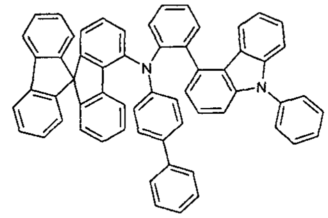

Formel 368

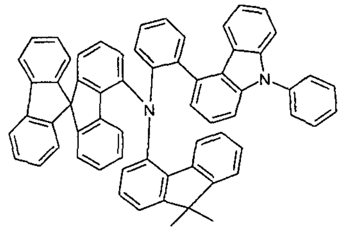

Formel 369

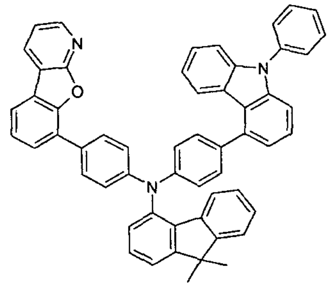

Formel 370

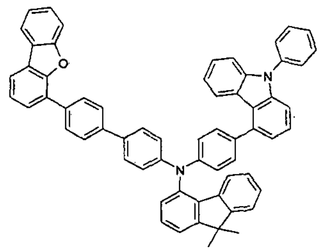

Formel 371

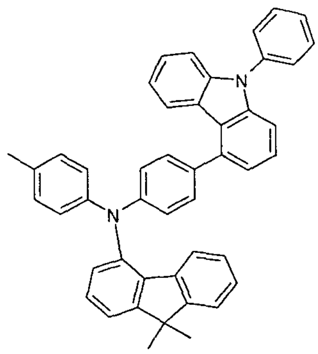

Formel 372

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (I), bei dem in einer Kupplungsreaktion eine Gruppe, umfassend mindestens einen Stickstoff-umfassenden heterocyclischen Rest, vorzugsweise ein Carbazol-Rest, mit einer Gruppe, umfassend mindestens einen Fluoren-Rest, verbunden wird. Hierbei kann eine Stickstoffumfassende heterocyclische Verbindung, vorzugsweise eine CarbazolVerbindung, die eine Aminogruppe, vorzugsweise eine sekundäre Aminogruppe umfasst, mit einer Gruppe, umfassend mindestens einen Fluoren-Rest, verbunden werden. Weiterhin kann eine Verbindung mit einer Aminogruppe, vorzugsweise einer sekundären Aminogruppe, umfassend mindestens eine Fluoren-Gruppe, mit einem Stickstoff-umfassenden heterocyclischen Rest, vorzugsweise einem Carbazol-Rest verbunden werden.

Die notwendigen Bedingungen hierfür sind dem Fachmann bekannt, wobei die ausführlichen Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität.

In allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemta, ohne dass hierdurch eine Beschränkung erfolgen soll. Die Teilschritte der einzelnen Schemata können hierbei beliebig kombiniert werden.

Ein bevorzugter Syntheseweg zur Herstellung der erfindungsgemäßen Verbindungen wird im Folgenden dargestellt. Der Syntheseweg umfasst zwei Kupplungsreaktionen: zunächst wird das Fluoren- bzw. Spirobifluorenderivat in einer ersten Buchwald-Kupplung mit einem Amin der Formel $Ar^3$-$NH_2$ (vgl. Formeln (I), (Ia), (Ib) oder (Ic) der erfindungsgemäßen Verbindungen) umgesetzt. Schließlich erfolgt eine zweite Buchwald-Kupplung, um den Molekülteil enthaltend die heterocyclische, stickstoffumfassende Gruppe, vorzugsweise die Carbazolgruppe einzuführen.

Der Syntheseweg wird im Folgenden beispielhaft anhand einer Verbindung der Formel (I) dargestellt (Schema 1). Es soll jedoch betont werden, dass auf diesem Syntheseweg gleichermaßen auch erfindungsgemäße Verbindungen der Formel (Ia), (Ib) oder (Ic) hergestellt werden können. Analog zu den dargestellten Fluorenyl-Ausgangsverbindungen können auch Spirobifluorenyl-Verbindungen eingesetzt werden, so dass erfindungsgemäße Verbindungen enthaltend Fluoren-Einheiten erhalten werden.

**Schema 1:**

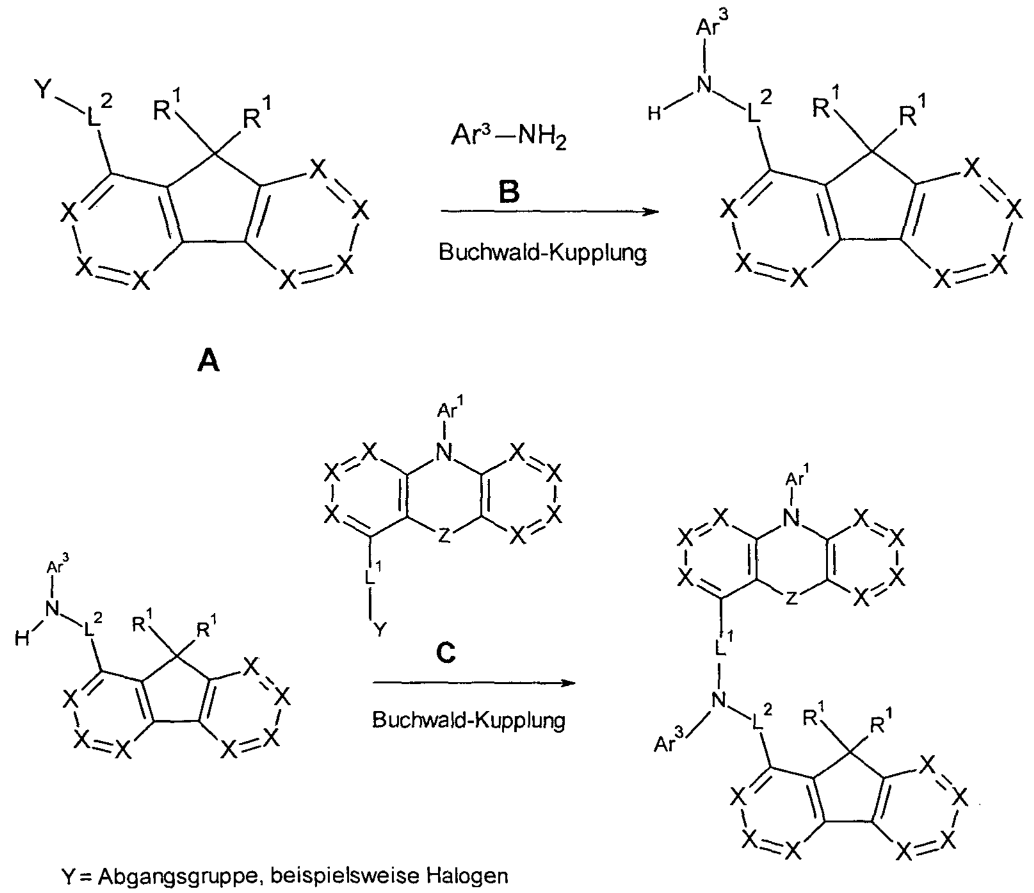


A

Y = Abgangsgruppe, beispielsweise Halogen

Synthesewege für die Ausgangsverbindungen **A, B** und **C**, welche in der Synthese der erfindungsgemäßen Verbindungen eingesetzt werden (vgl. Schema 1), sind dem Fachmann bekannt. Weiterhin werden in den Ausführungsbeispielen einige explizite Syntheseverfahren im Detail dargestellt.

Gegenstand der vorliegenden Erfindung ist somit ein Verfahren zur Herstellung einer Verbindung gemäß Formel (I), (Ia), (Ib) oder (Ic), dadurch gekennzeichnet, dass ein Fluorenyl- oder Spirobifluorenyl-Derivat in einer ersten Kupplungsreaktion mit einer Arylaminoverbindung umgesetzt wird, und das erhaltene Produkt in einer zweiten Kupplungsreaktion mit einer aromatischen, heterocyclischen Stickstoffverbindung, vorzugsweise einer Carbazolverbindung umgesetzt wird.

Die Kupplungsreaktionen stellen dabei bevorzugt Buchwald-Kupplungen dar.

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (I) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) oder erfindungsgemäße Verbindungen, wobei ein oder mehrere Bindungen der erfindungsgemäßen Verbindungen oder der Strukturen der Formel (I) zum Polymer, Oligomer oder Dendrimer vorhanden sind. Je nach Verknüpfung der Strukturen der Formel (I) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026),Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Ferner können die vorliegenden Verbindungen ein relativ geringes Molekulargewicht aufweisen. Ein weiterer Gegenstand der vorliegenden Erfindung ist demgemäß eine Verbindung umfassend ein oder mehrere Strukturen der Formel (I), die ein Molekulargewicht von vorzugsweise kleiner oder gleich 10000 g/mol, bevorzugt kleiner oder gleich 5000 g/mol, besonders bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1000 g/mol aufweist.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (I) bevorzugt, die eine Glasübergangstemperatur von mindestens 70°C, besonders bevorzugt von mindestens 110°C, ganz besonders bevorzugt von mindestens 120°C und insbesondere bevorzugt von mindestens 150°C aufweisen, bestimmt nach DIN 51005. Weiterhin ist festzuhalten, dass die erfindungsgemäßen Verbindungen, umfassend mindestens eine Struktur der Formel (I) bereits eine gesteigerte Glasübergangstemperatur besitzen.

Nochmals ein weiterer Gegenstand der vorliegenden Erfindung ist eine Formulierung, enthaltend eine erfindungsgemäße Verbindung bzw. ein erfindungsgemäßes Oligomer, Polymer oder Dendrimer und mindestens eine weitere Verbindung. Die weitere Verbindung kann vorzugsweise ein Lösemittel sein. Die weitere Verbindung kann aber auch eine weitere organische oder anorganische Verbindung sein, die ebenfalls in der elektronischen Vorrichtung eingesetzt

wird, beispielsweise ein Matrixmaterial. Diese weitere Verbindung kann auch polymer sein.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Nochmals ein weiterer Gegenstand der vorliegenden Erfindung ist eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, n-Dotanden, Wide-Band-Gap-Materialien, Elektronenblockiermaterialien und Lochblockiermaterialien.

Die vorliegenden Erfindung betrifft daher auch eine Zusammensetzung enthaltend wenigstens eine Verbindung umfassend Strukuren gemäß Formel (I) sowie wenigstens ein weiteres Matrixmaterial. Gemäß einem besonderen Aspekt der vorliegenden Erfindung weist das weitere Matrixmaterial elektronentransportierende Eigenschaften auf.

Ein weiterer Gegenstand der vorliegenden stellt eine Zusammensetzung dar, enthaltend wenigstens eine Verbindung, umfassend mindestens eine Struktur gemäß Formel (I) sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge O/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$\text{HOMO(eV)} = ((\text{HEh}*27.212)-0.9899)/1.1206$$

$$\text{LUMO(eV)} = ((\text{LEh}*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formel (I) sowie wenigstens einen phosphoreszierende Emitter, wobei unter dem Begriff phosphoreszierende Emitter auch phosphoreszierende Dotanden verstanden werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

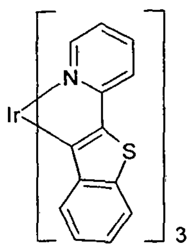
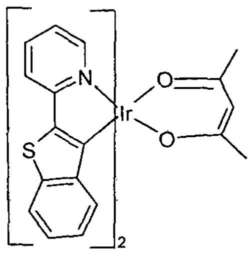
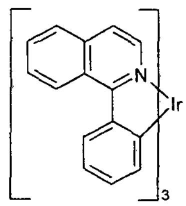

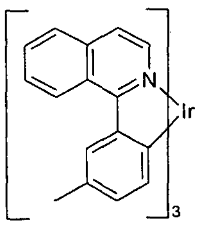
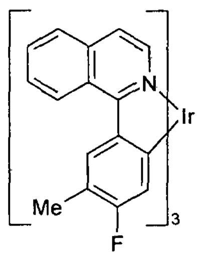
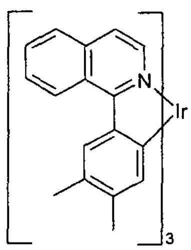

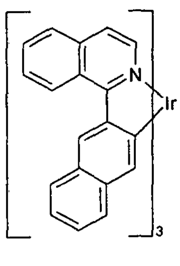
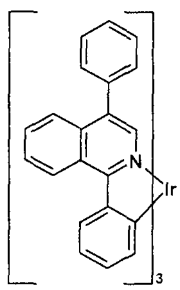
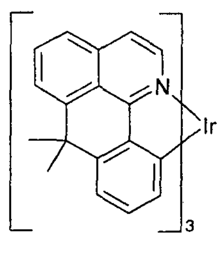

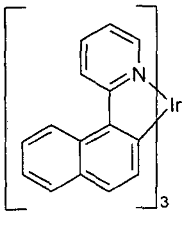 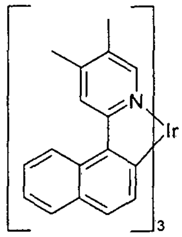 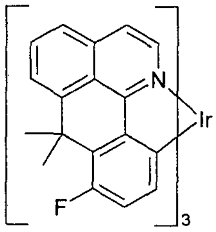

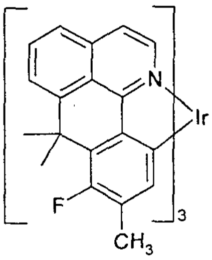 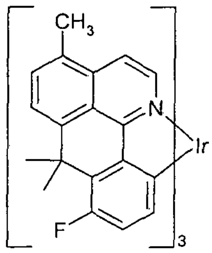 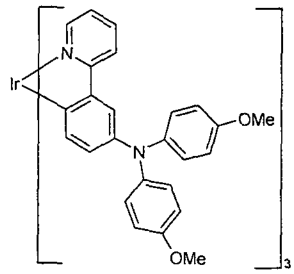

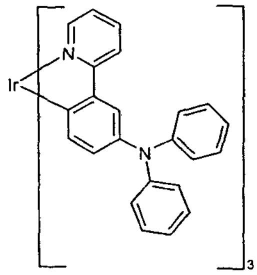 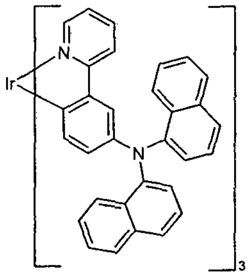 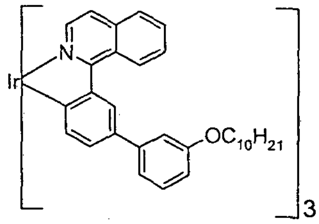

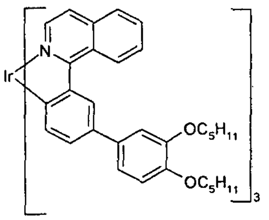 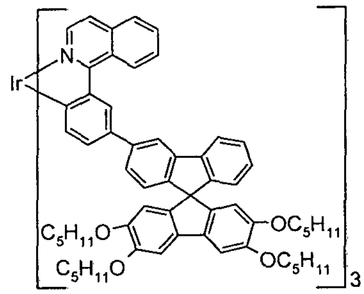 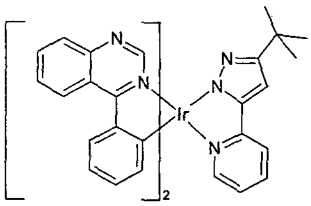

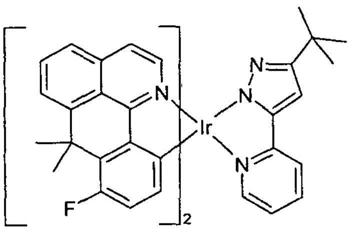 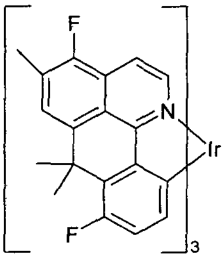 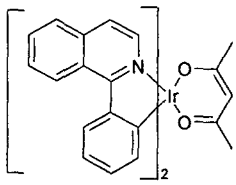

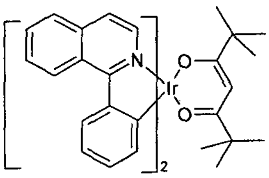 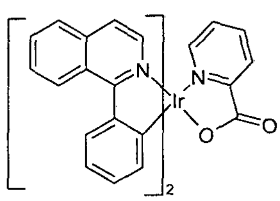 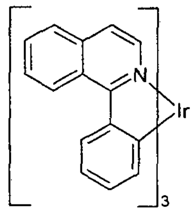

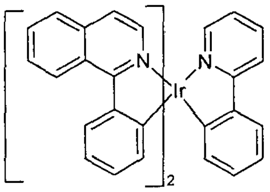
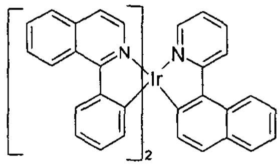
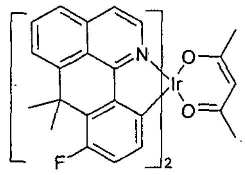

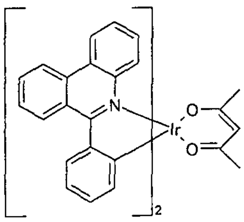
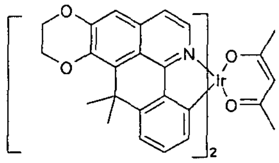
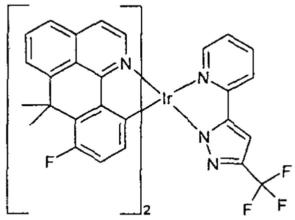

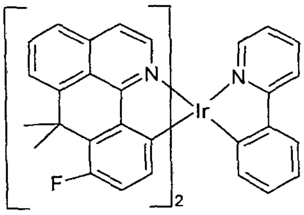
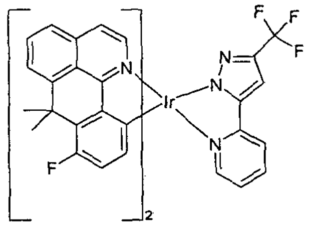
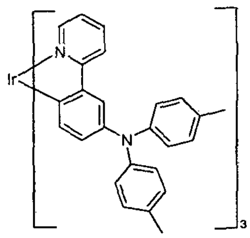

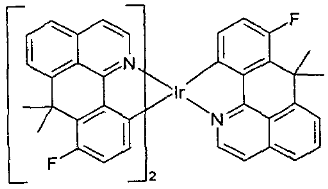
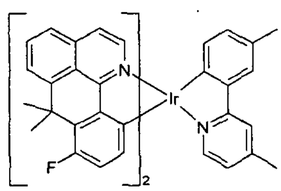
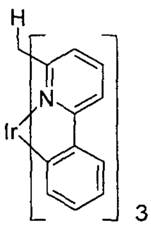

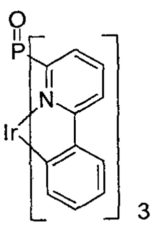
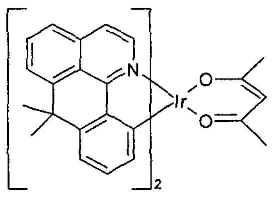
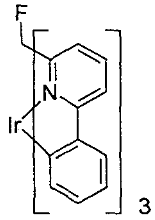

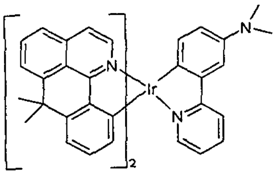
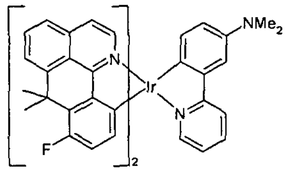
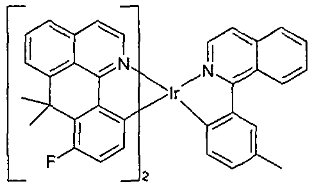

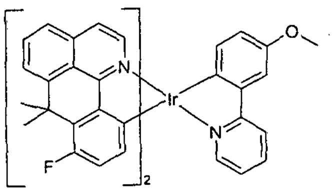

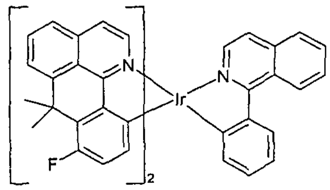

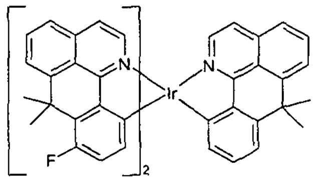

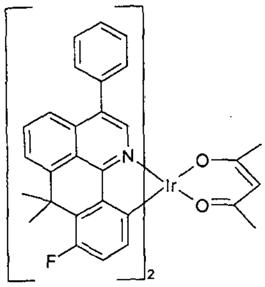

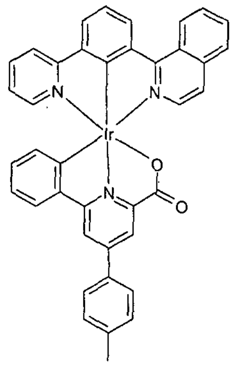

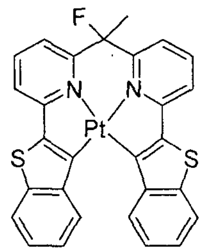

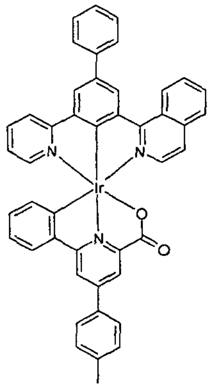

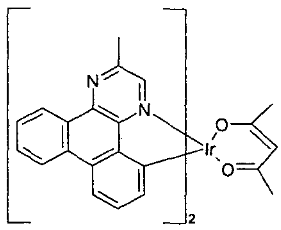

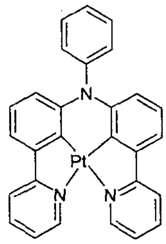

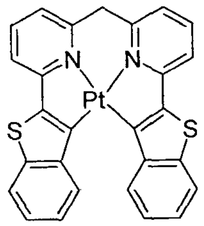

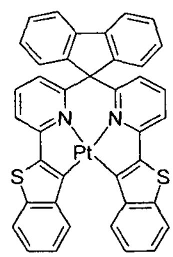

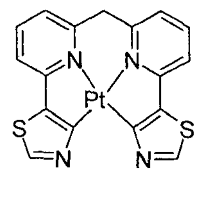

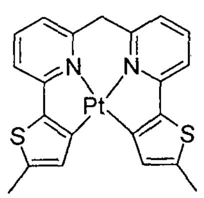

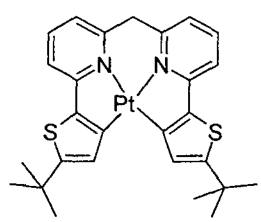

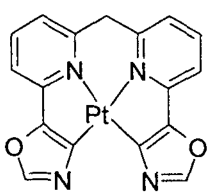

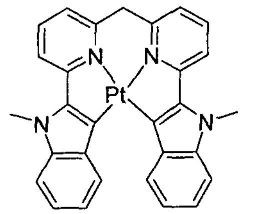

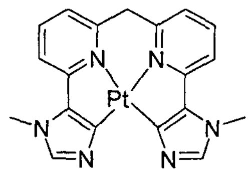

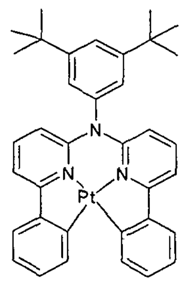

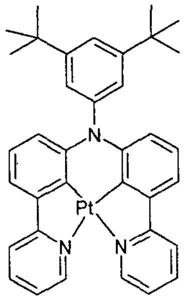

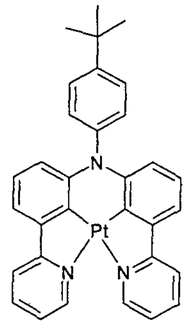

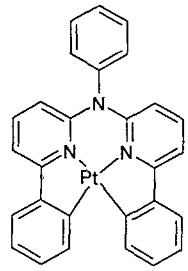

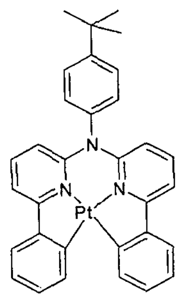

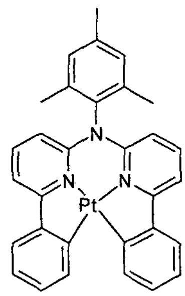

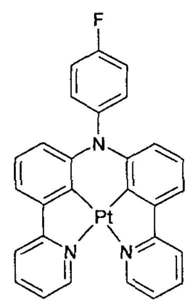

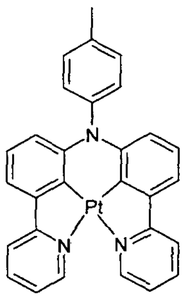

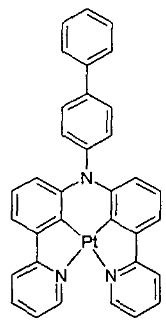

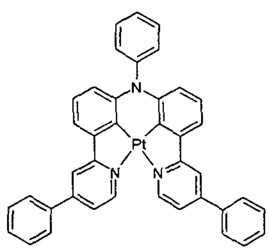

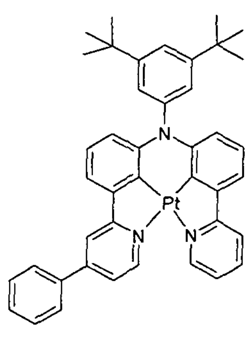

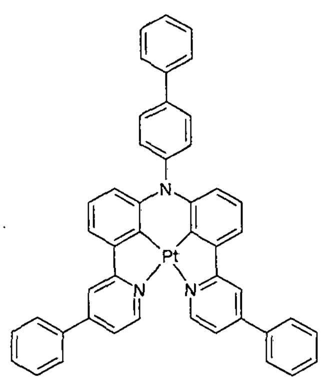

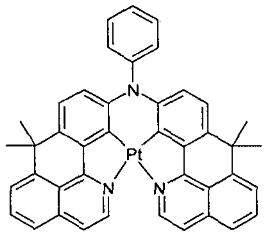

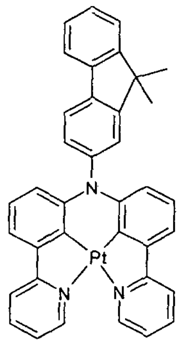
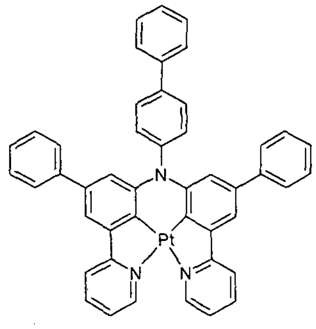
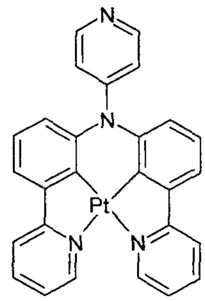

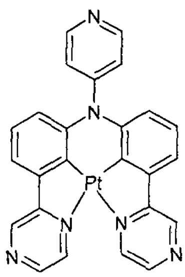
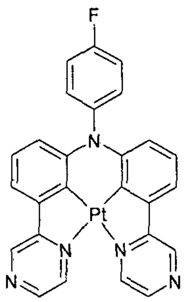
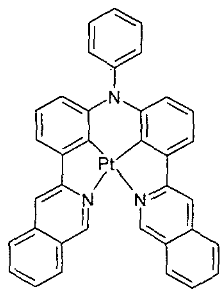

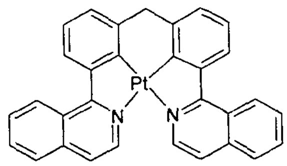
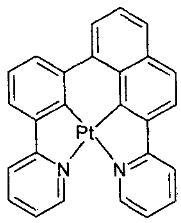
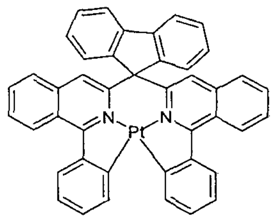

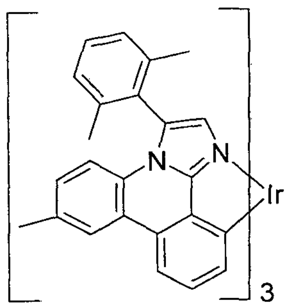
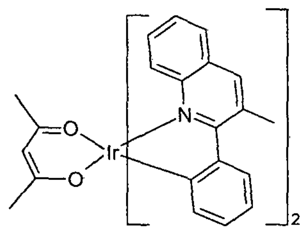
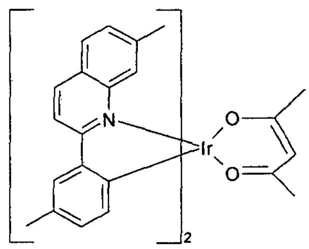

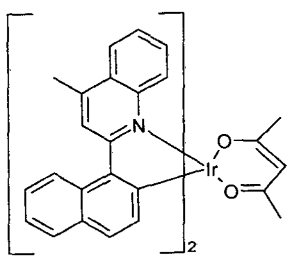
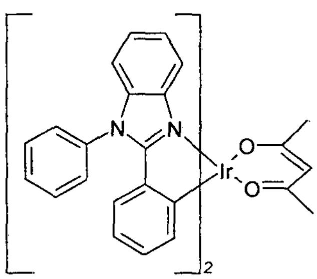
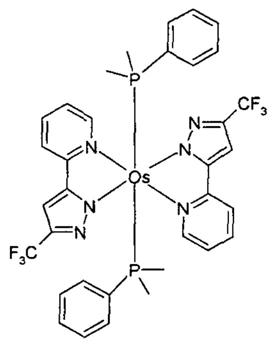

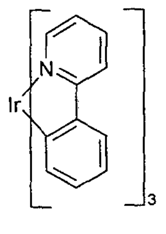
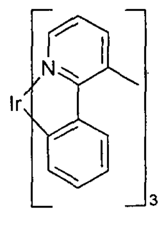
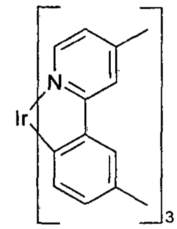
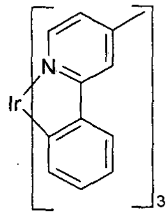
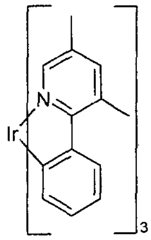
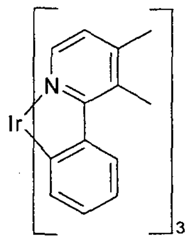
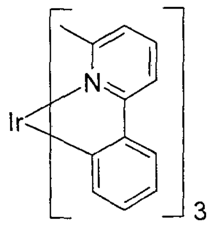
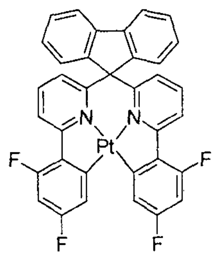
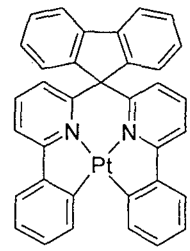
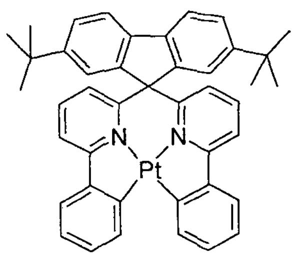
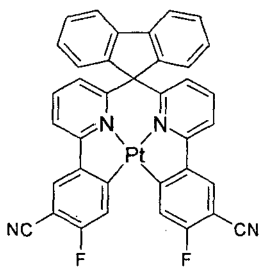
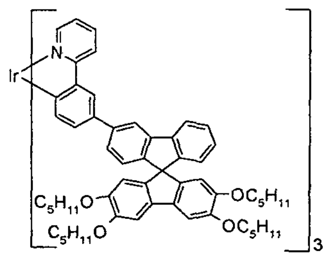
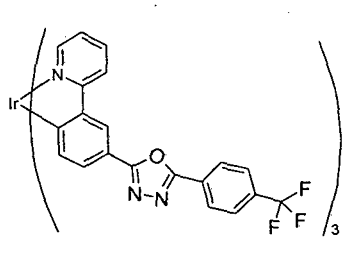
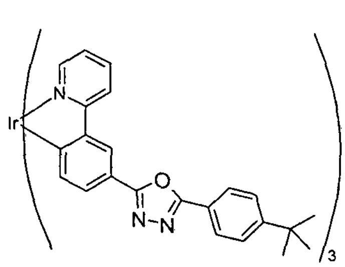
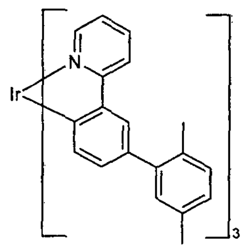
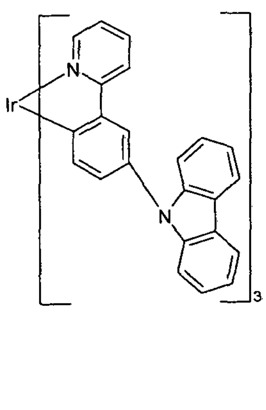
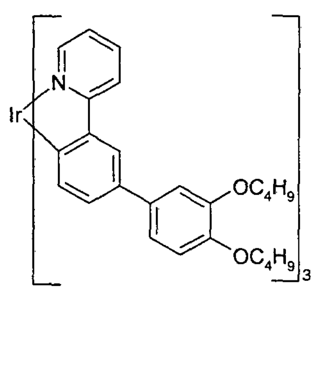
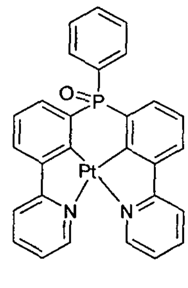

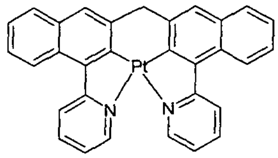
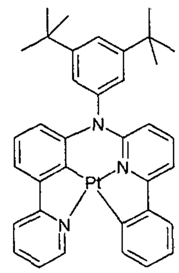
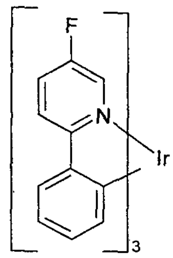
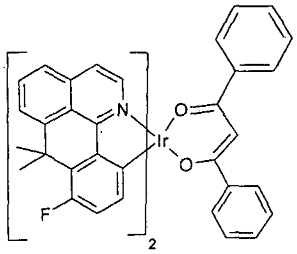
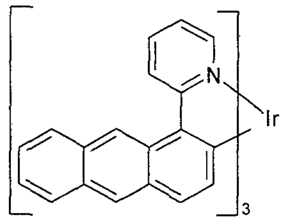
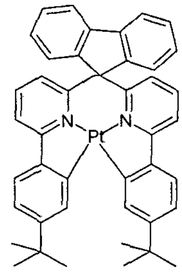
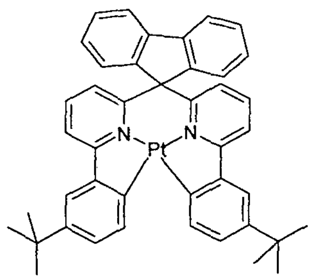
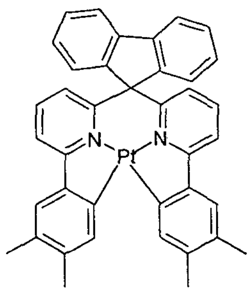
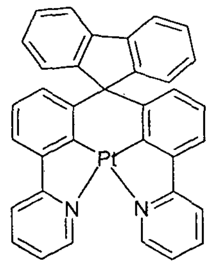
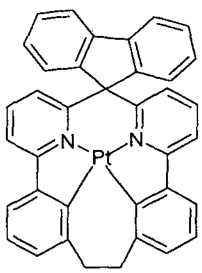
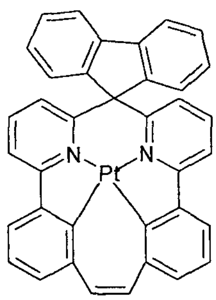
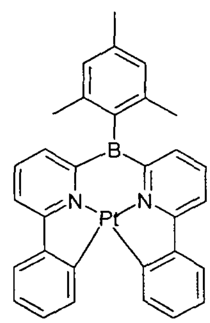
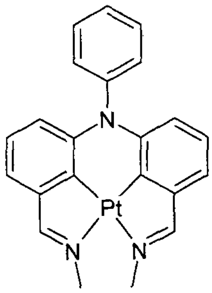
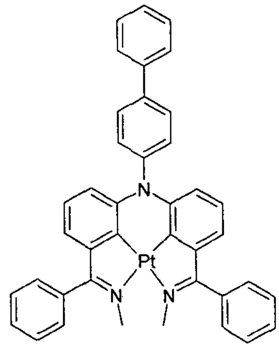
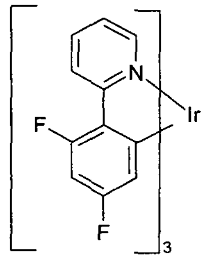

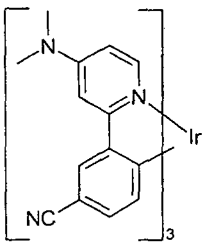
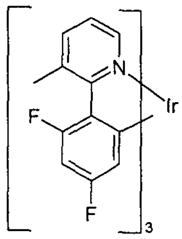
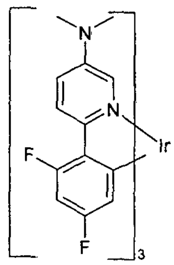

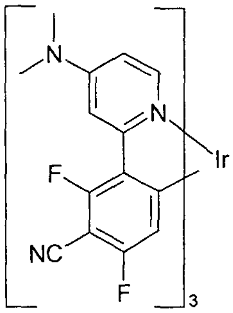
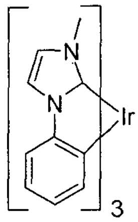
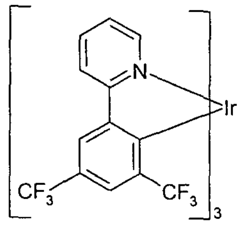

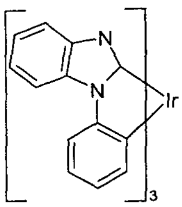
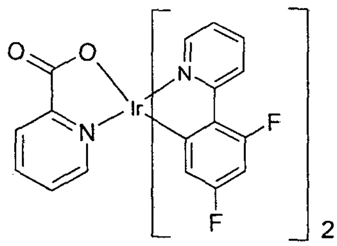
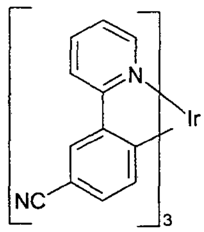

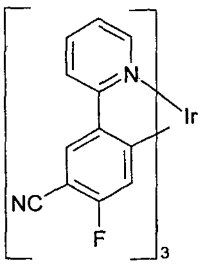
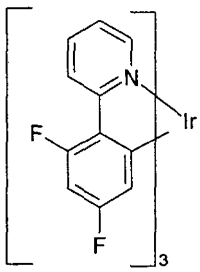
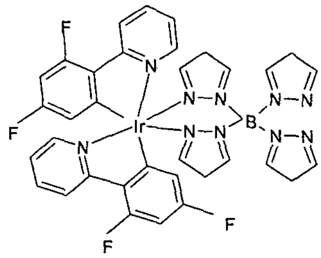

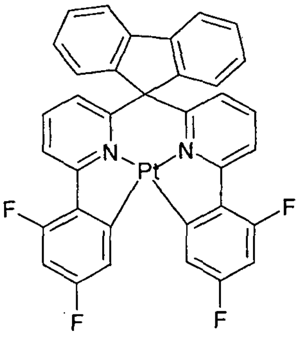
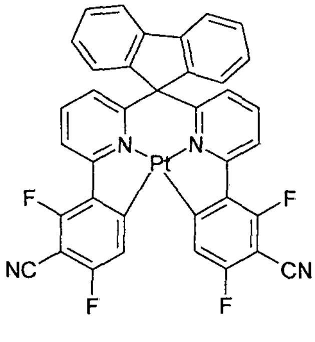
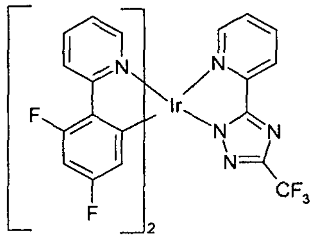

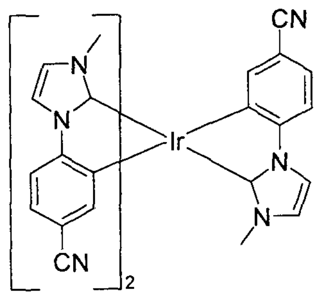
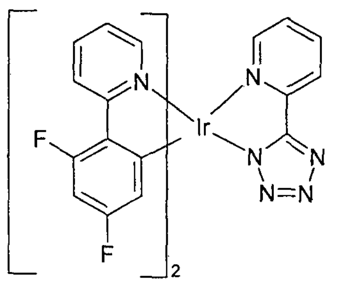
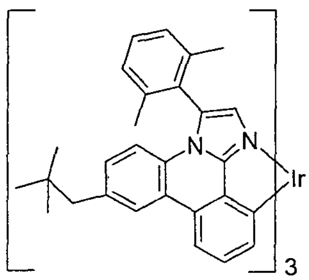

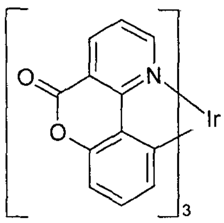
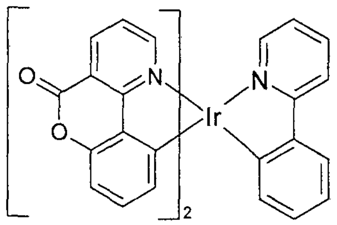
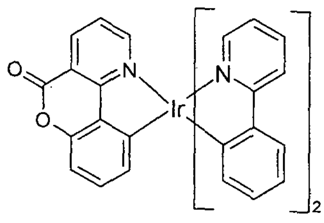

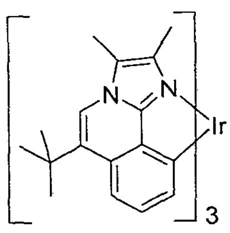
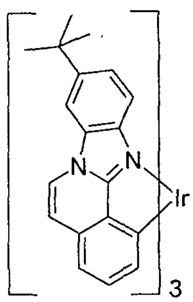
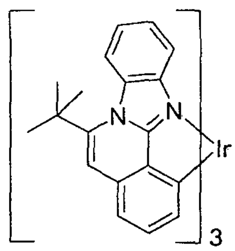

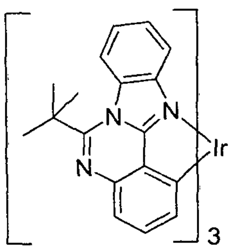
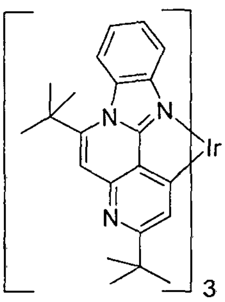
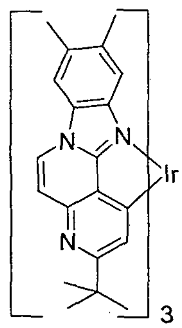

Die oben beschriebenen Verbindung, umfassend Strukturen der Formel (I), bzw. die oben aufgeführten bevorzugten Ausführungsformen können in einer elektronischen Vorrichtung bevorzugt als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine Verbindung, umfassend Strukturen der Formel (I), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs) oder organischen Laserdioden (O-Laser), enthaltend in mindestens einer Schicht mindestens eine Verbindung, umfassend Strukturen der Formel (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und

Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (I) bzw. die oben aufgeführten bevorzugten Ausführungsformen als Matrixmaterial, vorzugsweise als lochleitendes Matrixmaterial in einer oder mehreren emittierenden Schichten, bevorzugt in Kombination mit einem weiteren Matrixmaterial, vorzugsweise einem elektronenleitenden Matrixmaterial. Eine emittierende Schicht umfasst mindestens eine emittierende Verbindung.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Lactame, z.B. gemäß WO 2011/116865A1, WO 2013/064206A1, WO 2014/056567A1, WO 2014/094964A1, Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, oder verbrückte Carbazolderivate, z. B. gemäß US 2009/0136779, WO 2010/050778, WO 2011/042107 oder WO 2011/088877.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerte Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (I) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (I) in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und

20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren lochleitenden Schichten (HTL) umfasst, als lochleitende Verbindung. Vorzugsweise können eine oder mehrere erfindungsgemäße Verbindungen und/oder mindestens ein erfindungsgemäßes Oligomer, Polymer oder Dendrimer als Lochinjektionsmaterial in einer Lochinjektionsschicht oder als Elektronenblockiermaterial in einer Elektronenblockierschicht (EBL) eingesetzt werden. Vorzugsweise kann efindungsgemäße Verbindung umfassend Strukturen gemäß Formel (I) und/oder mindestens ein erfindungsgemäßes Oligomer, Polymer oder Dendrimer als Matrixmaterial, vorzugsweise als lochleitendes Matrixmaterial in einer oder mehreren emittierenden Schichten (EML) eingesetzt werden. Hierbei ist die Verwendung in einer HTL oder EML besonders bevorzugt.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung umfassend Strukturen gemäß Formel (I) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen umfassend Strukturen gemäß Formel (I) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend Verbindungen, Oligomere, Polymere oder Dendrimere mit Strukturen gemäß Formel (I), insbesondere als lochleitende Materialien, weisen eine sehr gute Lebensdauer auf.

2. Elektronischen Vorrichtungen, insbesondere organische Elektrolumineszenzvorrichtungen enthaltend Verbindungen, Oligomere, Polymere oder Dendrimere mit Strukturen gemäß Formel (I) als lochleitende Materialien weisen eine hervorragende Effizienz auf. Insbesondere ist die Effizienz deutlich höher gegenüber analogen Verbindungen, die keine Struktureinheit gemäß Formel (I) enthalten.

3. Die erfindungsgemäßen Verbindungen, Oligomere, Polymere oder Dendrimere mit Strukturen gemäß Formel (I) zeigen eine sehr hohe Stabilität und führen zu Verbindungen mit einer sehr hohen Lebensdauer.

4. Mit Verbindungen, Oligomere, Polymere oder Dendrimere mit Strukturen gemäß Formel (I) kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen, Oligomere, Polymere oder Dendrimere mit Strukturen gemäß Formel (I) in Schichten elektronischer Vorrichtungen, insbesondere organischer Elektrolumineszenzvorrichtungen führt zu einer hohen Mobilität der Elektron-Leiterstrukturen und/oder der Loch-Leiterstrukturen.

6. Verbindungen, Oligomere, Polymere oder Dendrimere mit Strukturen gemäß Formel (I) zeichnen sich durch eine ausgezeichnete thermische Stabilität aus, wobei Verbindungen mit einer Molmasse von weniger als ca. 1200 g/mol gut sublimierbar sind

7. Verbindungen, Oligomere, Polymere oder Dendrimere mit Strukturen gemäß Formel (I) weisen eine ausgezeichnete Glasfilmbildung auf.

8. Verbindungen, Oligomere, Polymere oder Dendrimere mit Strukturen gemäß Formel (I) bilden aus Lösungen sehr gute Filme.

9. Die Verbindungen, Oligomere, Polymere oder Dendrimere umfassend Strukturen gemäß Formel (I) weisen ein überraschend hohes HOMO-Niveau auf, wobei dies insbesondere Verbindungen gilt, die als lochleitende Materialien eingesetzt werden.

Diese oben genannten Vorteile gehen nicht mit einer Verschlechterung der weiteren elektronischen Eigenschaften einher.

Ein weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als als Lochtransportmaterial, Lochinjektionsmaterial, Lochblockiermaterial, Elektroneninjektionsmaterial, Elektronenblockiermaterial und/oder Elektronentransportmaterial, vorzugsweise als Lochtransportmaterial, Lochinjektionsmaterial, Lochblockiermaterial und/oder Matrixmaterial, besonders bevorzugt lochleitendes Matrixmaterial.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### Hestellungsbeispiele:

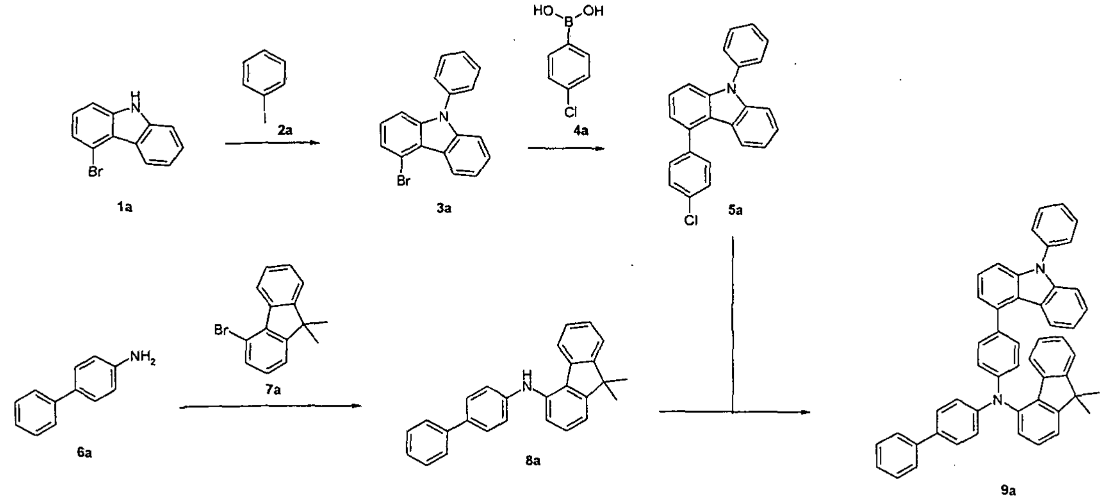


Darstellung von 4-Bromo-9-phenyl-9H-carbazol **3a**

In einem 500ml Vierhalskolben werden 15.0g (61.0mmol, 1.0eq) 4-Bromo-9H-carbazol **1a** (CAS 3652-89-9) zusammen mit 13.6ml (122 mmol, 2.0eq) lodbenzol **2a** und 16.8g (122mmol, 2.0eq) Kaliumcarbonat vorgelegt und in 180ml getrocknetem DMF gelöst. Nach 30 Minuten entgasen mittels eines Stickstoffstroms werden 1.38g (6.10mmol, 0.10eq) 1,3-Di(2-pyridyl)-1,3-propandion und 1.16g (6.10mmol, 0.10eq) Kupfer(I)-iodid zugegeben. Der Ansatz wird bei 110°C über Nacht gerührt und nach beendeter Reaktion das Lösungsmittel am Rotationsverdampfer entfernt. Der Rückstand wird in 250ml DCM aufgenommen, mit konz. Ammoniumchlorid-Lösung versetzt und über Celite filtriert. Anschließend werden die Phasen getrennt, die wässrige Phase zweimal mit je 100ml DCM extrahiert und die vereinten organischen Phasen abschließend mit Wasser gewaschen. Nach Trocknen über Natriumsulfat und Entfernung des Lösungsmittels im Vakuum wird der ölige Rückstand mit Heptan über Kieselgel filtriert und das Lösungsmittel erneut am Rotationsverdampfer entfernt. Es werden 19.0g (59.0mmol, 97%) eines farblosen Öls **3a** erhalten.

Analog werden dargestellt:

| Verbindung | Edukt **1** | Edukt **2** | Produkt **3** | Ausbeute [%] |
|---|---|---|---|---|
| **3b** | 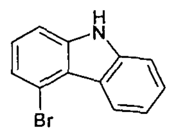 | 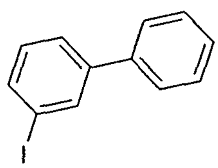 | 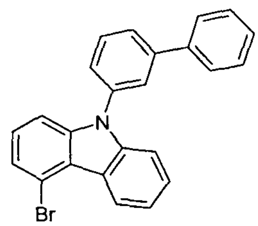 | 92 |
| **3c** | 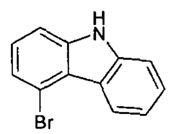 | 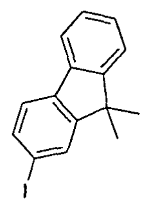 | 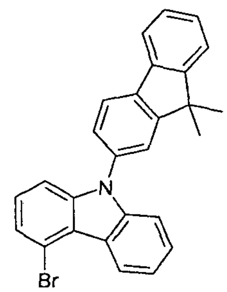 | 89 |

(fortgesetzt)

| Verbindung | Edukt **1** | Edukt **2** | Produkt **3** | Ausbeute [%] |
|---|---|---|---|---|
| **3d** | 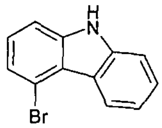 | 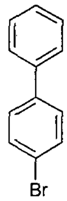 | 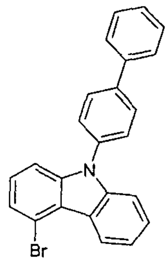 | 96 |
| **3e** | 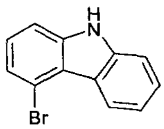 | 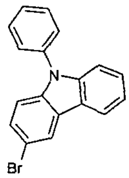 | 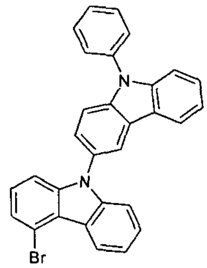 | 84 |

Darstellung von 4-(4-Chloro-phenyl)-9-phenyl-9H-carbazol **5a**

In einem 500ml Vierhalskolben werden 18.9g (58.7mmol, 1.0eq) 4-Bromo-9-phenyl-9H-carbazol **3,** 9.17g (58.7mol, 1.0eq) 4-Chlorphenylboronsäure (CAS 1679-18-1) und 6.22g (58.7mmol, 1.0eq) Natriumcarbonat werden in 150ml Toluol, 36ml Ethanol und 77ml Wasser gelöst. Nach 30-minütigem Entgasen mittels eines Stickstoffstroms werden 678mg (0.587mmol, 0.01eq) Tetrakis(triphenylphosphin)palladium zugegeben und der Ansatz über Nacht am Rückfluss erhitzt. Nach beendeter Reaktion werden die Phasen getrennt, die wässrige Phase dreifach mit Toluol extrahiert, die vereinten organischen Phasen anschließend mit Wasser gewaschen. Die organischen Phasen werden über Natriumsulfat getrocknet und die Lösung am Rotationsverdampfer eingeengt. Der Rückstand wird in 250ml Ethanol eingetragen und der entstandene Feststoff abgesaugt. Es werden 19.9g (56.4mmol, 96%) des gewünschten Produkts **5a** erhalten.

Analog werden hergestellt:

| Verbindung | Edukt **3** | Edukt **4** | Produkt **5** | Ausbeute [%] |
|---|---|---|---|---|
| **5b** | 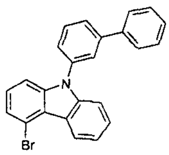 | 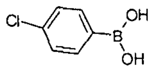 [1679-18-1] | 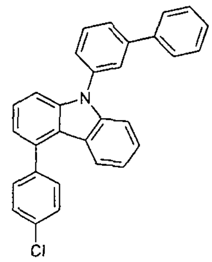 | 91 |
| **5c** | 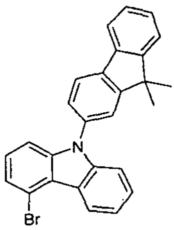 | 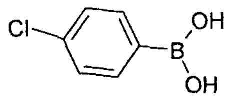 [1679-18-1] | 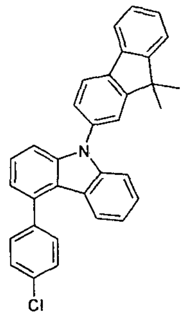 | 87 |

(fortgesetzt)

| Verbindung | Edukt **3** | Edukt **4** | Produkt **5** | Ausbeute [%] |
|---|---|---|---|---|
| **5d** | 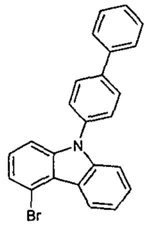 | 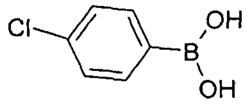 [1679-18-1] | 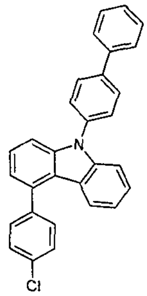 | 93 |
| **5e** | 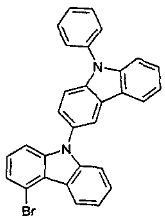 | 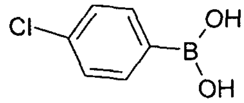 [1679-18-1] | 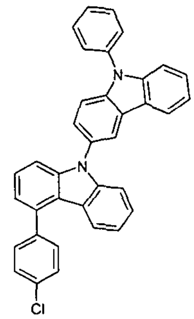 | 72 |
| **5f** | 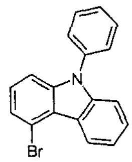 | 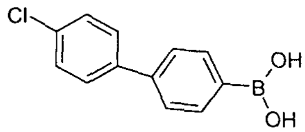 [364044-44-0] | 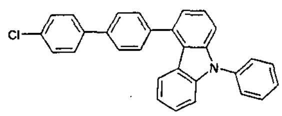 | 88 |
| **5g** | 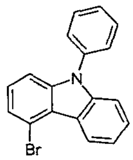 | 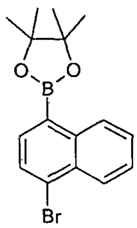 [1404070-40-1] | 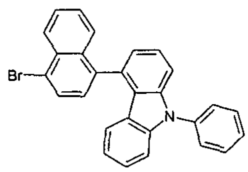 | 91 |
| **5h** | 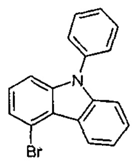 | 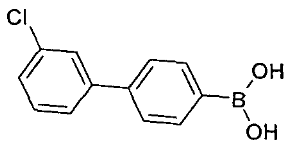 [1025496-32-5] | 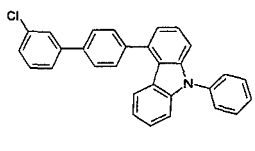 | 97 |
| **5i** | 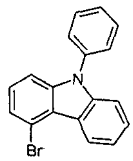 | 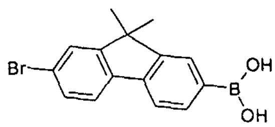 [1213768-48-9] | 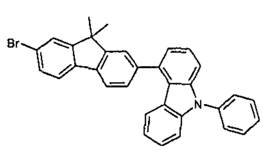 | 87 |

Darstellung von Biphenyl-4-yl-(9,9-dimethyl-9H-fluoren-4-yl)-amin **8a** (Variante A)

In einem 1L-Vierhalskolben werden 14.0g (51.4mmol, 1.0eq) 4-Brom-9,9-Dimethylfluoren **7a,** sowie 10.5g (60.8mmol, 1.2eq) Biphenyl-4-amin **6a** und 12.9g (134mmol, 2.6eq) Natrium-*tert*-butylat vorgelegt und in 300ml trockenem p-Xylol gelöst. Anschließend wird der Ansatz für 45 Minuten entgast und mit 346mg (1.54mmol, 0.03eq) Palladium(II)-acetat und 1.71g (3.09mmol, 0.06eq) 1,1'-Bis(diphenylphosphino)ferrocen zugegeben und auf über Nacht bei 140°C gerührt. Nach beendeter Reaktion wird das Lösungsmittel im Vakuum entfernt, der Rückstand in Dichlormethan aufgenommen und mit Heptan versetzt. De ausgefallene Feststoff wird abgesaugt und im Vakuumtrockenschrank getrocknet. Es werden 11.0g (30.5mmol, 59%) des gewünschten Produktes **8a** erhalten.

Analog werden hergestellt:

| Verbindung | Edukt **6** | Edukt **7** | Produkt **8** | Ausbeute [%] |
|---|---|---|---|---|
| **8b** | 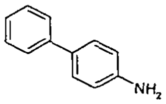 [92-67-1] | 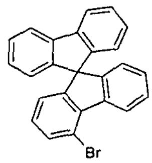 [1161009-88-6] | 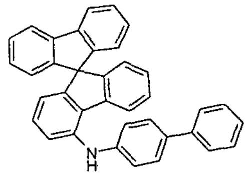 | 73 |
| **8c** | 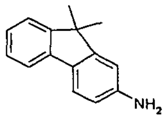 [108714-73-4] | 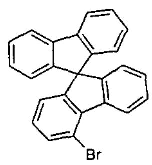 [1161009-88-6] | 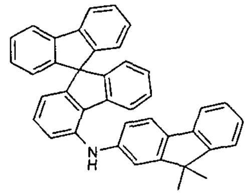 | 85 |
| **8d** | 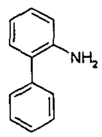 [90-41-5] | 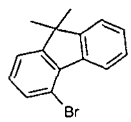 [942615-32-9] | 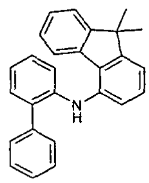 | 69 |
| **8e** | 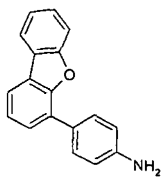 [578027 - 21-1] | 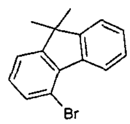 [942615-32-9] | 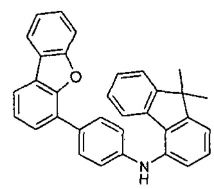 | 37 |
| **8f** | 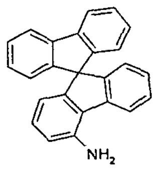 [1579281-06-3] | 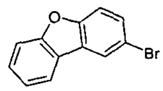 [86-76-0] | 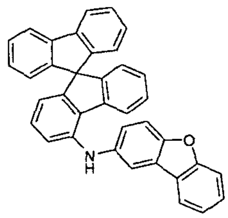 | 88 |
| **8g** | 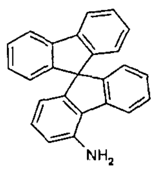 [1579281-06-3] | 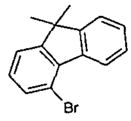 [942615-32-9] | 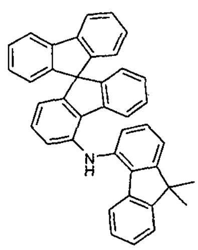 | 81 |

(fortgesetzt)

| Verbindung | Edukt **6** | Edukt **7** | Produkt **8** | Ausbeute [%] |
|---|---|---|---|---|
| **8h** | 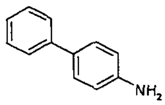 [92-67-1] | 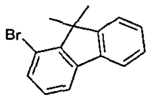 [1225053-54-2] | 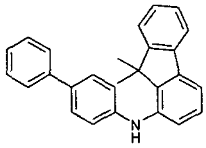 | 77 |
| **8i** | 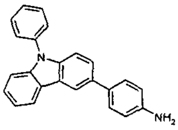 [1370037-59-5] | 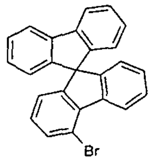 [1161009-88-6] | 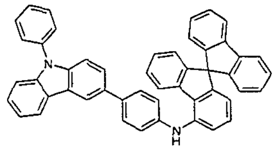 | 89 |
| **8j** | 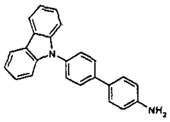 [207447-26-5] | 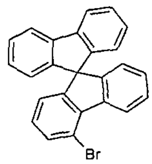 [1161009-88-6] | 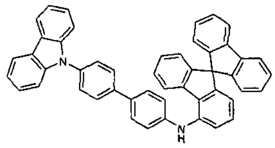 | 93 |
| **8k** | 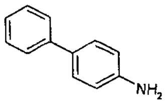 [92-67-1] | 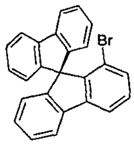 [1450933-18-2] | 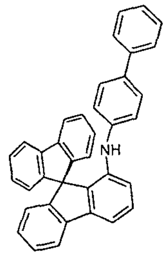 | 21 |
| **8l** | 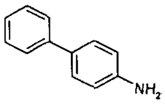 [92-67-1] | 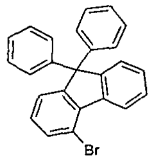 [713125-22-5] | 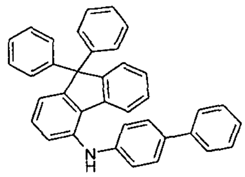 | 67 |
| **8m** | 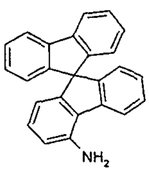 [1579281-06-3] | 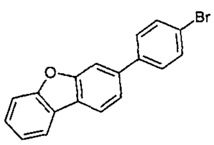 [86-76-0] | 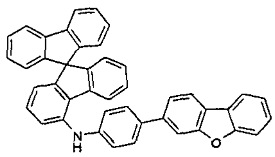 | 84 |
| **8n** | 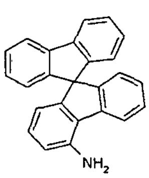 [1579281-06-3] | 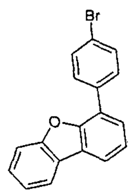 [955959-84-9] | 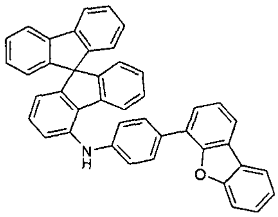 | 91 |

(fortgesetzt)

| Verbindung | Edukt **6** | Edukt **7** | Produkt **8** | Ausbeute [%] |
|---|---|---|---|---|
| **8o** | 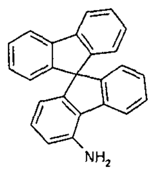 [1579281-06-3] | 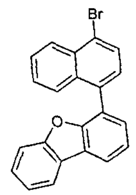 [1297532-85-4] | 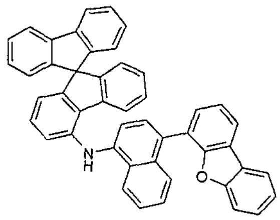 | 74 |

Darstellung von Biphenyl-4-yl-(9,9-dimethyl-9H-fluoren-4-yl)-[4-(9-phenyl-9H-carbazol-4-yl)-phenyl]-amin **9a** (Variante A)

Es werden 11.0g (30.5mmol, 1.0eq) des sekundären Amins **8a,** sowie 11.9g (33.6mmol, 1.1eq) 4-(4-Chlorophenyl)-9-phenyl-9H-carbazol **5a** in 250ml trockenem Toluol gelöst und ca. 30 Minuten mit Argon gesättigt. Anschließend werden 279mg (0.305mmol, 0.1eq) Tris(dibenyliden-aceton)dipalladium und 250mg (0.610mmol, 0.02eq) 2-Dicyclohexylphosphino-2',6'-methoxybiphenyl zugegeben und der Ansatz auf Rückfluss erhitzt. Nach beendeter Reaktion wird der Ansatz in einen Scheidetrichter überführt und zweimal mit 300ml Wasser extrahiert. Die wässrige Phase wird erneut mit Toluol ausgeschüttelt und die vereinten, organischen Phasen über Natriumsulfat getrocknet. Nach Entfernen des Lösungsmittels am Rotationsverdampfer wird ein harziger Feststoff erhalten, der in etwas Dichlormethan aufgenommen und in Ethanol eingetragen wird. Der erhaltene Feststoff wird mittels Heißextraktion und dreifacher Umkristallisation aus Toluol/Heptan aufgereinigt und abschließend sublimiert. Es werden 7.04g (10.4mmol, 34%) der Endstufe **9a** mit einer HPLC Reinheit >99.9% erhalten.

Analog werden hergestellt:

| Verbindung | Edukt **6** | Edukt **7** | Produkt **8** | Ausbeute [%] |
|---|---|---|---|---|
| **9b** | 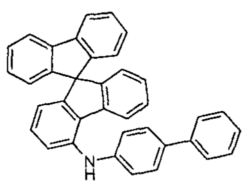 | 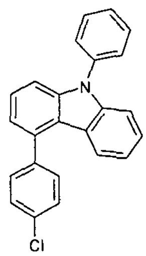 | 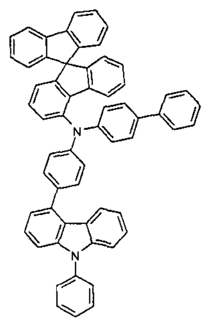 | 45 |
| **9c** | 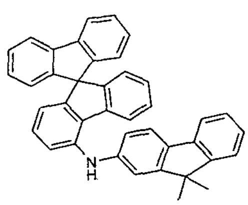 | 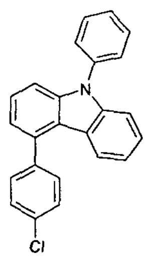 | 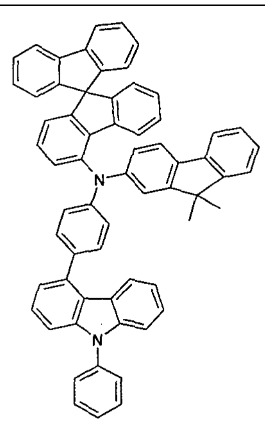 | 51 |

(fortgesetzt)

| Verbindung | Edukt **6** | Edukt **7** | Produkt **8** | Ausbeute [%] |
|---|---|---|---|---|
| **9d** | 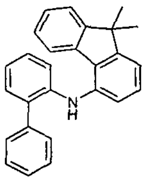 | 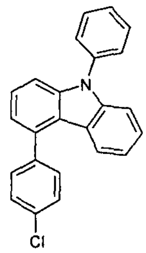 | 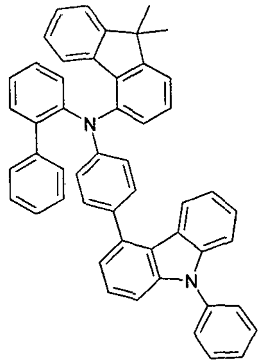 | 47 |
| **9e** | 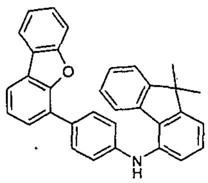 | 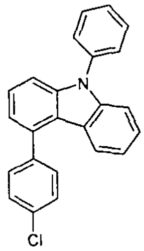 | 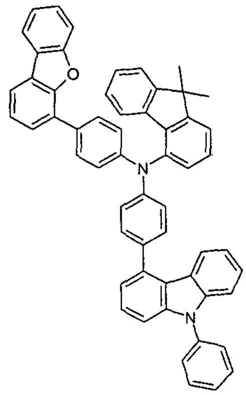 | 21 |
| **9f** | 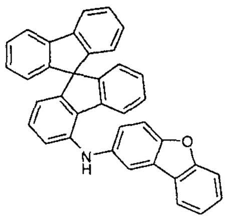 | 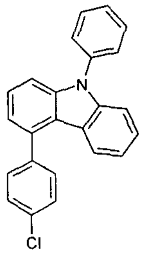 | 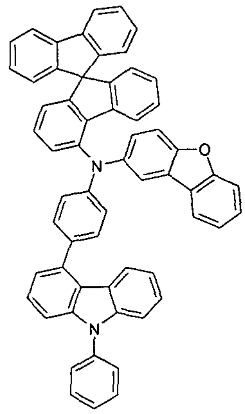 | 63 |
| **9g** | 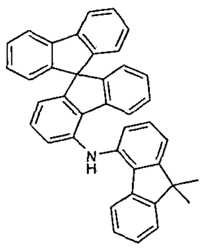 | 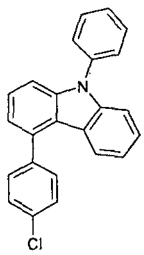 | 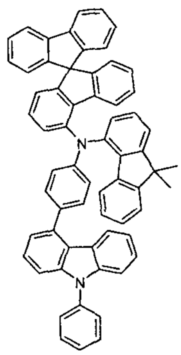 | 44 |

(fortgesetzt)

| Verbindung | Edukt **6** | Edukt **7** | Produkt **8** | Ausbeute [%] |
|---|---|---|---|---|
| **9h** | 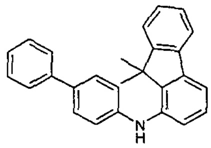 | 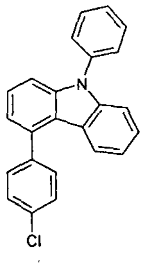 | 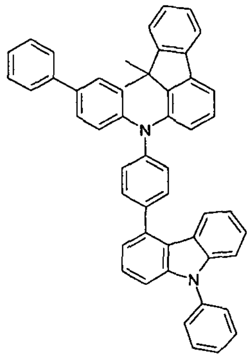 | 41 |
| **9i** | 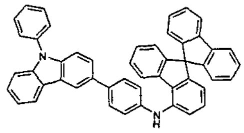 | 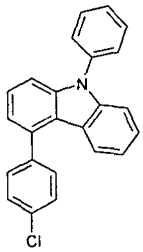 | 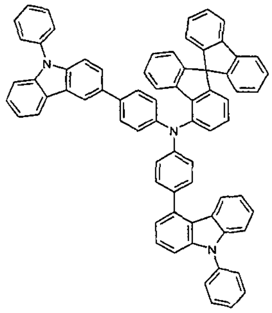 | 37 |
| **9j** | 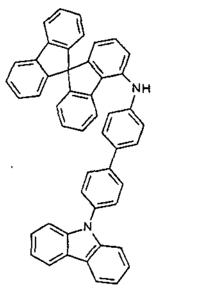 | 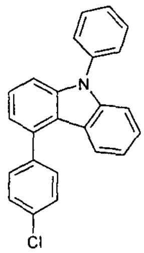 | 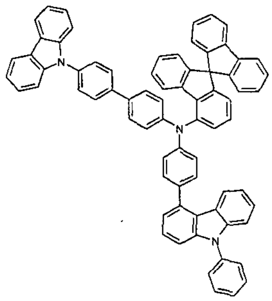 | 52 |
| **9k** | 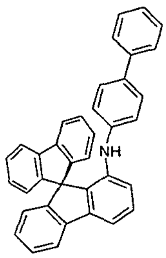 | 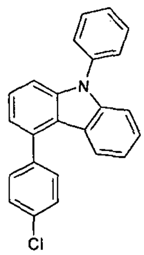 | 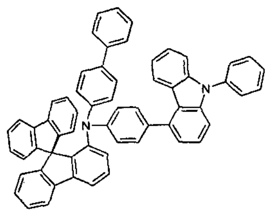 | 28 |

(fortgesetzt)

| Verbindung | Edukt **6** | Edukt **7** | Produkt **8** | Ausbeute [%] |
|---|---|---|---|---|
| **9l** | 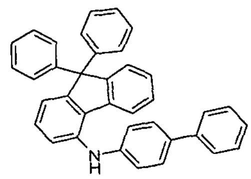 | 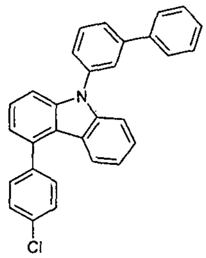 | 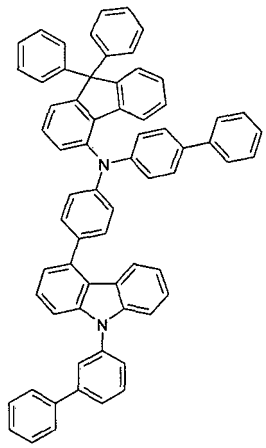 | 46 |
| **9m** | 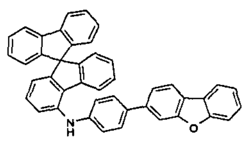 | 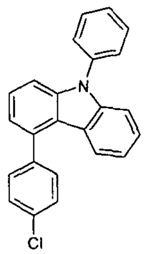 | 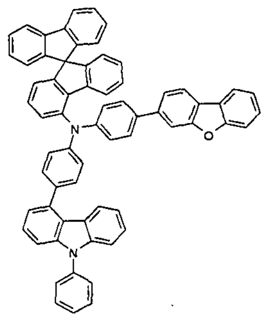 | 41 |
| **9n** | 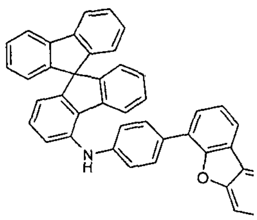 | 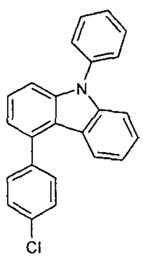 | 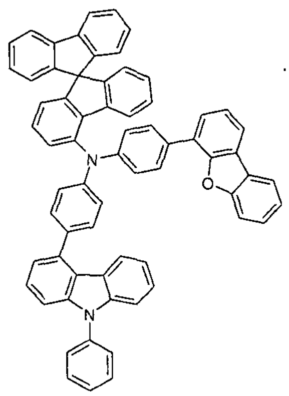 | 39 |
| **9o** | 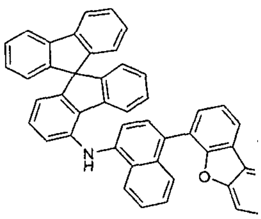 | 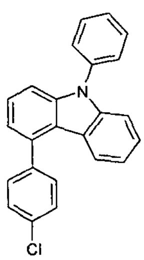 | 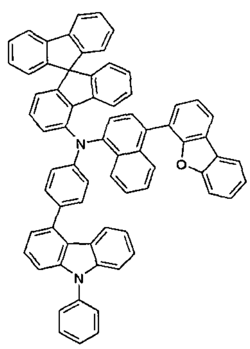 | 36 |

(fortgesetzt)

| Verbindung | Edukt **6** | Edukt **7** | Produkt **8** | Ausbeute [%] |
|---|---|---|---|---|
| **9p** | 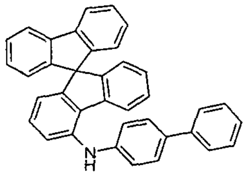 | 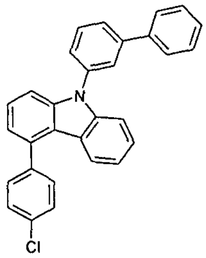 | 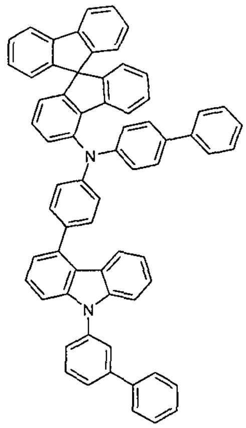 | 22 |
| **9q** | 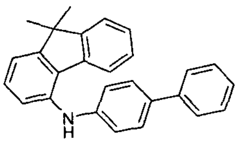 | 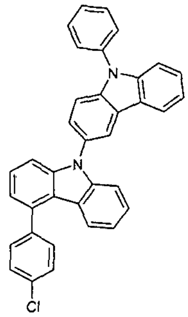 | 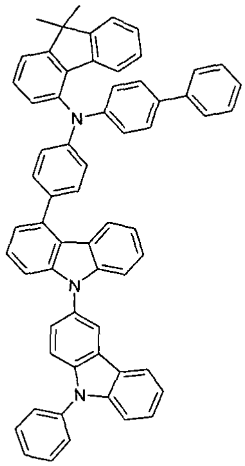 | 63 |
| **9r** | 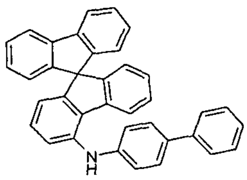 | 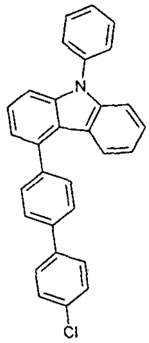 | 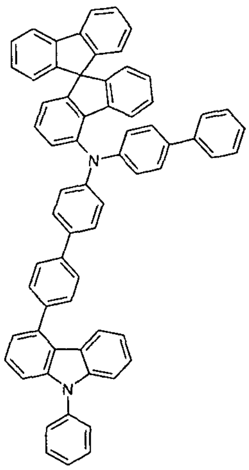 | 58 |

(fortgesetzt)

| Verbindung | Edukt **6** | Edukt **7** | Produkt **8** | Ausbeute [%] |
|---|---|---|---|---|
| **9s** | 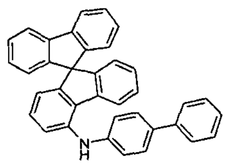 | 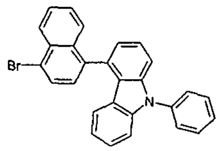 | 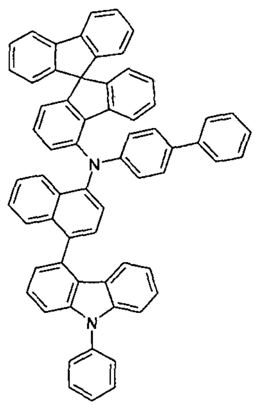 | 45 |
| **9t** | 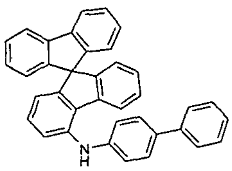 | 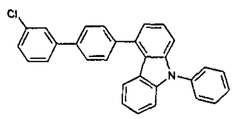 | 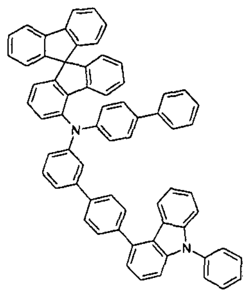 | 44 |
| **9u** | 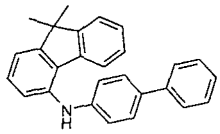 | 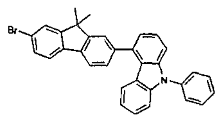 | 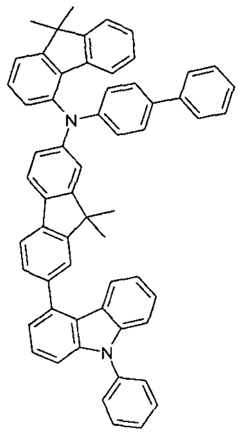 | 17 |

**Herstellung der OLEDs**

In den folgenden Beispielen V1 bis E14(siehe Tabellen 1 und 2) werden die Daten verschiedener OLEDs vorgestellt.

**Vorbehandlung für die Beispiele V1-E14:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly-(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht

immer aus mindestens einem Matrix-material (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:SdT:TEG1 (55%:35%:10%) bedeutet hierbei, dass das Material IC1 in einem Volumenanteil von 55%, SdT in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$. Als Lebensdauer LD wird die Zeit definiert, nach der die Leuchtdichte bei Betrieb mit konstantem Strom von der Startleuchtdichte auf einen gewissen Anteil $L_1$ absinkt. Eine Angabe von Lo;jo = 4000 cd/$m^2$ und $L_1$ = 70% in Tabelle 2 bedeutet, dass die in Spalte LD angegebene Lebensdauer der Zeit entspricht, nach der die Anfangsleuchtdichte von 4000 cd/$m^2$ auf 2800 cd/$m^2$ absinkt. Analog bedeutet $L_0$;$j_0$ = 20mA/$cm^2$, $L_1$= 80%, dass die Leuchtdichte bei Betrieb mit 20mA/$cm^2$ nach der Zeit LD auf 80% ihres Anfangswertes absinkt.

Die Daten der verschiedenen OLEDs sind in Tabelle 2 zusammengefasst. Das Beispiel V1 ist ein Vergleichsbeispiel gemäß dem Stand der Technik, die Beispiele E1-E14 zeigen Daten von erfindungsgemäßen OLEDs.

Im Folgenden werden einige der Beispiele näher erläutert, um die Vorteile der erfindungsgemäßen OLEDs zu verdeutlichen.

**Verwendung von erfindungsgemäßen Verbindungen als lochtransportierendes Matrixmaterial phosphoreszenter OLEDs**

Die erfindungsgemäßen Materialien ergeben bei Einsatz als lochtransportierendes Matrixmaterial in Kombination mit einer elektronenleitenden Verbindung (wie z.B. Verbindung IC1 in den unten aufgeführten Beispielen) in der Emissionsschicht (EML) in phosphoreszierenden OLEDs wesentliche Verbesserungen gegenüber dem Stand der Technik, vor allem bezüglich der Lebensdauer der OLEDs. Durch Einsatz der erfindungsgemäßen Verbindungen 9a und 9h lässt sich eine Verbesserung der Lebensdauer mehr als 50% gegenüber der Verbindung aus dem Stand der Technik SdT beobachten (Beispiele V1, E1 und E2 oder V2 und E15).

Tabelle 1: Aufbau der OLEDs

| Bsp. | HIL Dicke | IL Dicke | HTL Dicke | EBL Dicke | EML Dicke | HBL Dicke | ETL Dicke | EIL Dicke |
|---|---|---|---|---|---|---|---|---|
| V1 | SpA1 70nm | HATCN 5nm | SpMA1 70nm | --- | IC1:SdT:TEG1 (45%: 45%:10%) 30nm | IC1 10nm | ST2:LiQ (50%:50%) 30nm | --- |
| V2 | SpA1 70nm | HATCN 5nm | SpMA1 70nm | --- | IC1:SdT2:TEG1 (45%:45%:10%) 30nm | IC1 10nm | ST2:LiQ (50%:50%) 30nm | |
| E1 | SpA1 70nm | HATCN 5nm | SpMA1 70nm | --- | IC1:9a:TEG1 (45%: 45%:10%) 30nm | IC1 10nm | ST2:LiQ (50%:50%) 30nm | --- |
| E2 | SpA1 70nm | HATCN 5nm | SpMA1 70nm | --- | IC1:9h:TEG1 (45%: 45%:10%) 30nm | IC1 10nm | ST2:LiQ (50%:50%) 30nm | --- |
| E3 | SpA1 70nm | HATCN 5nm | SpMA1 80nm | 9b 10nm | IC1:IC3:TEG1 (65%: 30%:5%) 30nm | IC1 10nm | ST2:LiQ (50%:50%) 30nm | --- |
| E4 | SpA1 70nm | HATCN 5nm | SpMA1 80nm | 9c 10nm | IC1:IC3:TEG1 (65%: 30%:5%) 30nm | IC1 10nm | ST2:LiQ (50%:50%) 30nm | --- |

(fortgesetzt)

| Bsp. | HIL Dicke | IL Dicke | HTL Dicke | EBL Dicke | EML Dicke | HBL Dicke | ETL Dicke | EIL Dicke |
|---|---|---|---|---|---|---|---|---|
| E5 | SpA1 70nm | HATCN 5nm | SpMA1 90nm | --- | IC1:9f:TEG1 (65%: 30%:5%) 30nm | IC1 10nm | ST2:LiQ (50%:50%) 30nm | --- |
| E6 | SpA1 90nm | HATCN 5nm | SpMA1 110nm | 9g 20nm | IC1:TER1 (92%:8%) 40nm | --- | ST2:LiQ (50%:50%) 40nm | --- |
| E7 | SpA1 90nm | HATCN 5nm | SpMA1 130nm | --- | IC1:9i:TER1 (87%: 5%:8%) 40nm | --- | ST2:LiQ (50%:50%) 40nm | --- |
| E8 | SpA1 90nm | HATCN 5nm | SpMA1 110nm | 9j 20nm | IC1:9j:TER1 (82%: 10%:8%) 40nm | --- | ST2:LiQ (50%:50%) 40nm | --- |
| E9 | SpA1 90nm | HATCN 5nm | SpMA1 130nm | --- | IC1:9k:TER1 (87%: 5%:8%) 40nm | --- | ST2:LiQ (50%:50%) 40nm | --- |
| E10 | SpA1 70nm | HATCN 5nm | SpMA1 70nm | --- | IC1:9n:TEG1 (45%: 45%:10%) 30nm | IC1 10nm | ST2:LiQ (50%:50%) 30nm | --- |
| E11 | SpA1 70nm | HATCN 5nm | SpMA1 70nm | --- | IC1:9p:TEG1 (60%: 30%:10%) 30nm | IC1 10nm | ST2:LiQ (50%:50%) 30nm | --- |
| E12 | SpA1 70nm | HATCN 5nm | SpMA1 70nm | --- | IC1:9q:TEG1 (60%: 30%:10%) 30nm | IC1 10nm | ST2:LiQ (50%:50%) 30nm | --- |
| E13 | SpA1 70nm | HATCN 5nm | SpMA1 80nm | 9r 10nm | IC1:IC3:TEG1 (55%: 35%:10%) 30nm | IC1 10nm | ST2:LiQ (50%:50%) 30nm | --- |
| E14 | SpA1 70nm | HATCN 5nm | SpMA1 80nm | 9u 10nm | IC1:IC3:TEG1 (55%: 35%:10%) 30nm | IC1 10nm | ST2:LiQ (50%:50%) 30nm | --- |
| E15 | SpA1 70nm | HATCN 5nm | SpMA1 70nm | --- | IC1:9h:TEG1 (45%: 45%:10%) 30nm | IC1 10nm | ST2:LiQ (50%:50%) 30nm | |

Tabelle 2: Daten der OLEDs

| Bsp. | U1000 (V) | SE1000 (cd/A) | LE1000. (lm/W) | EQE 1000 | CIE x/y bei 1000 cd/m$^2$ | $L_0$; $j_0$ | $L_1$ % | LD (h) |
|---|---|---|---|---|---|---|---|---|
| V1 | 3.3 | 60 | 57 | 16.7% | 0.32/0.63 | 20 mA/cm$^2$ | 80 | 120 |
| V1 | 3.4 | 59 | 55 | 16.4% | 0.32/0.63 | 20 mA/cm$^2$ | 80 | 145 |
| E1 | 3.5 | 58 | 52 | 16.4% | 0.33/0.62 | 20 mA/cm$^2$ | 80 | 220 |
| E2 | 3.4 | 60 | 55 | 16.5% | 0.33/0.63 | 20 mA/cm$^2$ | 80 | 180 |

(fortgesetzt)

| Bsp. | U1000 (V) | SE1000 (cd/A) | LE1000. (lm/W) | EQE 1000 | CIE x/y bei 1000 cd/m2 | $L_0$; $j_0$ | $L_1$ % | LD (h) |
|---|---|---|---|---|---|---|---|---|
| E3 | 3.6 | 62 | 54 | 16.8% | 0.32/0.63 | 20 mA/cm2 | 80 | 200 |
| E4 | 3.4 | 59 | 55 | 16.6% | 0.32/0.63 | 20 mA/cm2 | 80 | 220 |
| E5 | 3.3 | 58 | 55 | 16.4% | 0.33/0.62 | 20 mA/cm2 | 80 | 130 |
| E6 | 4.4 | 12 | 9 | 13.1% | 0.67/0.33 | 4000 cd/m2 | 80 | 310 |
| E7 | 4.5 | 13 | 9 | 13.2% | 0.67/0.33 | 4000 cd/m2 | 80 | 510 |
| E8 | 4.6 | 13 | 9 | 13.0% | 0.66/0.34 | 4000 cd/m2 | 80 | 530 |
| E9 | 4.4 | 12 | 9 | 12.9% | 0.67/0.33 | 4000 cd/m2 | 80 | 490 |
| E10 | 3.5 | 58 | 52 | 16.3% | 0.34/0.62 | 20 mA/cm2 | 80 | 230 |
| E11 | 3.6 | 60 | 52 | 16.4% | 0.32/0.63 | 20 mA/cm2 | 80 | 190 |
| E12 | 3.6 | 61 | 53 | 16.5% | 0.34/0.62 | 20 mA/cm2 | 80 | 200 |
| E13 | 3.5 | 63 | 57 | 16.9% | 0.32/0.64 | 20 mA/cm2 | 80 | 170 |
| E14 | 3.4 | 58 | 54 | 16.2% | 0.33/0.63 | 20 mA/cm2 | 80 | 210 |
| E15 | 3.4 | 60 | 55 | 16.5% | 0.33/0.63 | 20 mA/cm2 | 80 | 180 |

Tabelle 3: Strukturformeln der Materialien für die OLEDs

| | |
|---|---|
| 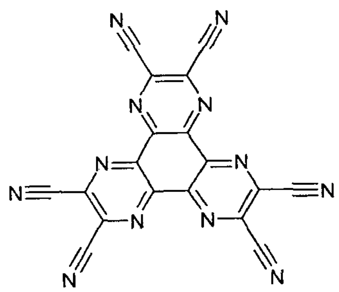 | 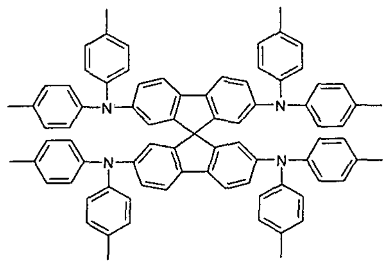 |
| HATCN | SpA1 |

(fortgesetzt)

| | |
|---|---|
| 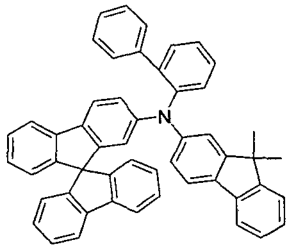 | 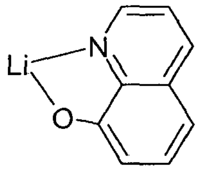 |
| SpMA1 | LiQ |
| 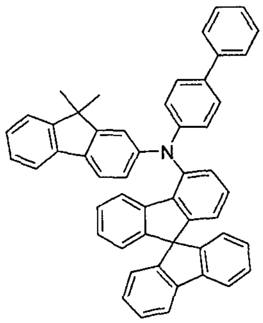 | 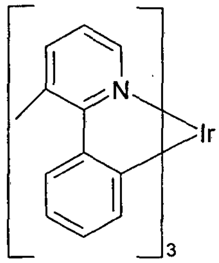 |
| SpMA2 | TEG1 |
| 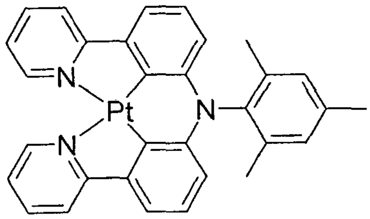 | 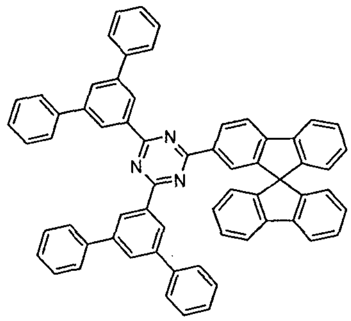 |
| TER1 | ST2 |
| 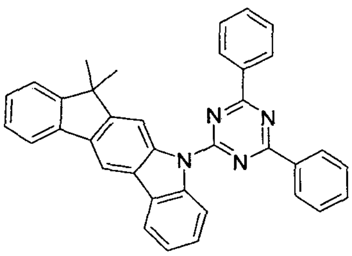 | 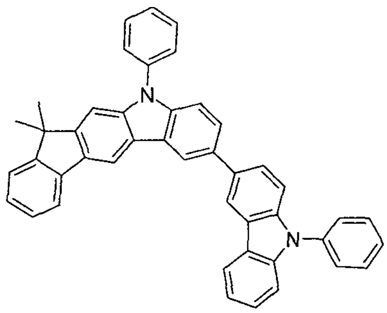 |
| IC1 | IC3 |

(fortgesetzt)

| | |
|---|---|
| 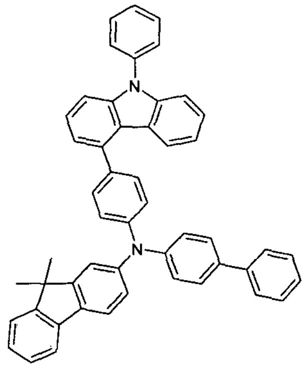 | 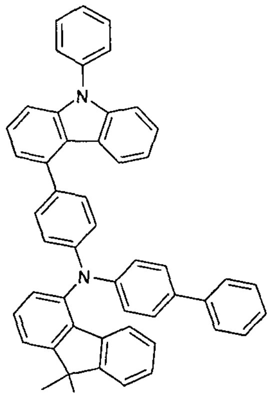 |
| SdT | 9a |
| 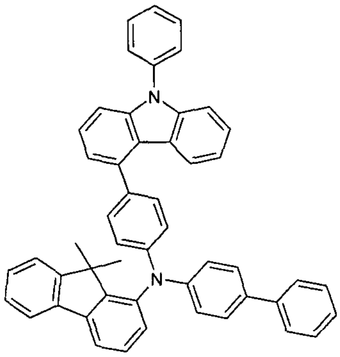 | 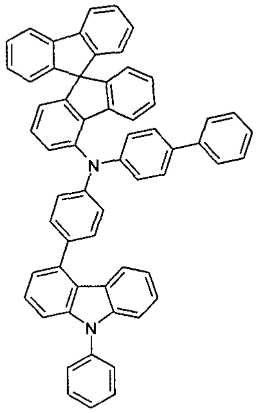 |
| 9h | 9b |
| 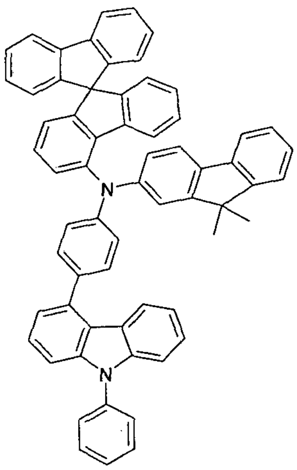 | 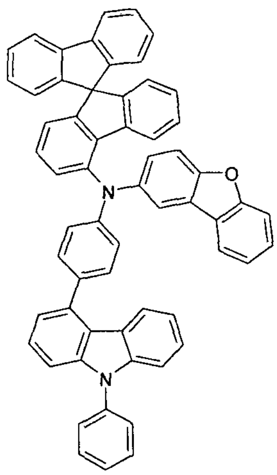 |
| 9c | 9f |

(fortgesetzt)

| | |
|---|---|
| 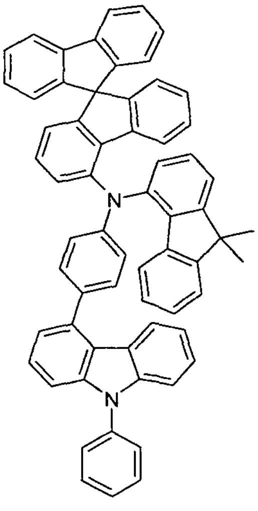 | 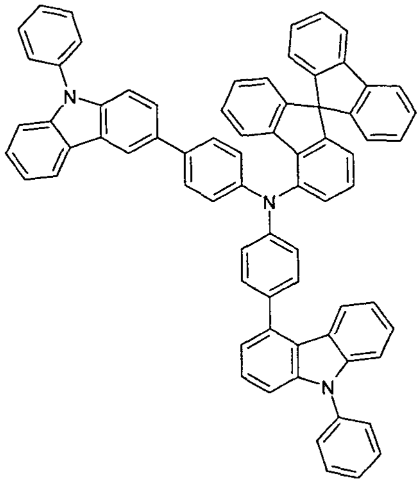 |
| 9g | 9i |
| 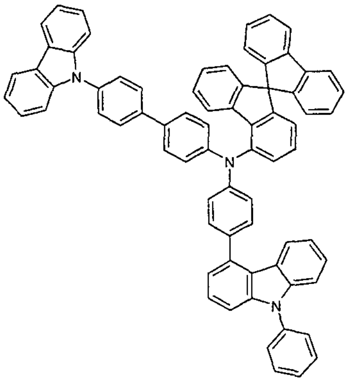 | 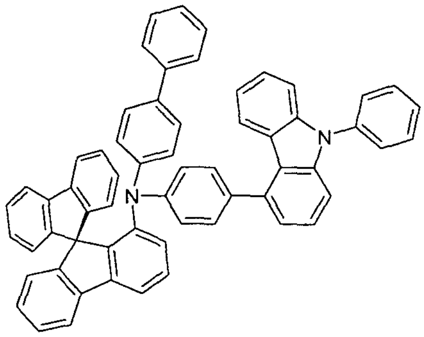 |
| 9j | 9k |
| 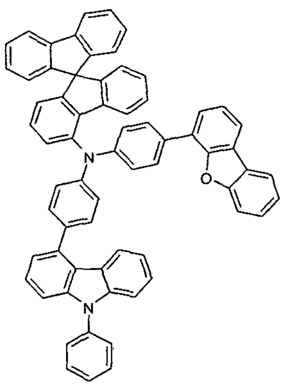 | 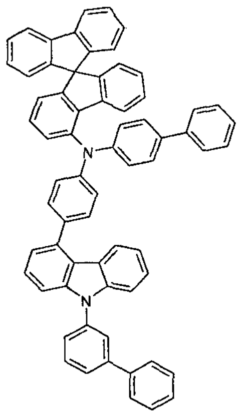 |
| 9n | 9p |

(fortgesetzt)

| | |
|---|---|
| 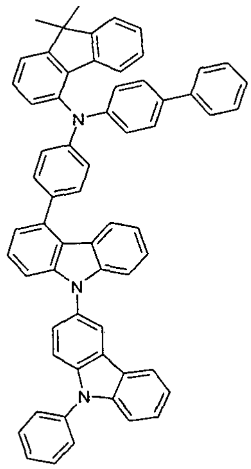 | 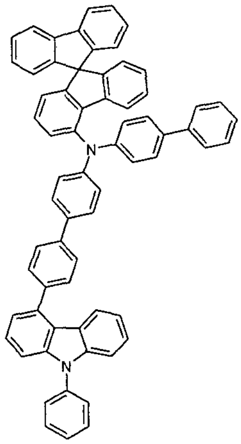 |
| 9q | 9r |
| 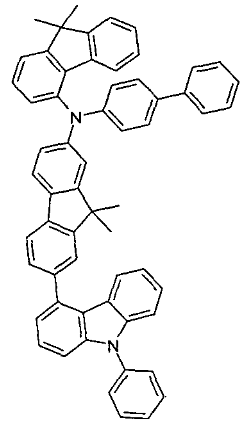 | 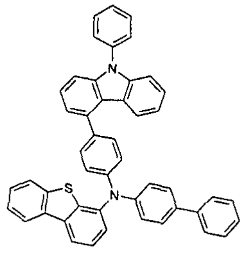 |
| 9u | SdT2 |

**Patentansprüche**

1. Verbindung, umfassend Strukturen der Formel (I)

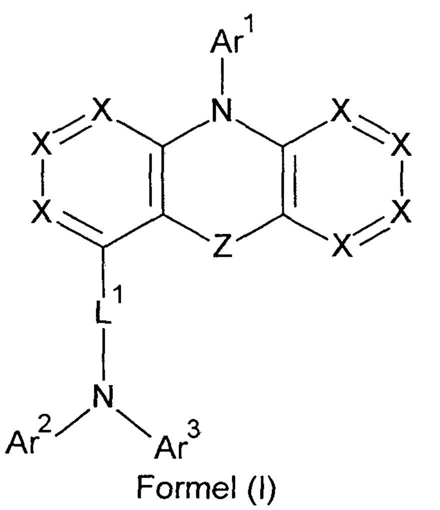

Formel (I)

wobei für die verwendeten Symbole gilt:

X ist bei jedem Auftreten gleich oder verschieden N oder $CR^1$, vorzugsweise $CR^1$, mit der Maßgabe, dass nicht mehr als zwei der Gruppen X in einem Cyclus für N stehen;
Z ist eine Bindung, $C(R^1)_2$, O oder S;
$L^1$ ist eine Bindung, ein aromatisches Ringsystem mit 6 bis 60 C-Atomen oder ein heteroaromatisches Ringsystem mit 3 bis 60 C-Atomen, welches jeweils durch einen oder mehrere Reste $R^1$ substituiert sein kann;
$Ar^1$, $Ar^2$, $Ar^3$ ist eine Arylgruppe mit 6 bis 40 C-Atomen oder eine Heteroarylgruppe mit 3 bis 40 C-Atomen, welche jeweils durch einen oder mehrere Reste $R^1$ substituiert sein kann;
$R^1$ ist bei jedem Auftreten gleich oder verschieden H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, $-C{\equiv}C-$, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden;
$R^2$ ist bei jedem Auftreten gleich oder verschieden H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, $-C{\equiv}C-$, $Si(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;
$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

mit der Maßgabe, dass
mindestens einer der Reste $Ar^2$ und/oder $Ar^3$ eine Gruppe der Formel (IIa) oder (IIb) darstellt

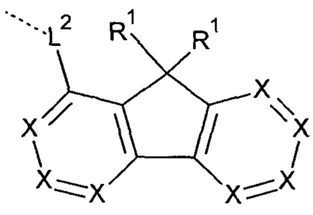

Formel (IIa)

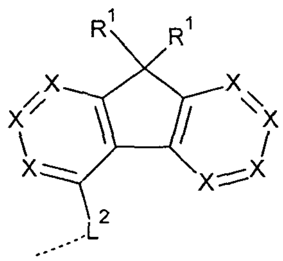

Formel (IIb)

worin

X bei jedem Auftreten gleich oder verschieden N oder $CR^1$ ist, vorzugsweise $CR^1$, mit der Maßgabe, dass nicht mehr als zwei der Gruppen X in einem Cyclus für N stehen;
$L^2$ eine Bindung, ein aromatisches Ringsystem mit 6 bis 60 C-Atomen oder ein heteroaromatisches Ringsystem mit 3 bis 60 C-Atomen ist, welches jeweils durch einen oder mehrere Reste $R^1$ substituiert sein kann;
$R^1$ die zuvor genannte Bedeutung hat und die gestrichelte Linie die Bindungsstelle darstellt, so dass $L^2$ an das gleiche Stickstoffatom bindet, wie $L^1$.

2. Verbindung gemäß Anspruch 1, **dadurch gekennzeichnet, dass** eine Struktur gemäß Formel (Ia) gebildet wird

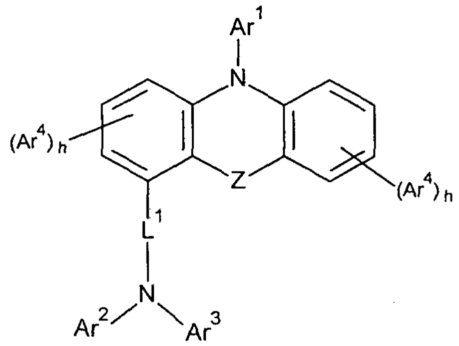

Formel (Ia)

worin

h bei jedem Auftreten unabhängig 0, 1, 2, 3 oder 4, vorzugsweise 0, 1 oder 2 und besonders bevorzugt 0 oder 1 ist;
$Ar^4$ eine Arylgruppe mit 6 bis 40 C-Atomen oder eine Heteroarylgruppe mit 3 bis 40 C-Atomen, welche jeweils durch einen oder mehrere Reste $R^1$ substituiert sein kann, und
die Symbole $Ar^1$, $Ar^2$, $Ar^3$, $L^1$ und Z die in Anspruch 1 genannte Bedeutung haben.

3. Verbindung gemäß Anspruch 1 oder 2, **dadurch gekennzeichnet, dass** die Gruppe Z für eine Bindung steht, so dass eine Struktur der Formel (Ib) oder (Ic) gebildet wird

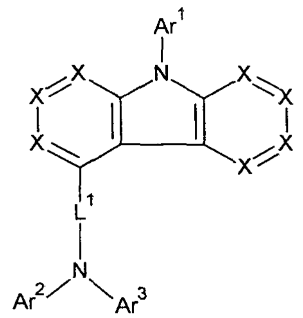

Formel (Ib)

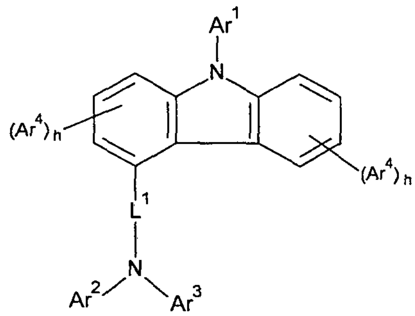

Formel (Ic)

worin die Symbole die in Anspruch 1 oder 2 dargelegte Bedeutung haben.

4. Verbindungen gemäß einem oder mehreren der Ansprüche 1 bis 3, **dadurch gekennzeichnet, dass** das die beiden Reste $R^1$, die an das C-Atom in Position 9 in der Fluorenstruktur gemäß Formel (IIa) oder (IIb) gebunden sind, jeweils ein aromatisches oder heteroaromatisches Ringsystem mit 5 bis 30 aromatischen Ringatomen, das mit einem oder mehreren Resten $R^1$ substituiert sein kann darstellen, wobei die zwei Ringsysteme miteinander verbunden sind.

5. Verbindungen gemäß Anspruch 4, **dadurch gekennzeichnet, dass** mindestens einer der Reste $Ar^2$ und/oder $Ar^3$ eine Gruppe der Formel (IIc) oder (IId) umfasst

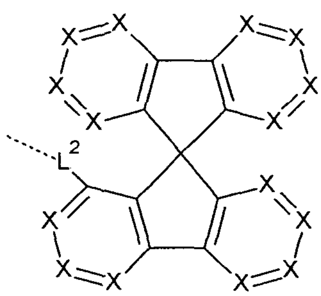

Formel (IIc)

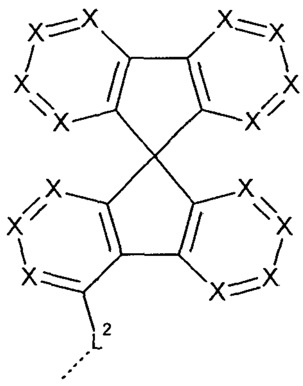

Formel (IId)

worin die Symbole die in Anspruch 1 dargelegte Bedeutung haben.

**6.** Verbindungen gemäß einem oder mehreren der Ansprüche 1 bis 3, **dadurch gekennzeichnet, dass** das die beiden Reste $R^1$, die an das C-Atom in Position 9 in der Fluorenstruktur gemäß Formel (IIa) oder (IIb) gebunden sind, jeweils ein aromatisches oder heteroaromatisches Ringsystem mit 5 bis 30 aromatischen Ringatomen, das mit einem oder mehreren Resten $R^1$ substituiert sein kann darstellen, wobei die zwei Ringsysteme nicht miteinander verbunden sind, oder eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C≡C-, $Si(R^2)_2$, C=O, C=S,
$C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können.

**7.** Verbindungen gemäß einem oder mehreren der Ansprüche 1 bis 3 oder 6, **dadurch gekennzeichnet, dass** mindestens einer der Reste $Ar^2$ und/oder $Ar^3$ eine Gruppe der Formel (IIe) oder (IIf) umfasst

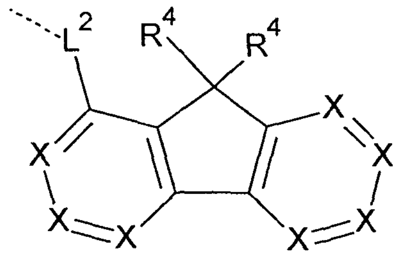

Formel (IIe)

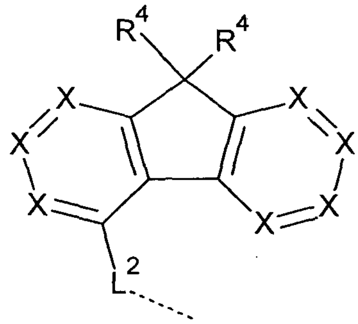

Formel (IIf)

worin die Symbole X und $L^2$ die in Anspruch 1 dargelegte Bedeutung haben und $R^4$ für H, ein aromatisches Ringsystem mit 6 bis 30 aromatischen Ringatomen, vorzugsweise eine Arylgruppe mit 6 bis 20 Kohlenstoffatomen, besonders bevorzugt Phenyl, Biphenyl, oder Naphthyl, oder eine Alkylgruppe mit 1 bis 20 Kohlenstoffatomen, vorzugsweise Methyl, Ethyl, Propyl oder Butyl, besonders bevorzugt Methyl steht.

**8.** Verbindungen gemäß einem oder mehreren der Ansprüche 1 bis 7, **dadurch gekennzeichnet, dass** in Formeln (I), (Ib), (IIa), (IIb), (IIc), (IId), (IIe) und Formel (IIf) nicht mehr als zwei, vorzugsweise nicht mehr als eine Gruppe X für N steht, vorzugsweise alle X für $CR^1$ stehen, wobei vorzugsweise höchstens 4, besonders bevorzugt höchstens 3 und speziell bevorzugt höchstens 2 der Gruppen $CR^1$ für die X steht, ungleich der Gruppe CH ist.

**9.** Verbindungen gemäß einem oder mehreren der Ansprüche 1 bis 8, **dadurch gekennzeichnet, dass** in den Strukturen gemäß Formeln (I), (Ib), (IIa), (IIb), (IIc), (IId), (IIe) oder Formel (IIf) mindestens ein Rest $R^1$, $Ar^2$, $Ar^3$ oder $Ar^4$ für eine Gruppe steht, die ausgewählt ist aus den Formeln ($R^1$-1) bis ($R^1$-72)

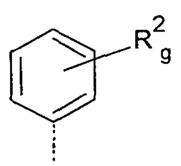

Formel ($R^1$-1)

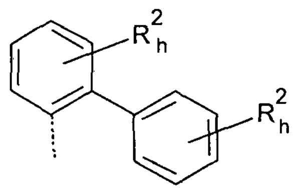

Formel ($R^1$-2)

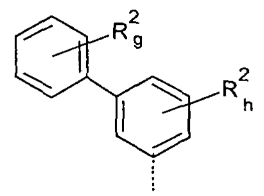

Formel ($R^1$-3)

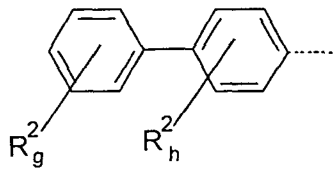

Formel ($R^1$-4)

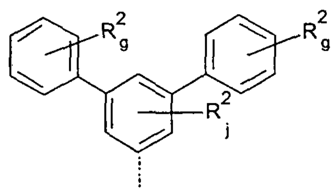

Formel ($R^1$-5)

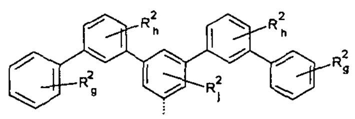

Formel ($R^1$-6)

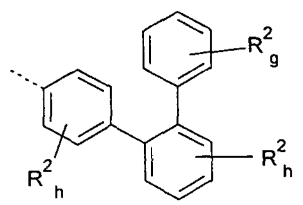

Formel ($R^1$-7)

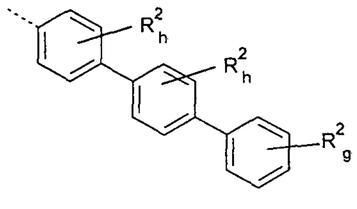

Formel ($R^1$-8)

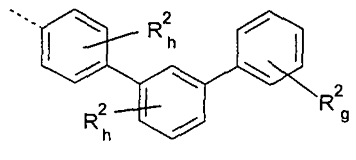

Formel ($R^1$-9)

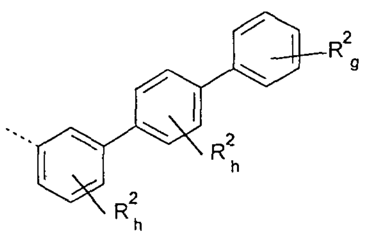

Formel ($R^1$-10)

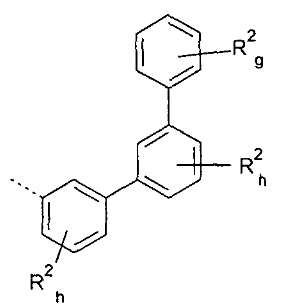

Formel ($R^1$-11)

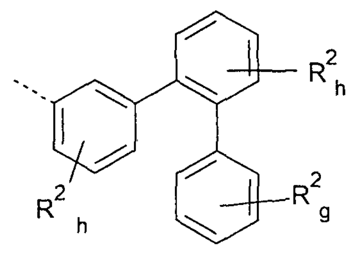

Formel ($R^1$-12)

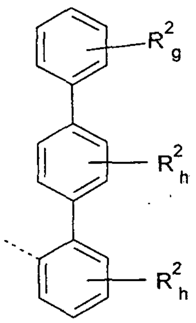

Formel ($R^1$-13)

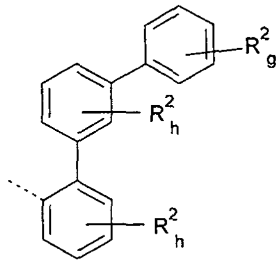

Formel ($R^1$-14)

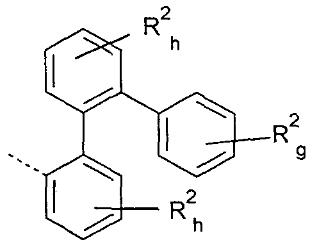

Formel ($R^1$-15)

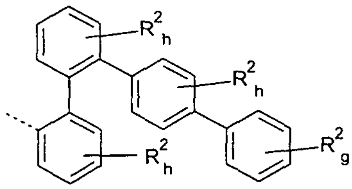

Formel ($R^1$-16)

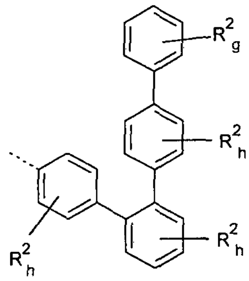

Formel ($R^1$-17)

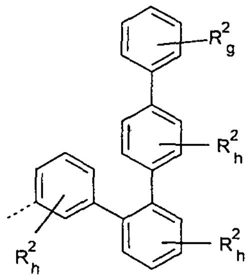

Formel ($R^1$-18)

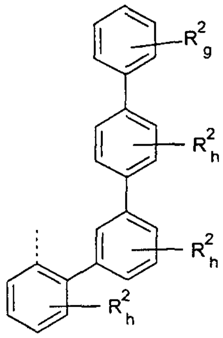

Formel ($R^1$-19)

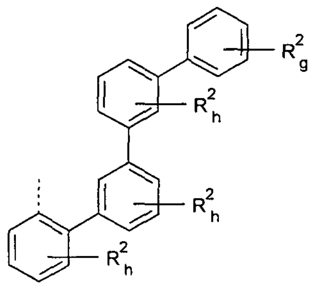

Formel ($R^1$-20)

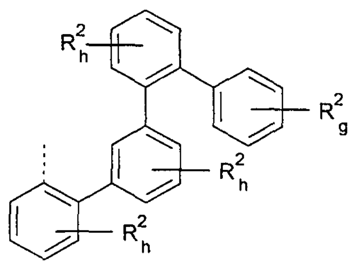

Formel ($R^1$-21)

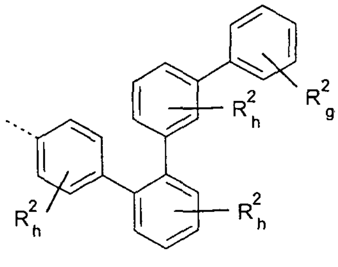

Formel ($R^1$-22)

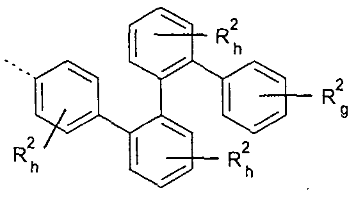

Formel ($R^1$-23)

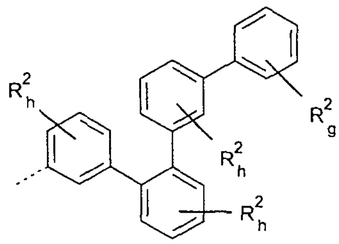

Formel ($R^1$-24)

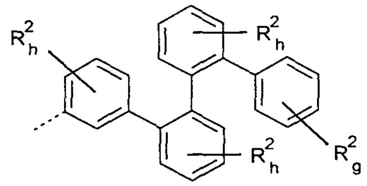

Formel ($R^1$-25)

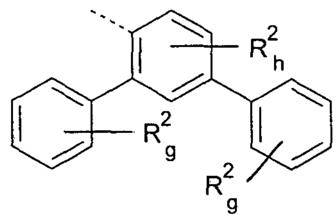

Formel ($R^1$-26)

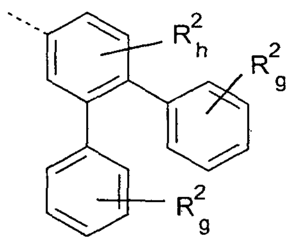

Formel ($R^1$-27)

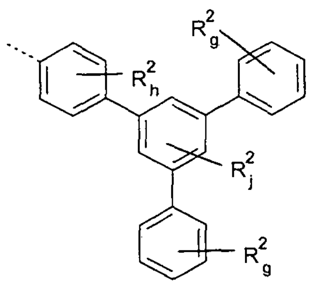

Formel ($R^1$-28)

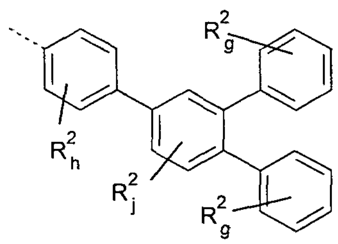

Formel ($R^1$-29)

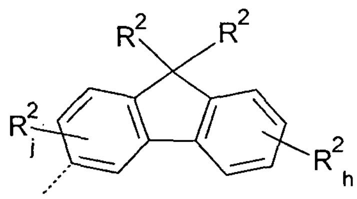

Formel ($R^1$-30)

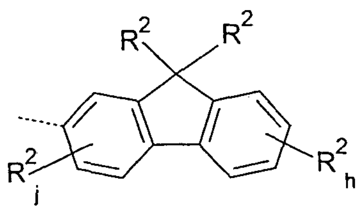

Formel ($R^1$-31)

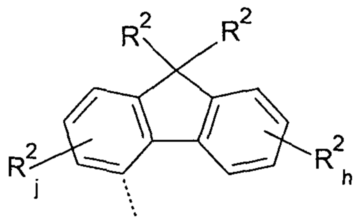

Formel ($R^1$-32)

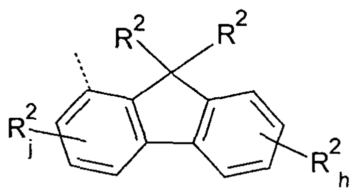

Formel ($R^1$-33)

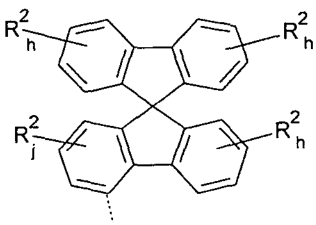

Formel ($R^1$-34)

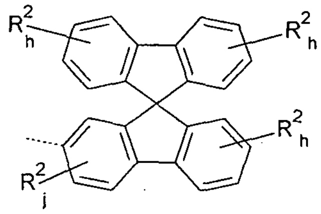

Formel ($R^1$-35)

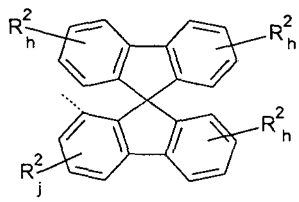

Formel ($R^1$-36)

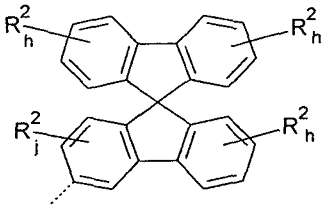

Formel ($R^1$-37)

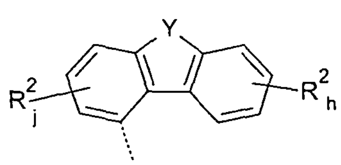

Formel ($R^1$-38)

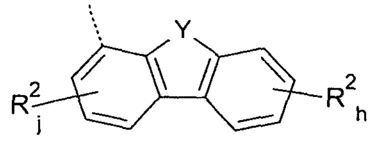

Formel ($R^1$-39)

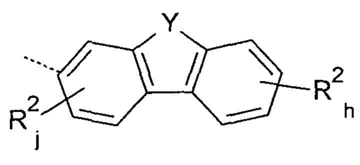

Formel ($R^1$-40)

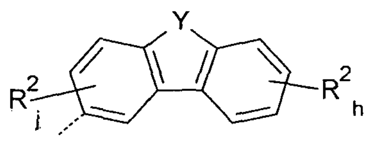

Formel ($R^1$-41)

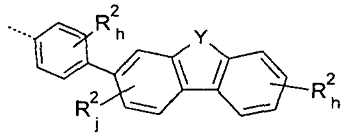

Formel ($R^1$-42)

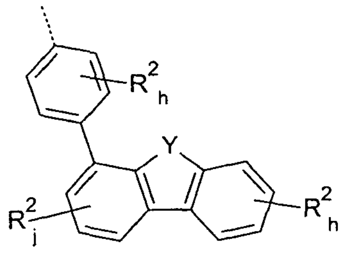

Formel ($R^1$-43)

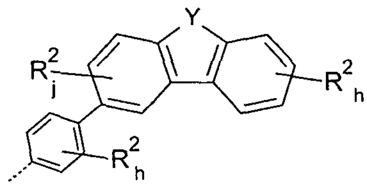

Formel ($R^1$-44)

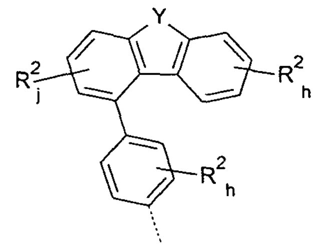

Formel ($R^1$-45)

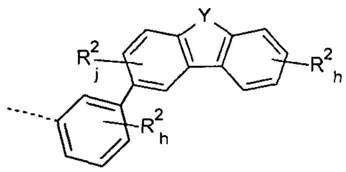

Formel ($R^1$-46)

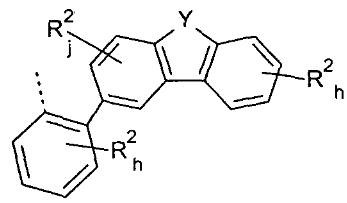

Formel ($R^1$-47)

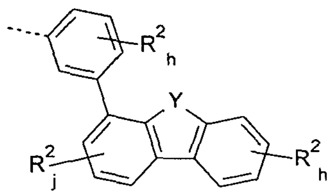

Formel ($R^1$-48)

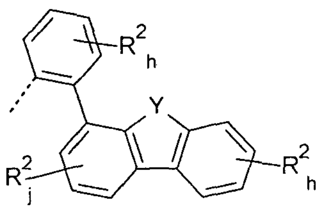

Formel ($R^1$-49)

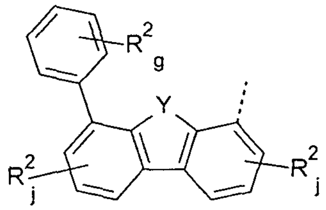

Formel ($R^1$-50)

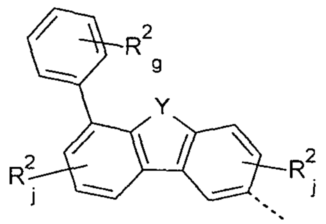

Formel ($R^1$-51)

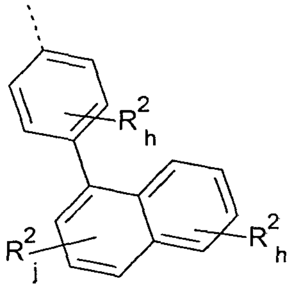

Formel ($R^1$-52)

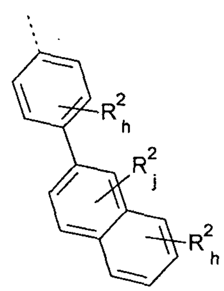

Formel ($R^1$-53)

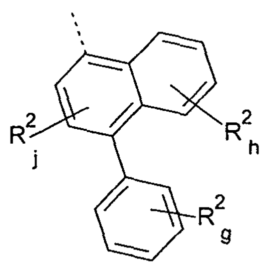

Formel ($R^1$-54)

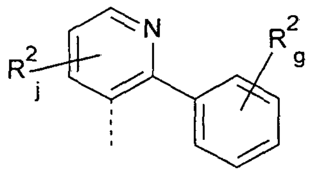
Formel ($R^1$-55)

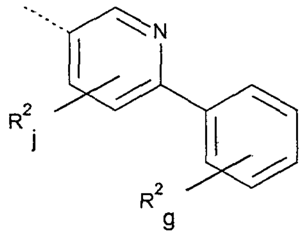
Formel ($R^1$-56)

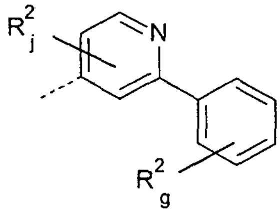
Formel ($R^1$-57)

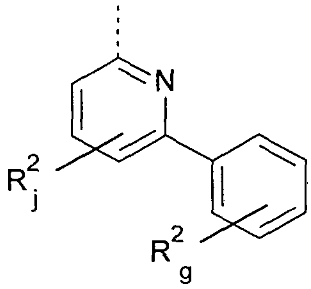
Formel ($R^1$-58)

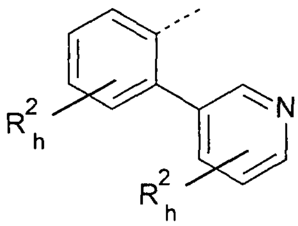
Formel ($R^1$-59)

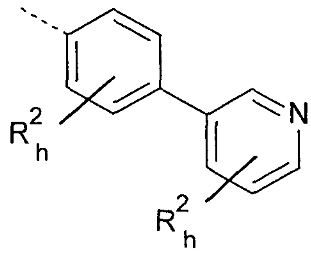
Formel ($R^1$-60)

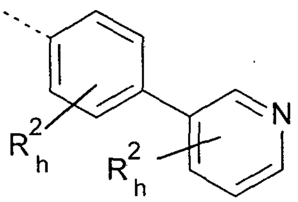
Formel ($R^1$-61)

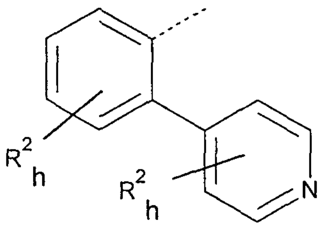
Formel ($R^1$-62)

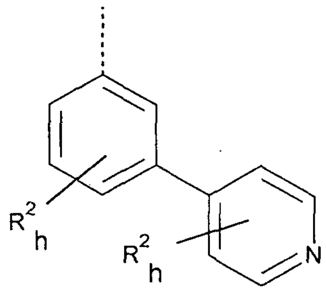
Formel ($R^1$-63)

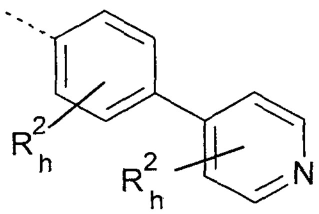
Formel ($R^1$-64)

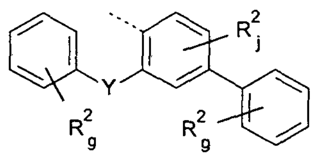
Formel ($R^1$-65)

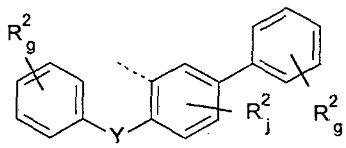
Formel ($R^1$-66)

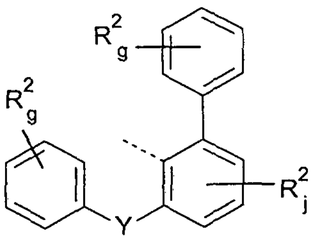
Formel ($R^1$-67)

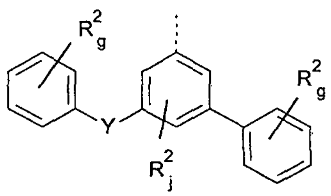
Formel ($R^1$-68)

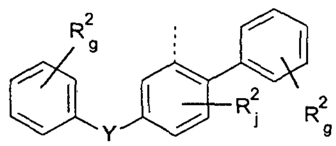
Formel ($R^1$-69)

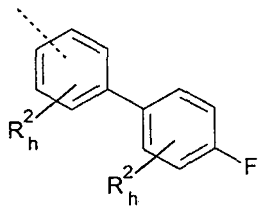

Formel ($R^1$-70)

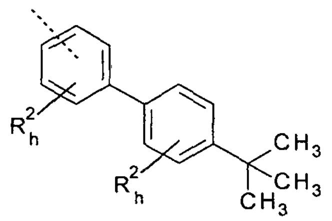

Formel ($R^1$-71)

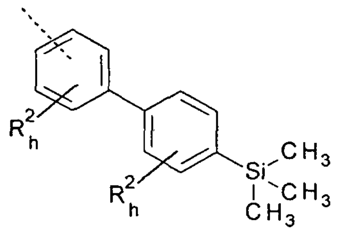

Formel ($R^1$-72)

wobei für die verwendeten Symbole gilt:

Y ist O, S oder $NR^2$, vorzugsweise O oder S;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5:

die gestrichelte Bindung markiert die Anbindungsposition; und

$R^2$ weist die in Anspruch 1 genannte Bedeutung auf

**10.** Verbindungen gemäß Anspruch 9, **dadurch gekennzeichnet, dass** die Summe der Indices g, h und j in den Strukturen der Formel ($R^1$-1) bis ($R^1$-72) jeweils höchstens 3, vorzugsweise höchstens 2 und besonders bevorzugt höchstens 1 beträgt.

**11.** Verbindungen gemäß einem oder mehreren der Ansprüche 1 bis 10, **dadurch gekennzeichnet, dass** in den Struktur gemäß Formeln (I), (IIa) und/oder (IIb) mindestens eine Gruppe $L^1$ und/oder $L^2$ für eine Gruppe steht, die ausgewählt ist aus den Formeln (L-1) bis (L- 78)

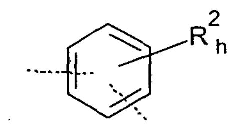

Formel (L-1)

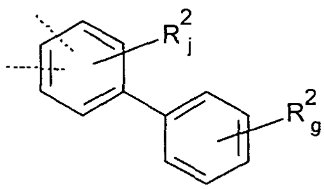

Formel (L-2)

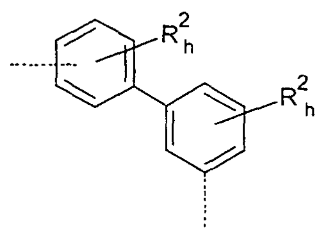

Formel (L-3)

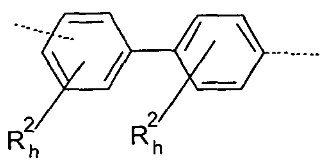

Formel (L-4)

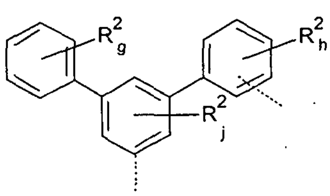

Formel (L-5)

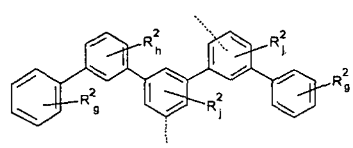

Formel (L-6)

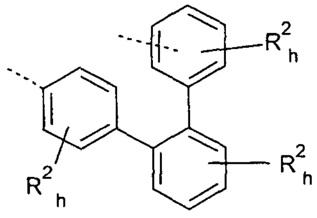

Formel (L-7)

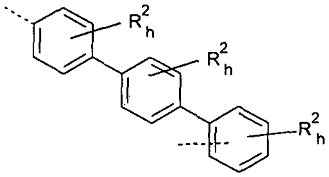

Formel (L-8)

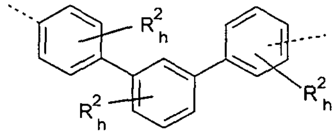

Formel (L-9)

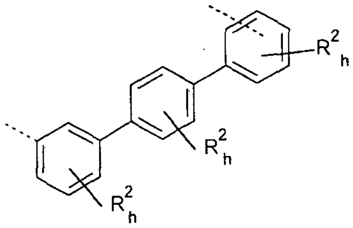

Formel (L-10)

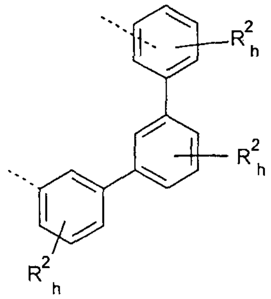

Formel (L-11)

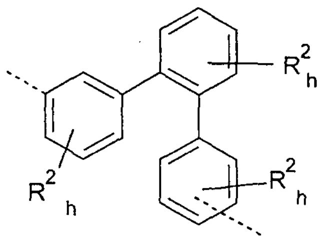

Formel (L-12)

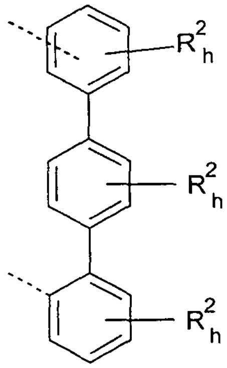

Formel (L-13)

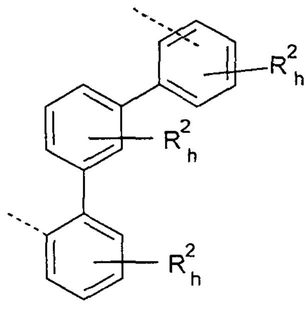

Formel (L-14)

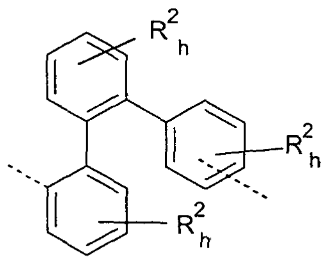

Formel (L-15)

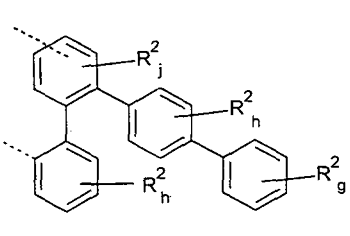

Formel (L-16)

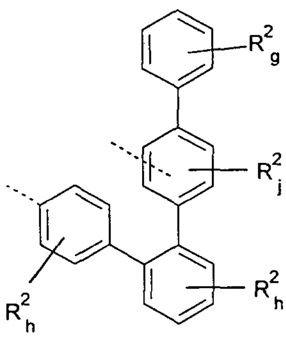

Formel (L-17)

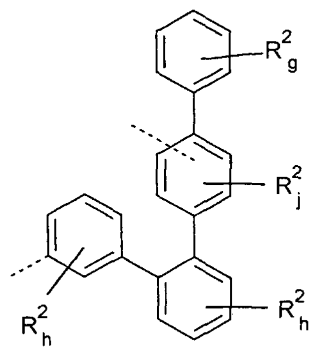

Formel (L-18)

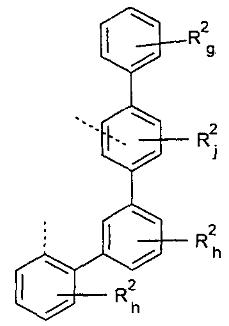

Formel (L-19)

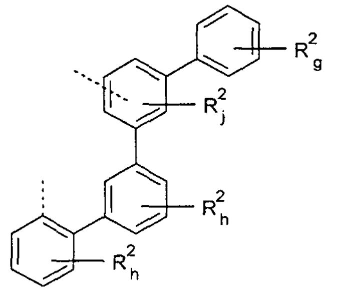

Formel (L-20)

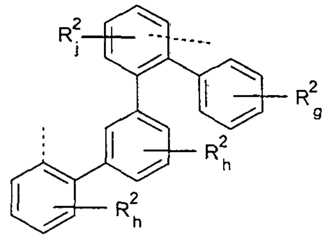

Formel (L-21)

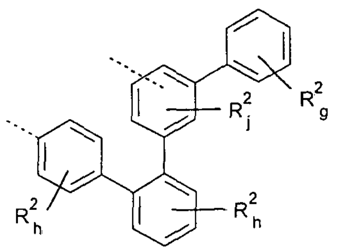

Formel (L-22)

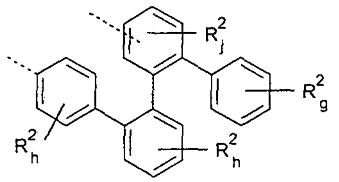

Formel (L-23)

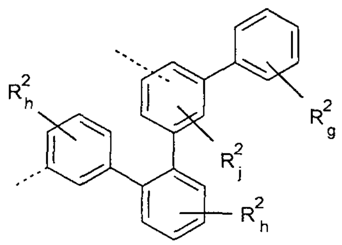

Formel (L-24)

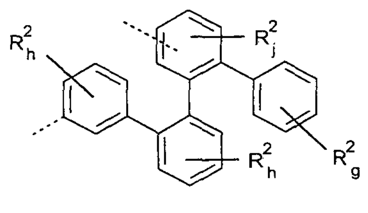

Formel (L-25)

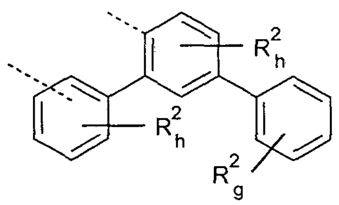

Formel (L-26)

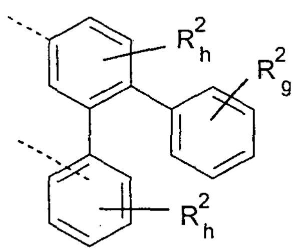

Formel (L-27)

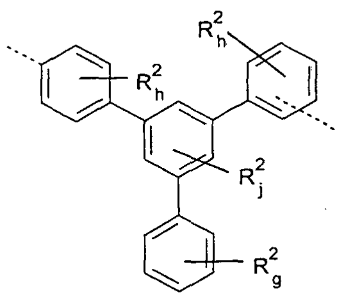

Formel (L-28)

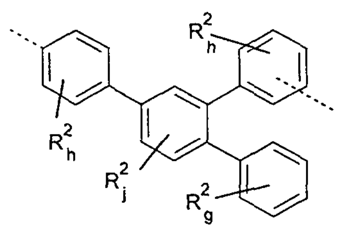

Formel (L-29)

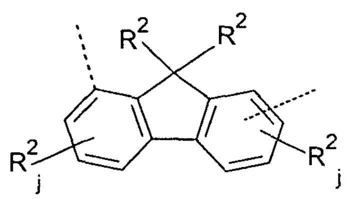

Formel (L-30)

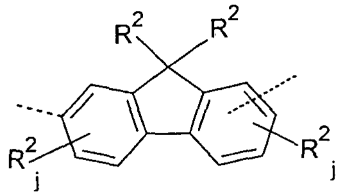

Formel (L-31)

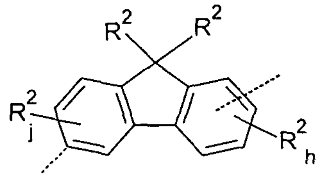

Formel (L-32)

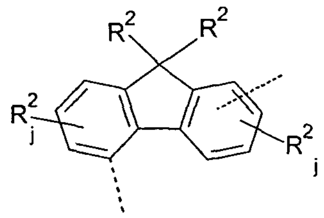

Formel (L-33)

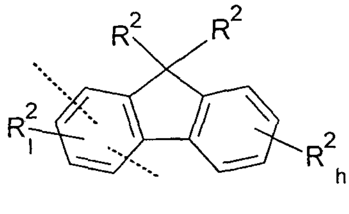

Formel (L-34)

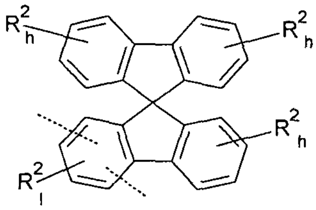

Formel (L-35)

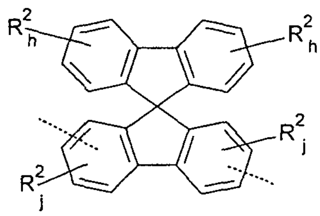

Formel (L-36)

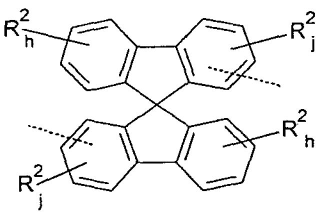

Formel (L-37)

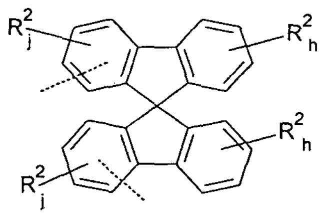

Formel (L-38)

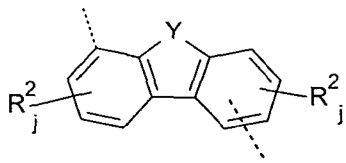

Formel (L-39)

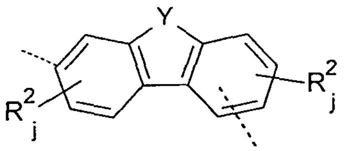

Formel (L-40)

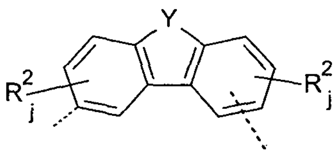

Formel (L-41)

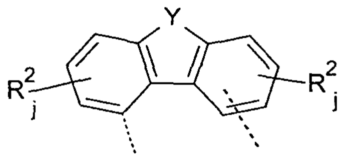

Formel (L-42)

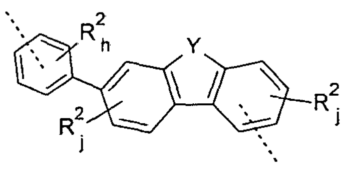

Formel (L-43)

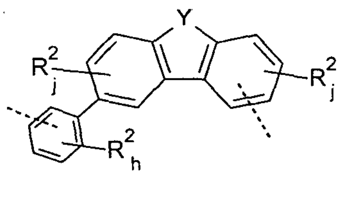

Formel (L-44)

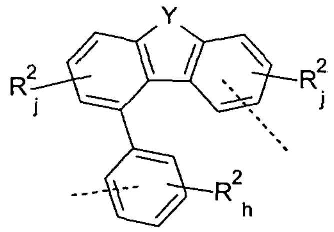

Formel (L-45)

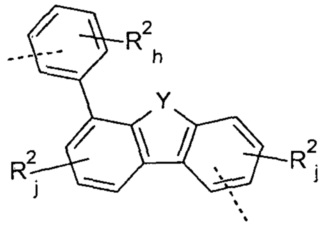

Formel (L-46)

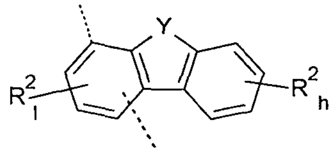

Formel (L-47)

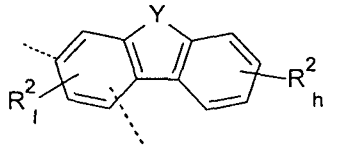

Formel (L-48)

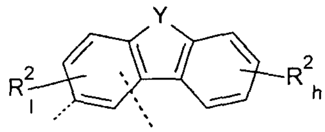

Formel (L-49)

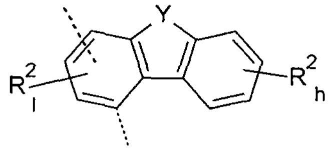

Formel (L-50)

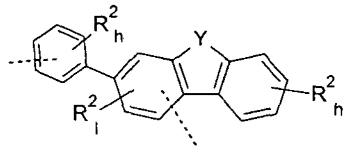

Formel (L-51)

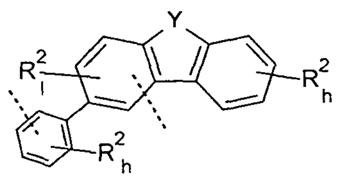

Formel (L-52)

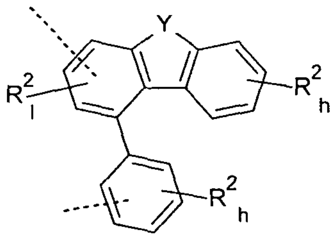

Formel (L-53)

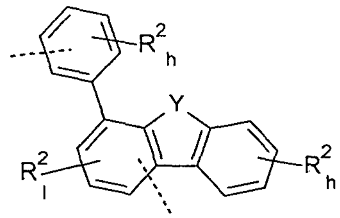

Formel (L-54)

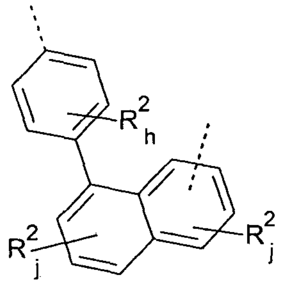

Formel (L-55)

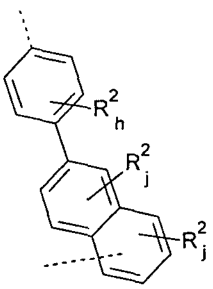

Formel (L-56)

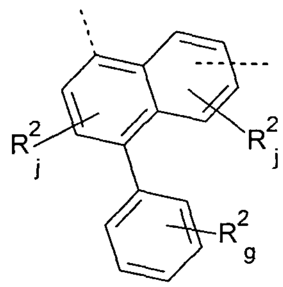

Formel (L-57)

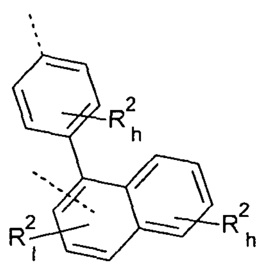

Formel (L-58)

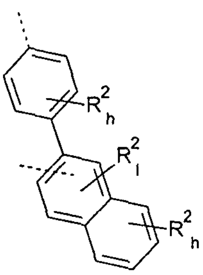

Formel (L-59)

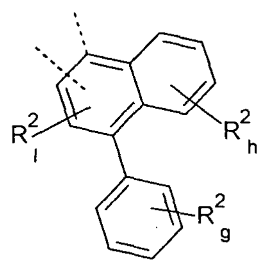

Formel (L-60)

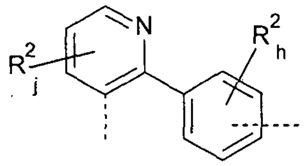

Formel (L-61)

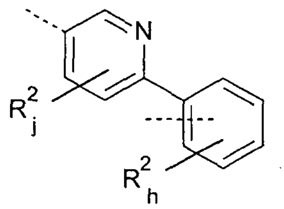

Formel (L-62)

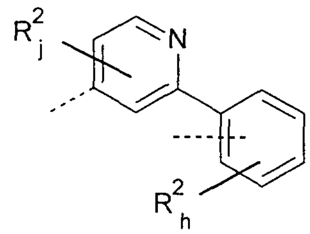

Formel (L-63)

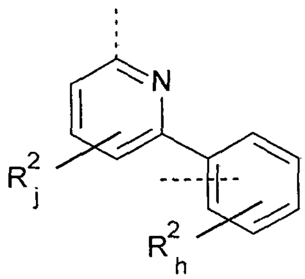

Formel (L-64)

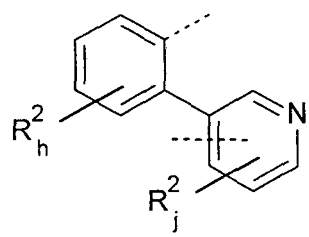

Formel (L-65)

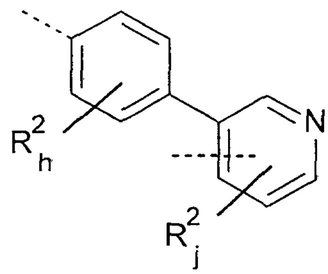

Formel (L-66)

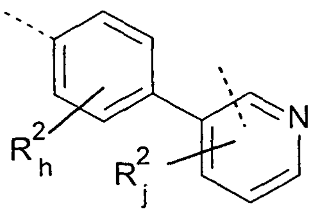

Formel (L-67)

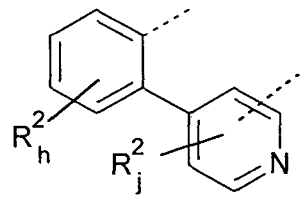

Formel (L-68)

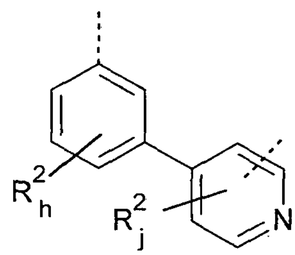

Formel (L-69)

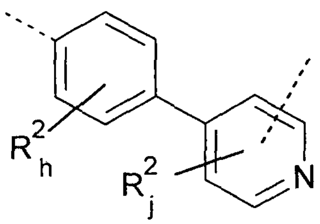

Formel (L-70)

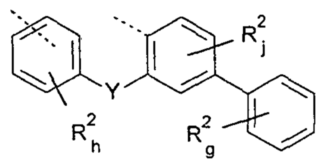

Formel (L-71)

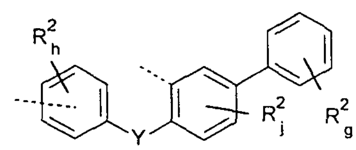

Formel (L-72)

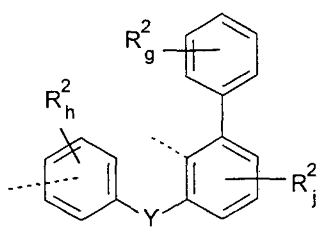

Formel (L-73)

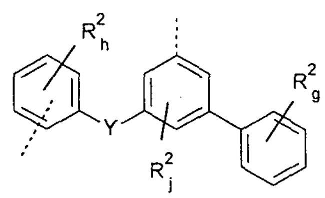

Formel (L-74)

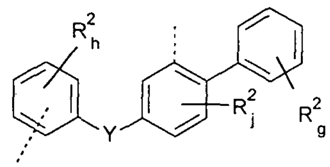

Formel (L-75)

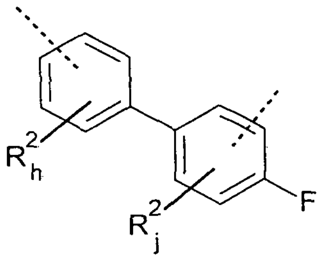

Formel (L-76)

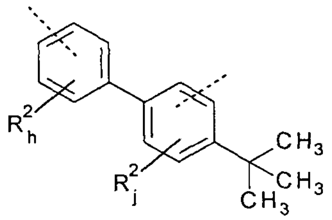

Formel (L-77)

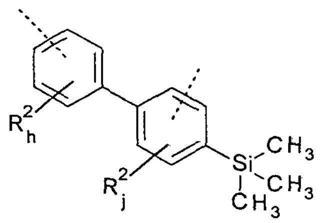

Formel (L-78)

wobei die gestrichelten Bindungen jeweils die Anbindungspositionen markieren, der Index l 0, 1 oder 2 ist, der Index g 0, 1, 2, 3, 4 oder 5 ist, j bei jedem Auftreten unabhängig 0, 1, 2 oder 3 ist; h bei jedem Auftreten unabhängig 0, 1, 2, 3 oder 4 ist; Y O, S oder $NR^2$, vorzugsweise O oder S ist; und $R^2$ die in Anspruch 1 genannte Bedeutung aufweist.

12. Verbindungen gemäß Anspruch 11, **dadurch gekennzeichnet, dass** die Summe der Indices l, g, h und j in den Strukturen der Formel (L-1) bis (L-78) jeweils höchstens 3, vorzugsweise höchstens 2 und besonders bevorzugt höchstens 1 beträgt.

13. Verbindungen gemäß Anspruch 11 oder 12, **dadurch gekennzeichnet, dass** in Struktur gemäß Formel (I) die Gruppe $L^1$ für eine Bindung und in Formel (IIa) und/oder (IIb) die Gruppe $L^2$ für eine Gruppe steht, die ausgewählt ist aus den Formeln (L-1) bis (L- 78), vorzugsweise aus den Formeln (L-1) bis (L- 5), wie in Anspruch 11 beschrieben.

14. Verbindungen gemäß Anspruch 11 oder 12, **dadurch gekennzeichnet, dass** in Struktur gemäß Formel (IIa) und/oder (IIb) die Gruppe $L^2$ für eine Bindung steht und in Formel (I) die Gruppe $L^1$ für eine Gruppe steht, die ausgewählt ist aus den Formeln (L-1) bis (L- 78), vorzugsweise aus den Formeln (L-1) bis (L- 5), wie in Anspruch 11 beschrieben.

15. Verbindungen gemäß einem oder mehreren der Ansprüche 1 bis 10, **dadurch gekennzeichnet, dass** in Struktur gemäß Formel (I), (IIa) und/oder (IIb) die Gruppen $L^1$ und $L^2$ für eine Bindung stehen.

16. Verbindungen gemäß einem oder mehreren der Ansprüche 1 bis 10, **dadurch gekennzeichnet, dass** in Struktur gemäß Formel (I), (IIa) und/oder (IIb) die Gruppen $L^1$ und $L^2$ für eine Gruppe stehen, die ausgewählt ist aus den Formeln (L-1) bis (L- 78), vorzugsweise aus den Formeln (L-1) bis (L- 5), wie in Anspruch 11 beschrieben.

17. Verbindungen gemäß einem oder mehreren der Ansprüche 1 bis 16, **dadurch gekennzeichnet, dass** $Ar^1$ und $Ar^3$ ein aromatisches Ringsystem mit 6 bis 12 aromatischen Ringatomen darstellt, welches mit einem oder mehreren Resten $R^1$ substituiert sein kann.

18. Oligomer, Polymer oder Dendrimer enthaltend eine oder mehrere Verbindungen gemäß einem oder mehreren der Ansprüche 1 bis 17, wobei ein oder mehrere Bindungen der Verbindung zum Polymer, Oligomer oder Dendrimer vorhanden sind.

19. Zusammensetzung enthaltend wenigstens eine Verbindung gemäß einem oder mehreren der Ansprüche 1 bis 17 und/oder ein Oligomer, Polymer oder Dendrimer gemäß Anspruch 18 und wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

20. Formulierung enthaltend mindestens eine Verbindung gemäß einem oder mehreren der Ansprüche 1 bis 17, ein Oligomer, Polymer oder Dendrimer gemäß Anspruch 18 und/oder wenigstens eine Zusammensetzung nach Anspruch 19 und mindestens ein Lösungsmittel.

21. Verfahren zur Herstellung einer Verbindung gemäß einem oder mehreren der Ansprüche 1 bis 17 oder eines Oligomers, Polymers oder Dendrimers gemäß Anspruch 18, **dadurch gekennzeichnet, dass** in einer Kupplungsreaktion eine Gruppe, umfassend mindestens einen Carbazol-Rest, mit einer Gruppe, umfassend mindestens einen Fluoren-Rest, verbunden wird.

**22.** Verwendung einer Verbindung gemäß einem oder mehreren der Ansprüche 1 bis 17, eines Oligomers, Polymers oder Dendrimers gemäß Anspruch 18 oder einer Zusammensetzung nach Anspruch 19 in einer elektronischen Vorrichtung als Matrix-Material, Elektronenblockiermaterial, Lochinjektionsmaterial und/oder Lochtransportmaterial, bevorzugt als Matrix-Material.

**23.** Elektronische Vorrichtung, enthaltend mindestens eine Verbindung gemäß einem oder mehreren der Ansprüche 1 bis 17, ein Oligomer, Polymer oder Dendrimer gemäß Anspruch 18 oder eine Zusammensetzung gemäß Anspruch 19, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

**1.** Compound comprising structures of the formula (I)

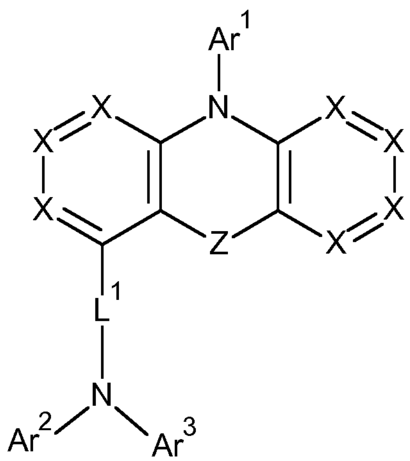

formula (I)

where the following applies to the symbols used:

X is on each occurence, identically or differently, N or $CR^1$, preferably $CR^1$, with the proviso that not more than two of the groups X in a ring stand for N;
Z is a bond, $C(R^1)_2$, O or S;
$L^1$ is a bond, an aromatic ring system having 6 to 60 C atoms or a heteroaromatic ring system having 3 to 60 C atoms, which may in each case be substituted by one or more radicals $R^1$;
$Ar^1$, $Ar^2$, $Ar^3$ are an aryl group having 6 to 40 C atoms or a heteroaryl group having 3 to 40 C atoms, which may in each case be substituted by one or more radicals $R^1$;
$R^1$ is on each occurence, identically or differently, H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^2$, where one or more non-adjacent $CH_2$ groups may be replaced by $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or a combination of these systems; two or more adjacent substituents $R^1$ may also form a mono- or polycyclic, aliphatic or aromatic ring system with one another;
$R^2$ is on each occurence, identically or differently, H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, a straight-

chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by $-R^3C=CR^3-$, -C≡C-, $Si(R^3)_2$, C=O, C=S, $C=NR^3$, -C(=O)O-, $-C(=O)NR^3-$, $NR^3$, $P(=O)(R^3)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals $R^3$, or a combination of these systems; two or more adjacent substituents $R^2$ may also form a mono- or polycyclic, aliphatic or aromatic ring system with one another;

$R^3$ is on each occurence, identically or differently, H, D, F or an aliphatic, aromatic and/or heteroaromatic hydrocarbon radical having 1 to 20 C atoms, in which, in addition, H atoms may be replaced by F; two or more adjacent substituents $R^3$ may also form a mono- or polycyclic, aliphatic or aromatic ring system with one another;

with the proviso that
at least one of the radicals $Ar^2$ and/or $Ar^3$ represents a group of the formula (IIa) or (IIb)

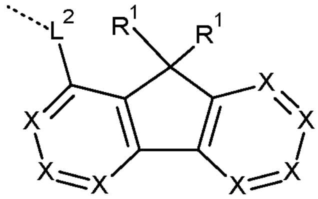

formula (IIa)

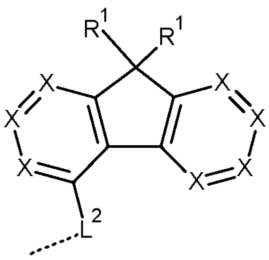

formula (IIb)

in which

X is on each occurence, identically or differently, N or $CR^1$, preferably $CR^1$, with the proviso that not more than two of the groups X in a ring stand for N;

$L^2$ is a bond, an aromatic ring system having 6 to 60 C atoms or a heteroaromatic ring system having 3 to 60 C atoms, which may in each case be substituted by one or more radicals $R^1$;

$R^1$ has the meaning given above and the dashed line represents the bonding site, so that $L^2$ is bonded to the same nitrogen atom as $L^1$.

2. Compound according to Claim 1, **characterised in that** a structure of the formula (Ia) is formed

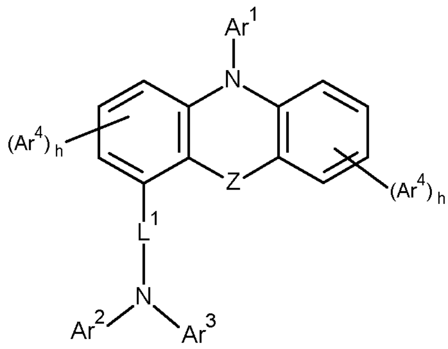

formula (Ia)

in which

h is on each occurrence, independently, 0, 1, 2, 3 or 4, preferably 0, 1 or 2 and particularly preferably 0 or 1;
$Ar^4$ is an aryl group having 6 to 40 C atoms or a heteroaryl group having 3 to 40 C atoms, which may in each case be substituted by one or more radicals $R^1$, and
the symbols $Ar^1$, $Ar^2$, $Ar^3$, $L^1$ and Z have the meaning given in Claim 1.

**3.** Compound according to Claim 1 or 2, **characterised in that** the group Z stands for a bond, so that a structure of the formula (Ib) or (Ic) is formed

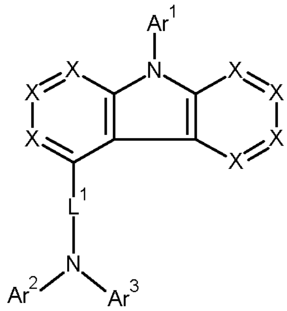

formula (Ib)

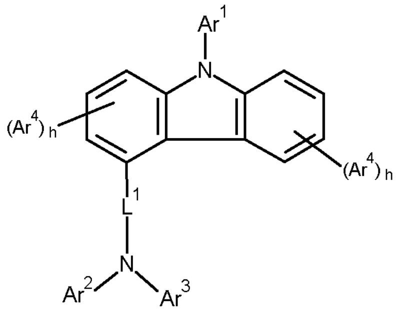

formula (Ic)

in which the symbols have the meaning indicated in Claim 1 or 2.

**4.** Compounds according to one or more of Claims 1 to 3, **characterised in that** the two radicals $R^1$ which are bonded to the C atom in position 9 in the fluorene structure of the formula (IIa) or (IIb) in each case represent an aromatic or heteroaromatic ring system having 5 to 30 aromatic ring atoms, which may be substituted by one or more radicals $R^1$, where the two ring systems are connected to one another.

**5.** Compounds according to Claim 4, **characterised in that** at least one of the radicals $Ar^2$ and/or $Ar^3$ comprises a

group of the formula (IIc) or (IId)

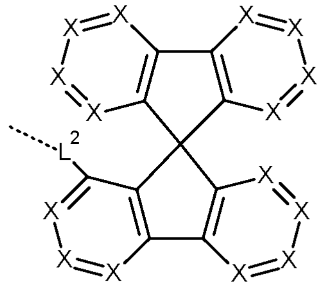

formula (IIc)

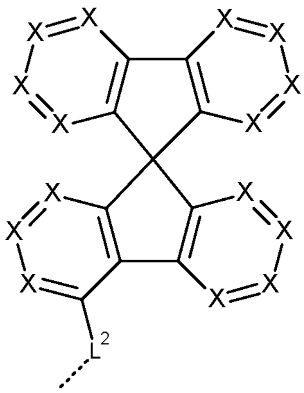

formula (IId)

in which the symbols have the meanng indicated in Claim 1.

**6.** Compounds according to one or more of Claims 1 to 3, **characterised in that** the two radicals $R^1$ which are bonded to the C atom in position 9 in the fluorene structure of the formula (IIa) or (IIb) in each case represent an aromatic or heteroaromatic ring system having 5 to 30 aromatic ring atoms, which may be substituted by one or more radicals $R^1$, where the two ring systems are not connected to one another, or a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^2$, where one or more non-adjacent $CH_2$ groups may be replaced by $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO or $SO_2$ and where one or more H atoms may be replaced by D, F, Cl, Br, I, CN or $NO_2$.

**7.** Compounds according to one or more of Claims 1 to 3 and 6, characterisaed in that at least one of the radicals $Ar^2$ and/or $Ar^3$ comprises a group of the formula (IIe) or (IIf)

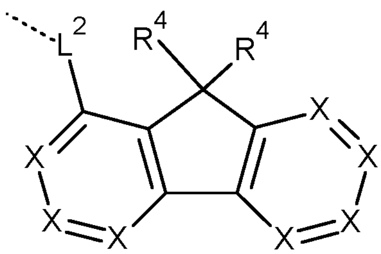

formula (IIe)

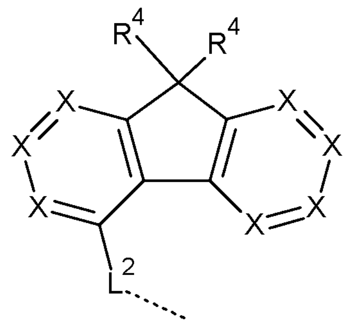

formula (IIf)

in which the symbols X and $L^2$ have the meaning indicated in Claim 1 and $R^4$ stands for H, an aromatic ring system having 6 to 30 aromatic ring atoms, preferably an aryl group having 6 to 20 carbon atoms, particularly preferably phenyl, biphenyl or naphthyl, or an alkyl group having 1 to 20 carbon atoms, preferably methyl, ethyl, propyl or butyl, particularly preferably methyl.

**8.** Compounds according to one or more of Claims 1 to 7, **characterised in that**, in the formulae (I), (Ib), (IIa), (IIb), (IIc), (IId), (IIe) and formula (IIf), not more than two, preferably not more than one group X stands for N, preferably all X stand for $CR^1$, where preferably at most 4, particularly preferably at most 3 and especially preferably at most 2 of the groups $CR^1$ for which X stands are not the CH group.

**9.** Compounds according to one or more of Claims 1 to 8, **characterised in that**, in the structures of the formulae (I), (Ib), (IIa), (IIb), (IIc), (IId), (IIe) or formula (IIf), at least one radical $R^1$, $Ar^2$, $Ar^3$ or $Ar^4$ stands for a group selected from the formulae ($R^1$-1) to ($R^1$-72)

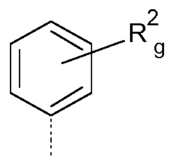

formula ($R^1$-1)

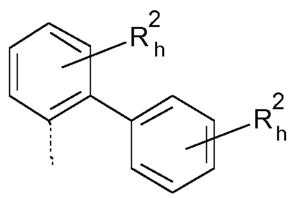

formula ($R^1$-2)

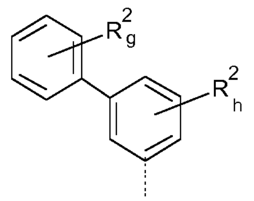

formula ($R^1$-3)

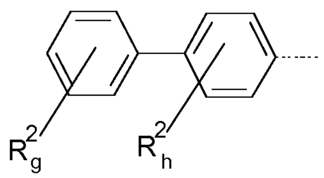

formula ($R^1$-4)

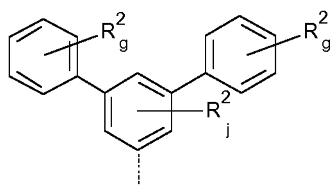

formula ($R^1$-5)

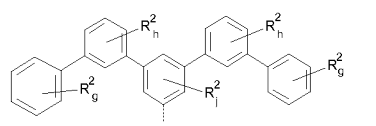

formula ($R^1$-6)

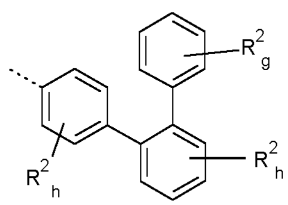

formula ($R^1$-7)

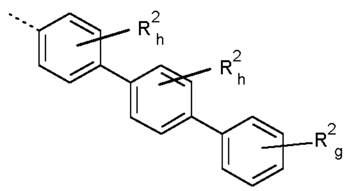

formula ($R^1$-8)

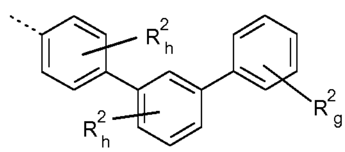

formula ($R^1$-9)

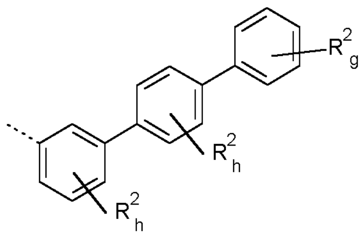

formula ($R^1$-10)

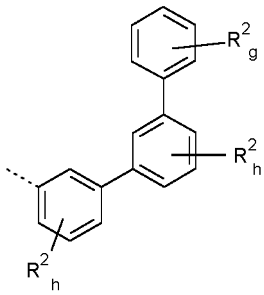

formula ($R^1$-11)

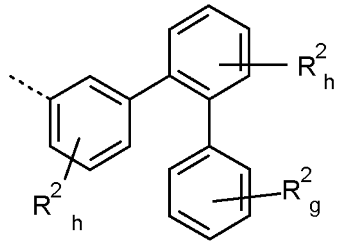

formula ($R^1$-12)

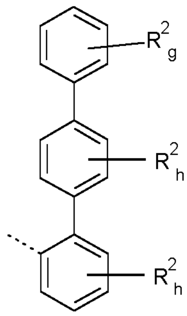

formula ($R^1$-13)

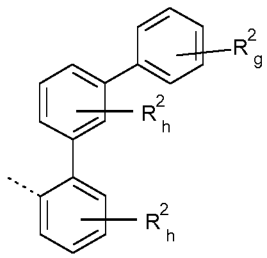

formula ($R^1$-14)

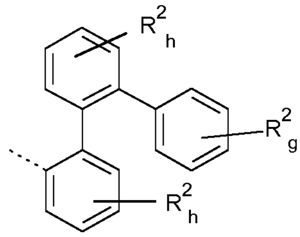

formula ($R^1$-15)

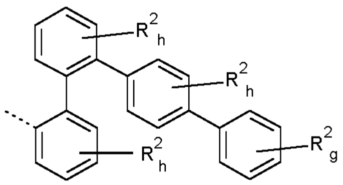

formula ($R^1$-16)

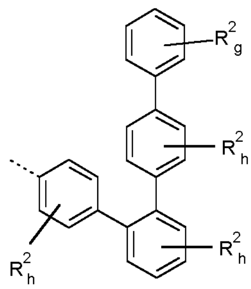

formula ($R^1$-17)

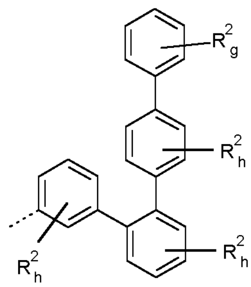

formula ($R^1$-18)

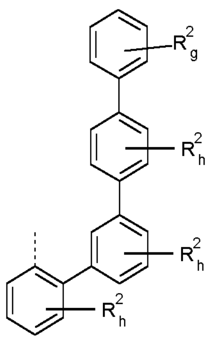

formula ($R^1$-19)

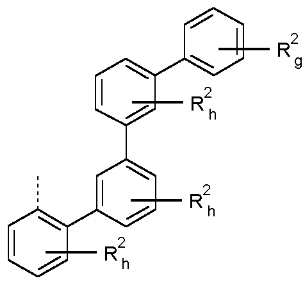

formula ($R^1$-20)

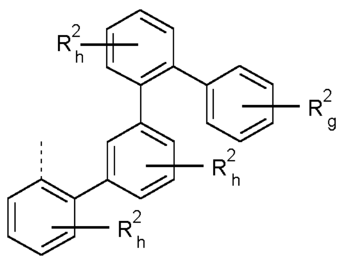

formula ($R^1$-21)

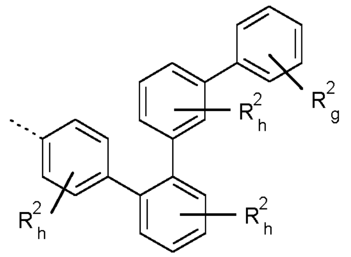

formula ($R^1$-22)

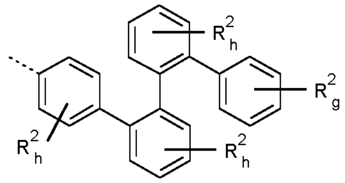

formula ($R^1$-23)

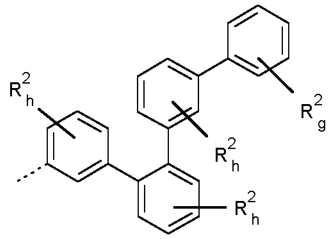

formula ($R^1$-24)

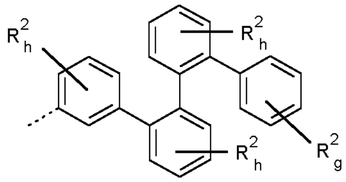

formula ($R^1$-25)

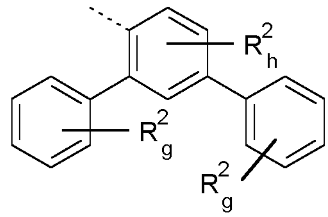

formula ($R^1$-26)

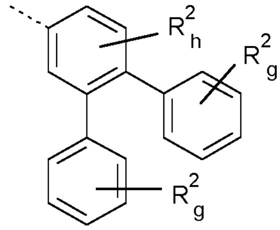

formula ($R^1$-27)

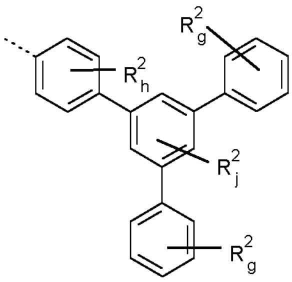

formula ($R^1$-28)

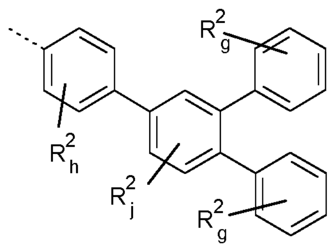

formula ($R^1$-29)

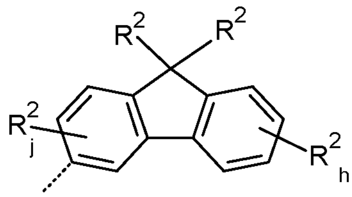

formula ($R^1$-30)

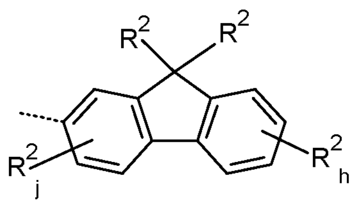

formula ($R^1$-31)

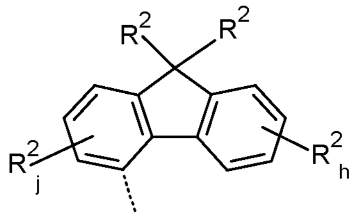

formula ($R^1$-32)

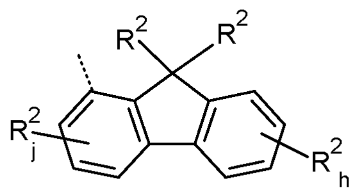

formula ($R^1$-33)

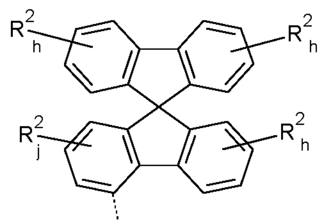

formula ($R^1$-34)

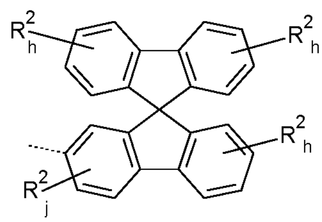

formula ($R^1$-35)

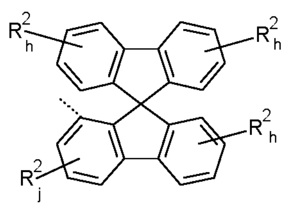

formula ($R^1$-36)

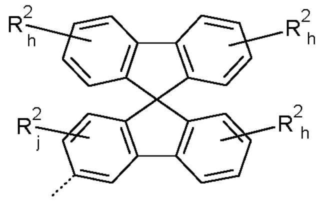

formula ($R^1$-37)

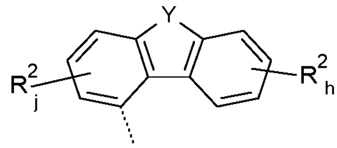

formula ($R^1$-38)

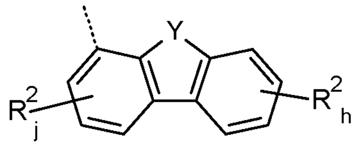

formula ($R^1$-39)

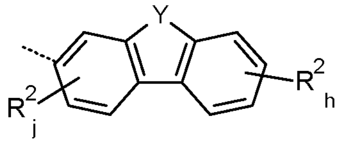

formula ($R^1$-40)

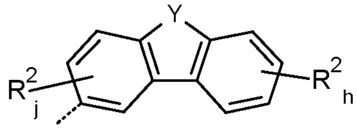

formula ($R^1$-41)

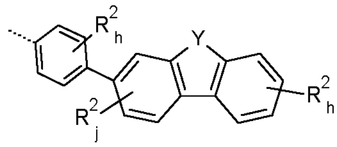

formula ($R^1$-42)

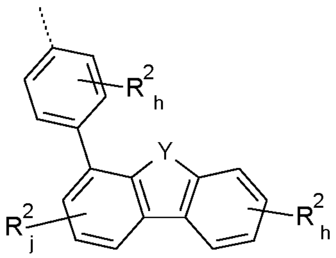

formula ($R^1$-43)

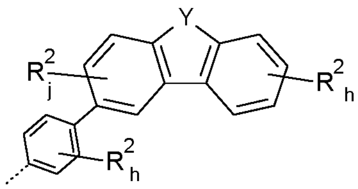

formula ($R^1$-44)

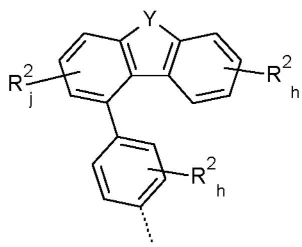

formula ($R^1$-45)

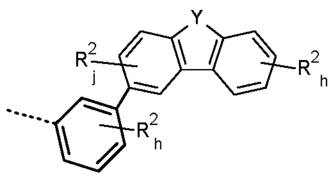

formula ($R^1$-46)

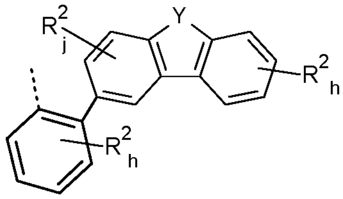

formula ($R^1$-47)

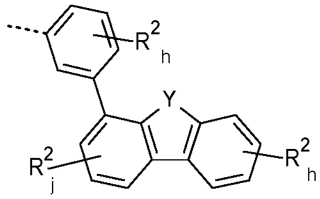

formula ($R^1$-48)

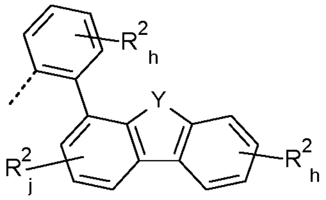

formula ($R^1$-49)

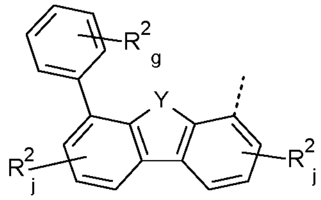

formula ($R^1$-50)

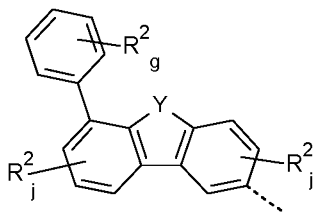

formula ($R^1$-51)

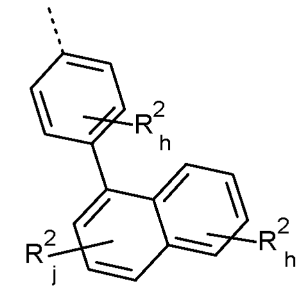

formula ($R^1$-52)

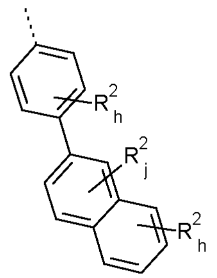

formula ($R^1$-53)

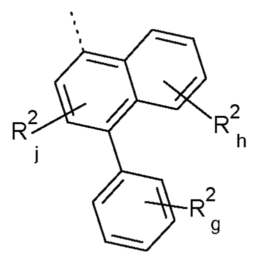

formula ($R^1$-54)

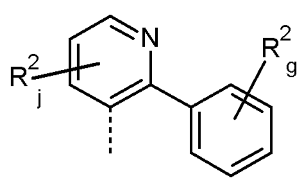

formula ($R^1$-55)

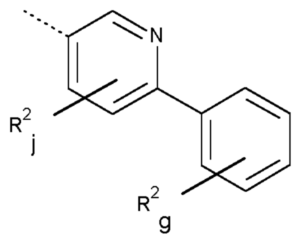

formula ($R^1$-56)

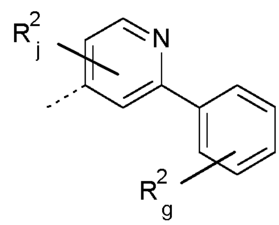

formula ($R^1$-57)

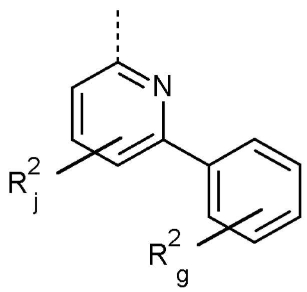

formula ($R^1$-58)

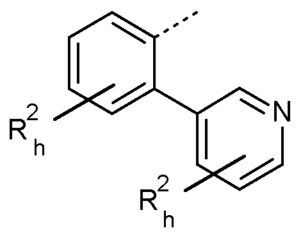

formula ($R^1$-59)

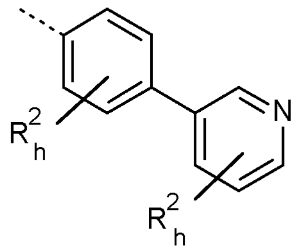

formula ($R^1$-60)

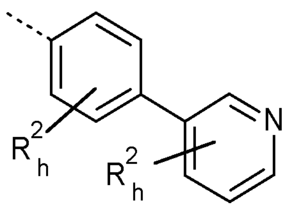

formula ($R^1$-61)

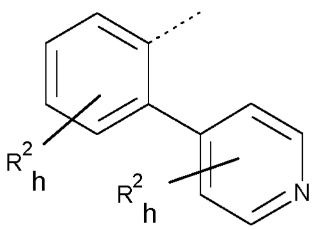

formula ($R^1$-62)

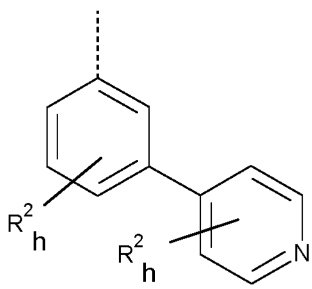

formula ($R^1$-63)

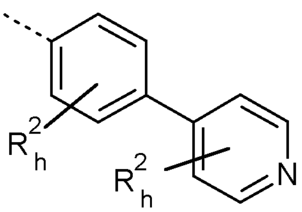

formula ($R^1$-64)

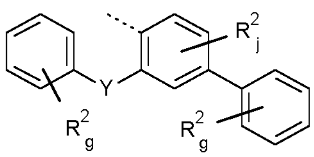

formula ($R^1$-65)

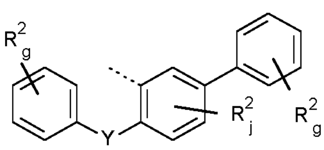

formula ($R^1$-66)

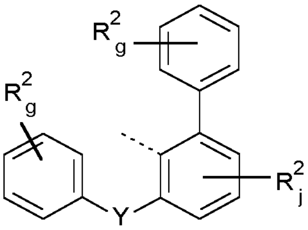

formula ($R^1$-67)

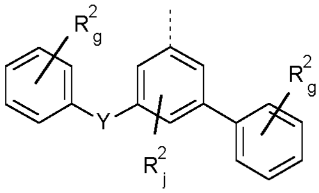

formula ($R^1$-68)

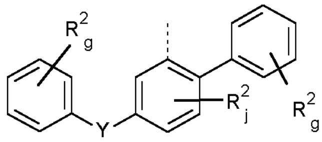

formula ($R^1$-69)

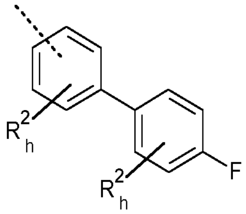

formula ($R^1$-70)

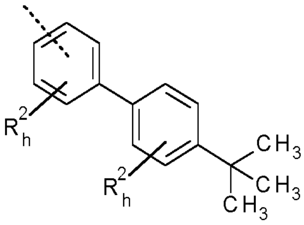

formula ($R^1$-71)

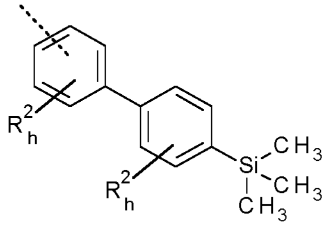

formula ($R^1$-72)

where the following applies to the symbols used:

Y is O, S or $NR^2$, preferably O or S;
j is on each occurence, independently, 0, 1, 2 or 3;
h is on each occurence, independently, 0, 1, 2, 3 or 4;
g is on each occurence, independently, 0, 1, 2, 3, 4 or 5;

the dashed bond marks the bonding position; and

$R^2$ has the meaning given in Claim 1.

**10.** Compounds according to Claim 9, **characterised in that** the sum of the indices g, h and j in the structures of the formulae ($R^1$-1) to ($R^1$-72) is in each case at most 3, preferably at most 2 and particularly preferably at most 1.

**11.** Compounds according to one or more of Claims 1 to 10, **characterised in that**, in the structures of the formulae (I), (IIa) and/or (IIb), at least one group $L^1$ and/or $L^2$ stands for a group selected from the formulae (L-1) to (L-78)

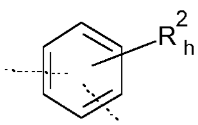

formula (L-1)

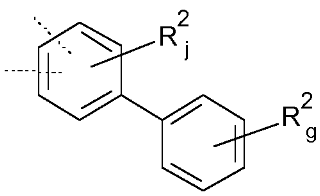

formula (L-2)

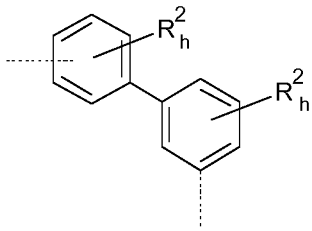

formula (L-3)

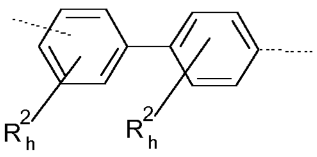

formula (L-4)

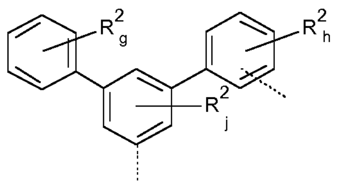

formula (L-5)

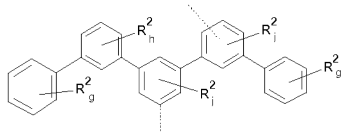

formula (L-6)

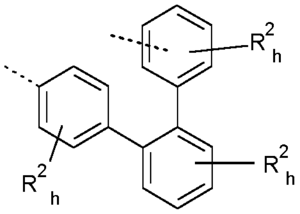

formula (L-7)

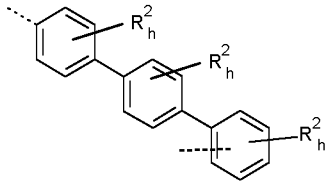

formula (L-8)

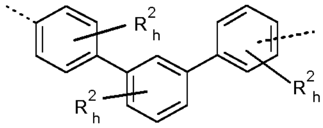

formula (L-9)

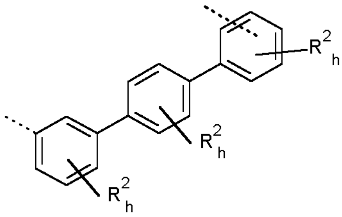

formula (L-10)

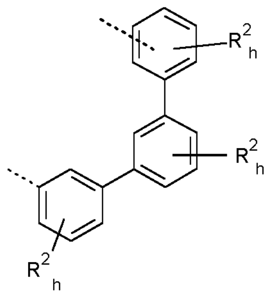

formula (L-11)

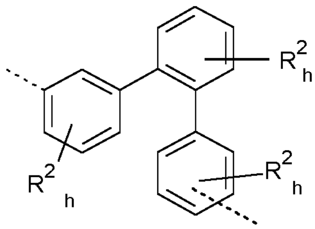

formula (L-12)

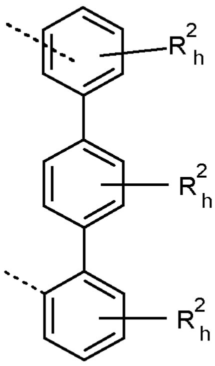

formula (L-13)

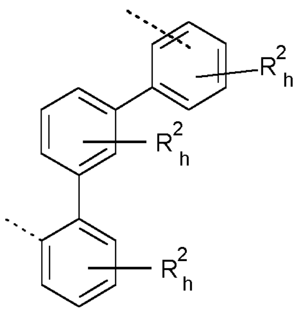

formula (L-14)

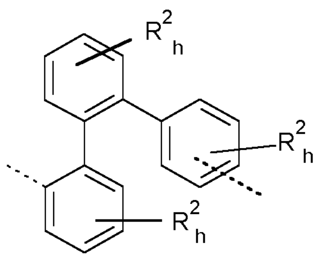

formula (L-15)

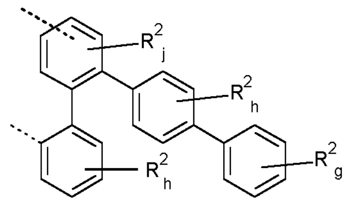

formula (L-16)

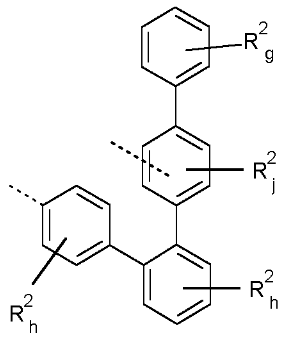

formula (L-17)

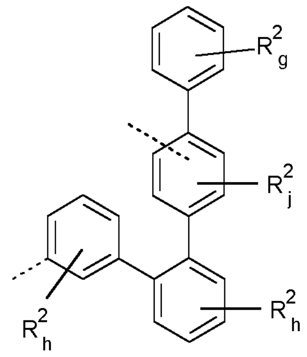

formula (L-18)

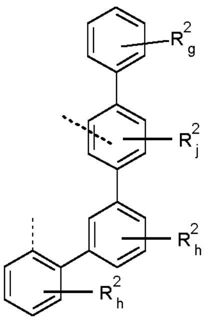

formula (L-19)

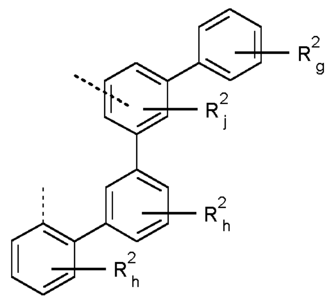

formula (L-20)

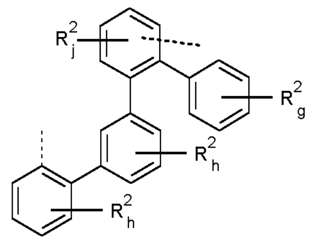

formula (L-21)

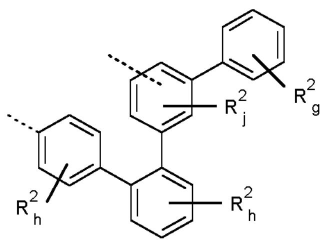

formula (L-22)

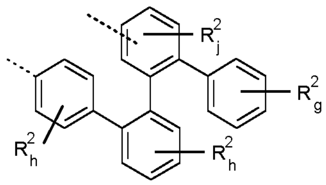

formula (L-23)

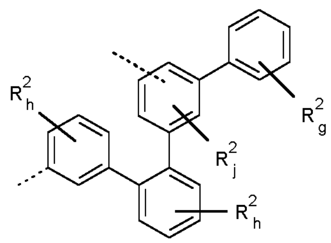

formula (L-24)

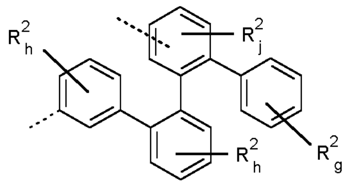

formula (L-25)

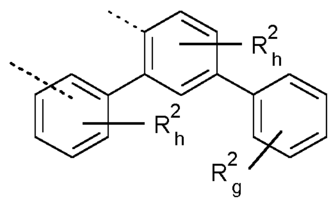

formula (L-26)

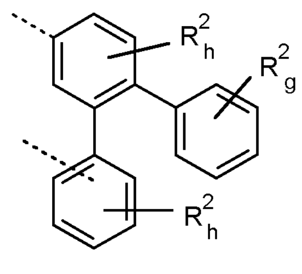

formula (L-27)

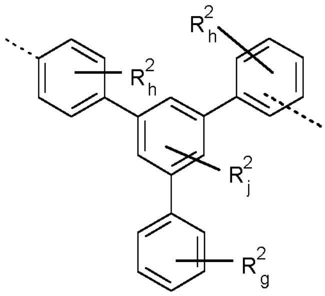

formula (L-28)

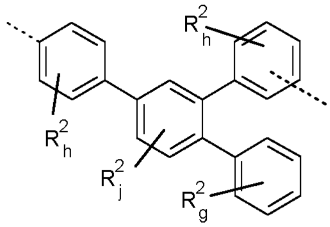

formula (L-29)

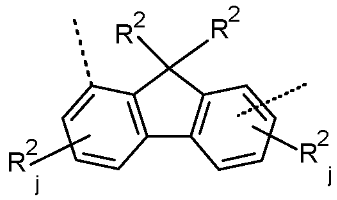

formula (L-30)

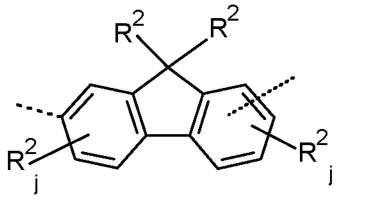

formula (L-31)

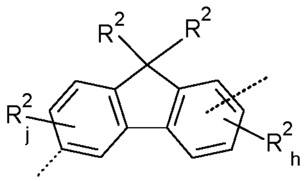

formula (L-32)

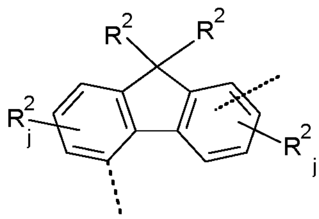

formula (L-33)

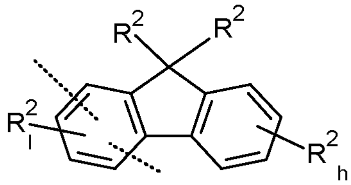

formula (L-34)

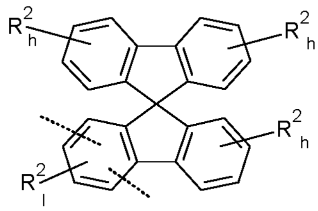

formula (L-35)

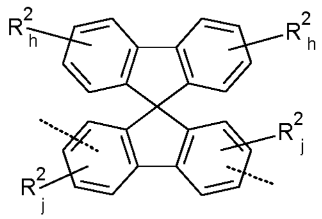

formula (L-36)

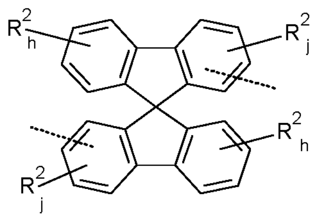

formula (L-37)

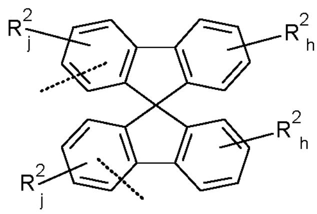

formula (L-38)

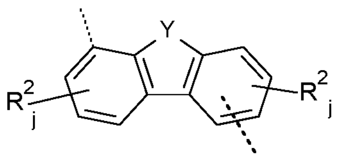

formula (L-39)

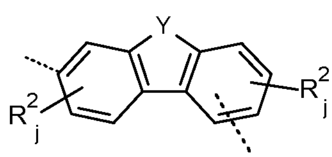

formula (L-40)

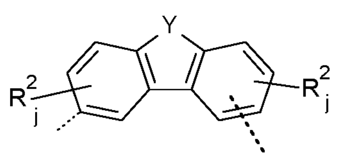

formula (L-41)

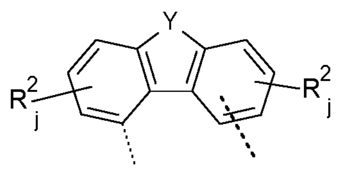

formula (L-42)

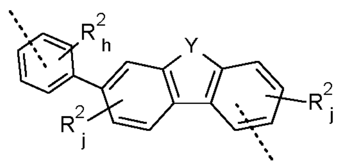
formula (L-43)

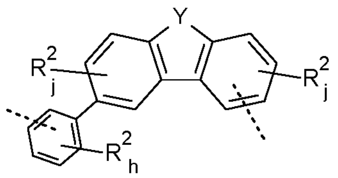
formula (L-44)

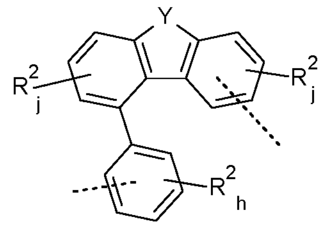
formula (L-45)

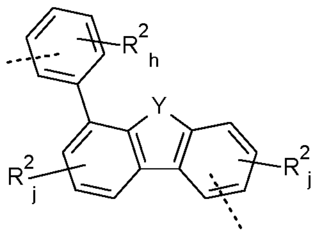
formula (L-46)

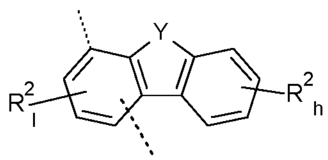
formula (L-47)

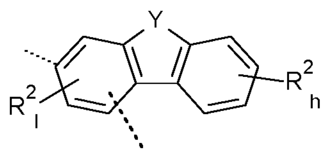
formula (L-48)

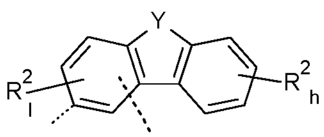
formula (L-49)

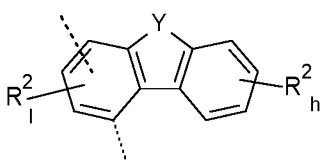
formula (L-50)

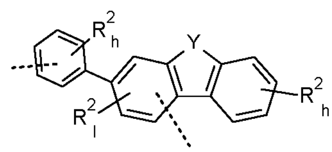
formula (L-51)

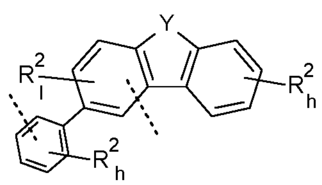
formula (L-52)

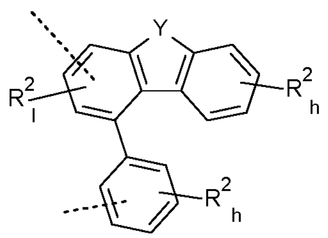
formula (L-53)

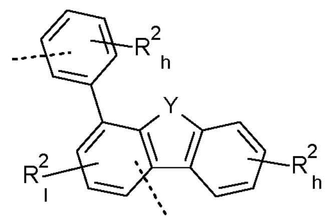
formula (L-54)

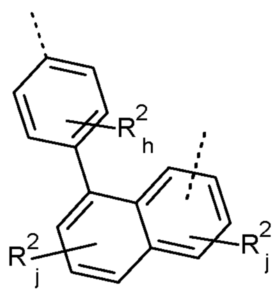
formula (L-55)

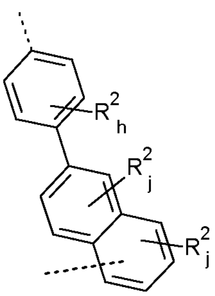
formula (L-56)

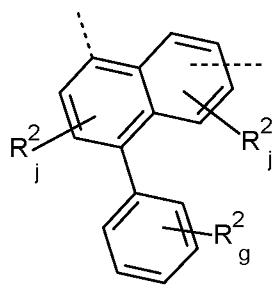
formula (L-57)

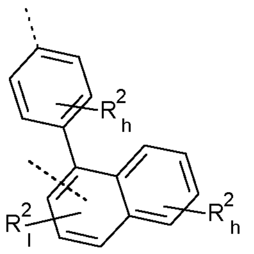

formula (L-58)

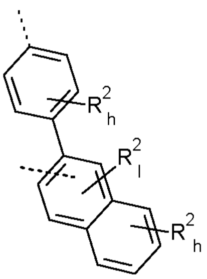

formula (L-59)

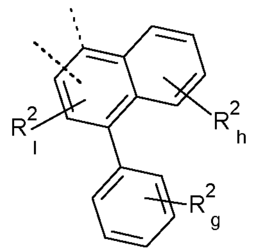

formula (L-60)

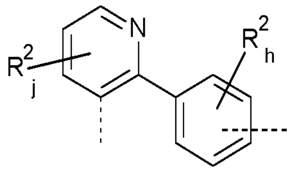

formula (L-61)

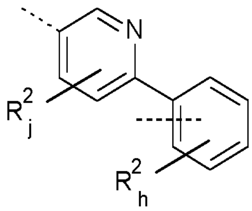

formula (L-62)

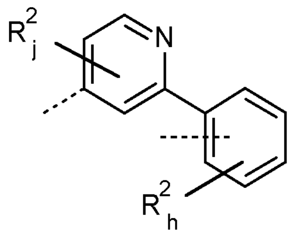

formula (L-63)

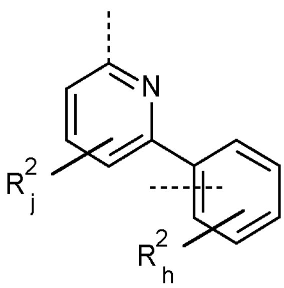

formula (L-64)

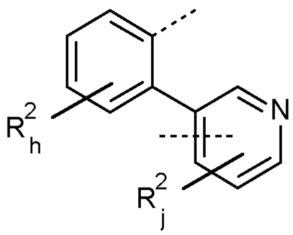

formula (L-65)

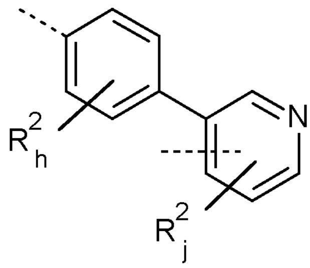

formula (L-66)

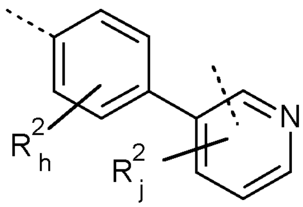

formula (L-67)

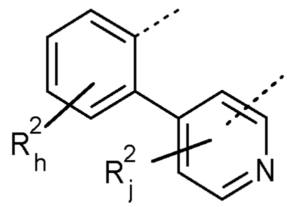

formula (L-68)

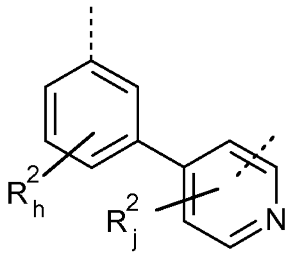

formula (L-69)

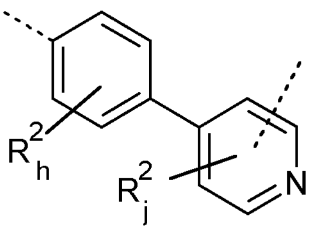

formula (L-70)

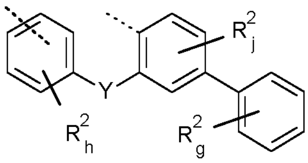

formula (L-71)

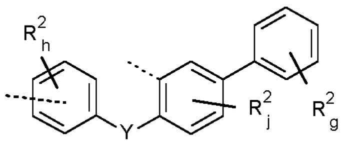

formula (L-72)

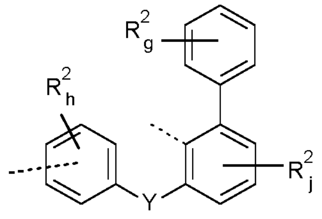

formula (L-73)

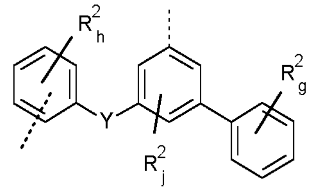

formula (L-74)

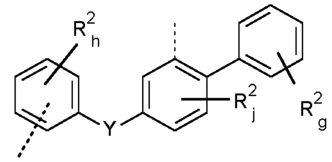

formula (L-75)

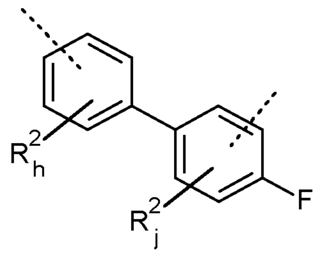

formula (L-76)

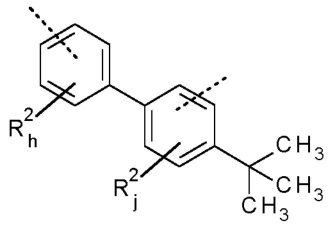

formula (L-77)

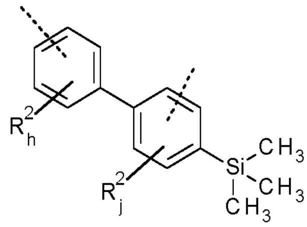

formula (L-78)

where the dashed bonds in each case mark the bonding positions, the index l is 0, 1 or 2, the index g is 0, 1, 2, 3, 4 or 5, j is on each occurence, independently, 0, 1, 2 or 3; h is on each occurence, independently, 0, 1, 2, 3 or 4; Y is O, S or $NR^2$, preferably O or S; and $R^2$ has the meaning given in Claim 1.

**12.** Compounds according to Claim 11, **characterised in that** the sum of the indices l, g, h and j in the structures of the formulae (L-1) to (L-78) is in each case at most 3, preferably at most 2 and particularly preferably at most 1.

**13.** Compounds according to Claim 11 or 12, **characterised in that**, in the structure of the formula (I), the group $L^1$ stands for a bond and, in the formula (IIa) and/or (IIb), the group $L^2$ stands for a group selected from the formulae (L-1) to (L-78), preferably from the formulae (L-1) to (L-5), as described in Claim 11.

**14.** Compounds according to Claim 11 or 12, **characterised in that**, in the structures of the formulae (IIa) and/or (IIb), the group $L^2$ stands for a bond and, in the formula (I), the group $L^1$ stands for a group selected from the formulae (L-1) to (L-78), preferably from the formulae (L-1) to (L-5), as described in Claim 11.

**15.** Compounds according to one or more of Claims 1 to 10, **characterised in that**, in the structures of the formulae (I), (IIa) and/or (IIb), the groups $L^1$ and $L^2$ stand for a bond.

**16.** Compounds according to one or more of Claims 1 to 10, **characterised in that**, in the structures of the formulae (I), (IIa) and/or (IIb), the groups $L^1$ and $L^2$ stand for a group selected from the formulae (L-1) to (L-78), preferably from the formulae (L-1) to (L-5), as described in Claim 11.

**17.** Compounds according to one or more of Claims 1 to 16, **characterised in that** $Ar^1$ and $Ar^3$ represent an aromatic ring system having 6 to 12 aromatic ring atoms, which may be substituted by one or more radicals $R^1$.

**18.** Oligomer, polymer or dendrimer containing one or more compounds according to one or more of Claims 1 to 17, where one or more bonds are present from the compound to the polymer, oligomer or dendrimer.

**19.** Composition comprising at least one compound according to one or more of Claims 1 to 17 and/or an oligomer, polymer or dendrimer according to Claim 18 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

**20.** Formulation comprising at least one compound according to one or more of Claims 1 to 17, an oligomer, polymer or dendrimer according to Claim 18 and/or at least one composition according to Claim 19 and at least one solvent.

21. Process for the preparation of a compound according to one or more of Claims 1 to 17 or an oligomer, polymer or dendrimer according to Claim 18, **characterised in** tha a group comprising at least one carbazole radical is connected to a group comprising at least one fluorene radical in a coupling reaction.

22. Use of a compound according to one or more of Claims 1 to 17, an oligomer, polymer or dendrimer according to Claim 18 or a composition according to Claim 19 as matrix material, electron-blocking material, hole-injection material and/or hole-transport material, preferably as matrix material, in an electronic device.

23. Electronic device containing at least one compound according to one or more of Claims 1 to 17, an oligomer, polymer or dendrimer according to Claim 18 or a composition according to Claim 19, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells and organic laser diodes.

## Revendications

1. Composé comprenant des structures de la formule (I) :

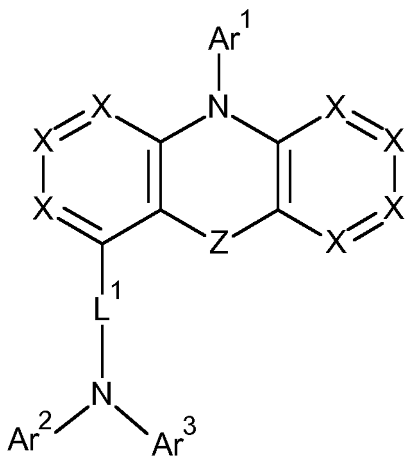

formule (I)

dans laquelle ce qui suit s'applique aux symboles qui sont utilisés :

X est pour chaque occurrence, de manière identique ou différente, N ou $CR^1$, de préférence $CR^1$, étant entendu que pas plus de deux des groupes X dans un cycle représentent N ;
Z est une liaison, $C(R^1)_2$, O ou S ;
$L^1$ est une liaison, un système de cycle aromatique qui comporte de 6 à 60 atomes de C ou un système de cycle hétéroaromatique qui comporte de 3 à 60 atomes de C, lequel peut dans chaque cas être substitué par un radical ou par plusieurs radicaux $R^1$ ;
$Ar^1$, $Ar^2$, $Ar^3$ sont un groupe aryle qui comporte de 6 à 40 atomes de C ou un groupe hétéroaryle qui comporte de 3 à 40 atomes de C, lequel peut dans chaque cas être substitué par un radical ou par plusieurs radicaux $R^1$ ;
$R^1$ est pour chaque occurrence, de manière identique ou différente, H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO ou $SO_2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 40 atomes de cycle aromatique, lequel peut dans chaque cas être substitué par un radical ou par plusieurs radicaux $R^2$, ou un groupe aryloxy ou hétéroaryloxy qui comporte de 5 à 40 atomes de cycle

aromatique, lequel peut être substitué par un radical ou par plusieurs radicaux $R^2$, ou une combinaison de ces systèmes ; deux substituants $R^1$ adjacents ou plus peuvent également former un système de cycle aliphatique ou aromatique mono- ou polycyclique l'un avec l'autre ou les uns avec les autres ;

$R^2$ est pour chaque occurrence, de manière identique ou différente, H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $-R^3C=CR^3-$, -C≡C-, $Si(R^3)_2$, C=O, C=S, $C=NR^3$, -C(=O)O-, $-C(=O)NR^3-$, $NR^3$, $P(=O)(R^3)$, -O-, -S-, SO ou $SO_2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 40 atomes de cycle aromatique, lequel peut dans chaque cas être substitué par un radical ou par plusieurs radicaux $R^3$, ou un groupe aryloxy ou hétéroaryloxy qui comporte de 5 à 40 atomes de cycle aromatique, lequel peut être substitué par un radical ou par plusieurs radicaux $R^3$, ou une combinaison de ces systèmes ; deux substituants $R^2$ adjacents ou plus peuvent également former un système de cycle aliphatique ou aromatique mono- ou polycyclique l'un avec l'autre ou les uns avec les autres ;

$R^3$ est pour chaque occurrence, de manière identique ou différente, H, D, F ou un radical hydrocarbone aliphatique, aromatique et/ou hétéroaromatique qui comporte de 1 à 20 atome(s) de C, où, en outre, des atomes de H peuvent être remplacés par F ; deux substituants $R^3$ adjacents ou plus peuvent également former un système de cycle aliphatique ou aromatique mono- ou polycyclique l'un avec l'autre ou les uns avec les autres ;

étant entendu que :

au moins l'un des radicaux $Ar^2$ et/ou $Ar^3$ représente un groupe de la formule (IIa) ou (IIb) :

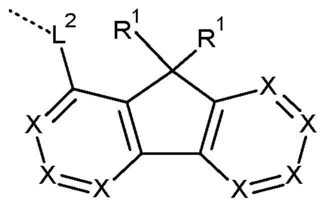

formule (IIa)

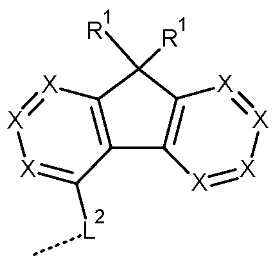

formule (IIb)

dans lesquelles :

X est pour chaque occurrence, de manière identique ou différente, N ou $CR^1$, de préférence $CR^1$, étant entendu que pas plus de deux des groupes X dans un cycle représentent N ;

$L^2$ est une liaison, un système de cycle aromatique qui comporte de 6 à 60 atomes de C ou un système de cycle hétéroaromatique qui comporte de 3 à 60 atomes de C, lequel peut dans chaque cas être substitué par un radical ou par plusieurs radicaux $R^1$ ;

$R^1$ présente la signification qui a été donnée ci-avant et la ligne en pointillés représente le site de liaison, de sorte que $L^2$ est lié au même atome d'azote que $L^1$.

**2.** Composé selon la revendication 1, **caractérisé en ce qu'**une structure de la formule (Ia) est formée :

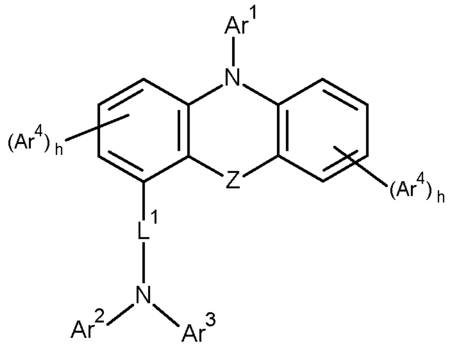

formule (Ia)

dans laquelle :

h est pour chaque occurrence, de manière indépendante, 0, 1, 2, 3 ou 4, de préférence 0, 1 ou 2 et de manière particulièrement préférable, 0 ou 1 ;
$Ar^4$ est un groupe aryle qui comporte de 6 à 40 atomes de C ou un groupe hétéroaryle qui comporte de 3 à 40 atomes de C, lequel peut dans chaque cas être substitué par un radical ou par plusieurs radicaux $R^1$ ; et
les symboles $Ar^1$, $Ar^2$, $Ar^3$, $L^1$ et Z présentent la signification qui a été donnée selon la revendication 1.

**3.** Composé selon la revendication 1 ou 2, **caractérisé en ce que** le groupe Z représente une liaison, de sorte qu'une structure de la formule (Ib) ou (Ic) est formée :

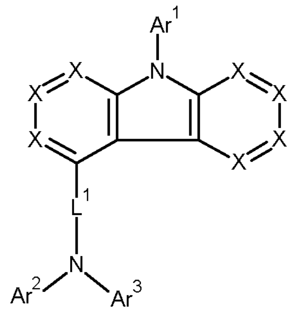

formule (Ib)

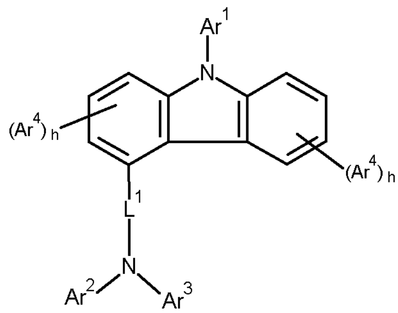

formule (Ic)

dans lesquelles les symboles présentent la signification qui a été indiquée selon la revendication 1 ou 2.

**4.** Composés selon une ou plusieurs des revendications 1 à 3, **caractérisés en ce que** les deux radicaux $R^1$ qui sont liés à l'atome de C à la position 9 dans la structure fluorène de la formule (IIa) ou (IIb) représentent dans chaque

cas un système de cycle aromatique ou hétéroaromatique qui comporte de 5 à 30 atomes de cycle aromatique, lequel peut être substitué par un radical ou par plusieurs radicaux $R^1$, où les deux systèmes de cycle sont connectés l'un à l'autre.

**5.** Composés selon la revendication 4, **caractérisés en ce qu'**au moins l'un des radicaux $Ar^2$ et/ou $Ar^3$ comprend un groupe de la formule (IIc) ou (IId) :

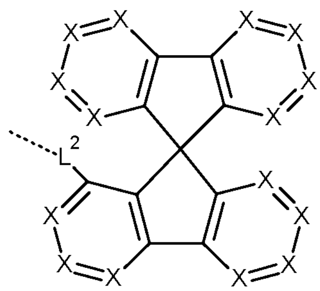

formule (IIc)

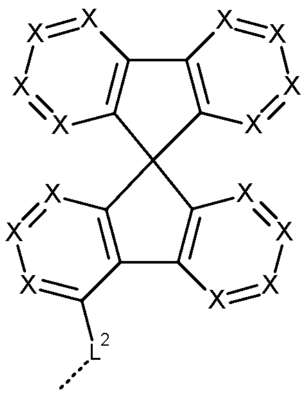

formule (IId)

dans lesquelles les symboles présentent la signification qui a été indiquée selon la revendication 1.

**6.** Composés selon une ou plusieurs des revendications 1 à 3, **caractérisés en ce que** les deux radicaux $R^1$ qui sont liés à l'atome de C à la position 9 dans la structure fluorène de la formule (IIa) ou (IIb) représentent dans chaque cas un système de cycle aromatique ou hétéroaromatique qui comporte de 5 à 30 atomes de cycle aromatique, lequel peut être substitué par un radical ou par plusieurs radicaux $R^1$, où les deux systèmes de cycle ne sont pas connectés l'un à l'autre, ou un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $-R^2C=CR^2-$, -C≡C-, $Si(R^2)_2$, C=O, C=S, $C=NR^2$, -C(=O)O-, $-C(=O)NR^2-$ $NR^2$, $P(=O)(R^2)$ -O-, -S-, SO ou $SO_2$ et où un ou plusieurs atome(s) de H peut/peuvent être remplacé(s) par D, F, Cl, Br, I, CN ou $NO_2$.

**7.** Composés selon une ou plusieurs des revendications 1 à 3 et 6, **caractérisés en ce qu'**au moins l'un des radicaux $Ar^2$ et/ou $Ar^3$ comprend un groupe de la formule (IIe) ou (IIf) :

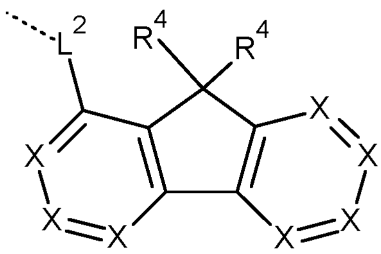

formule (IIe)

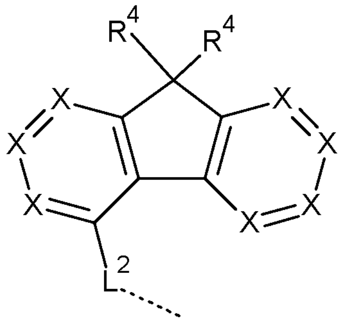

formule (IIf)

dans lesquelles les symboles X et $L^2$ présentent la signification qui a été indiquée selon la revendication 1 et $R^4$ représente H, un système de cycle aromatique qui comporte de 6 à 30 atomes de cycle aromatique, de préférence un groupe aryle qui comporte de 6 à 20 atomes de carbone, de façon particulièrement préférable, un groupe phényle, biphényle ou naphtyle, ou un groupe alkyle qui comporte de 1 à 20 atome(s) de carbone, de préférence méthyle, éthyle, propyle ou butyle, de façon particulièrement préférable, méthyle.

8. Composés selon une ou plusieurs des revendications 1 à 7, **caractérisés en ce que**, dans les formules (I), (Ib), (IIa), (IIb), (IIc), (IId), (IIe) et dans la formule (IIf), pas plus de deux groupes X, de préférence pas plus d'un groupe X représente(nt) N, de préférence tous les groupes X représentent $CR^1$, où, de préférence, au plus 4, de façon particulièrement préférable, au plus 3 et de façon tout particulièrement préférable, au plus 2 des groupes $CR^1$ que X représente ne sont pas le groupe CH.

9. Composés selon une ou plusieurs des revendications 1 à 8, **caractérisés en ce que**, dans les structures des formules (I), (Ib), (IIa), (IIb), (IIc), (IId), (IIe) ou de la formule (IIf), au moins un radical $R^1$, $Ar^2$, $Ar^3$ ou $Ar^4$ représente un groupe qui est sélectionné parmi les formules ($R^1$-1) à ($R^1$-72) :

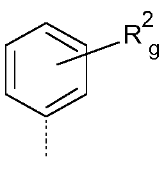

formule ($R^1$-1)

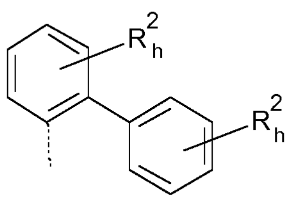

formule ($R^1$-2)

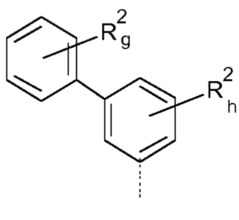

formule ($R^1$-3)

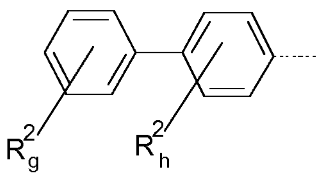

formule ($R^1$-4)

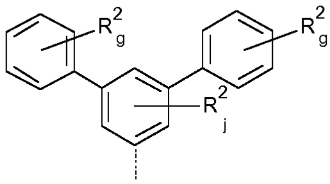

formule ($R^1$-5)

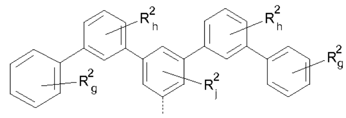

formule ($R^1$-6)

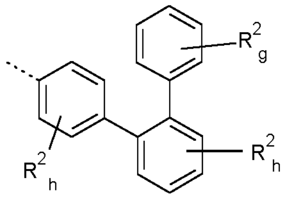

formule ($R^1$-7)

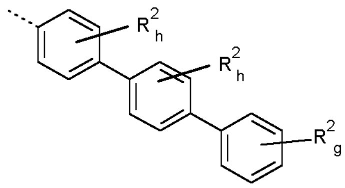

formule ($R^1$-8)

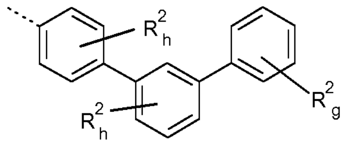

formule ($R^1$-9)

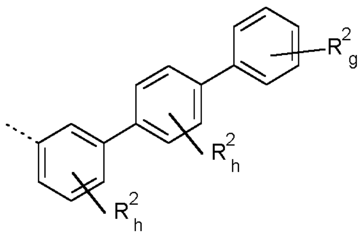

formule ($R^1$-10)

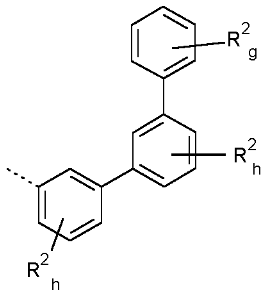

formule ($R^1$-11)

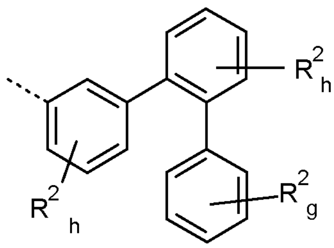

formule ($R^1$-12)

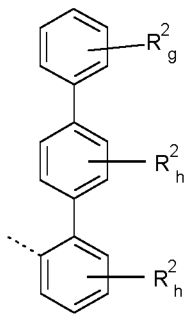

formule ($R^1$-13)

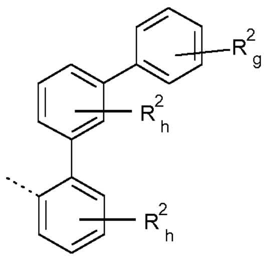

formule ($R^1$-14)

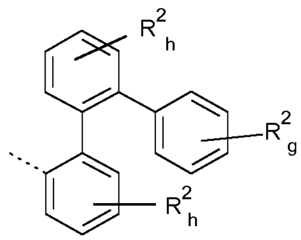

formule ($R^1$-15)

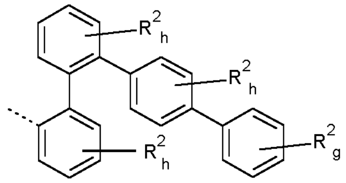

formule ($R^1$-16)

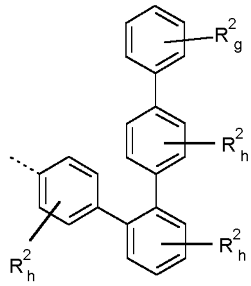

formule ($R^1$-17)

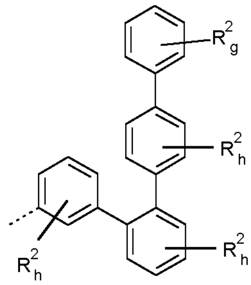

formule ($R^1$-18)

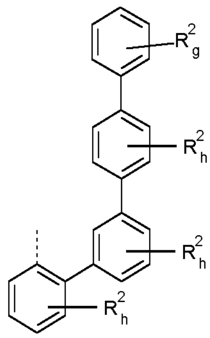

formule ($R^1$-19)

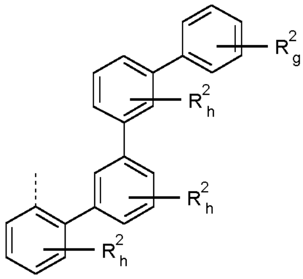

formule ($R^1$-20)

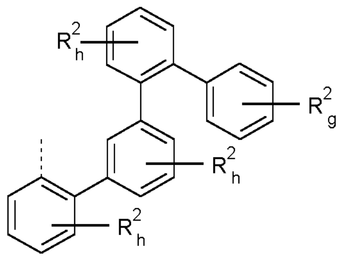

formule ($R^1$-21)

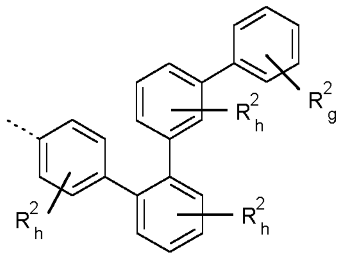

formule ($R^1$-22)

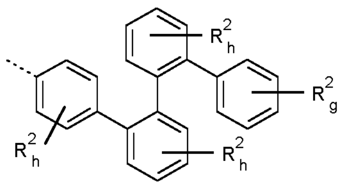

formule ($R^1$-23)

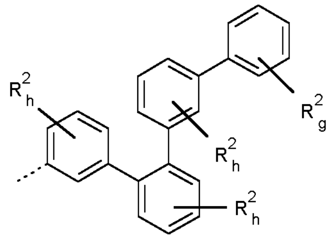

formule ($R^1$-24)

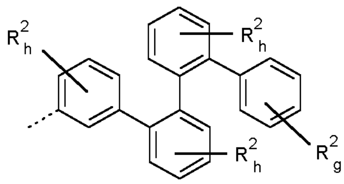

formule ($R^1$-25)

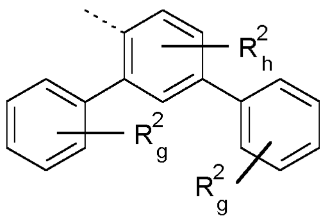

formule ($R^1$-26)

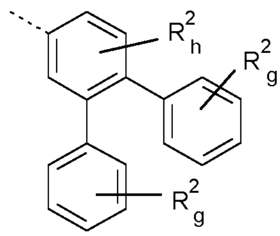

formule ($R^1$-27)

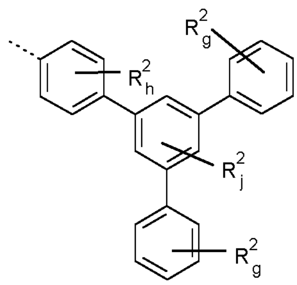

formule ($R^1$-28)

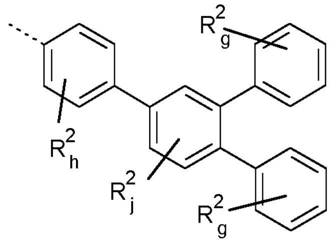

formule ($R^1$-29)

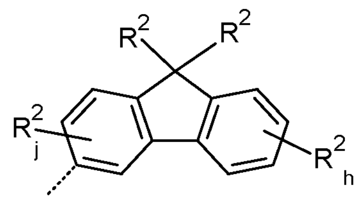

formule ($R^1$-30)

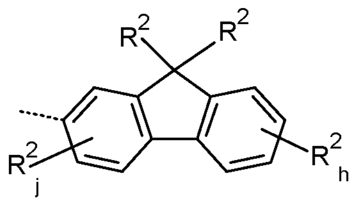

formule ($R^1$-31)

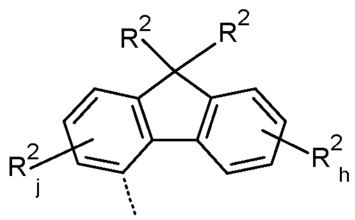

formule ($R^1$-32)

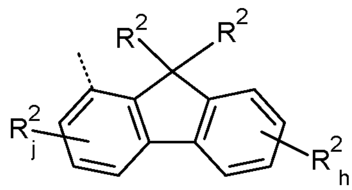

formule ($R^1$-33)

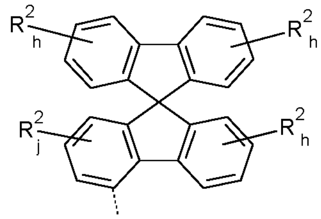

formule ($R^1$-34)

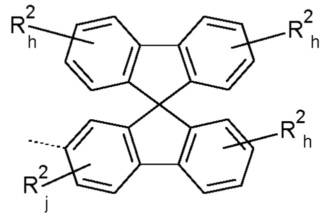

formule ($R^1$-35)

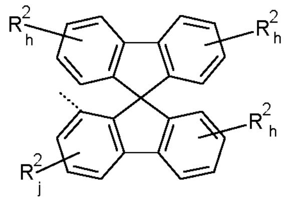

formule ($R^1$-36)

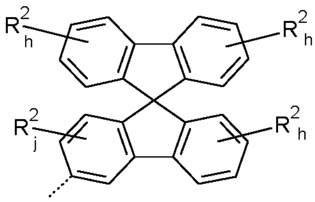

formule ($R^1$-37)

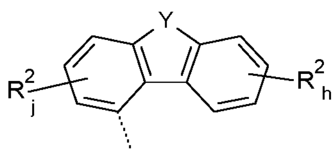

formule ($R^1$-38)

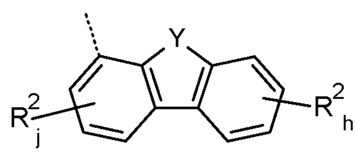

formule ($R^1$-39)

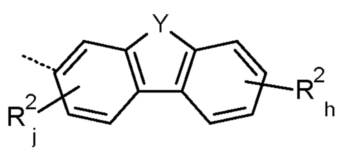

formule ($R^1$-40)

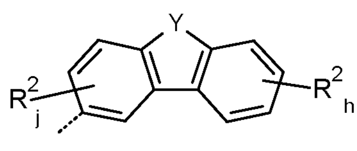

formule ($R^1$-41)

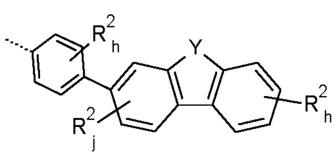

formule ($R^1$-42)

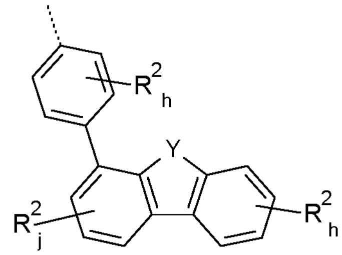

formule ($R^1$-43)

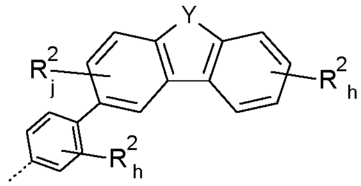

formule ($R^1$-44)

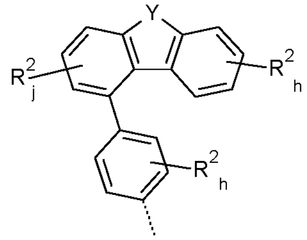

formule ($R^1$-45)

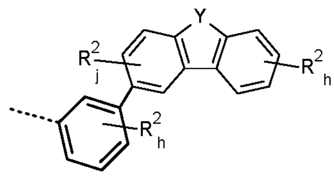

formule ($R^1$-46)

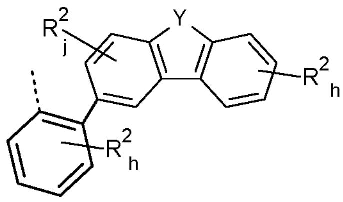

formule ($R^1$-47)

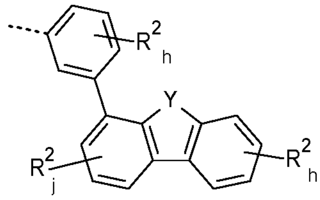

formule ($R^1$-48)

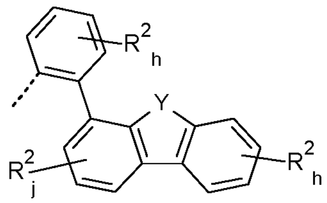

formule ($R^1$-49)

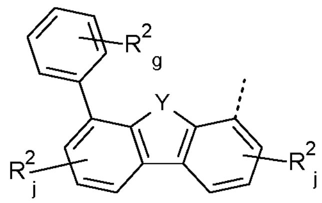

formule ($R^1$-50)

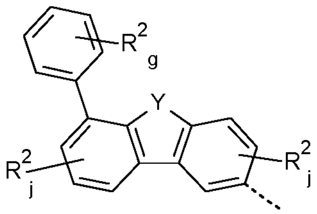

formule ($R^1$-51)

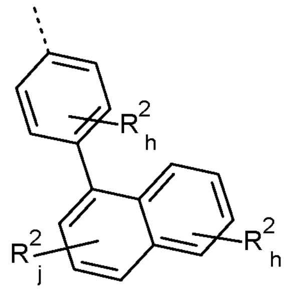

formule ($R^1$-52)

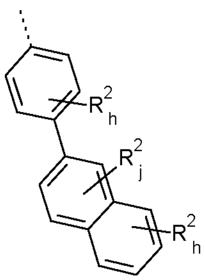

formule ($R^1$-53)

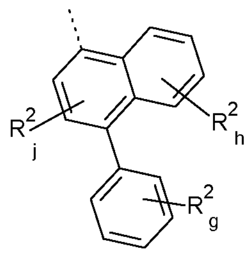

formule ($R^1$-54)

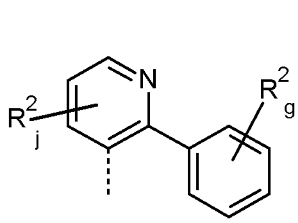

formule ($R^1$-55)

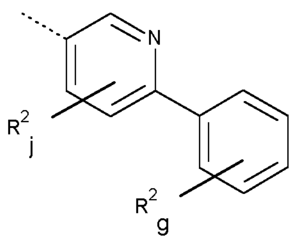

formule ($R^1$-56)

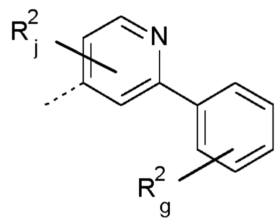

formule ($R^1$-57)

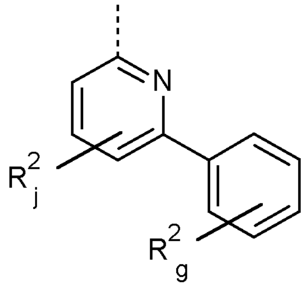
formule ($R^1$-58)

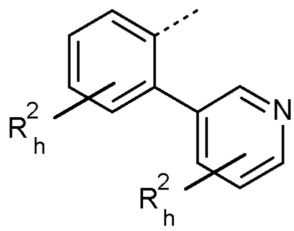
formule ($R^1$-59)

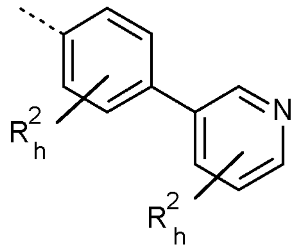
formule ($R^1$-60)

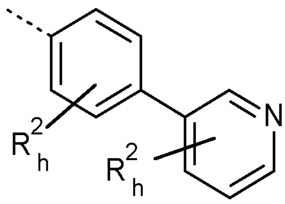
formule ($R^1$-61)

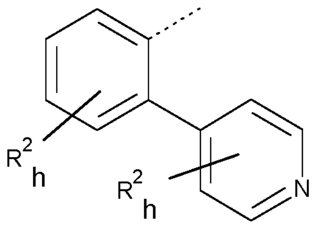
formule ($R^1$-62)

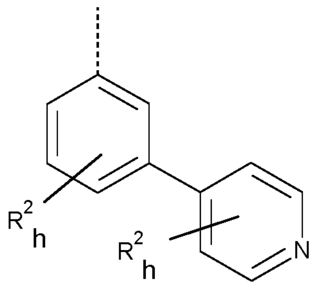
formule ($R^1$-63)

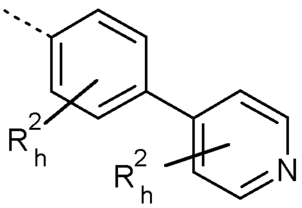
formule ($R^1$-64)

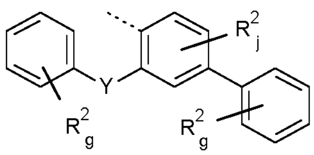
formule ($R^1$-65)

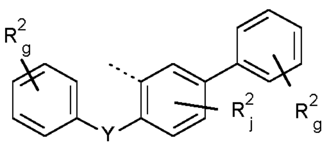
formule ($R^1$-66)

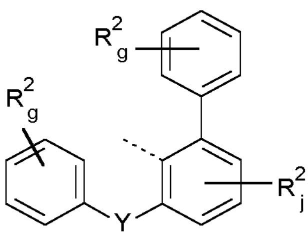
formule ($R^1$-67)

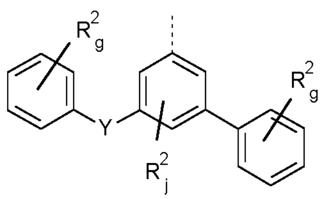
formule ($R^1$-68)

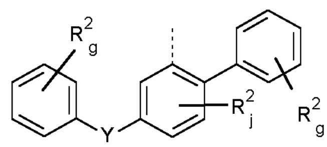
formule ($R^1$-69)

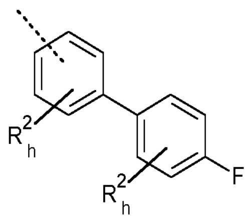
formule ($R^1$-70)

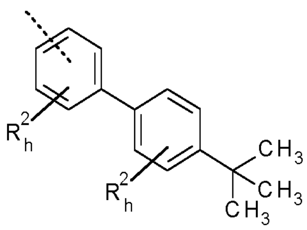
formule ($R^1$-71)

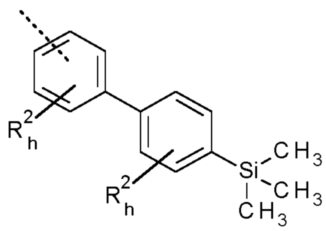
formule ($R^1$-72)

dans lesquelles ce qui suit s'applique aux symboles qui sont utilisés :

Y est O, S ou $NR^2$, de préférence O ou S ;
j est pour chaque occurrence, de manière indépendante, 0, 1, 2 ou 3 ;
h est pour chaque occurrence, de manière indépendante, 0, 1, 2, 3 ou 4 ;
g est pour chaque occurrence, de manière indépendante, 0, 1, 2, 3, 4 ou 5 ;

la liaison en pointillés indique la position de liaison ; et
$R^2$ présente la signification qui a été donnée selon la revendication 1.

**10.** Composés selon la revendication 9, **caractérisés en ce que** la somme des indices g, h et j dans les structures des formules ($R^1$-1) à ($R^1$-72) est dans chaque cas d'au plus 3, de préférence d'au plus 2 et de façon particulièrement préférable, d'au plus 1.

**11.** Composés selon une ou plusieurs des revendications 1 à 10, **caractérisés en ce que**, dans les structures des formules (I), (IIa) et/ou (IIb), au moins un groupe $L^1$ et/ou $L^2$ représente un groupe qui est sélectionné parmi les formules (L-1) à (L-78) :

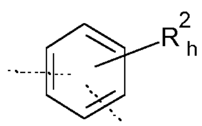

formule (L-1)

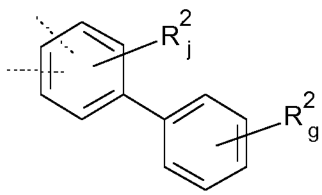

formule (L-2)

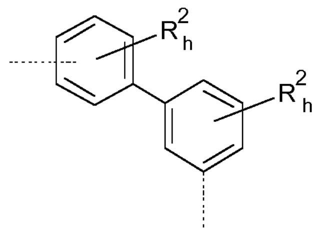

formule (L-3)

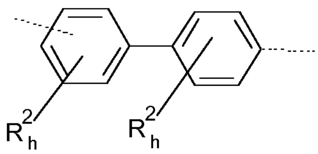

formule (L-4)

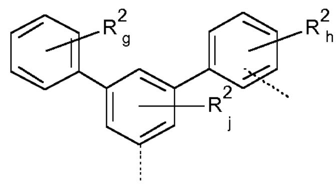

formule (L-5)

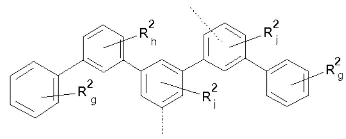

formule (L-6)

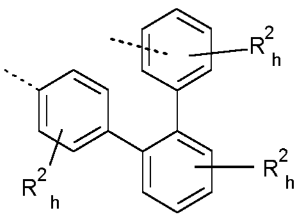

formule (L-7)

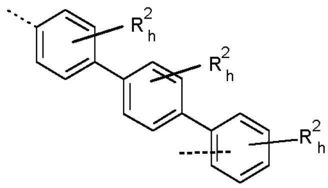

formule (L-8)

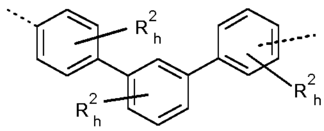

formule (L-9)

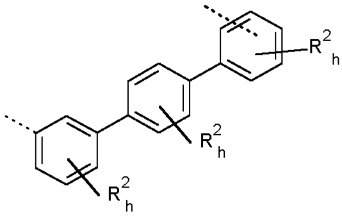

formule (L-10)

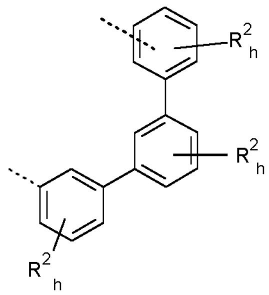

formule (L-11)

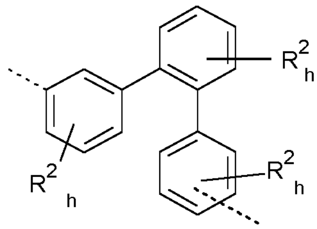

formule (L-12)

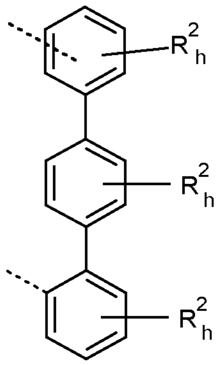

formule (L-13)

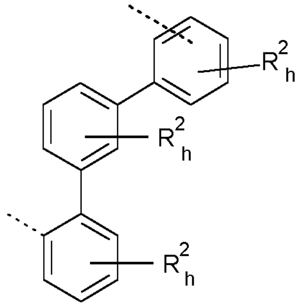

formule (L-14)

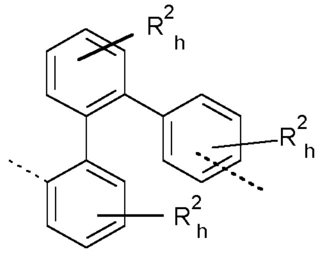

formule (L-15)

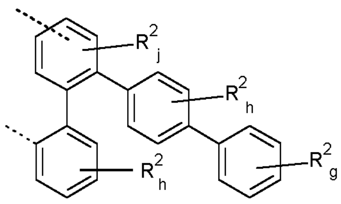

formule (L-16)

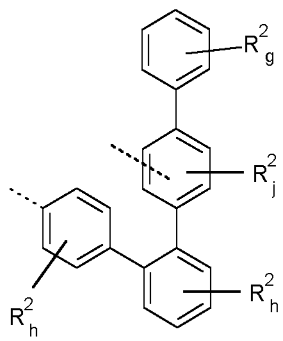

formule (L-17)

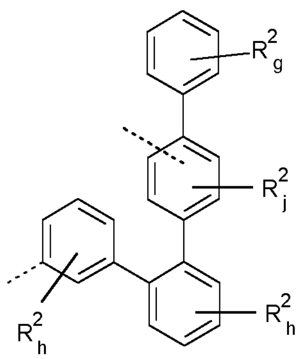

formule (L-18)

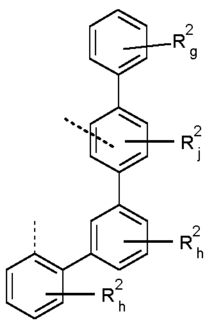

formule (L-19)

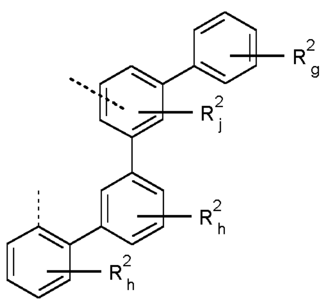

formule (L-20)

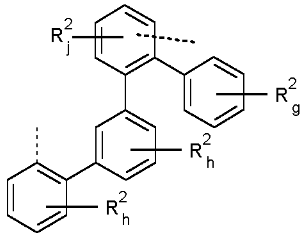

formule (L-21)

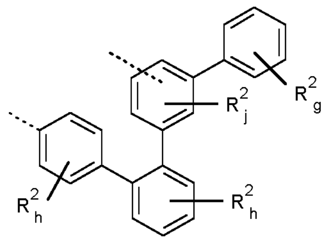

formule (L-22)

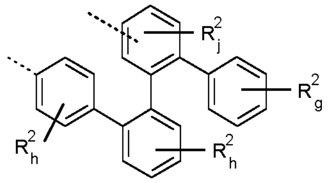

formule (L-23)

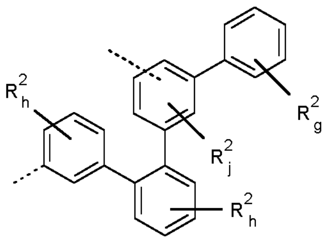

formule (L-24)

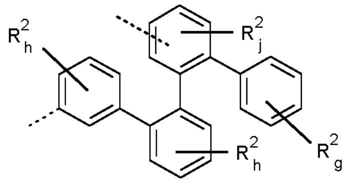

formule (L-25)

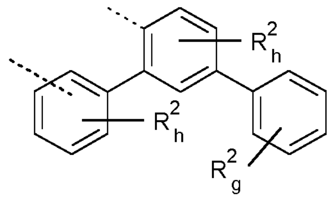

formule (L-26)

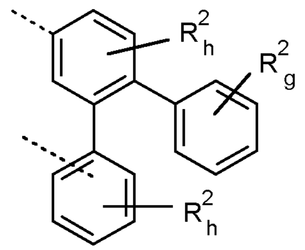

formule (L-27)

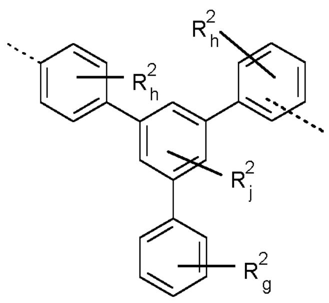

formule (L-28)

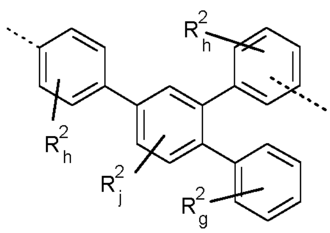

formule (L-29)

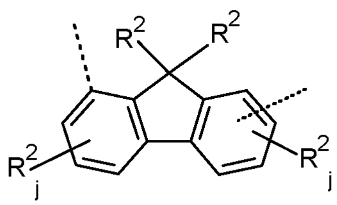

formule (L-30)

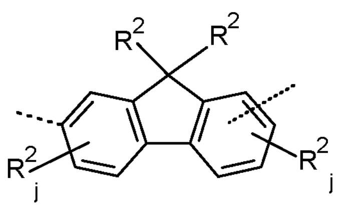

formule (L-31)

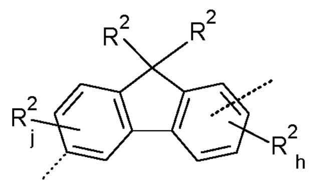

formule (L-32)

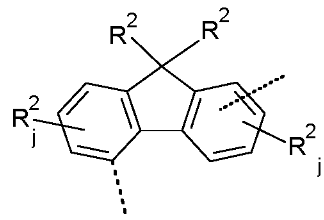

formule (L-33)

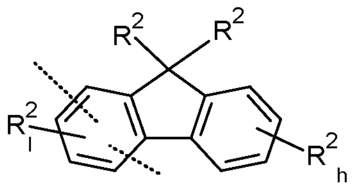

formule (L-34)

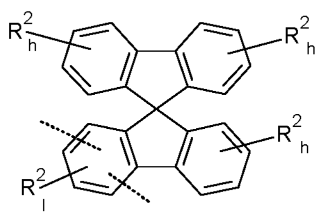

formule (L-35)

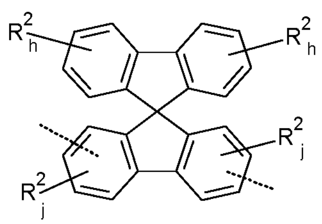

formule (L-36)

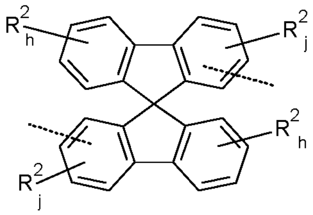

formule (L-37)

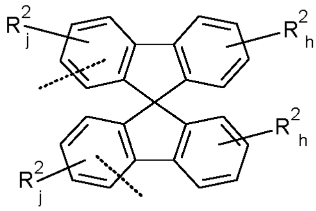

formule (L-38)

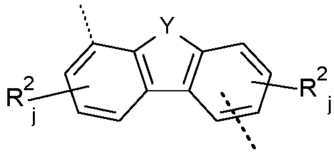

formule (L-39)

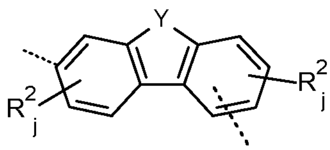

formule (L-40)

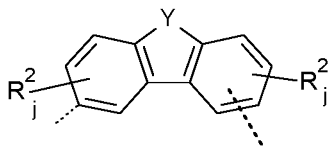

formule (L-41)

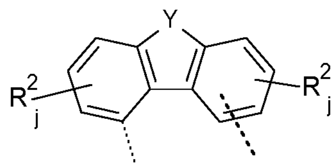

formule (L-42)

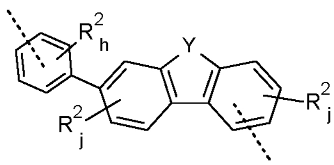

formule (L-43)

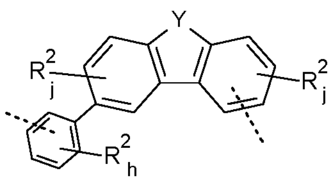

formule (L-44)

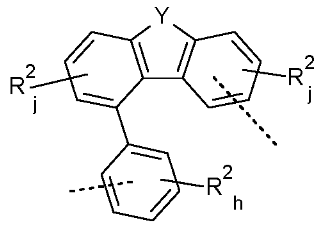

formule (L-45)

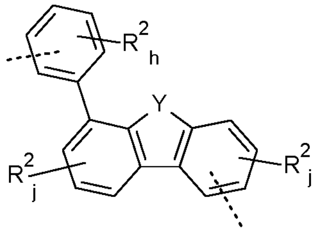

formule (L-46)

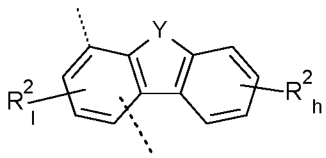

formule (L-47)

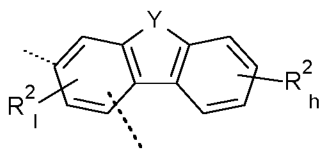

formule (L-48)

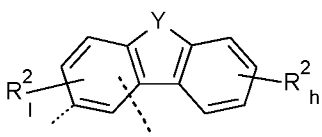

formule (L-49)

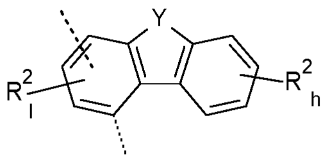

formule (L-50)

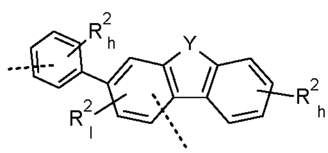

formule (L-51)

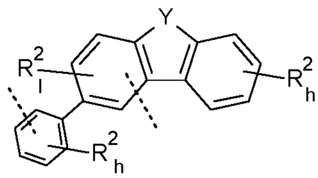

formule (L-52)

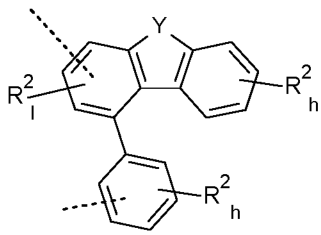

formule (L-53)

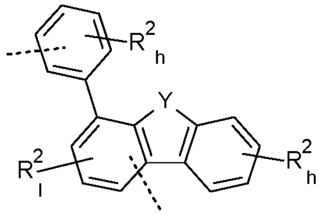

formule (L-54)

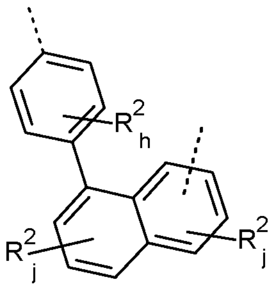

formule (L-55)

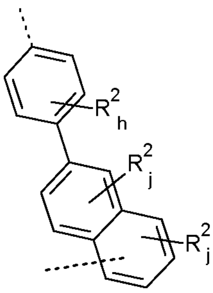

formule (L-56)

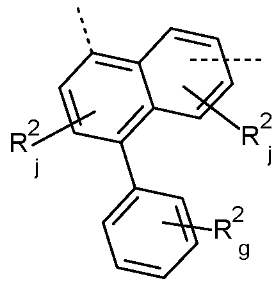

formule (L-57)

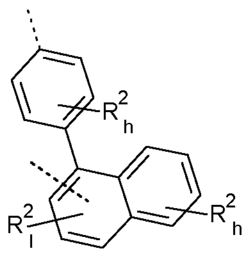

formule (L-58)

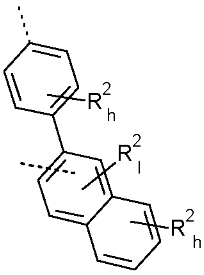

formule (L-59)

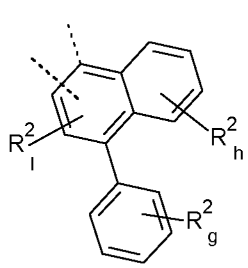

formule (L-60)

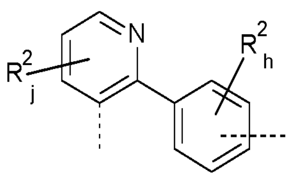

formule (L-61)

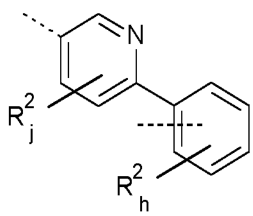

formule (L-62)

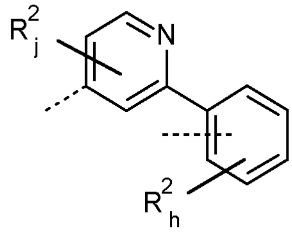

formule (L-63)

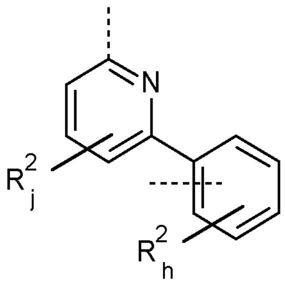
formule (L-64)

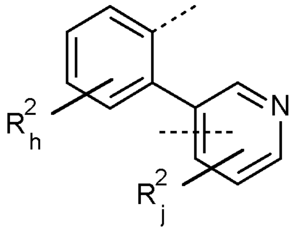
formule (L-65)

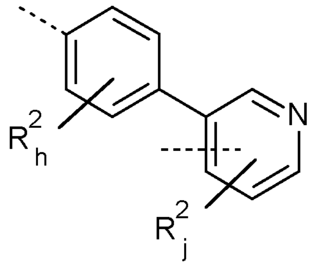
formule (L-66)

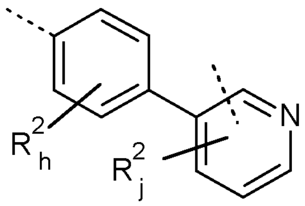
formule (L-67)

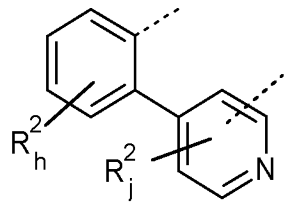
formule (L-68)

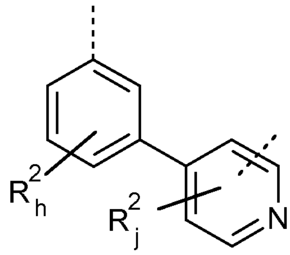
formule (L-69)

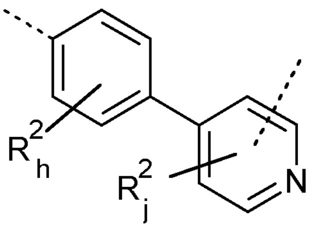
formule (L-70)

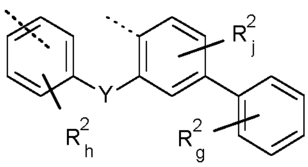
formule (L-71)

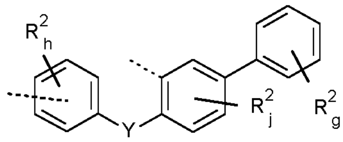
formule (L-72)

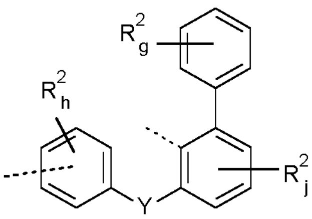
formule (L-73)

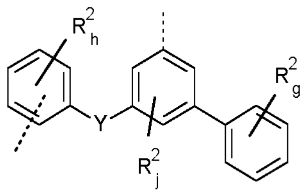
formule (L-74)

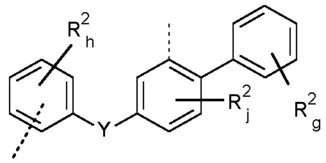
formule (L-75)

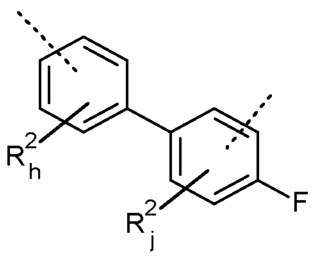
formule (L-76)

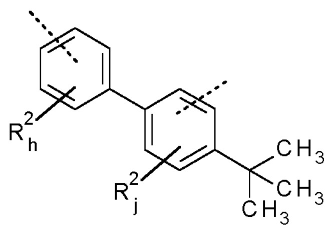
formule (L-77)

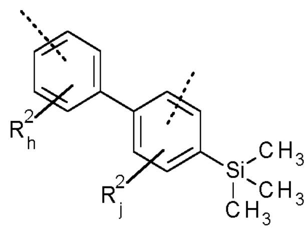
formule (L-78)

dans lesquelles les liaisons en pointillés indiquent dans chaque cas les positions de liaison, l'indice l est 0, 1 ou 2,

l'indice g est 0, 1, 2, 3, 4 ou 5, j est pour chaque occurrence, de manière indépendante, 0, 1, 2 ou 3 ; h est pour chaque occurrence, de manière indépendante, 0, 1, 2, 3 ou 4 ; Y est O, S ou $NR^2$, de préférence O ou S ; et $R^2$ présente la signification qui a été donnée selon la revendication 1.

**12.** Composés selon la revendication 11, **caractérisés en ce que** la somme des indices l, g, h et j dans les structures des formules (L-1) à (L-78) est dans chaque cas d'au plus 3, de préférence d'au plus 2 et de façon particulièrement préférable, d'au plus 1.

**13.** Composés selon la revendication 11 ou 12, **caractérisés en ce que**, dans la structure de la formule (I), le groupe $L^1$ représente une liaison et, dans la formule (IIa) et/ou (IIb), le groupe $L^2$ représente un groupe qui est sélectionné parmi les formules (L-1) à (L-78), de préférence parmi les formules (L-1) à (L-5), comme il a été décrit selon la revendication 11.

**14.** Composés selon la revendication 11 ou 12, **caractérisés en ce que**, dans les structures des formules (IIa) et/ou (IIb), le groupe $L^2$ représente une liaison et, dans la formule (I), le groupe $L^1$ représente un groupe qui est sélectionné parmi les formules (L-1) à (L-78), de préférence parmi les formules (L-1) à (L-5), comme il a été décrit selon la revendication 11.

**15.** Composés selon une ou plusieurs des revendications 1 à 10, **caractérisés en ce que**, dans les structures des formules (I), (IIa) et/ou (IIb), les groupes $L^1$ et $L^2$ représentent une liaison.

**16.** Composés selon une ou plusieurs des revendications 1 à 10, **caractérisés en ce que**, dans les structures des formules (I), (IIa) et/ou (IIb), les groupes $L^1$ et $L^2$ représentent un groupe qui est sélectionné parmi les formules (L-1) à (L-78), de préférence parmi les formules (L-1) à (L-5), comme il a été décrit selon la revendication 11.

**17.** Composés selon une ou plusieurs des revendications 1 à 16, **caractérisés en ce que** $Ar^1$ et $Ar^3$ représentent un système de cycle aromatique qui comporte de 6 à 12 atomes de cycle aromatique, lequel peut être substitué par un radical ou par plusieurs radicaux $R^1$.

**18.** Oligomère, polymère ou dendrimère contenant un ou plusieurs composé(s) selon une ou plusieurs des revendications 1 à 17, où une ou plusieurs liaison(s) est/sont présente(s) depuis le composé jusqu'au polymère, jusqu'à l'oligomère ou jusqu'au dendrimère.

**19.** Composition comprenant au moins un composé selon une ou plusieurs des revendications 1 à 17 et/ou un oligomère, un polymère ou un dendrimère selon la revendication 18 et au moins un autre composé qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

**20.** Formulation comprenant au moins un composé selon une ou plusieurs des revendications 1 à 17, un oligomère, un polymère ou un dendrimère selon la revendication 18 et/ou au moins une composition selon la revendication 19 et au moins un solvant.

**21.** Procédé pour la préparation d'un composé selon une ou plusieurs des revendications 1 à 17 ou d'un oligomère, d'un polymère ou d'un dendrimère selon la revendication 18, **caractérisé en ce qu'**un groupe qui comprend au moins un radical carbazole est connecté à un groupe qui comprend au moins un radical fluorène au moyen d'une réaction de couplage.

**22.** Utilisation d'un composé selon une ou plusieurs des revendications 1 à 17, d'un oligomère, d'un polymère ou d'un dendrimère selon la revendication 18 ou d'une composition selon la revendication 19 en tant que matériau de matrice, matériau de blocage d'électrons, matériau d'injection de trous et/ou matériau de transport de trous, de préférence en tant que matériau de matrice, dans un dispositif électronique.

**23.** Dispositif électronique contenant au moins un composé selon une ou plusieurs des revendications 1 à 17, un oligomère, un polymère ou un dendrimère selon la revendication 18 ou une composition selon la revendication 19, dans lequel le dispositif électronique est de préférence sélectionné parmi le groupe qui est constitué par les dispositifs électroluminescents organiques, les circuits intégrés organiques, les transistors à effet de champ organiques, les

transistors à film mince organiques, les transistors à émission de lumière ou luminescents organiques, les cellules solaires organiques, les détecteurs optiques organiques, les photorécepteurs organiques, les dispositifs à extinction de champ organiques, les cellules électrochimiques à émission de lumière ou luminescentes et les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9013148 A1
- WO 2014088047 A1
- WO 2013120577 A1
- WO 2013183851 A1
- EP 2468725 A1
- KR 20130134426 A
- KR 101395080 B1
- EP 2772483 A1
- JP 2014101275 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2005011013 A
- WO 2011116865 A1
- WO 2013064206 A1
- WO 2014056567 A1
- WO 2014094964 A1
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- US 20090134784 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 2005111172 A
- WO 2006117052 A
- WO 2010054729 A
- WO 2010054730 A
- WO 2010015306 A
- EP 652273 A
- WO 2009062578 A
- WO 2009148015 A
- US 20090136779 A
- WO 2010050778 A
- WO 2011042107 A
- WO 2011088877 A
- WO 2010108579 A


### In der Beschreibung aufgeführte Nicht-Patentliteratur


- **M. S. ARNOLD et al.** *Appl. Phys. Lett.,* 2008, vol. 92, 053301